# EUROPEAN PATENT SPECIFICATION

(11) **EP 2 114 160 B1**
(45) Date of publication and mention of the grant of the patent: **16.11.2016**
(21) Application number: 08729376.7
(22) Date of filing: 08.02.2008
(51) Int. Cl.: A61K 31/56, A01N 45/00, A61K 45/06, A61P 25/00

(54) **ANTAGONISTS OF A NON-SELECTIVE CATION CHANNEL IN NEURAL CELLS**
ANTAGONISTEN EINES NICHT SELEKTIVEN KATIONENKANALS IN NERVENZELLEN
ANTAGONISTES D'UN CANAL CATIONIQUE NON SÉLECTIF DANS DES CELLULES NEURALES

(30) Priority: 09.02.2007 US 889065 P; 17.07.2007 US 950170 P
(43) Date of publication of application: 11.11.2009
(73) Proprietor: University of Maryland, Baltimore, Baltimore, MD 21201-1691 (US); The United States of America, as represented by the Department of Veterans Affairs, Washington, D.C. 20420 (US)
(72) Inventor: SIMARD, J., Marc, Baltimore, MD 21212-3421 (US); GERZANICH, Vladimir, Baltimore, MD 21212 (US)
(74) Representative: Newman, Alastair James Morley
(86) International application number: PCT/US2008/053405
(87) International publication number: WO 2008/098160

(56) References cited:
- WO-A2-03/057843
- US-A1- 2006 276 411
- US-A1- 2006 276 411
- SIMARD J M ET AL: "Newly expressed SUR1-regulated NCCa-ATP channel mediates cerebral edema after ischemic stroke", NATURE MEDICINE, NATURE PUBLISHING GROUP, NEW YORK, NY, US, vol. 12, no. 4, 1 April 2006 (2006-04-01), pages 433-440, XP008117381, ISSN: 1078-8956, DOI: 10.1038/NM1390 [retrieved on 2006-03-19]
- MANLEY G T ET AL: "AUQPORIN-4 DELETION IN MICE REDUCES BRAIN EDEMA AFTER ACUTE WATER INTOXICATION AND ISCHEMIC STROKE", NATURE MEDICINE, NATURE PUBLISHING GROUP, NEW YORK, NY, US, vol. 6, no. 2, 1 February 2000 (2000-02-01), pages 159-163, XP000938619, ISSN: 1078-8956, DOI: 10.1038/72256
- FAGAN ET AL: "Targets for vascular protection after acute ischemic stroke", STROKE,, vol. 35, no. 9, 1 September 2004 (2004-09-01), pages 2220-2225, XP008116004, DOI: 10.1161/01.STR.0000138023.60272.9E
- ROSENBERG G A ET AL: "TIMP-2 reduces proteolytic opening of blood-brain barrier by type IV collagenase", BRAIN RESEARCH, ELSEVIER, AMSTERDAM, NL, vol. 576, no. 2, 3 April 1992 (1992-04-03) , pages 203-207, XP024273625, ISSN: 0006-8993, DOI: 10.1016/0006-8993(92)90681-X [retrieved on 1992-04-03]
- GURSOY-OZDEMIR ET AL: "Role of Endothelial Nitric Oxide Generation and Peroxynitrite Formation in Reperfusion Injury After Focal Cerebral Ischemia", STROKE,, vol. 31, no. 8, 1 August 2000 (2000-08-01) , pages 1974-1980, XP008116161,
- NILIUS ET AL.: 'Transient receptor potential cation channels in disease' PHYSIOLOGICAL REVIEWS vol. 87, no. 1, January 2007, pages 165 - 217, XP008116003
- ULLRICH ET AL.: 'Comparison of functional properties of the Ca2+-activated cation channels TRPM4 and TRPM5 from mice' CELL CALCIUM vol. 37, no. 3, March 2005, pages 267 - 278, XP004724069
- MORRIS ET AL.: 'Extension of the therapeutic window for recombinant tissue plasminogen activator with argatroban in a rat model of embolic stroke' STROKE vol. 32, no. 11, November 2001, pages 2635 - 2640, XP008116007
- MANLEY ET AL.: 'Aquaporin-4 deletion in mice reduces brain edema after acute water intoxication and ischemic stroke' NATURE MEDICINE vol. 6, no. 2, February 2000, pages 159 - 163, XP000938619
- FAGAN ET AL.: 'Targets for vascular protection after acute ischemic stroke' STROKE vol. 35, no. 9, September 2004, pages 2220 - 2225, XP008116004
- PISANO C. ET AL.: 'Undersulfated, low-molecular-weight glycol-split heparin as an antiangiogenic VEGF antagonist' GLYCOBIOLOGY vol. 15, no. 2, February 2005, pages 1C - 6C, XP008116008
- ROSENBERG ET AL.: 'TIMP-2 reduces proteolytic opening of blood-brain barrier by type IV collagenase' BRAIN RESEARCH vol. 576, no. 2, 03 April 1992, pages 203 - 207, XP024273625
- GURSOY-OZDEMIR ET AL.: 'Role of Endothelial Nitric Oxide Generation and Peroxynitrite Formation in Reperfusion Injury After Focal Cerebral Ischemia' STROKE vol. 31, no. 8, August 2000, pages 1974 - 1980, XP008116161

### SEQUENCE LISTING

<110> UNIVERSITY OF MARYLAND, BALTIMORE Simard, J. Marc Gerzanich, Volodymyr
<120> ANTAGONISTS OF A NON-SELECTIVE CATION CHANNEL IN NEURAL CELLS
<130> HO-P03431WO0
<140> TBA
   <141> 2008-02-08
<150> 60/889,065
   <151> 2007-02-09
<150> 60/950,170
   <151> 2007-07-17
<160> 6
<170> PatentIn version 3.4
<210> 1
   <211> 20
   <212> DNA
   <213> Artificial Sequence
<220>
   <223> Synthetic Primer
<400> 1
   gtgtgcatcg ctgtcccaca 20
<210> 2
   <211> 20
   <212> DNA
   <213> Artificial Sequence
<220>
   <223> Synthetic Primer
<400> 2
   ctgcgatagc actcgccaaa 20
<210> 3
   <211> 10
   <212> DNA
   <213> Artificial Sequence
<220>
   <223> Synthetic Primer
<220>
   <221> misc_feature
   <222> (5)..(6)
   <223> n is a, c, g, or t
<400> 3
   gggrnnyycc 10
<210> 4
   <211> 10
   <212> DNA
   <213> Artificial Sequence
<220>
   <223> Synthetic Primer
<220>
   <221> misc_feature
   <222> (5)..(6)
   <223> n is a, c, g, or t
<400> 4
   cccrnnyycc 10
<210> 5
   <211> 4826
   <212> DNA
   <213> Human
<400> 5
   agctgagccc gagcccagac cgcgcccgcg ccgccatgcc cctggccttc tgcggcagcg     60
   agaaccactc ggccgcctac cgggtggacc agggggtcct caacaacggc tgctttgtgg     120
   acgcgctcaa cgtggtgccg cacgtcttcc tactcttcat caccttcccc atcctcttca    180
   ttggatgggg aagtcagagc tccaaggtgc acatccacca cagcacatgg cttcatttcc    240
   ctgggcacaa cctgcggtgg atcctgacct tcatgctgct cttcgtcctg gtgtgtgaga    300
   ttgcagaggg catcctgtct gatggggtga ccgaatccca ccatctgcac ctgtacatgc    360
   cagccgggat ggcgttcatg gctgctgtca cctccgtggt ctactatcac aacatcgaga    420
   cttccaactt ccccaagctg ctaattgccc tgctggtgta ttggaccctg gccttcatca    480
   ccaagaccat caagtttgtc aagttcttgg accacgccat cggcttctcg cagctacgct    540
   tctgcctcac agggctgctg gtgatcctct atgggatgct gctcctcgtg gaggtcaatg    600
   tcatcagggt gaggagatac atcttcttca agacaccgag ggaggtgaag cctcccgagg    660
   acctgcaaga cctgggggta cgcttcctgc agcccttcgt gaatctgctg tccaaaggca    720
   cctactggtg gatgaacgcc ttcatcaaga ctgcccacaa gaagcccatc gacttgcgag    780
   ccatcgggaa gctgcccatc gccatgaggg ccctcaccaa ctaccaacgg ctctgcgagg    840
   cctttgacgc ccaggtgcgg aaggacattc agggcactca aggtgcccgg gccatctggc    900
   aggcactcag ccatgccttc gggaggcgcc tggtcctcag cagcactttc cgcatcttgg    960
   ccgacctgct gggcttcgcc gggccactgt gcatctttgg gatcgtggac caccttggga   1020
   aggagaacga cgtcttccag cccaagacac aatttctcgg ggtttacttt gtctcatccc   1080
   aagagttcct tgccaatgcc tacgtcttag ctgtgcttct gttccttgcc ctcctactgc   1140
   aaaggacatt tctgcaagca tcctactatg tggccattga aactggaatt aacttgagag 1200
   gagcaataca gaccaagatt tacaataaaa ttatgcacct gtccacctcc aacctgtcca 1260
   tgggagaaat gactgctgga cagatctgta atctggttgc catcgacacc aatcagctca 1320
   tgtggttttt cttcttgtgc ccaaacctct gggctatgcc agtacagatc attgtgggtg 1380
   tgattctcct ctactacata ctcggagtca gtgccttaat tggagcagct gtcatcattc 1440
   tactggctcc tgtccagtac ttcgtggcca ccaagctgtc tcaggcccag cggagcacac 1500
   tggagtattc caatgagcgg ctgaagcaga ccaacgagat gctccgcggc atcaagctgc 1560
   tgaagctgta cgcctgggag aacatcttcc gcacgcgggt ggagacgacc cgcaggaagg 1620
   agatgaccag cctcagggcc tttgccatct atacctccat ctccattttc atgaacacgg 1680
   ccatccccat tgcagctgtc ctcataactt tcgtgggcca tgtcagcttc ttcaaagagg 1740
   ccgacttctc gccctccgtg gcctttgcct ccctctccct cttccatatc ttggtcacac 1800
   cgctgttcct gctgtccagt gtggtccgat ctaccgtcaa agctctagtg agcgtgcaaa 1860
   agctaagcga gttcctgtcc agtgcagaga tccgtgagga gcagtgtgcc ccccatgagc 1920
   ccacacctca gggcccagcc agcaagtacc aggcggtgcc cctcagggtt gtgaaccgca 1980
   agcgtccagc ccgggaggat tgtcggggcc tcaccggccc actgcagagc ctggtcccca 2040
   gtgcagatgg cgatgctgac aactgctgtg tccagatcat gggaggctac ttcacgtgga 2100
   ccccagatgg aatccccaca ctgtccaaca tcaccattcg tatcccccga ggccagctga 2160
   ctatgatcgt ggggcaggtg ggctgcggca agtcctcgct ccttctagcc gcactggggg 2220
   agatgcagaa ggtctcaggg gctgtcttct ggagcagcct tcctgacagc gagataggag 2280
   aggaccccag cccagagcgg gagacagcga ccgacttgga tatcaggaag agaggccccg 2340
   tggcctatgc ttcgcagaaa ccatggctgc taaatgccac tgtggaggag aacatcatct 2400
   ttgagagtcc cttcaacaaa caacggtaca agatggtcat tgaagcctgc tctctgcagc 2460
   cagacatcga catcctgccc catggagacc agacccagat tggggaacgg ggcatcaacc 2520
   tgtctggtgg tcaacgccag cgaatcagtg tggcccgagc cctctaccag cacgccaacg 2580
   ttgtcttctt ggatgacccc ttctcagctc tggatatcca tctgagtgac cacttaatgc 2640
   aggccggcat ccttgagctg ctccgggacg acaagaggac agtggtctta gtgacccaca 2700
   agctacagta cctgccccat gcagactgga tcattgccat gaaggatggc accatccaga 2760
   gggagggtac cctcaaggac ttccagaggt ctgaatgcca gctctttgag cactggaaga 2820
   ccctcatgaa ccgacaggac caagagctgg agaaggagac tgtcacagag agaaaagcca 2880
   cagagccacc ccagggccta tctcgtgcca tgtcctcgag ggatggcctt ctgcaggatg 2940
   aggaagagga ggaagaggag gcagctgaga gcgaggagga tgacaacctg tcgtccatgc 3000
   tgcaccagcg tgctgagatc ccatggcgag cctgcgccaa gtacctgtcc tccgccggca 3060
   tcctgctcct gtcgttgctg gtcttctcac agctgctcaa gcacatggtc ctggtggcca 3120
   tcgactactg gctggccaag tggaccgaca gcgccctgac cctgacccct gcagccagga 3180
   actgctccct cagccaggag tgcaccctcg accagactgt ctatgccatg gtgttcacgg 3240
   tgctctgcag cctgggcatt gtgctgtgcc tcgtcacgtc tgtcactgtg gagtggacag 3300
   ggctgaaggt ggccaagaga ctgcaccgca gcctgctaaa ccggatcatc ctagccccca 3360
   tgaggttttt tgagaccacg ccccttggga gcatcctgaa cagattttca tctgactgta 3420
   acaccatcga ccagcacatc ccatccacgc tggagtgcct gagccgctcc accctgctct 3480
   gtgtctcagc cctggccgtc atctcctatg tcacacctgt gttcctcgtg gccctcttgc 3540
   ccctggccat cgtgtgctac ttcatccaga agtacttccg ggtggcgtcc agggacctgc 3600
   agcagctgga tgacaccacc cagcttccac ttctctcaca ctttgccgaa accgtagaag 3660
   gactcaccac catccgggcc ttcaggtatg aggcccggtt ccagcagaag cttctcgaat 3720
   acacagactc caacaacatt gcttccctct tcctcacagc tgccaacaga tggctggaag 3780
   tccgaatgga gtacatcggt gcatgtgtgg tgctcatcgc agcggtgacc tccatctcca 3840
   actccctgca cagggagctc tctgctggcc tggtgggcct gggccttacc tacgccctaa 3900
   tggtctccaa ctacctcaac tggatggtga ggaacctggc agacatggag ctccagctgg 3960
   gggctgtgaa gcgcatccat gggctcctga aaaccgaggc agagagctac gaggggctcc 4020
   tggcaccatc gctgatccca aagaactggc cagaccaagg gaagatccag atccagaacc 4080
   tgagcgtgcg ctacgacagc tccctgaagc cggtgctgaa gcacgtcaat gccctcatct 4140
   cccctggaca gaagatcggg atctgcggcc gcaccggcag tgggaagtcc tccttctctc 4200
   ttgccttctt ccgcatggtg gacacgttcg aagggcacat catcattgat ggcattgaca 4260
   tcgccaaact gccgctgcac accctgcgct cacgcctctc catcatcctg caggaccccg 4320
   tcctcttcag cggcaccatc cgatttaacc tggaccctga gaggaagtgc tcagatagca 4380
   cactgtggga ggccctggaa atcgcccagc tgaagctggt ggtgaaggca ctgccaggag 4440
   gcctcgatgc catcatcaca gaaggcgggg agaatttcag ccagggacag aggcagctgt 4500
   tctgcctggc ccgggccttc gtgaggaaga ccagcatctt catcatggac gaggccacgg 4560
   cttccattga catggccacg gaaaacatcc tccaaaaggt ggtgatgaca gccttcgcag 4620
   accgcactgt ggtcaccatc gcgcatcgag tgcacaccat cctgagtgca gacctggtga 4680
   tcgtcctgaa gcggggtgcc atccttgagt tcgataagcc agagaagctg ctcagccgga 4740
   aggacagcgt cttcgcctcc ttcgtccgtg cagacaagtg acctgccaga gcccaagtgc 4800
   catcccacat tcggaccctg cccata 4826
<210> 6
   <211> 10
   <212> PRT
   <213> Artificial Sequence
<220>
   <223> Synthetic Peptide
<400> 6
   Cys Thr Thr His Trp Gly Phe Thr Leu Cys
   1               5                   10

## Description

### FIELD OF THE INVENTION

The present invention generally relates to inhibition of neural cell swelling and treating or preventing spinal cord injury stemming from neural cell swelling.

### BACKGROUND OF THE INVENTION

Injury to the nervous system has serious consequences. Following traumatic brain injury and stroke, for example, the normal response of the surrounding brain is to mount a cellular response that includes formation of reactive astrocytes that are believed to be important to "contain" and "clean-up" the injury site. Swelling of neural cells is part of the cytotoxic or cell swelling response that characterizes brain damage in cerebral ischemia and traumatic brain injury, and is a major cause of morbidity and mortality. See, Staub *et al.,* 1993; Kimelberg *et al.,* 1995. A number of mediators have been identified that initiate swelling of neural cells, including elevation of extracellular K⁺, acidosis, release of neurotransmitters and free fatty acids. See, Kempski *et al.,* 1991; Rutledge and Kimelberg, 1996; Mongin *et al.,* 1999. Cytotoxic edema is a well-recognized phenomenon clinically that causes brain swelling, which worsens outcome and increases morbidity and mortality in brain injury and stroke.

### Spinal cord injury - the clinical problem

Acute spinal cord injury (SCI) results in physical disruption of spinal cord neurons and axons leading to deficits in motor, sensory, and autonomic function. This is a debilitating neurological disorder common in young adults that often requires life-long therapy and rehabilitative care, placing a significant burden on healthcare systems. The fact that SCI impacts mostly young people makes the tragedy all the more horrific, and the cost to society in terms of lost "person-years" all the more enormous. Sadly, many patients exhibit neuropathologically and clinically complete cord injuries following SCI. However, many others have neuropathologically incomplete lesions (Hayes and Kakulas, 1997; Tator and Fehlings, 1991). giving hope that proper treatment to minimize secondary injury may reduce the functional impact.

### Secondary injury - progressive hemorrhagic necrosis (PHN)

The concept of secondary injury in SCI arises from the observation that the volume of injured tissue increases with time after injury, *i.e.,* the lesion itself expands and evolves over time. Whereas primary injured tissues are irrevocably damaged from the very beginning, right after impact, tissues that are destined to become "secondarily" injured are considered to be potentially salvageable. Secondary injury in SCI has been reviewed in a classic paper by Tator (1991), as well as in more recent reviews (Kwon *et al.,* 2004), wherein the overall concept of secondary injury is validated. Older observations based on histological studies that gave rise to the concept of lesion-evolution have been confirmed with non-invasive MRI (Bilgen *et al.,* 2000; Ohta *et al.,* 1999; Sasaki *et al.,* 1978; Weirich *et al.,* 1990).

Numerous mechanisms of secondary injury are recognized, including edema, ischemia, oxidative stress and inflammation. In SCI, however, one pathological entity in particular is recognized that is relatively unique to the spinal cord and that has especially devastating consequences - progressive hemorrhagic necrosis (PHN) (Fitch *et al.,* 1999; Kraus, 1996; Nelson *et al.,* 1977; Tator, 1991; Tator and Fehlings, 1991; Tator and Koyanagi, 1997).

PHN is a rather mysterious condition, first recognized over 3 decades ago, that has thus far eluded understanding and treatment. Following impact, petechial hemorrhages form in surrounding tissues and later emerge in more distant tissues, eventually coalescing into the characteristic lesion of hemorrhagic necrosis. The specific time course and magnitude of these changes remain to be determined, but papers by Khan *et al.* (1985) and Kawata *et al.* (1993) nicely describe the progressive increase in hemorrhage in the cord. After injury, a small hemorrhagic lesion involving primarily the capillary-rich central gray matter is observed at 15 min, but hemorrhage, necrosis and edema in the central gray matter enlarge progressively over a period of 3-24 h (Balentine, 1978; Iizuka *et al.,* 1987; Kawata *et al.,* 1993). The white matter surrounding the hemorrhagic gray matter shows a variety of abnormalities, including decreased H&E staining, disrupted myelin, and axonal and periaxonal swelling. Tator and Koyanagi (1997) noted that white matter lesions extend far from the injury site, especially in the posterior columns. The evolution of hemorrhage and necrosis has been referred to as "autodestruction", and it is this that forms the key observation that defines PHN. PHN eventually causes loss of vital spinal cord tissue and, in some species including humans, leads to post-traumatic cystic cavitation surrounded by glial scar tissue.

### Mechanisms of delayed hemorrhage and PHN

Tator and Koyanagi (1997) expressed the view that obstruction of small intramedullary vessels by the initial mechanical stress or secondary injury may be responsible for PHN. Kawata and colleagues (1993) attributed the progressive changes to leukocyte infiltration around the injured area leading to plugging of capillaries. Most importantly, damage to the endothelium of spinal cord capillaries and postcapillary venules has been regarded as a major factor in the pathogenesis of PHN (Griffiths *et al.,* 1978; Kapadia, 1984; Nelson *et al.,* 1977). That endothelium is involved is essentially certain, given that petechial hemorrhages, the primary characteristic of PHN, arise from nothing less than catastrophic failure of capillary or venular integrity. However, no molecular mechanism for progressive dysfunction of endothelium has heretofore been identified.

"Hemorrhagic conversion" is a term familiar to many from the stroke literature, but not from the SCI literature. Hemorrhagic conversion describes the process of conversion from a bland infarct into a hemorrhagic infarct, and is typically associated with post-ischemic reperfusion, either spontaneous or induced by thrombolytic therapy. The molecular pathology involved in hemorrhagic conversion has yet to be fully elucidated, but considerable work has implicated enzymatic destruction of capillaries by matrix-metalloproteinases (MMP) released by invading neutrophils (Gidday *et al.,* 2005; Justicia *et al.,* 2003; Lorenzl *et al.,* 2003; Romanic *et al.,* 1998). Maladaptive activation of MMP compromises the structural integrity of capillaries, leading to formation of petechial hemorrhages. In ischemic stroke, MMP inhibitors reduce hemorrhagic conversion following thrombolytic-induced reperfusion. MMPs are also implicated in spinal cord injury (de *et al.,* 2000; Duchossoy *et al.,* 2001; Duchossoy *et al.,* 2001; Goussev *et al.,* 2003; Hsu *et al.,* 2006; Noble *et al.,* 2002; Wells *et al.,* 2003). In SCI, however, their role has been studied predominantly in the context of delayed tissue healing, and no evidence has been put forth to suggest their involvement in PHN.

### Therapies in SCI

No cure exists for the primary injury in SCI, but research has identified various pharmacological compounds that specifically antagonize secondary injury mechanisms responsible for worsened outcome in SCI. Several compounds including methylprednisolone, GM-1 ganglioside, thyrotropin releasing hormone, nimodipine, and gacyclidine have been tested in prospective randomized clinical trials of SCI, with only methylprednisolone and GM-1 ganglioside showing evidence of a modest benefit (Fehlings and Baptiste, 2005). At present, high dose methylprednisolone steroid therapy is the only pharmacological therapy shown to have efficacy in a Phase Three randomized trial when it can be administered within eight hours of injury (Bracken, 2002; Bracken *et al.,* 1997; Bracken *et al.,* 1998).

Of the numerous treatments assessed in SCI, very few have been shown to actually decrease the hemorrhage and tissue loss associated with PHN. Methylprednisolone, the only approved therapy for SCI, improves edema, but does not alter the development of PHN (Merola *et al.,* 2002). A number of compounds have shown beneficial effects related to sparing of white matter, including the NMDA antagonist, MK801 (Faden *et al.,* 1988), the AMPA antagonist, GYKI 52466 (Colak *et al.,* 2003), Na⁺ channel blockers (Schwartz and Fehlings, 2001; Teng and Wrathall, 1997), minocycline (Teng *et al.,* 2004), and estrogen (Chaovipoch *et al.,* 2006).

However, no treatment has been reported that reduces PHN and lesion volume, and that improves neurobehavioral function to the extent that is observed with the highly selective but exemplary SUR1 (sulfonylurea receptor 1) antagonists, glibenclamide and repaglinide, as well as with antisense-oligodeoxynucleotide (AS-ODN) directed against SUR1. It is useful that the molecular mechanisms targeted by these 3 agents - SUR1 and the SUR1-regulated NC_{Ca-ATP} channel, are characterized to further elucidate their role in PHN.

Other and further objects, features, and advantages will be apparent from the following description of the present exemplary embodiments of the invention, which are given for the purpose of disclosure.

### SUMMARY OF THE INVENTION

The NC_{Ca-ATP} channel is expressed at least in neurons, glia and neural endothelial cells after brain trauma, for example. This unique non-selective cation channel is activated by intracellular calcium and blocked by intracellular ATP (NC_{Ca-ATP} channel), and can be expressed in, for example, neural cells, such as neuronal cells, neuroglia cells (also termed glia, or glial cells, e.g., astrocyte, ependymal cell, oligodentrocyte and microglia) or neural endothelial cells *(e.g.,* capillary endothelial cells) in which the cells have been or are exposed to a traumatic insult, for example, an acute neuronal insult (e.g., hypoxia, ischemia, tissue compression, mechanical distortion, cerebral edema or cell swelling), toxic compounds or metabolites, an acute injury, cancer, brain abscess, *etc.*

In particular aspects, the NC_{Ca-ATP} channel includes a SUR1 receptor and a TRPM4 channel. It has a single-channel conductance to potassium ion (K⁺) between 20 and 50 pS at physiological K concentrations. The NC_{Ca-ATP} channel is also stimulated by Ca²⁺ on the cytoplasmic side of the cell membrane in a physiological concentration range, where concentration range is from 10⁻⁸ to 10⁻⁵ M, in specific embodiments. The NC_{Ca-ATP} channel is also inhibited by cytoplasmic ATP in a physiological concentration range, where the concentration range is from 10⁻¹ mM to 5 mM, in certain cases. The NC_{Ca-ATP} channel is also permeable at least to the following cations; K⁺, Cs⁺, Li⁺, Na⁺; to the extent that the permeability ratio between any two of the cations is greater than 0.5 and less than 2, for example. In specific embodiments, NC_{Ca-ATP} channel has the following characteristics: 1) it is a non-selective monovalent cation channel; 2) it is activated by an increase in intracellular calcium or by a decrease in intracellular ATP, or both; and 3) it is regulated by SUR1.

The regulation and/or modulation of this NC_{Ca-ATP} channel can be used to treat various diseases and/or conditions, for example acute neuronal insults (e.g., stroke, an ischemic/hypoxic insult, a traumatic or mechanical injury) and diseases or conditions leading to formation of a gliotic capsule. The modulation and/or regulation of the channel results from administration of an antagonist or inhibitor of the channel, in specific embodiments. Thus, depending upon the disease, a composition (an antagonist, which may also be referred to as an inhibitor) is administered to block or inhibit at least in part the channel to prevent cell death, for example to treat at least cerebral edema that results from ischemia due to tissue trauma or to increased tissue pressure. In at least these instances, the channel is blocked to prevent or reduce or modulate depolarization of the cells.

In certain aspects, antagonists of one or more proteins that comprise the channel and/or antagonists for proteins that modulate activity of the channel are utilized. The channel is expressed on neuronal cells, neuroglia cells, neural epithelial cells, neural endothelial cells, or a combination thereof, for example. In specific embodiments, an inhibitor of the channel directly or indireclty inhibits the activity of the channel, for example by inhibiting the influx of cations, such as Na⁺, into the cells, this inhibition thereby preventing depolarization of the cells. Inhibition of the influx of Na⁺ into the cells thereby at least prevents or reduces cytotoxic edema and/or ionic edema, and prevents or reduces hemorrhagic conversion. Thus, this treatment reduces cell death or necrotic death of at least neuronal, neuroglial, and/or neural endothelial cells.

In certain embodiments of the invention, the methods and compositions are useful for treating and/or preventing hemorrhage. The hemorrhage may be primary hemorrhage and/or secondary hemorrhage. The hemorrhage may be in the brain and/or the spinal cord, for example, including after injury thereto. In specific embodiments, the hemorrhage is intracerebral hemorrhage (ICH) or subarachnoid hemorrhage (SAII), for example.

The present invention provides an antagonist of TRPM4 (a transient receptor potential cation channel, subfamily M, member 4) for use in a method of (a) inhibiting neural cell swelling in an individual having traumatic brain injury, traumatic spinal cord injury, cerebral ischemia, spinal cord ischemia, central nervous system (CNS) damage, peripheral nervous system (PNS) damage, cerebral hypoxia, spinal cord hypoxia, or cerebral edema; or (b) treating or preventing spinal cord injury stemming from neural cell swelling.

Typically, the antagonist of TRPM4 is a nucleic acid, a protein, a small molecule, or a combination thereof. Typically, the nucleic acid is a TRPM4 siRNA. Typically, the method further comprises delivering to the individual a therapeutically effective amount of an additional therapeutic compound selected from: a) a SUR1 antagonist; b) one or more cation channel blockers; c) one or more of a compound selected from one or more antagonists of vascular endothelial growth factor (VEGF), one or more antagonists of matrix metalloprotease (MMP), one or more antagonists of nitric oxide synthase (NOS), one or more antagonists of thrombin, aquaporin, a biologically active derivative thereof, and a combination thereof; and d) a combination thereof. Typically, the TRPM4 antagonist and the additional therapeutic compound are delivered to the individual successively; or the TRPM4 antagonist is delivered to the individual prior to delivery of the additional therapeutic compound; or the TRPM4 antagonist is delivered to the individual subsequent to delivery of the additional therapeutic compound. Typically, the TRPM4 antagonist and the additional therapeutic compound are delivered to the individual concomitantly; or the TRPM4 antagonist and the additional therapeutic compound are delivered as a mixture; or the TRPM4 antagonist and the additional therapeutic compound act synergistically in the individual.

In some embodiments, the invention also encompasses the use of such antagonist compounds in singular or combinatorial compositions. In one embodiment, the therapeutic combinatorial composition can be administered to and/or into the brain, for example. Such administration to the brain includes injection directly into the brain, for example, particularly in the case where the brain has been rendered accessible to injection due to trauma or surgery to the skull, for example. The invention provides the therapeutic use of compounds that block the pore of the NC_{Ca-ATP} channel, such as flufenamic acid and other blockers of TRPM4.

The invention encompasses antagonists of TRPM4, including small molecules, large molecules, proteins, (including antibodies), as well as nucleotide sequences that can be used to inhibit expression of the genes that encode the pore-forming subunits of the NC_{Ca-ATP} channel (*e.g*., antisense and ribozyme molecules). In certain cases, an antagonist of the NC_{Ca-ATP} channel includes one or more compounds capable of one or more of the following: (1) blocking the channel; (2) preventing channel opening; (3) inhibiting the channel; (4) reducing the magnitude of current flow through the channel; (5) inhibiting transcriptional expression of the channel; (6) inhibiting post-translational assembly and/or trafficking of channel subunits and/or (7) increasing the closed time and/or closing rate of the channel, for example.

In certain embodiments of the invention, there are methods of inhibiting neural cell swelling in an individual having traumatic brain injury, cerebral ischemia, central nervous system (CNS) damage, peripheral nervous system (PNS) damage, cerebral hypoxia, or cerebral edema by inhibiting expression of TRPM4. The expression may be inhibited directly by inhibiting a transcription factor that regulates expression of TRPM4 or it may be inhibited indirectly by modulating expression and/or activity of an upstream or downstream effector of TRPM4. In specific embodiments, modulation of one or more gene products results in inhibition of TRPM4. For example, utilization of an inhibitor of PIP₂, or degradation of PIP₂, an activator of phospholipase C, estrogen or an estrogen analog, a protein kinase C (PKC)δ activator, such as PMA, an inhibitor of TNFα, an inhibitor of HIF1α, and/or an NFκB inhibitor may be employed to inhibit the neural cell swelling and, therefore, may be used in methods of treating traumatic brain injury, cerebral ischemia, central nervous system (CNS) damage, peripheral nervous system (PNS) damage, cerebral hypoxia, and/or cerebral edema, for example. In specific embodiments, activation of the phospholipase C (PLC)-coupled M1 muscarinic receptor and/or pharmacological depletion of cellular PIP2 inhibits TRPM4.

The NC_{Ca-ATP} channel can be inhibited by an NC_{Ca-ATP} channel inhibitor, an NC_{CaATP} channel blocker, a type 1 sulfonylurea receptor (SUR1) antagonist, SUR1 inhibitor, a TRPM4 inhibitor, or a compound capable of reducing the magnitude of membrane current through the channel. More specifically, the exemplary SUR1 antagonist may be selected from the group consisting of mitiglinide, iptakalim, endosulfines, glibenclamide, tolbutamide, repaglinide, nateglinide, meglitinide, LY397364, LY389382, glyclazide, glimepiride, estrogen, estrogen related-compounds (estradiol, estrone, estriol, genistein, non-steroidal estrogen (e.g., diethystilbestrol), phytoestrogen *(e.g.,* coumestrol), zearalenone, *etc.),* and compounds known to inhibit or block K_{ATP} channels. MgADP can also be used to inhibit the channel. Compounds known to inhibit K_{ATP} channels include, but are not limited to, tolbutamide, glyburide (1[p-2[5-chloro-O-anisamido)ethyl] phenyl] sulfonyl] -3-cyclohexyl-3-urea); chlopropamide (1-[[(p-chlorophenyl)sulfonyl]-3-propylurea; glipizide (1-cyclohexyl-3[[p-[2(5-methylpyrazine carboxamido)ethyl] phenyl] sulfonyl] urea); or tolazamide(benzenesulfonamide-N-[[(hexahydro-1H-azepin-1yl)amino] carbonyl] -4-methyl). In additional embodiments, non-sulfonylurea compounds that block the pore of the channel, such as flufenamic acid, may be employed in the invention. In other embodiments, agents such as 2, 3-butanedione, 5-hydroxydecanoic acid, and/or quinine, and therapeutically equivalent salts and derivatives thereof, may be employed in the invention. In specific embodiments, the channel is inhibited by caffeine or tetracaine, for example.

The antagonist can be administered systemically, alimentarily (e.g., orally, buccally, rectally or sublingually); parenterally (*e.g*., intravenously, intradermally, intramuscularly, intraarterially, intrathecally, subcutaneously, intraperitoneally, intraventricularly); by intracavity; intravesicularly; intrapleurally; and/or topically *(e.g.,* transdermally), mucosally, or by direct injection into the brain parenchyma.

Another embodiment of the present invention comprises a method of reducing mortality of a subject suffering from a stroke, comprising administering to the subject a singular or combinatorial therapeutic composition effective at least in part to inhibit NC_{Ca-ATP} channels which is an antagonist of TRPM4 in at least a neuronal cell, a neuroglia cell, a neural endothelial cell or a combination thereof. The compound reduces stroke size and reduces edema located in the peri-infarct tissue. Still further, another embodiment comprises a method of reducing edema in a peri-infarct tissue area of a subject comprising administering to the subject a singular or combinatorial therapeutic composition effective to inhibit NC_{Ca-ATP} channels which is an antagonist of TRPM4 at least in a neuronal cell, a neuroglial cell, a neural endothelial cell, or a combination thereof. Further embodiments comprise a method of treating a subject at risk for developing a stroke, comprising administering to the subject a singular or combinatorial therapeutic composition effective at least in part to inhibit a NC_{Ca-ATP} channel which is an antagonist of TRPM4 in a neural cell, such as a neuronal cell, a neuroglia cell, a neural endothelial cell or a combination thereof.

In certain embodiments, the subject is undergoing treatment for a condition that increases the subject's risk for developing a stroke, such as a cardiac condition, for example. The treatment, for example, may comprise the use of thrombolytic agents to treat myocardial infarctions. Still further, the subject may be at risk for developing a stroke because the subject suffers from atrial fibrillation or a clotting disorder, for example. Other subjects that are at risk for developing a stroke include subjects that are at risk of developing pulmonary emboli, subjects undergoing surgery (e.g., vascular surgery or neurological surgery), or subjects undergoing treatments that increase their risk for developing a stroke, for example; the treatment may comprise cerebral/endovascular treatment, angiography or stent placement. Other subjects at risk include premature infants at risk for developing germinal matrix hemorrhage especially with mechanical ventilation. In other embodiments, the subject may be undergoing treatment for vascular disease that could place the spinal cord at risk for ischemia, such as surgery requiring aortic cross-clamping, surgery for abdominal aortic aneurysm, *etc.* In other embodiments, the patient may be undergoing surgery for a spinal or spinal cord condition, including discectomy, fusion, laminectomy, extradural or intradural surgery for tumor or mass, *etc.,* that would place the spinal cord at risk of injury. In some embodiments of the invention, the subject has a chronic condition, whereas in other embodiments of the invention, the subject does not have a chronic condition, such as a short-term condition. In other embodiments, the subject may have no medical condition either chronic or short-term, but may be placing himself or herself at risk for head injury, brain injury or spinal spinal cord injury by engaging in a dangerous sport such as football, soccer, racing, skiing, horseback riding, *etc.,* or by being part of a military force.

Another embodiment of the present invention comprises a method of treating a subject at risk for developing cerebral edema comprising administering to the subject a singular or combinatorial therapeutic composition effective at least in part to inhibit a NC_{Ca-ATP} channel which is an antagonist of TRPM4 in at least a neuronal cell, a neuroglia cell, a neural endothelial cell, or a combination thereof. The subject at risk may be suffering from an arterial-venous malformation, or a mass-occupying lesion *(e.g.,* hematoma), or may be involved in activities that have an increased risk of brain trauma compared to the general population.

In further embodiments, the compound that inhibits the NC_{Ca-ATP} channel which is an antagonist of TRPM4 can be administered in combination with, for example, a thrombolytic agent (*e.g*., tissue plasminogen activator (tPA), urokinase, prourokinase, streptokinase, anistreplase, reteplase, tenecteplase), an anticoagulant or antiplatelet *(e.g.,* aspirin, warfarin or coumadin), statins, diuretics, vasodilators *(e.g.,* nitroglycerin), mannitol, diazoxide and/or similar compounds that stimulate or promote ischemic precondition. Yet further, another embodiment comprises a pharmaceutical composition comprising a thrombolytic agent *(e.g.,* tissue plasminogen activator (tPA), urokinase, prourokinase, streptokinase, anistreplase, reteplase, tenecteplase), an anticoagulant or antiplatelet *(e.g.,* aspirin, warfarin or coumadin), statins, diuretics, vasodilators, mannitol, diazoxide or similar compounds that stimulate or promote ischemic precondition or a pharmaceutically acceptable salt thereof and a compound that inhibits a NC_{Ca-ATP} channel which is an antagonist of TRPM4 or a pharmaceutically acceptable salt thereof. This pharmaceutical composition can be considered neuroprotective, in specific embodiments. For example, the pharmaceutical composition comprising a combination of the thrombolytic agent and a compound that inhibits a NC_{Ca-ATP} channel is neuroprotective, because it increases the therapeutic window for the administration of the thrombolytic agent by several hours; for example, the therapeutic window for administration of thrombolytic agents may be increased by several hours *(e.g.* about 4-about 8 hrs) by co-administering one or more antagonists of the NC_{Ca-ATP} channel which are TRPM4 channel antagonists.

Still further, another embodiment comprises a method of treating acute cerebral ischemia in a subject comprising administering to a subject an amount of a thrombolytic agent or a pharmaceutically acceptable salt thereof in combination with an amount of a compound that inhibits a NC_{Ca-ATP} channel which is an antagonist of TRPM4 or a pharmaceutically acceptable salt thereof. In certain embodiments, the thrombolytic agent is a tissue plasminogen activator (tPA), urokinase, prourokinase, streptokinase, anistreplase, reteplase, tenecteplase or any combination thereof. The TRPM4 antagonist can be administered by any standard parenteral or alimentary route; for example the TRPM4 antagonist may be administered as a bolus injection or as an infusion or a combination thereof.

An effective amount of an inhibitor of NC_{Ca-ATP} channel which is an antagonist of TRPM4 that may be administered to an individual or a cell in a tissue or organ thereof includes a dose of about 0.0001 nM to about 2000 µM, for example. More specifically, doses of an antagonist to be administered are from about 0.01 nM to about 2000µM; about 0.01 µM to about 0.05 µM; about 0.05 µM to about 1.0 µM; about 1.0 µM to about 1.5 µM; about 1.5 µM to about 2.0 µM; about 2.0 µM to about 3.0 µM; about 3.0 µM to about 4.0 µM; about 4.0 µM to about 5.0 µM; about 5.0 µM to about 10 µM; about 10 µM to about 50 µM; about 50 µM to about 100 µM; about 100 µM to about 200 µM; about 200 µM to about 300 µM; about 300 µM to about 500µM; about 500 µM to about 1000 µM; about 1000 µM to about 1500 µM and about 1500 µM to about 2000 µM, for example. Of course, all of these amounts are exemplary, and any amount in-between these dosages is also expected to be of use in the invention.

An effective amount of an inhibitor of the NC_{Ca-ATP} channel which is an antagonist of TRPM4 as a treatment varies depending upon the host treated and the particular mode of administration. In one embodiment of the invention the dose range of the antagonist of the NC_{Ca-ATP} channel which is an antagonist of TRPM4 will be about 0.01 µg/kg body weight to about 20,000 µg/kg body weight.

In specific embodiments, the dosage is less than 0.8 mg/kg. In particular aspects, the dosage range may be from 0.005 mg/kg to 0.8 mg/kg body weight, 0.006 mg/kg to 0.8 mg/kg body weight, 0.075 mg/kg to 0.8 mg/kg body weight, 0.08 mg/kg to 0.8 mg/kg body weight, 0.09 mg/kg to 0.8 mg/kg body weight, 0.005 mg/kg to 0.75 mg/kg body weight, 0.005 mg/kg to 0.7 mg/kg body weight, 0.005 mg/kg to 0.65 mg/kg body weight, 0.005 mg/kg to 0.5 mg/kg body weight, 0.09 mg/kg to 0.8 mg/kg body weight, 0.1 mg/kg to 0.75 mg/kg body weight, 0.1 mg/kg to 0.70 mg/kg body weight, 0.1 mg/kg to 0.65 mg/kg body weight, 0.1 mg/kg to 0.6 mg/kg body weight, 0.1 mg/kg to 0.55 mg/kg body weight, 0.1 mg/kg to 0.5 mg/kg body weight, 0.1 mg/kg to 0.45 mg/kg body weight, 0.1 mg/kg to 0.4 mg/kg body weight, 0.1 mg/kg to 0.35 mg/kg body weight, 0.1 mg/kg to 0.3 mg/kg body weight, 0.1 mg/kg to 0.25 mg/kg body weight, 0.1 mg/kg to 0.2 mg/kg body weight, or 0.1 mg/kg to 0.15 mg/kg body weight, for example.

In specific embodiments, the dosage range may be from 0.2 mg/kg to 0.8 mg/kg body weight, 0.2 mg/kg to 0.75 mg/kg body weight, 0.2 mg/kg to 0.70 mg/kg body weight, 0.2 mg/kg to 0.65 mg/kg body weight, 0.2 mg/kg to 0.6 mg/kg body weight, 0.2 mg/kg to 0.55 mg/kg body weight, 0.2 mg/kg to 0.5 mg/kg body weight, 0.2 mg/kg to 0.45 mg/kg body weight, 0.2 mg/kg to 0.4 mg/kg body weight, 0.2 mg/kg to 0.35 mg/kg body weight, 0.2 mg/kg to 0.3 mg/kg body weight, or 0.2 mg/kg to 0.25 mg/kg body weight, for example.

In further specific embodiments, the dosage range may be from 0.3 mg/kg to 0.8 mg/kg body weight, 0.3 mg/kg to 0.75 mg/kg body weight, 0.3 mg/kg to 0.70 mg/kg body weight, 0.3 mg/kg to 0.65 mg/kg body weight, 0.3 mg/kg to 0.6 mg/kg body weight, 0.3 mg/kg to 0.55 mg/kg body weight, 0.3 mg/kg to 0.5 mg/kg body weight, 0.3 mg/kg to 0.45 mg/kg body weight, 0.3 mg/kg to 0.4 mg/kg body weight, or 0.3 mg/kg to 0.35 mg/kg body weight, for example.

In specific embodiments, the dosage range may be from 0.4 mg/kg to 0.8 mg/kg body weight, 0.4 mg/kg to 0.75 mg/kg body weight, 0.4 mg/kg to 0.70 mg/kg body weight, 0.4 mg/kg to 0.65 mg/kg body weight, 0.4 mg/kg to 0.6 mg/kg body weight, 0.4 mg/kg to 0.55 mg/kg body weight, 0.4 mg/kg to 0.5 mg/kg body weight, or 0.4 mg/kg to 0.45 mg/kg body weight, for example.

In specific embodiments, the dosage range may be from 0.5 mg/kg to 0.8 mg/kg body weight, 0.5 mg/kg to 0.75 mg/kg body weight, 0.5 mg/kg to 0.70 mg/kg body weight, 0.5 mg/kg to 0.65 mg/kg body weight, 0.5 mg/kg to 0.6 mg/kg body weight, or 0.5 mg/kg to 0.55 mg/kg body weight, for example. In specific embodiments, the dosage range may be from 0.6 mg/kg to 0.8 mg/kg body weight, 0.6 mg/kg to 0.75 mg/kg body weight, 0.6 mg/kg to 0.70 mg/kg body weight, or 0.6 mg/kg to 0.65 mg/kg body weight, for example. In specific embodiments, the dosage range may be from 0.7 mg/kg to 0.8 mg/kg body weight or 0.7 mg/kg to 0.75 mg/kg body weight, for example. In specific embodiments the dose range may be from 0.001 mg/day to 3.5 mg/day. In other embodiments, the dose range may be from 0.001 mg/day to 10mg/day. In other embodiments, the dose range may be from 0.001 mg/day to 20mg/day.

Further, those of skill will recognize that a variety of different dosage levels will be of use, for example, 0.0001 µg/kg, 0.0002 µg/kg, 0.0003 µg/kg, 0.0004 µg/kg, 0.005 µg/kg, 0.0007 µg/kg, 0.001 µg/kg, 0.1 µg/kg, 1.0 µg/kg, 1.5 µg/kg, 2.0 µg/kg, 5.0 µg/kg, 10.0 µg/kg, 15.0 µg/kg, 30.0 µg/kg, 50 µg/kg, 75 µg/kg, 80 µg/kg, 90 µg/kg, 100 µg/kg, 120 µg/kg, 140 µg/kg, 150 µg/kg, 160 µg/kg, 180 µg/kg, 200 µg/kg, 225 µg/kg, 250 µg/kg, 275 µg/kg, 300 µg/kg, 325 µg/kg, 350 µg/kg, 375 µg/kg, 400 µg/kg, 450 µg/kg, 500 µg/kg, 550 µg/kg, 600 µg/kg, 700 µg/kg, 750 µg/kg, 800 µg/kg, 900 µg/kg, 1 mg/kg, 5 mg/kg, 10 mg/kg, 12 mg/kg, 15 mg/kg, 20 mg/kg, and/or 30 mg/kg. In particular embodiments, there may be dosing of from very low ranges (e.g. 1 mg/kg/day or less; 5 mg/kg bolus; or 1 mg/kg/day) to moderate doses *(e.g.* 2 mg bolus, 15 mg/day) to high doses *(e.g.* 5 mg bolus, 30-40 mg/day; and even higher). Of course, all of these dosages are exemplary, and any dosage in-between these dosages is also expected to be of use in the invention. Any of the above dosage ranges or dosage levels may be employed for an agonist or antagonist, or both, of NC_{Ca-ATP} channel or related-compounds thereof.

An effective amount of a therapeutic composition of the invention, including an antagonist of NC _{Ca-ATP} channel which is an antagonist of TRPM4 and/or the additional therapeutic compound, that may be administered to a cell includes a dose of about 0.0001 nM to about 2000 µM, for example. More specifically, doses to be administered are from about 0.01 nM to about 2000µM; about 0.01 µM to about 0.05 µM; about 0.05 µM to about 1.0 µM; about 1.0 µM to about 1.5 µM; about 1.5 µM to about 2.0 µM; about 2.0 µM to about 3.0 µM; about 3.0 µM to about 4.0 µM; about 4.0 µ M to about 5.0 µM; about 5.0 µM to about 10 µM; about 10 µM to about 50 µM; about 50 µM to about 100 µM; about 100 µM to about 200 µM; about 200 µM to about 300 µM; about 300 µM to about 500µM; about 500 µM to about 1000 µM; about 1000 µM to about 1500 µM and about 1500 µM to about 2000 µM, for example. Of course, all of these amounts are exemplary, and any amount in-between these dosages is also expected to be of use in the invention.

An effective amount of an antagonist of the NC_{Ca-ATP} channel which is an antagonist of TRPM4 as a treatment varies depending upon the host treated and the particular mode of administration. In one embodiment of the invention, the dose range of the therapeutic combinatorial composition of the invention, including an antagonist of NC_{Ca-ATP} channel which is an antagonist of TRPM4 and/or the additional therapeutic compound, is about 0.01 µg/kg body weight to about 20,000 µg/kg body weight. The term "body weight" is applicable when an animal is being treated. When isolated cells are being treated, "body weight" as used herein should read to mean "total cell body weight". The term "total body weight" may be used to apply to both isolated cell and animal treatment. All concentrations and treatment levels are expressed as "body weight" or simply "kg" in this application are also considered to cover the analogous "total cell body weight" and "total body weight" concentrations. However, those of skill will recognize the utility of a variety of dosage range, for example, 0.01 µg/kg body weight to 20,000 µg/kg body weight, 0.02 µg/kg body weight to 15,000 µg/kg body weight, 0.03 µg/kg body weight to 10,000 µg/kg body weight, 0.04 µg/kg body weight to 5,000 µg/kg body weight, 0.05 µg/kg body weight to 2,500 µg/kg body weight, 0.06 µg/kg body weight to 1,000 µg/kg body weight, 0.07 µg/kg body weight to 500 µg/kg body weight, 0.08 µg/kg body weight to 400 µg/kg body weight, 0.09 µg/kg body weight to 200 µg/kg body weight or 0.1 µg/kg body weight to 100 µg/kg body weight. Further, those of skill will recognize that a variety of different dosage levels are of use, for example, 0.0001 µg/kg, 0.0002 µg/kg, 0.0003 µg/kg, 0.0004 µg/kg, 0.005 µg/kg, 0.0007 µg/kg, 0.001 µg/kg, 0.1 µg/kg, 1.0 µg/kg, 1.5 µg/kg, 2.0 µg/kg, 5.0 µg/kg, 10.0 µg/kg, 15.0 µg/kg, 30.0 µg/kg, 50 µg/kg, 75 µg/kg, 80 µg/kg, 90 µg/kg, 100 µg/kg, 120 µg/kg, 140 µg/kg, 150 µg/kg, 160 µg/kg, 180 µg/kg, 200 µg/kg, 225 µg/kg, 250 µg/kg, 275 µg/kg, 300 µg/kg, 325 µg/kg, 350 µg/kg, 375 µg/kg, 400 µg/kg, 450 µg/kg, 500 µg/kg, 550 µg/kg, 600 µg/kg, 700 µg/kg, 750 µg/kg, 800 µg/kg, 900 µg/kg, 1 mg/kg, 5 mg/kg, 10 mg/kg, 12 mg/kg, 15 mg/kg, 20 mg/kg, and/or 30 mg/kg.

In particular embodiments, there may be dosing of from very low ranges *(e.g.* for glyburide 1 mg/day or less) to moderate doses (*e.g.* 3.5 mg/day) to high doses *(e.g.* 10-40 mg/day; and even higher). Of course, all of these dosages are exemplary, and any dosage in-between these dosages is also expected to be of use in the invention. Any of the above dosage ranges or dosage levels may be employed for an antagonist of NC_{Ca-ATP} channel which is an antagonist of TRPM4.

In a particular embodiment, the dosage is about 0.5 mg/day too about 10 mg/day.

In certain embodiments, the amount of the combinatorial therapeutic composition administered to the subject is in the range of about 0.0001 µg/kg/day to about 20 mg/kg/day, about 0.01 µg/kg/day to about 100 µg/kg/day, or about 100 µg/kg/day to about 20 mg/kg/day. Still further, the combinatorial therapeutic composition may be administered to the subject in the form of a treatment in which the treatment may comprise the amount of the combinatorial therapeutic composition or the dose of the combinatorial therapeutic composition that is administered per day (1, 2, 3, 4, *etc*.), week (1, 2, 3, 4, 5, *etc*.), month (1, 2, 3, 4, 5, *etc*.), *etc.* Treatments may be administered such that the amount of combinatorial therapeutic composition administered to the subject is in the range of about 0.0001 µg/kg/treatment to about 20 mg/kg/treatment, about 0.01 µg/kg/treatment to about 100 µg/kg/treatment, or about 100 µg/kg/treatment to about 20 mg/kg/treatment.

A typical dosing regime consists of a loading dose designed to reach a target agent plasma level followed by an infusion of up to 7 days to maintain that target level. One skilled in the art will recognize that the pharmacokinetics of each agent will determine the relationship between the load dose and infusion rate for a targeted agent plasma level. In one example, for intravenous glyburide administration, a 15.7 µg bolus (also called a loading dose) is followed by a maintenance dose of 0.3 µg/min (432 µg/day) for 120 hours (5 days). This dose regime is predicted to result in a steady-state plasma concentration of 4.07 ng/mL. In another example for intravenous glyburide, a 117 µg bolus dose is followed by a maintenance dose of 2.1 µg/min (3 mg/day) for 3 days. This dose is predicted to result in a steady-state plasma concentration of 28.3 ng/mL. In yet another example for glyburide, a 665 µg bolus dose is followed by a maintenance dose of 11.8 µg/min (17 mg/day) for 120 hours (5 days). This dose is predicted to result in a steady-state plasma concentration of 160.2 ng/mL. Once the pharmacokinetic parameters for an agent are known, loading dose and infusion dose for any specified targeted plasma level can be calculated. As an illustrative case for glyburide, the bolus is generally 30-90 times, for example 40-80 times, such as 50-60 times, the amount of the maintenance dose, and one of skill in the art can determine such parameters for other compounds based on the guidance herein.

In cases where combination therapies are utilized, the components of the combination may be of any kind. In specific embodiments, the components are provided to an individual substantially concomitantly, whereas in other cases the components are provided at separate times. The ratio of the components may be determined empirically, as is routine in the art. Exemplary ratios include at least about the following: 1:1, 1:2, 1:3, 1:4, 1:5, 1:6, 1:7, 1:8, 1:9, 1:10, 1:20, 1:25, 1:30, 1:40, 1:50, 1:60, 1:70, 1:80, 1:90, 1:100, 1:500, 1:750, 1:1000, 1:10000, and so forth.

In another embodiment, there is a kit, housed in a suitable container, that comprises an inhibitor of NC_{Ca-ATP} channel which is an antagonist of TRPM4 and, in some cases, one or more of a cation channel blocker and/or an antagonist of VEGF, MMP, NOS, or thrombin, for example. The kit may also comprise suitable tools to administer compositions of the invention to an individual.

In one embodiment, there is a composition comprising a compound that inhibits a NC_{Ca-ATP} channel which is an antagonist of TRPM4 and an additional therapeutic compound, wherein the additional therapeutic compound is selected from the group consisting of: a) one or more cation channel blockers; and b) one or more of a compound selected from the group consisting of one or more antagonists of vascular endothelial growth factor (VEGF), one or more antagonists of matrix metalloprotease (MMP), one or more antagonists of nitric oxide synthase (NOS), one or more antagonists of thrombin, aquaporin, or a biologically active derivative thereof, wherein the NC_{Ca-ATP} channel has the following characteristics: 1) it is a non-selective monovalent cation channel; 2) it is activated by an increase in intracellular calcium or by a decrease in intracellular ATP, or both; and 3) it is regulated by a SUR1.

Compounds that inhibit the NC_{Ca-ATP} channel may be SUR1 antagonists, such as, for example, mitiglinide, iptakalim, endosulfines, glibenclamide, tolbutamide, repaglinide, nateglinide, meglitinide, midaglizole, LY397364, LY389382, glyclazide, glimepiride, estrogen, estradiol, estrone, estriol, genistein, diethystilbestrol, coumestrol, zearalenone, a compound that inhibits K_{ATP} channels, and a combination thereof. In a specific embodiment, the cation channel blocker is selected from the group consisting of pinkolant, rimonabant, a fenamate (such as flufenamic acid, mefenamic acid, meclofenamic acid, or niflumic acid), and SKF 96365 (SK&F 96365) 1-(beta-[3-(4-methoxy-phenyl)propoxy]-4-methoxyphenethyl)-1H- imidazole hydrochloride, and a biologically active derivative thereof. In specific embodiments, the compound that inhibits the NC_{Ca-ATP} channel is a TRPM4 antagonist, such as a nucleic acid, including a siRNA; a protein; a small molecule; or a combination thereof.

In a further specific embodiment, one or more antagonists of vascular endothelial growth factor (VEGF) are soluble neuropilin 1 (NRP-1), undersulfated LMW glycol-split heparin, VEGF TrapR1R2, Bevacizumab, HuMV833, s-Flt-1, s-Flk-1, s-Flt-1/Flk-1, NM-3, GFB 116, or a combination or mixture thereof. In an additional specific embodiment, the undersulfated, LMW glycol-split heparin comprises ST2184. In an additional specific embodiment, the one or more antagonists of matrix metalloprotease (MMP) are (2R)-2-[(4-biphenylsulfonyl)amino]-3-phenylproprionic acid, GM-6001, TIMP-1, TIMP-2, RS 132908, batimastat, marimastat, a peptide inhibitor that comprises the amino acid sequence HWGF, or a mixture or combination thereof.

In one aspect of the invention, the one or more antagonists of nitric oxide synthase (NOS) are aminoguanidine (AG), 2-amino-5,6-dihydro-6-methyl-4H-1,3 thiazine (AMT), S-ethylisothiourea (EIT), asymmetric dimethylarginine (ADMA), N-nitro-L-arginine methylester (L-NAME), nitro-L-arginine (L-NA), N-(3-aminomethyl) benzylacetamidine dihydrochloride (1400W), NG-monomethyl-L-arginine (L-NMMA), 7-nitroindazole (7-NINA), N-nitro-L-arginine (L-NNA), or a mixture or combination thereof. In another aspect of the invention, the one or more antagonists of thrombin are ivalirudi, hirudin, SSR182289, antithrombin III, thrombomodulin, lepirudin, P-PACK II (d-Phenylalanyl-L-Phenylalanylarginine- chloro-methyl ketone 2 HCl), (BNas-Gly-(pAM)Phe-Pip), Argatroban, and mixtures or combinations thereof.

In an embodiment of the present invention, there is a method of inhibiting neural cell swelling in an individual having traumatic brain injury, cerebral eschemia, central nervous system (CNS) damage, peripheral nervous system (PNS) damage, cerebral hypoxia, or cerebral edema, comprising delivering to the individual a therapeutically effective amount of a composition of the invention.

In a specific embodiment, the compound that inhibits the NC_{Ca-ATP} channel which is an antagonist of TRPM4 and the additional therapeutic compound are delivered to the individual successively. In another specific embodiment, the compound that inhibits the NC_{Ca-ATP} channel which is an antagonist of TRPM4 is delivered to the individual prior to delivery of the additional therapeutic compound. In a further specific embodiment, the compound that inhibits the NC_{Ca-ATP} channel which is an antagonist of TRPM4 is delivered to the individual subsequent to delivery of the additional therapeutic compound. In another aspect, the compound that inhibits the NC_{Ca-ATP} channel which is an antagonist of TRPM4 and the additional therapeutic compound are delivered to the individual concomitantly. In an additional aspect, the compound that inhibits the NC_{Ca-ATP} channel which is an antagonist of TRPM4 and the additional therapeutic compound being delivered as a mixture. In an additional embodiment, the compound that inhibits the NC_{Ca-ATP} channel which is an antagonist of TRPM4 and the additional therapeutic compound act synergistically in the individual. In a particular case, the compound that inhibits the NC_{Ca-ATP} channel which is an antagonist of TRPM4 and/or the additional therapeutic compound is delivered to the individual at a certain dosage or range thereof, such as is provided in exemplary disclosure elsewhere herein.

In a specific embodiment, the compound that inhibits the NC_{Ca-ATP} channel is glibenclamide, and the maximum dosage of glibenclamide for the individual is about 20 mg/day. In a further specific embodiment, the compound that inhibits the NC_{Ca-ATP} channel is glibenclamide, and the dosage of glibenclamide for the individual is between about 2.5 mg/day and about 20 mg/day. In an additional specific embodiment, the compound that inhibits the NC_{Ca-ATP} channel is glibenclamide, and the dosage of glibenclamide for the individual is between about 5 mg/day and about 15 mg/day. In another specific embodiment, the compound that inhibits the NC_{Ca-ATP} channel is glibenclamide, and the dosage of glibenclamide for the individual is between about 5 mg/day and about 10 mg/day. In a still further specific embodiment, the compound that inhibits the NC_{Ca-ATP} channel is glibenclamide, and the dosage of glibenclamide for the individual is about 7 mg/day. In particular cases, the dosage is about 0.5 mg/day too about 10 mg/day.

In an additional embodiment, there is a kit comprising a composition of the invention, wherein the compound that inhibits the NC_{Ca-ATP} channel which is an antagonist of TRPM4 and the additional therapeutic compound are housed in one or more suitable containers.

In one exemplary embodiment concerning singular therapeutic compositions of the invention, there is a method of inhibiting neural cell swelling in an individual having traumatic brain injury, cerebral ischemia, central nervous system (CNS) damage, peripheral nervous system (PNS) damage, cerebral hypoxia, or cerebral edema, comprising delivering to the individual a therapeutically effective amount of an antagonist of TRPM4. In specific embodiments, the antagonist of TRPM4 is a nucleic acid (such as a TRPM4 siRNA, for example), a protein, a small molecule, or a combination thereof. In particular aspects, the method further comprises delivering to the individual a therapeutically effective amount of an additional therapeutic compound selected from the group consisting of: a) a SUR1 antagonist; b) one or more cation channel blockers; b) one or more of a compound selected from the group consisting of one or more antagonists of vascular endothelial growth factor (VEGF), one or more antagonists of matrix metalloprotease (MMP), one or more antagonists of nitric oxide synthase (NOS), one or more antagonists of thrombin, aquaporin, a biologically active derivative thereof, and a combination thereof; and d) a combination thereof.

In specific cases, the TRPM4 antagonist and the additional therapeutic compound are delivered to the individual successively, such as the TRPM4 antagonist being delivered to the individual prior to delivery of the additional therapeutic compound, the TRPM4 antagonist being delivered to the individual subsequent to delivery of the additional therapeutic compound, or the TRPM4 antagonist and the additional therapeutic compound being delivered to the individual concomitantly. In certain cases, the TRPM4 antagonist and the additional therapeutic compound are delivered as a mixture, and in particular aspects, the TRPM4 antagonist and the additional therapeutic compound act synergistically in the individual.

In some embodiments, several pathways to neural cell death are involved in ischemic stroke, and all require monovalent or divalent cation influx, implicating non-selective cation (NC) channels. NC channels are also involved in the dysfunction of vascular endothelial cells that leads to formation of edema following cerebral ischemia. Non-specific blockers of NC channels, including pinokalant (LOE 908 MS) and rimonabant (SR141716A), for example, have beneficial effects in rodent models of ischemic stroke and are useful in treatment methods of the invention.

In other embodiments of the invention, focal cerebral ischemia and post-ischemic reperfusion cause cerebral capillary dysfunction, resulting in edema formation and hemorrhagic conversion. In specific embodiments, the invention generally concerns the central role of Starling's principle, which states that edema formation is determined by the "driving force" and capillary "permeability pore". In particular aspects related to the invention, movements of fluids are driven largely without new expenditure of energy by the ischemic brain. In one embodiment, the progressive changes in osmotic and hydrostatic conductivity of abnormal capillaries is organized into 3 phases: formation of ionic edema, formation of vasogenic edema, and catastrophic failure with hemorrhagic conversion. In particular embodiments, ischemia-induced capillary dysfunction is attributed to *de novo* synthesis of a specific ensemble of proteins that determine the terms for osmotic and hydraulic conductivity in Starling's equation, and whose expression is driven by a distinct transcriptional program.

The foregoing has outlined rather broadly the features and technical advantages of the present invention in order that the detailed description of the invention that follows may be better understood. Additional features and advantages of the invention will be described hereinafter which form the subject of the claims of the invention. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following exemplary descriptions taken in conjunction with the accompanying exemplary drawings.

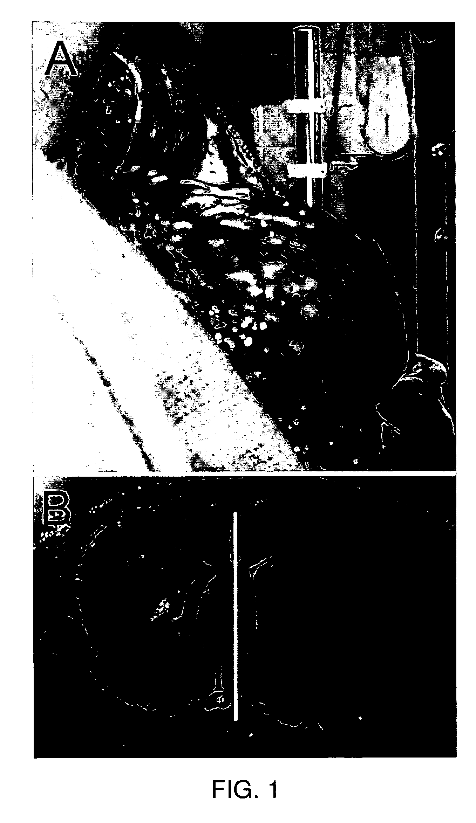

FIGS. 1A-1B show brain swelling after middle cerebral artery occlusion in human and rat. FIG. 1A: Intra-operative photograph showing massive brain swelling causing herniation of the brain out of the skull following decompressive craniectomy. FIG. 1B: Photograph of coronal section of rat head following middle cerebral artery occlusion; post-mortem perfusion with Evans blue and India ink shows regions with persistent circulation (darker areas, left) versus regions without appreciable circulation (pink area, right); white line from the superior sagittal sinus to the clivus indicates the midline, showing extensive shift due to massive swelling of the involved hemisphere.

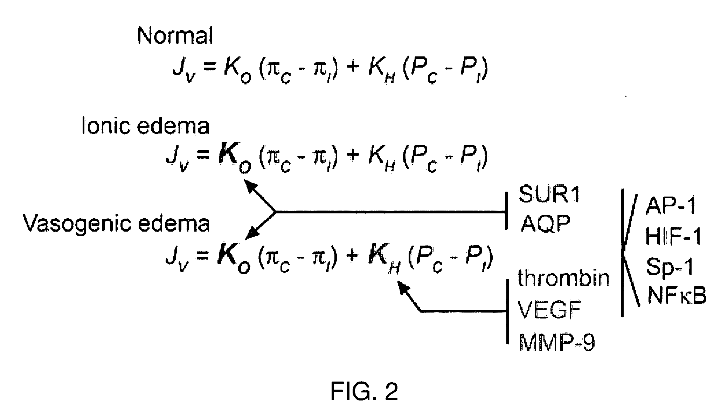

FIG. 2 provides Starling's equation, classically stated as Jv = Kf [(P*_{c}*-P*ᵢ*)-(π_{c} -πᵢ)], which describes capillary permeability under normal and pathological conditions. Formulated in 1896 by the British physiologist Ernest Starling, the Starling equation describes the role of hydrostatic and osmotic forces in the movement of fluid across capillary endothelial cells. According to Starling's equation, the movement of fluid depends on five variables: capillary hydrostatic pressure (P_{c}), interstitial hydrostatic pressure (Pi), capillary osmotic pressure (π_{c}), interstitial osmotic pressure (πᵢ), and a filtration coefficient (K_{f}). Here, two distinct "filtration" coefficients, the hydraulic conductivity (K_{H}), and the osmotic conductivity (K_{O}), are used to describe the situation in brain capillaries. The equation gives the net filtration or net fluid movement (Jᵥ), with outward force being positive, meaning that fluid will tend to leave the capillary. The filtration coefficients, K_{H} and Ko, determine edema formation. Normally, values of K_{O} and K_{H} are small or close to zero, and no edema forms. With ionic edema, K_{O} >> 0 and K_{H} ≈ 0, with the change in K_{O} being due to up-regulation of Na⁺ flux pathways such as the SUR1-regulated NC_{Ca-ATP} channel and possibly aquaporin (AQP) channels. With vasogenic edema, K_{O} >> 0 and K_{H} >> 0, with the increase in K_{H} being due to up-regulation of prothrombin, VEGF and MMP-9. Up-regulation of various edema-associated proteins can be attributed, at least in part, to activation of a transcriptional program involving AP-1, HIF-1, Sp-1 and NF-κB. Note that the driving forces for fluid movement are not generated by the ischemic brain; rather, hydrostatic pressure, P, is generated by the heart, and osmotic pressure, π, arises from potential energy stored in electrochemical gradients established before onset of ischemia.

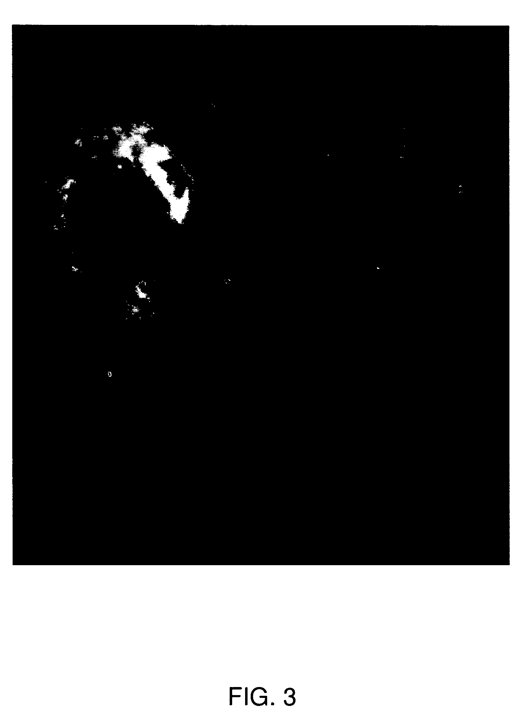

FIG. 3 shows that SUR1, the regulatory subunit of the NC_{Ca-ATP} channel, is up-regulated in focal cerebral ischemia. Capillary (left) labeled for von Willebrand factor and for SUR1, next to a dying neuron with blebs (right) that labels strongly for SUR1; nuclei labeled with DAPI; brain tissue from the core of the infarct 6 h after middle cerebral artery occlusion.

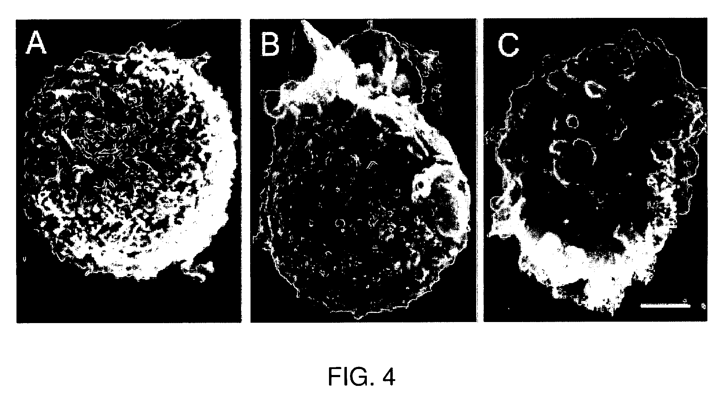

FIGS. 4A-4C show cell blebbing after NaN₃-induced ATP depletion. Scanning electron micrographs of freshly isolated native reactive astrocytes. Formaldehyde-glutaraldehyde fixation was initiated under control conditions (4A), 5 min after exposure to 1 mM NaN₃ (4B), and 25 min after exposure to 1 mM NaN₃ (4C). Bar, 12 µm.

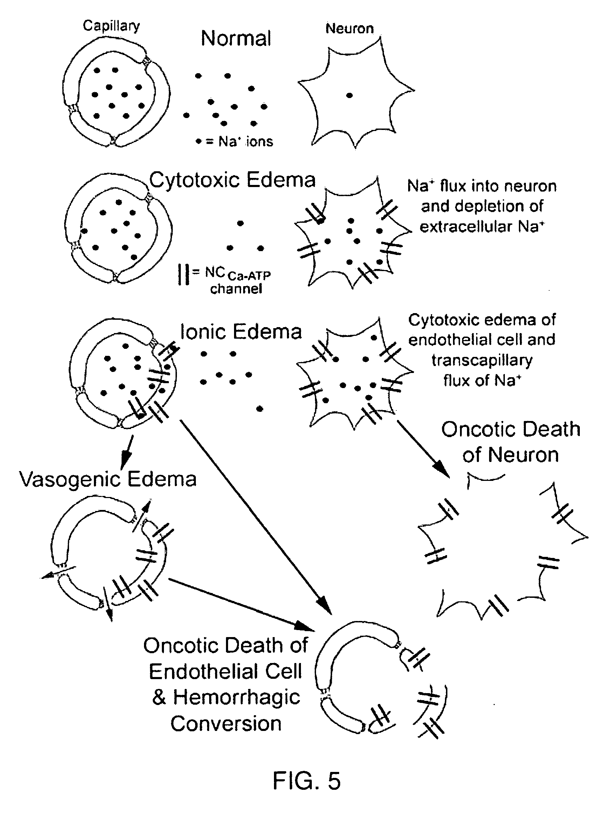

FIG. 5 provides an exemplary schematic diagram illustrating various types of edema progressing to hemorrhagic conversion. Normally, Na⁺ concentrations in serum and in extracellular space are the same, and much higher than inside the neuron. Cytotoxic edema of neurons is due to entry of Na⁺ into ischemic neurons *via* pathways such as NC_{Ca-ATP} channels, depleting extracellular Na⁺ and thereby setting up a concentration gradient between intravascular and extracellular compartments. Ionic edema results from cytotoxic edema of endothelial cells, due to expression of cation channels on both the luminal and abluminal side, allowing Na⁺ from the intravascular compartment to traverse the capillary wall and replenish Na⁺ in the extracellular space. Vasogenic edema results from degradation of tight junctions between endothelial cells, transforming capillaries into "fenestrated" capillaries that allow extravasation (outward filtration) of proteinatious fluid. Oncotic death of neuron is the ultimate consequence of cytotoxic edema. Oncotic death of endothelial cells results in complete loss of capillary integrity and in extravasation of blood. *i.e*., hemorrhagic conversion.

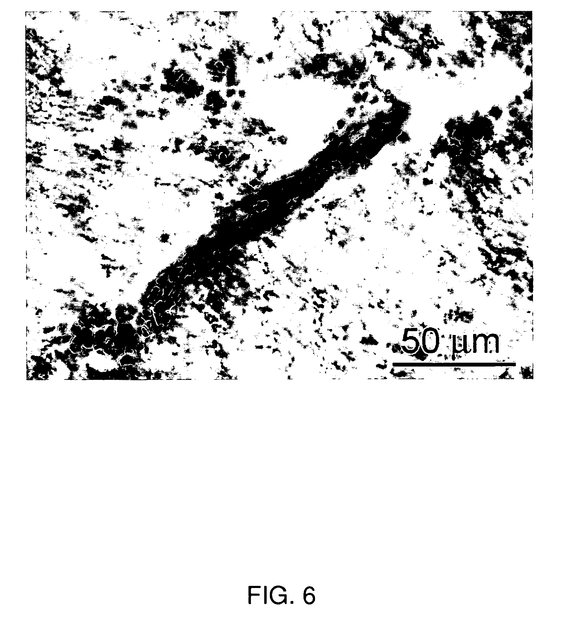

FIG. 6 shows hemorrhagic conversion with petechial hemorrhage is associated with transcriptional up-regulation of sulfonylurea receptor 1 (SUR1) in ischemic CNS tissues. *In situ* hybridization for SUR1 (azure) shows strong labeling with antisense probe in a microvessel surrounded by extravasated erythrocytes (red); control tissue labeled with antisense probe and ischemic tissue labeled with sense probe showed little or no labeling.

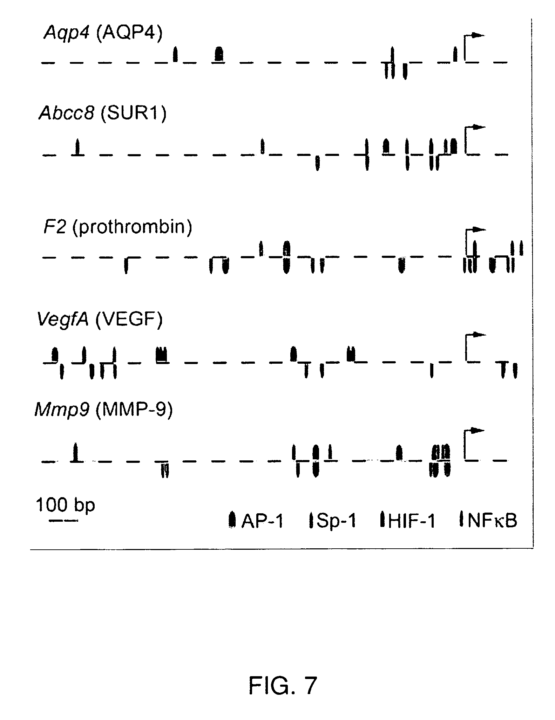

FIG. 7 shows that a distinct transcriptional program may account for sequential changes in ischemia-induced changes in BBB permeability. The promoter regions of five genes (italicized) for proteins (in parentheses) involved in edema, *Aqp4* (AQP4), *Abcc8* (SUR1), *F*2 (prothrombin), *VegfA* (VEGF) and *Mmp9* (MMP-9), were analyzed for potential consensus sequence binding sites for the transcription factors, AP-1, Sp-1, HIF-1 and NF-κB, using Gene2Promoter and MatInspector applets (see Genomatrix website). Promoter size was estimated as 1500 bp upstream and 200 bp downstream of the start codon (marked by a right-angle arrow). The "core sequence" of a matrix was defined as the (usually) 4 consecutive highest conserved positions of the matrix. The maximum core similarity of 1.0 is only reached when the highest conserved bases of a matrix match exactly in the sequence. More important than the core similarity is the matrix similarity, which takes into account all bases over the whole matrix length. A perfect match to the matrix receives a score of 1.0 (each sequence position corresponds to the highest conserved nucleotide at that position in the matrix), a "good" match to the matrix has a similarity >0.80. The number of putative binding sites and the range for values of matrix similarity (in parentheses) for Sp-1, AP-1, NF-κB and HIF-1, respectively, were for Aqp4: 3 (0.88-0.89), 2 (0.70-0.84), 3 (0.92-0.94), 1 (0.87); for Abcc8: 7 (0.88-1.00), 1 (0.89), 5 (0.85-1.00), 2 (0.99); for F2: 3 (0.88-0.94), 6 (0.82-0.92), 8 (0.83-0.99), 2 (0.92-0.96); for VegfA: 10 (0.85-1.00), 2 (0.73-0.92), 3 (0.85-0.91), 4 (0.89-0.96); for MMP9: 7 (0.81-0.99), 13 (0.72-1.00), 2 (0.87-0.97), 1 (0.90). The location of these putative binding sites on each promoter region is shown, with binding sites on the positive and negative strands indicated by upward and downward symbols, respectively (some symbols overlap, making the number of binding sites shown appear to be less than the number given).

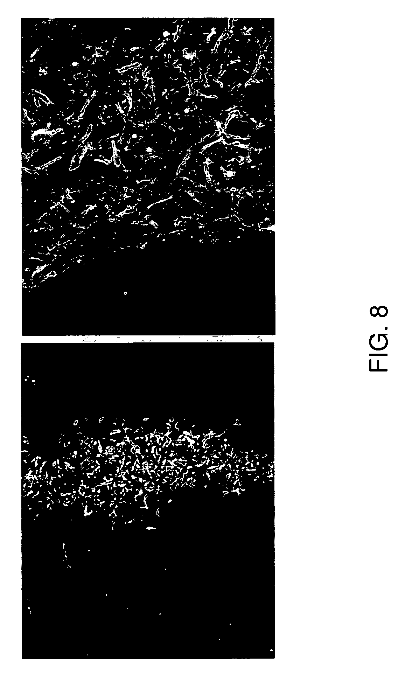

FIG. 8 shows TRPM4 immunolabeling in gliotic capsule. Inner zone of gliotic capsule imaged 28 days after implantation of a gelatin sponge into the parietal lobe of a rat, immunolabeled for TRPM4, shown at 20x and 40x; implant site is to the left.

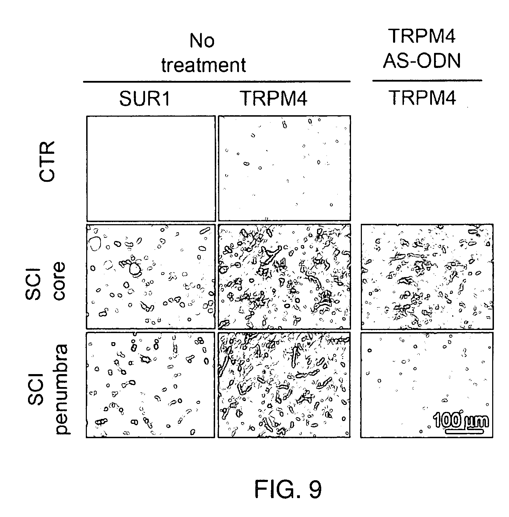

FIG. 9 shows immunolabeling for SUR1 and TRPM4 in cervical spinal cord injury (SCI). Labeling for SUR1 and TRPM4 is minimal in uninjured spinal cord (CTR). Twenty four hours following severe cervical SCI, labeling for SUR1 and TRPM4 is strong in various cells in the core, as well as in capillaries in penumbral tissues outside the core. Treatment with antisense oligodeoxynucleotide (AS-ODN) significantly reduces TRPM4 labeling in the core, with residual labeling present only in reactive astrocytes, but not in capillaries in either the core or penumbra.

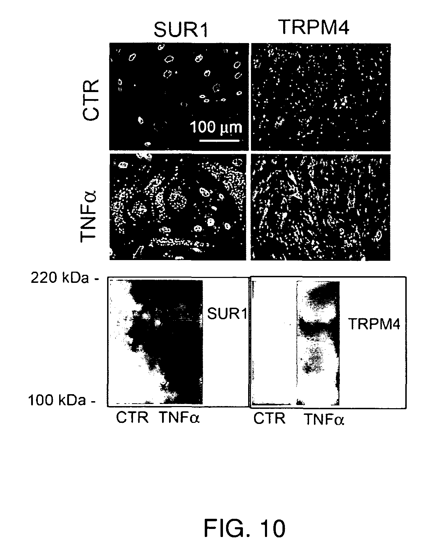

FIG. 10 demonstrates immunolabeling and Western blots for SUR1 and TRPM4 in bEnd.3 cells. Labeling for SUR1 and TRPM4 is minimal in untreated control cells (CTR). Six hours following exposure to TNFα, labeling for SUR1 and TRPM4 is prominent in all cells. Western blots for SUR1 and TRPM4 show little signal under control conditions (CTR), but prominent up-regulation of SUR1 and TRPM4 6 h after exposure to TNFα.

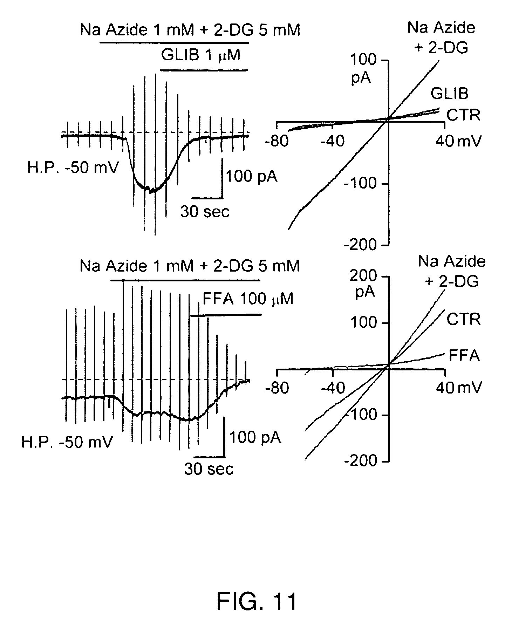

FIG. 11 demonstrates NC_{Ca-ATP} channel currents in bEnd.3 cells. Whole cell patch clamp of bEnd.3 cells exposed to TNFα for 12-15 hr to induce expression of NC_{Ca-ATP} channels. Application of Na azide plus 2-deoxyglucose to deplete cellular ATP turns on a strong inward current at the holding potential of -50 mV. Ramp pulses reveal that the new current is ohmic and reverses near 0 mV, consistent with an ATP-sensitive non-selective cation current. Application of glibenclamide blocks this current, as expected for an SUR1-regulated channel. Application of flufenamic acid also blocks this current, as expected for TRPM4.

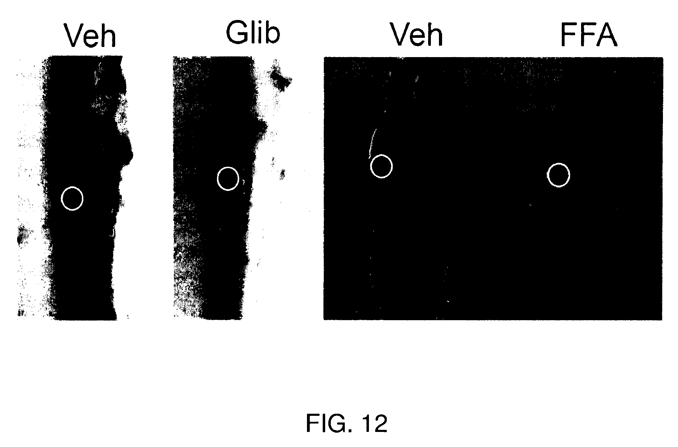

FIG. 12 provides NC_{Ca-ATP} channel currents in bEnd.3 cells. Whole cell patch clamp of bEnd.3 cells exposed to TNFα for 12-15 hr to induce expression of NC_{Ca-ATP} channels. Application of Na azide plus 2-deoxyglucose to deplete cellular ATP turns on a strong inward current at the holding potential of -50 mV. Ramp pulses reveal that the new current is ohmic and reverses near 0 mV, consistent with an ATP-sensitive non-selective cation current. Application of glibenclamide blocks this current, as expected for an SUR1-regulated channel. Application of flufenamic acid also blocks this current, as expected for TRPM4. Veh= vehicle.

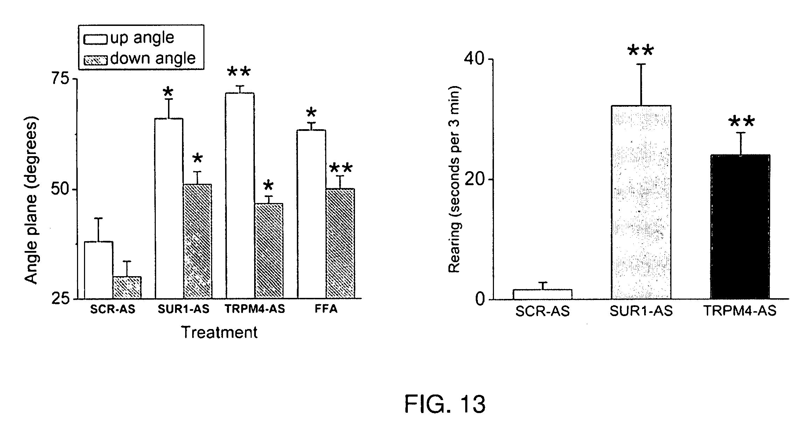

FIG. 13 demonstrates improvements in neurobehavioral function by inhibition of SUR1 and TRPM4. Performance on up-angled and down-angled plane (left) and rearing behavior (right) are improved post-SCI in animals treated with antisense oligodeoxynucleotide directed against SUR1 or against TRPM4. Also, performance on up-angled and down-angled plane is improved post-SCI in animals treated with flufenamic acid (left).

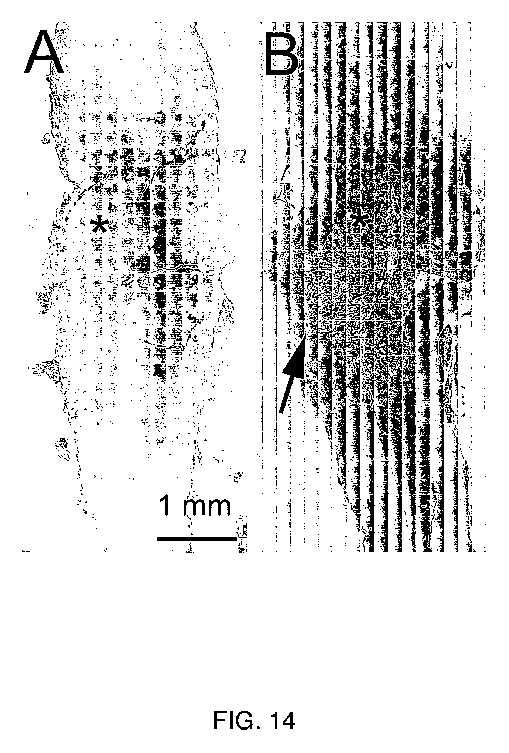

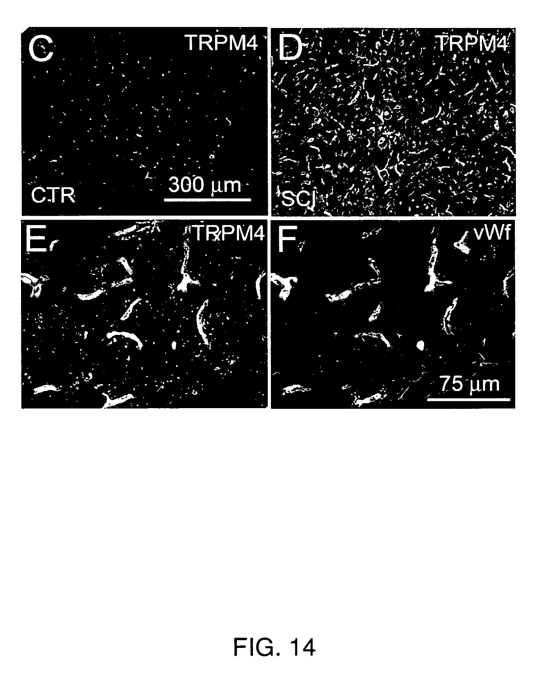

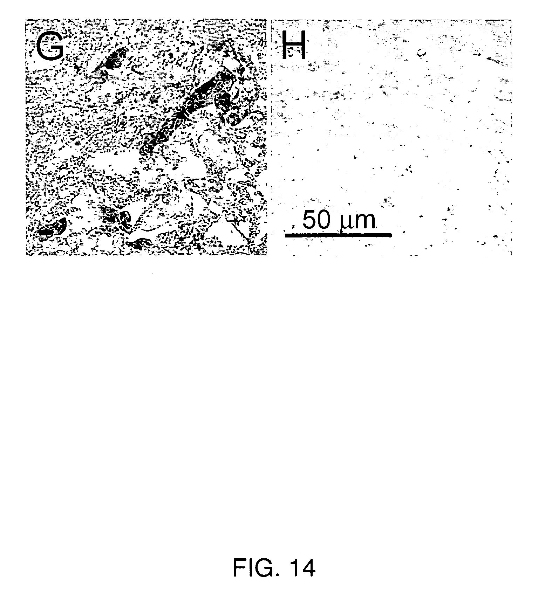

FIG. 14 demonstrates that TRPM4 is up-regulated in capillaries in SCI. A,B: Immunohistochemical localization of TRPM4 in control and 24 h post-SCI, with montages constructed from multiple individual images, and positive labeling shown in black pseudocolor; arrow points to impact site; red asterisks show sampling areas for panels C-F. C-E: Magnified views of TRPM4 immunolabeled sections taken from control (C) and from the "penumbra" (D,E). F: Immunolabeling of capillaries with von Willebrand factor; same field as E. G,H: *In situ* hybridization for TRPM4 in the penumbra 24 h post-SCI using antisense (AS) (G) and sense (SE) (H) probes. Images of immunohistochemistry and *in situ* hybridization are representative of findings in 3 rats/group.

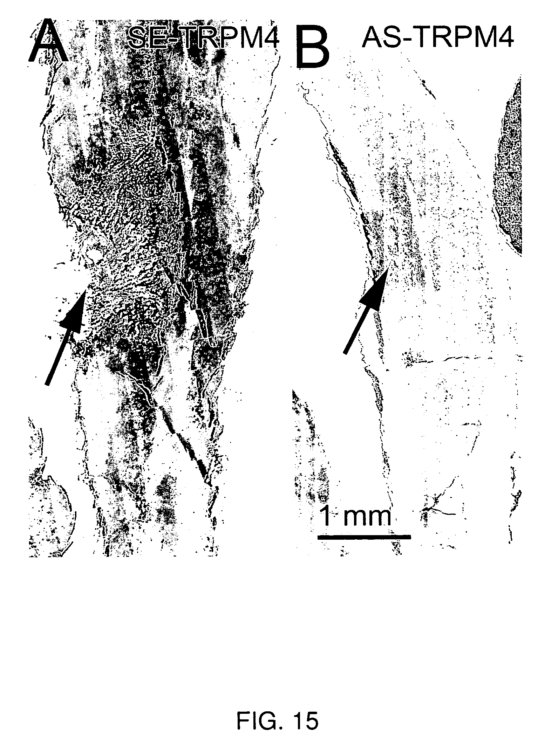

FIG. 15 shows that TRPM4 up-regulation post-SCI is prevented by gene suppression using AS-ODN. A,B: Montages showing immunohisto-chemical localization of TRPM4 24 h post-SCI in a rat treated with sense (SE) ODN (A) or with antisense (AS) ODN (B); i.v. infusions of ODN were started 48 h before SCI; arrows point to impact sites.

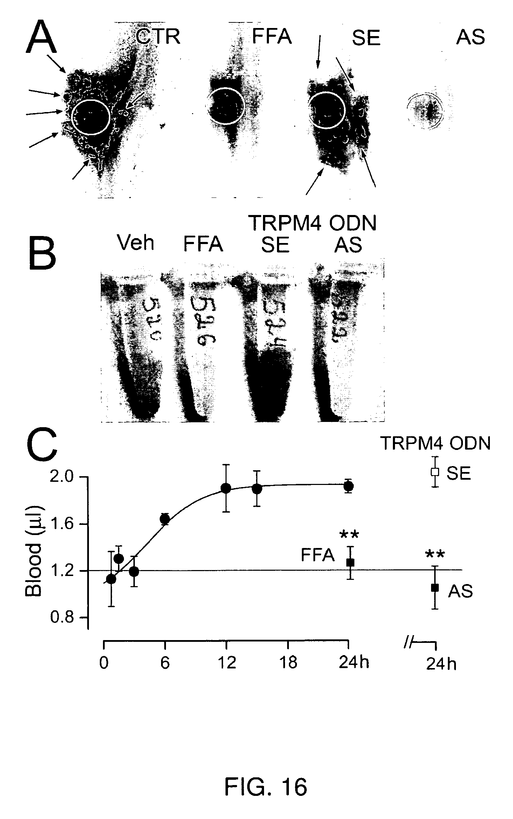

FIG. 16 shows that progressive hemorrhagic necrosis is prevented by TRPM4 blockers. A,B: Cord sections (A) and cord homogenates (B) from control rats (CTR or vehicle-treated), and rats treated with flufenamic acid (FFA), sense (SE) ODN, antisense (AS) ODN; arrows point to distant petechial hemorrhages. C: Quantification of extravasated blood in cord homogenates in controls (•), in rats treated post-SCI with FFA (n=3), or post-SCI with SE-ODN (n=4) or AS-ODN (n=5).

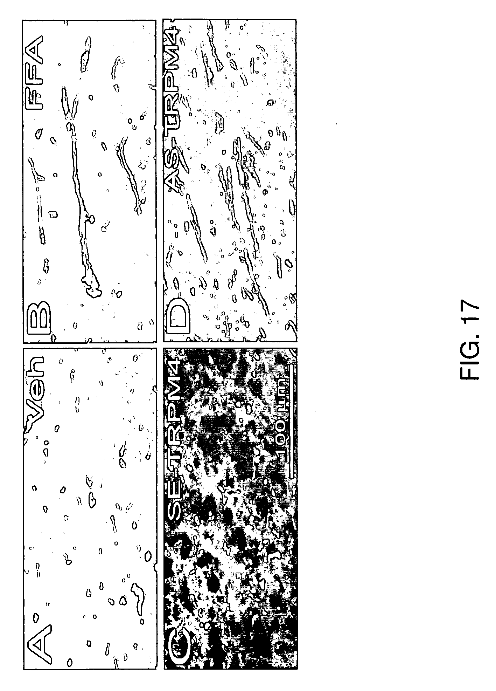

FIG. 17 demonstrates that capillary fragmentation is prevented by TRPM4 blockers. A-D: Sections immunolabeled for vimentin to show capillaries near the impact site in rats treated with vehicle (A), flufenamic acid (FFA) (B), sense (SE) ODN (C) or antisense (AS) ODN (D); note fragmented capillaries in A & C vs. elongated capillaries in B & D.

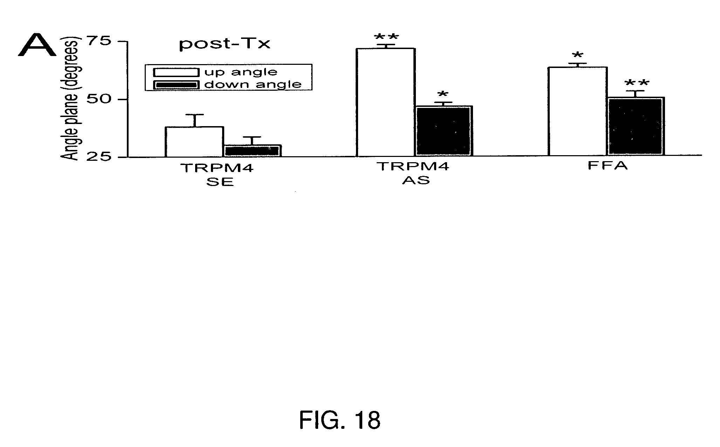

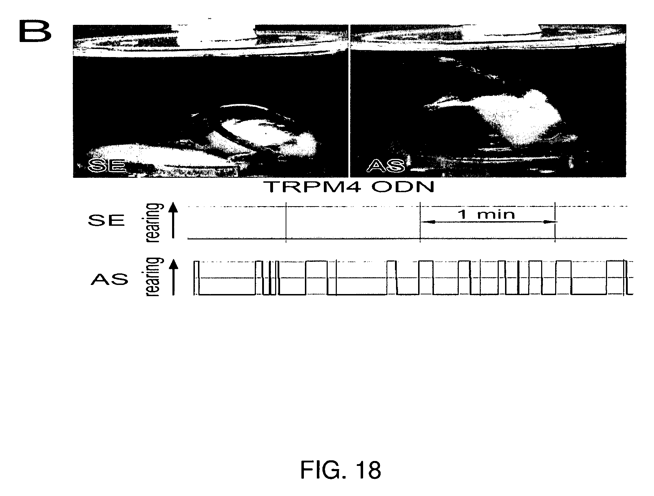

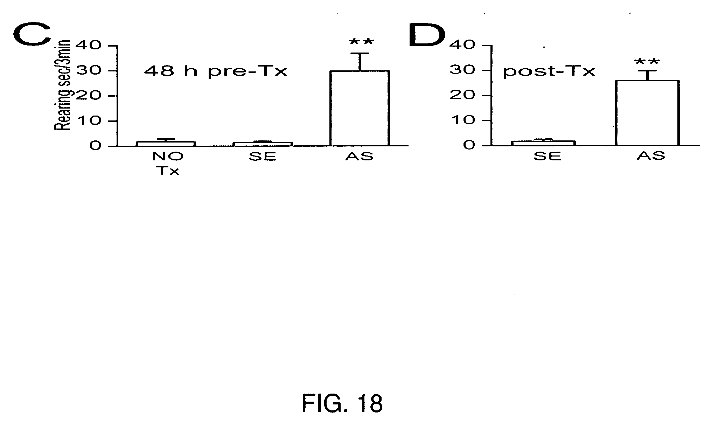

FIG. 18 shows that flufenamic acid (FFA) and TRPM4 AS-ODN improve neurobehavioral function post SCI. A: Performance on inclined plane 24 h post-SCI in rats treated after SCI with TRPM4 SE-ODN, AS-ODN and FFA. B-D: Rearing behavior 24 h post-SCI in rats either pre-treated for 48 h (C) or treated post-SCI (D) with TRPM4 SE-ODN versus AS-ODN.

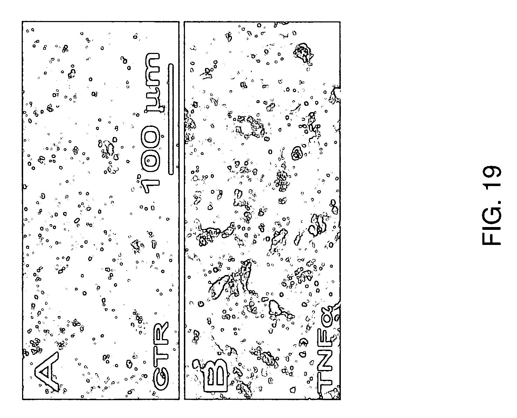

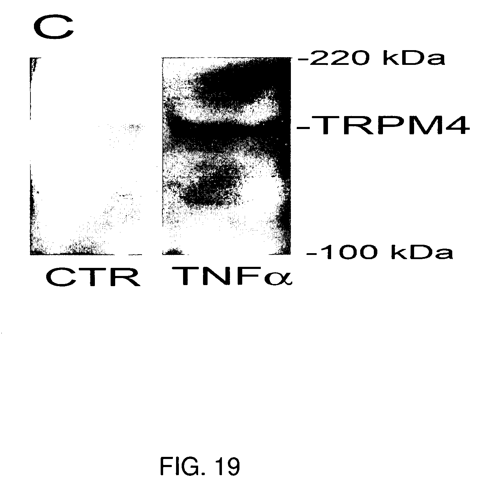

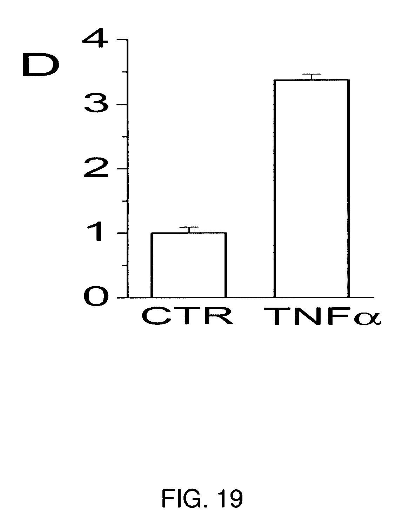

FIG. 19 shows that TNFα causes up-regulation of TRPM4 protein in bEnd.3 cells. A,B: Immunolabeling for TRPM4 in bEnd.3 cells under control conditions (A) and after 6-h exposure to 20 ng/mL TNFα (B). C,D: Immunoblots (C) and densitometric analysis of immunoblots (D) for TRPM4 in lysates form bEnd.3 cells under control conditions and after 6-h exposure to 20 ng/mL TNFα, as indicated; n=3; P<0.01.

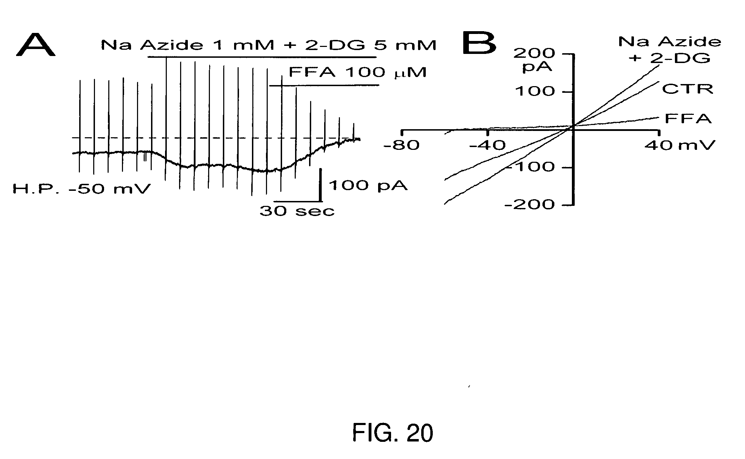

FIG. 20 demonstrates that TNFα causes up-regulation of NC_{Ca-ATP} (TRPM4) current in bEnd.3 cells. A,B: Macroscopic currents (nystatin whole cell) in bEnd.3 cells after 6-h exposure to 20 ng/mL TNFα (A,B), but not in control cells (not shown), were activated by depleting ATP with Na azide plus 2-DG, reversed near 0 mV and were blocked by flufenamic acid (FFA).

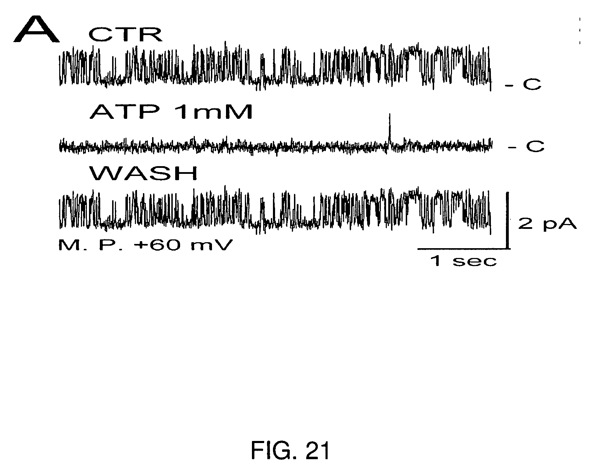

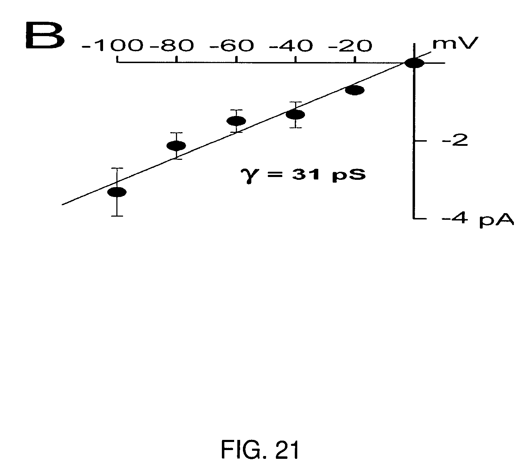

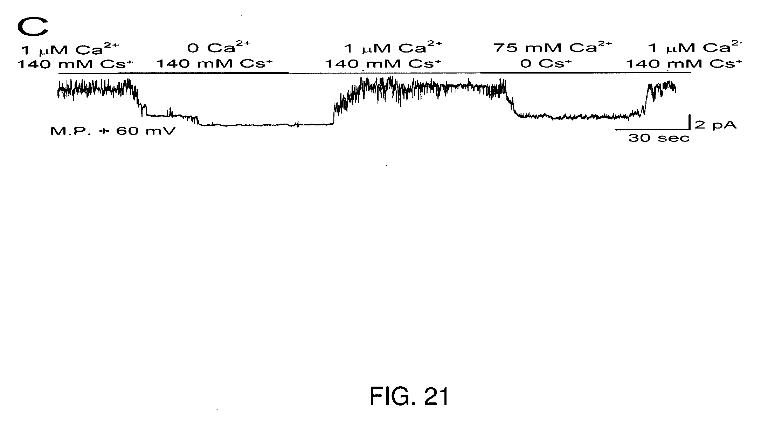

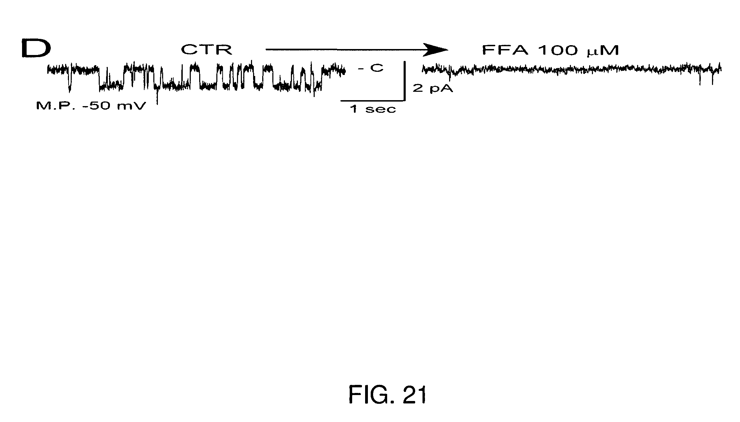

FIG. 21 demonstrates the biophysical properties of the NC_{Ca-ATP} channel in bEnd.3 cells after 6-h exposure to 20 ng/mL TNFα are identical to TRPM4. A: recording of inside-out patch showing 31 pS channel studied with Cs⁺ as the only permeant cation; the channel was reversibly blocked by ATP. B: Plot of single channel conductance. C: Outward cationic single channel currents at the membrane potential of +60 mV, in an inside-out patch with multiple channels, recorded in the presence on the cytoplasmic side of 0 CaCl₂/140 mM CsCl, 1 µM CaCl₂/140 mM CsCl and 75 mM CaCl₂/0 CsCl as indicated, showing that: (i) Cs⁺ is permeable; (ii) physiological levels of Ca²⁺ are required for channel activity; (iii) Ca²⁺ is not permeable; (iv) Cl- is not permeable. D: Single channel activity recorded in the absence of ATP, blocked by the TRPM4 blocker, flufenamic acid (FFA).

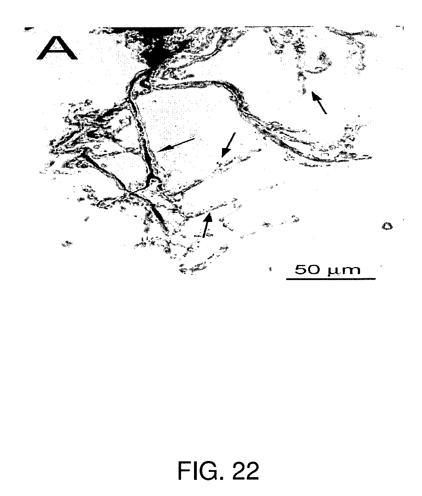

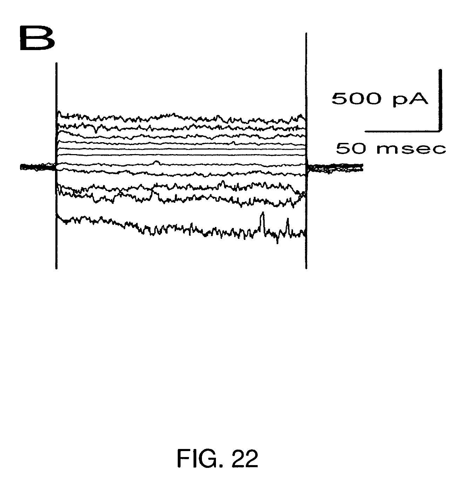

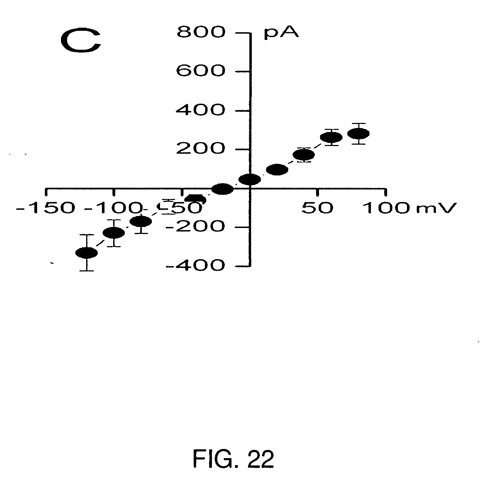

FIG. 22 shows the following: A: Phase-contrast micrograph showing magnetic particles (black clumps) inside of spinal cord precapillary arterioles, along with attached capillaries. B,C: Whole-cell currents (n=4) during step pulses (-140 to +80 mV, 20 mV intervals) in capillary endothelial cells still attached to freshly isolated spinal cord microvascular complexes, as in A. Standard physiological solutions inside and out, with no ATP in the pipette.

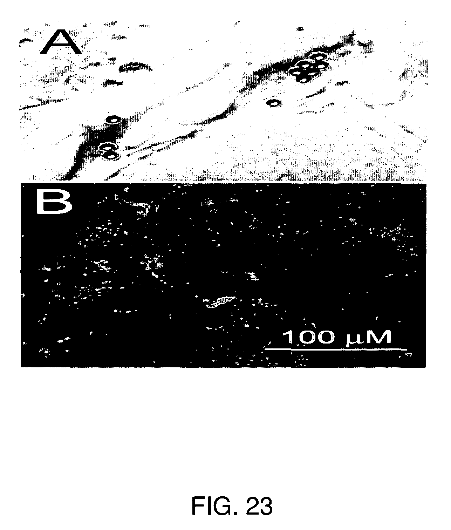

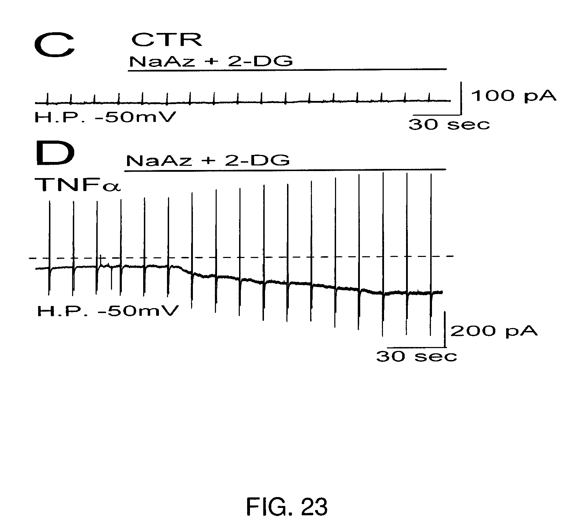

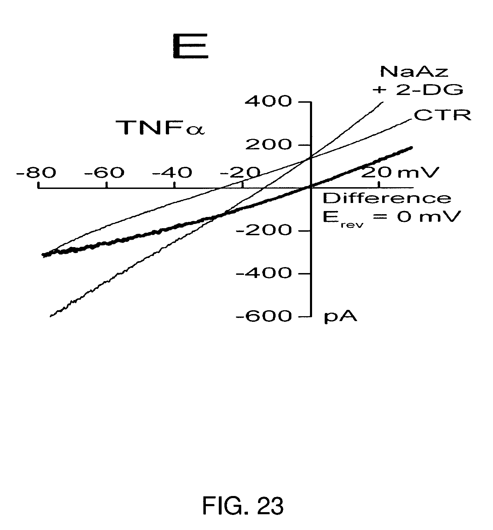

FIG. 23 demonstrates primary cultured murine spinal cord capillary endothelial (scEnd) cells express NC_{Ca-ATP} channel currents when exposed to 20 ng/mL TNFα for 6 h. A,B: Phase contrast and immunofluorescence images of primary cultured scEnd cells labeled with CD31(+) beads (A) or von Willebrand factor (B, green). C-E: In scEnd cells exposed to 20 ng/mL TNFα for 6-h (D), but not in control cells (C), current that reversed near 0 mV (the difference current) was activated by depleting ATP with Na azide plus 2-DG (E), consistent with NC_{Ca-ATP} (TRPM4).

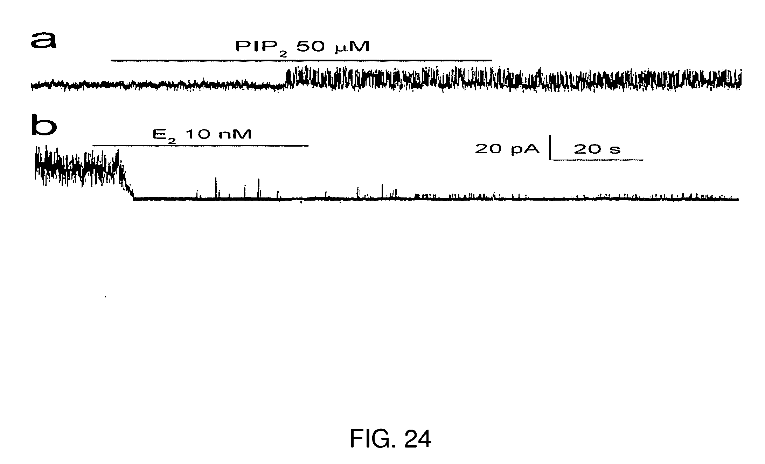

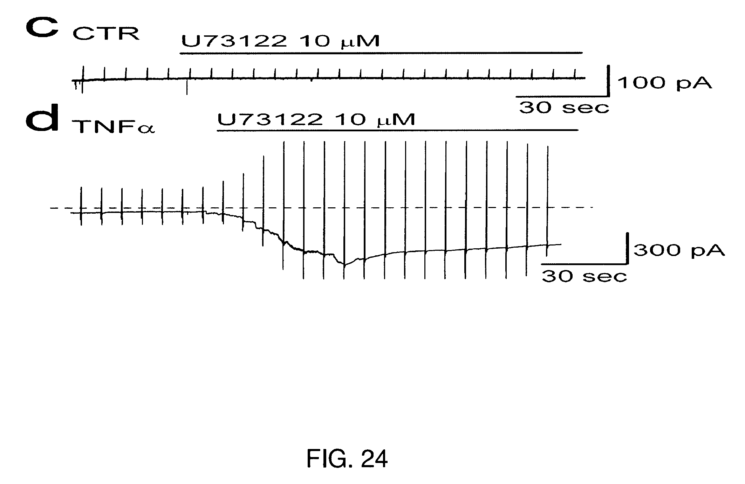

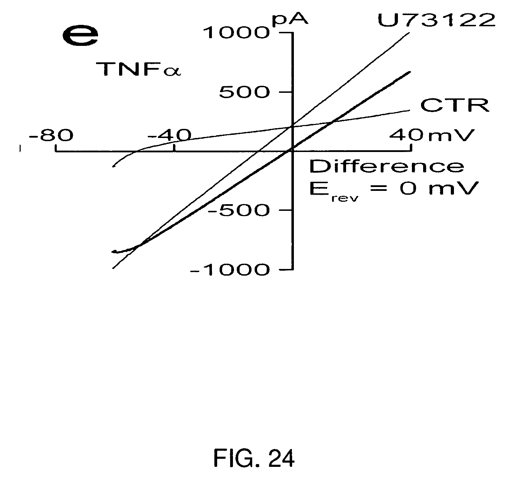

FIG. 24 provides modulation of NC_{Ca-ATP} (TRPM4) channel activity by PIP₂. a: Inside-out patch from astrocyte with channel activity blocked by 10 mM ATP in the bath, with release of inhibition by application of PIP₂. b: Inside-out patch from astrocyte with strong channel activity due to low concentration of ATP in the bath (100 nM), showing channel inhibition due to presumed PIP₂ depletion caused by activating phospholipase C with estrogen (E2). c-e: whole cell recordings of control (c) and TNFα-treated (d,e) bEnd.3 cells showing, in TNFα-treated cells only, channel activation due to presumed PIP₂ augmentation caused by inhibiting phospholipase C with U73122.

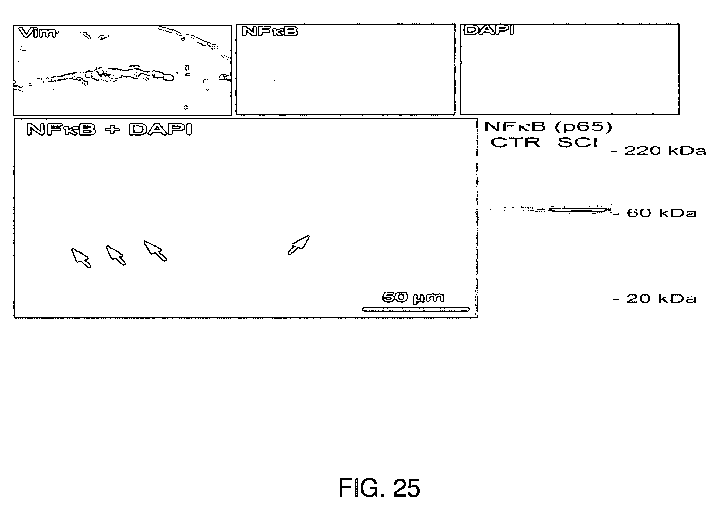

FIG. 25 demonstrates NFκB nuclear localization post-SCI. Co-labeled sections from penumbra 45 min post-SCI immunolabeled for vimentin (Vim) and NFκB and stained with DAPI. Pink nuclei in the composite image indicate NFκB nuclear localization in prevenular capillaries (arrows). A nuclear Western for p65 is also shown.

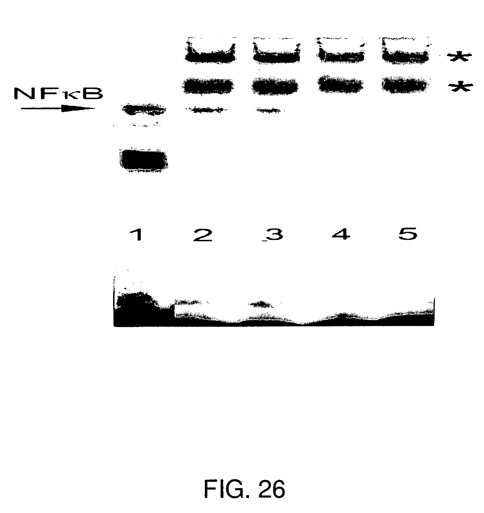

FIG. 26 shows NFκB binds to rat TRPM4 promoter. Lane 1: (positive control) EMSA of the TNFα-stimulated HeLa nuclear extract with biotinylated oligonucleotide (ON) encompassing NFκB binding site from HIV-1 promoter. Lanes 3,4: EMSA of 6 h post-injury spinal cord nuclear extracts from two rats with biotinylated ON encompassing NFκB binding consensus sequence of the rat TRPM4 promoter. Lanes 5,6: same as Lanes 3,4 plus 200-fold excess of non-biotinylated competitor. Arrow indicates NFκB/ON complex; asterisks: non specific signal.

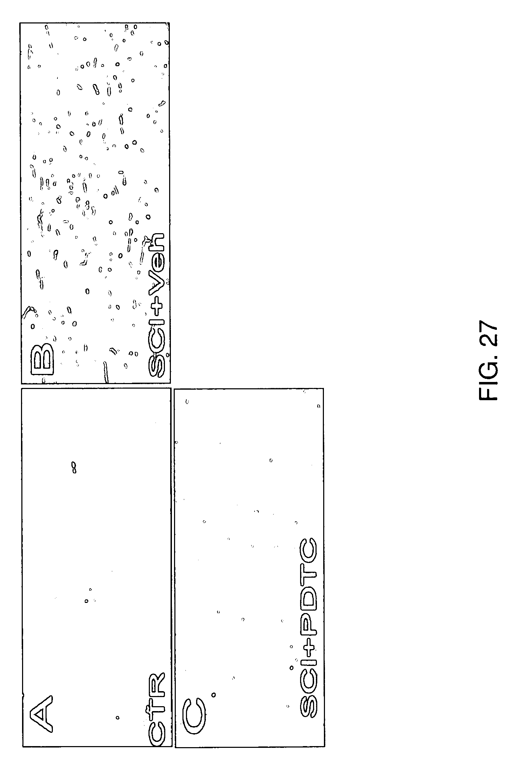

FIG. 27 shows that inhibiting NFκB reduces TRPM4 expression post-SCI. A-C: Cord sections immunolabeled for TRPM4 from a control rat (A), and from rats 24 h post-SCI that were treated with vehicle (B) or with the NFκB inhibitor, pyrrolidine dithiocarbamate (PDTC, 100 mg/kg, ip) (C).

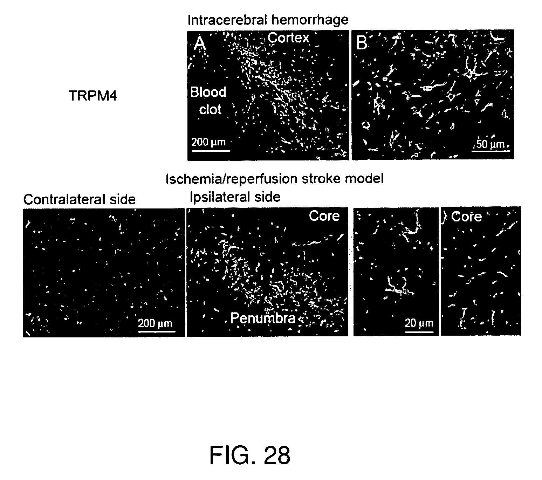

FIG. 28 demonstrates that TRPM4 is upregulated in cells and capillaries of penumbral tissues in rat models of hemorrhagic stroke (upper panels) and in ischemic stroke (lower panels).

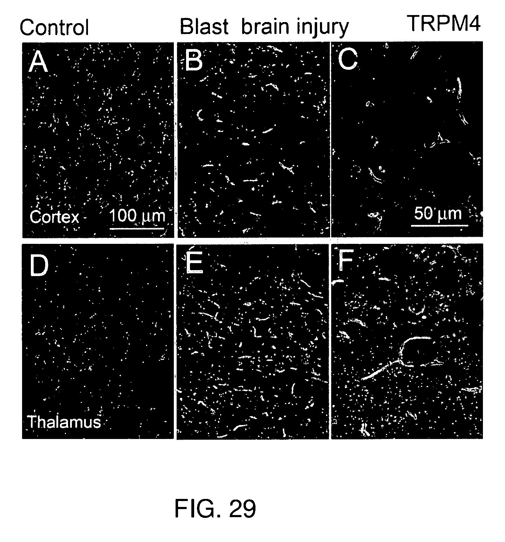

FIG. 29 shows that TRPM4 is upregulated in cortex and thalamus in a rat model of traumatic brain injury induced by gunshot blast.

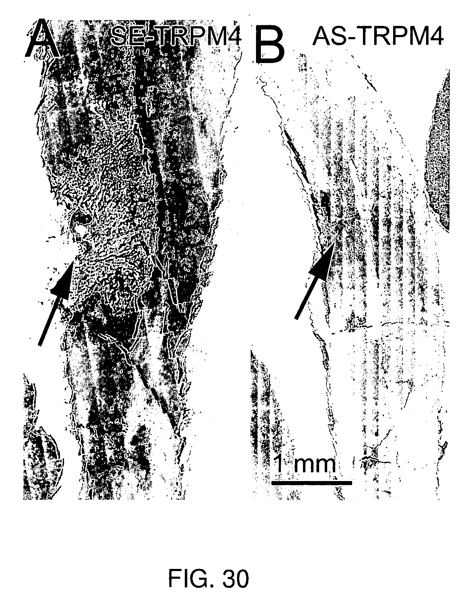

FIGS. 30A-30B show that TRPM4 up-regulation post-SCI is prevented by gene suppression using AS-ODN. FIGS. 30A,30B: Montages showing immunohistochemical localization of TRPM4 24 h post-SCI in a rat treated with sense (SE) ODN (30A) or with antisense (AS) ODN (30B); i.v. infusions of ODN were started 48 h before SCI; arrows point to impact sites.

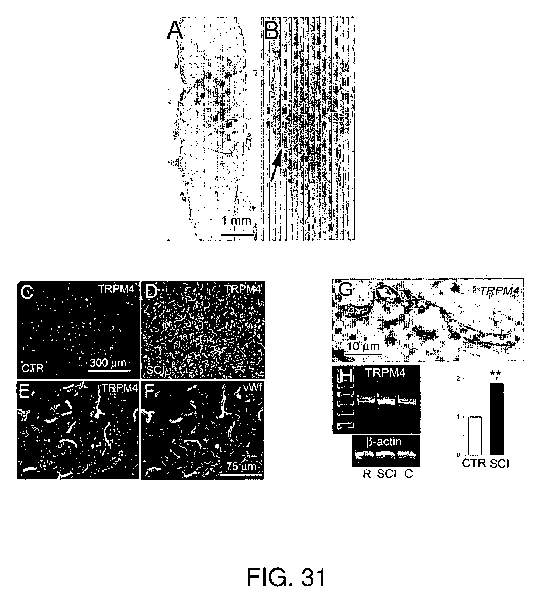

FIGS. 31A-31H show that TRPM4 is up-regulated in capillaries in SCI. 31A,31B: Immunohistochemical localization of TRPM4 in control and 24 h post-SCI, with montages constructed from multiple individual images, and positive labeling shown in black pseudocolor; arrow points to impact site; red asterisks show sampling areas for panels 31C-31F. 31C-31E: Magnified views of TRPM4 immunolabeled sections taken from control (C) and from the "penumbra" (31D,31E). 31F: Immunolabeling of capillaries with vonWillebrand factor; same field as 31E. 31G: In situ hybridization for TRPM4 in the penumbra 24 h post-SCI. 31H: PCR of spinal cord tissue from control (CTR) and post-SCI from 3 regions, the impact site (SCI) and rostral (R), caudal (C). Images of immunohistochemistry and in situ hybridization are representative of findings in 3 rats/group.

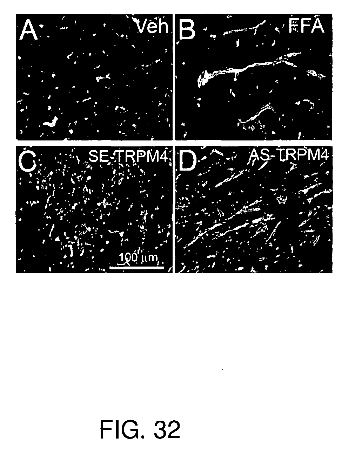

FIGS. 32A-32D demonstrate that capillary fragmentation is prevented by TRPM4 blockers. A-D: Sections immunolabeled for vimentin to show capillaries near the impact site in rats treated with vehicle (32A), flufenamic acid (FFA) (32B), sense (SE) ODN (32C) or antisense (AS) ODN (32D); note fragmented capillaries in 32A and 32C *vs.* elongated capillaries in 32B and 32D.

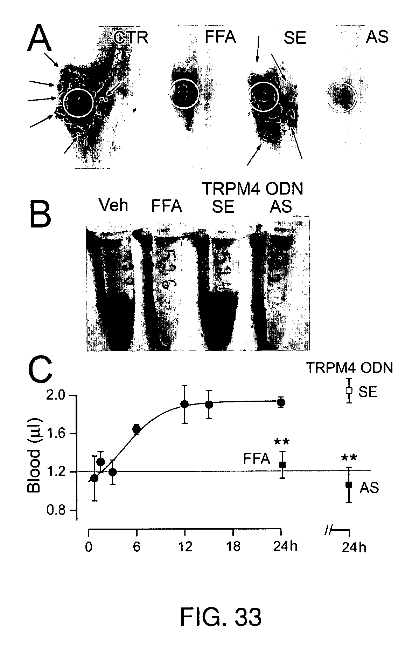

FIGS. 33A-33C illustrate that progressive hemorrhage is prevented by TRPM4 blockers. 33A,33B: Cord sections (33A) and cord homogenates (33B) from control rats (CTR or vehicle-treated), and rats treated with flufenamic acid (FFA), sense (SE) ODN, antisense (AS) ODN; arrows point to distant petechial hemorrhages. 33C: Quantification of extravasated blood in cord homogenates in controls (•), in rats treated post-SCI with FFA (n=3), or post-SCI with SE-ODN (n=4) or AS-ODN (n=5).

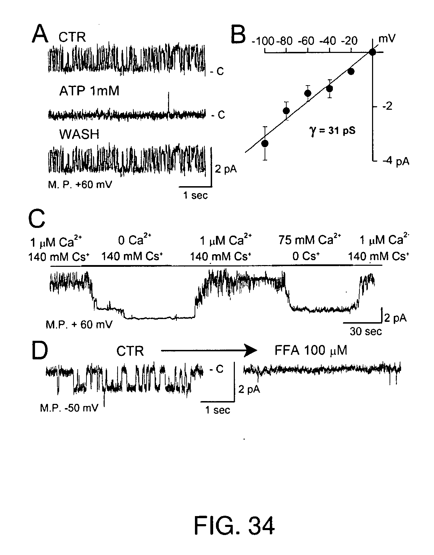

FIGS 34A-34D show that the biophysical properties of the NC_{Ca-ATP} channel in bEnd.3 cells after exposure to TNFα are identical to TRPM4. 34A: recording of inside-out patch showing 31 pS channel studied with Cs⁺ as the only permeant cation; the channel was reversibly blocked by ATP. 34B: Plot of single channel conductance. 34C: Outward cationic single channel currents at the membrane potential of +60 mV, in an inside-out patch with multiple channels, recorded in the presence on the cytoplasmic side of 0 CaCl₂/140 mM CsCl, 1 µM CaCl₂/140 mM CsCl and 75 mM CaCl₂/0 CsCl as indicated, showing that: (i) Cs⁺ is permeable; (ii) physiological levels of Ca²⁺ are required for channel activity; (iii) Ca²⁺ is not permeable; (iv) Cl-is not permeable. 34D: Single channel activity recorded in the absence of ATP, blocked by the TRPM4 blocker, flufenamic acid (FFA).

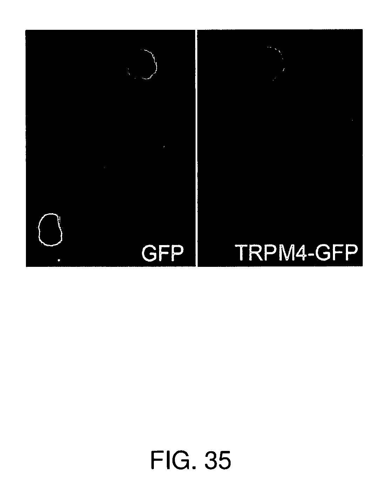

FIG. 35 demonstrates that TRPM4 expression and opening predisposes to oncotic/necrotic cell death. Plasmids of mTRPM4-GFP and enhanced green fluorescence protein (EGFP; Clontech) were transfected into COS7 cells. ATP was depleted using Na azide plus 2-DG, then cells were assayed with propidium iodide as a marker of oncotic/necrotic death. ATP depletion resulted in oncotic/necrotic death of 80% of cells transfected with TRPM4 *νs*. 2% of cells transfected with control EGFP.

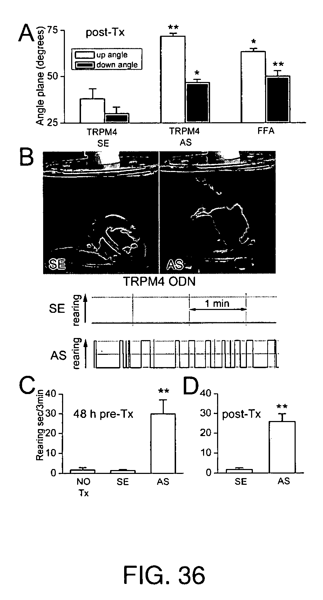

FIGS. 36A-36D provide that flufenamic acid (FFA) and TRPM4 AS-ODN improve neurobehavioral function post SCI. 36A: Performance on inclined plane 24 h post-SCI in rats treated after SCI with TRPM4 SE-ODN, AS-ODN and FFA. 36B-36D: Rearing behavior 24 h post-SCI in rats either pre-treated for 48 h (36C) or treated post-SCI (36D) with TRPM4 SE-ODN versus AS-ODN.

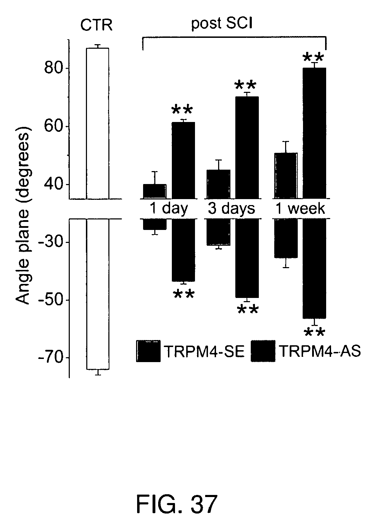

FIG. 37 demonstrates that an exemplary TRPM4-AS improves neuro-behavioral performance in rats post-SCI. Performance on up-angled and down-angled plane at various times post-SCI in rats administered TRPM4 antisense (AS) or TRPM4 sense (SE); n=7-8 per group; **, P<0.01.

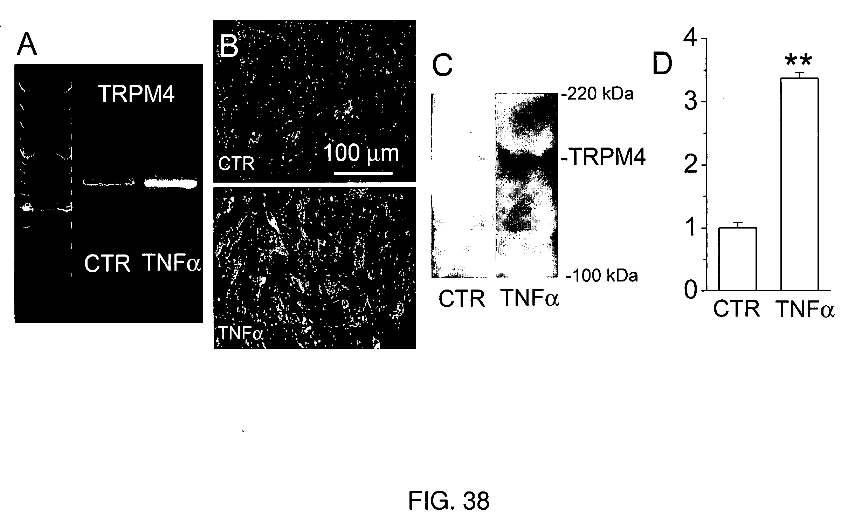

FIGS. 38A-38D demonstrate that TNFα causes up-regulation of TRPM4 mRNA and protein in bEnd.3 cells. 38A-38D: PCR (38A), immunolabeling (38B), and Western blots (38C,38D) for TRPM4 in bEnd.3 cells under control conditions and after 6-h exposure to 20 ng/mL TNFα; n=3; **, P<0.01.

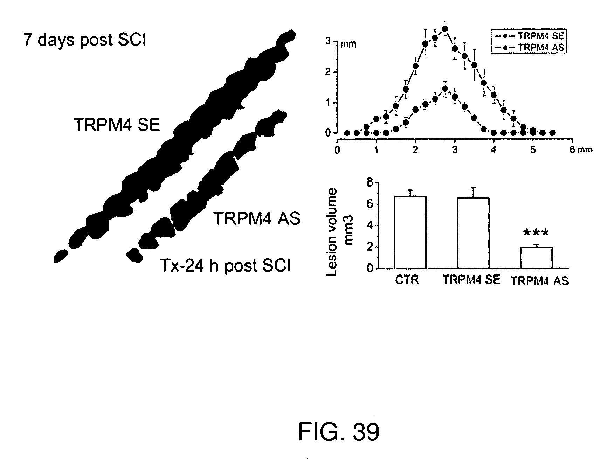

FIG. 39 illustrates that exemplary TRPM4-AS reduces lesion volume in rats post-SCI. Outlines of lesions in rats administered TRPM4 antisense (AS) or TRPM4 sense (SE) are shown at left; lesion areas (above) and lesion volumes (below) are shown at right; n=7-8 per group; **, P<0.001.

### DETAILED DESCRIPTION OF THE INVENTION

This Application makes reference to at least U.S. Application Serial No. 10/391,561, filed on March 20, 2003; U.S. Application Serial No. 11/099,332, filed April 5, 2005; U.S. Application Serial No. 11/229,236, filed September 16, 2005; and U.S. Application Serial No. 11/359,946, filed February 22, 2006; U.S. Patent Application Serial No. 11/574,793, filed July 25, 2005; U.S. Patent Application Serial No. 60/880,119, filed January 12, 2007; U.S. Patent Application Serial No. 60/889,065, filed February 9, 2007; U.S. Patent Application Serial No. 60/945,811, filed June 22, 2007; U.S. Patent Application Serial No. 60/945,825, filed June 22, 2007; and PCT/US2008/050922, filed January 11, 2008.

### I. Definitions

The use of the word "a" or "an" when used in conjunction with the term "comprising" in the claims and/or the specification may mean "one," but it is also consistent with the meaning of "one or more," "at least one," and "one or more than one." Some embodiments of the invention may consist of or consist essentially of one or more elements, method steps, and/or methods of the invention. It is contemplated that any method or composition described herein can be implemented with respect to any other method or composition described herein.

Some of the preferred embodiments of the present invention will be described in detail with reference to the attached drawings. This invention may be embodied in many different forms and should not be construed as being limited to the embodiments set forth herein.

The use of the term "or" in the claims is used to mean "and/or" unless explicitly indicated to refer to alternatives only or the alternatives are mutually exclusive, although the disclosure supports a definition that refers to only alternatives and "and/or."

As used herein, the term "acute" refers to the onset of a health effect, usually the effect is a rapid onset that is considered brief, not prolonged.

As used herein, the term "acute cerebral ischemia" refers to a cerebral ischemic event that has a rapid onset and is not prolonged. The terms "acute cerebral ischemia" and "stroke" can be used interchangeably.

As used herein, the term "antagonist" refers to a biological or chemical agent that acts within the body to reduce the physiological activity of another native chemical or biological substance. In the present invention, the antagonist blocks, inhibits, reduces and/or decreases the activity of a NC_{Ca-ATP} channel of a neural cell, such as a neuronal cell, a neuroglia cell or a neural endothelial cell *(e.g.,* capillary endothelial cells). In the present invention, the antagonist combines, binds, associates with a NC_{Ca-ATP} channel of a neural cell, such as a neuronal cell, a neuroglia cell or a neural endothelial cell *(e.g.,* capillary endothelial cells), such that the NC_{Ca-ATP} channel is closed (deactivated), meaning reduced biological activity with respect to the biological activity in the diseased state. In certain embodiments, the antagonist combines, binds and/or associates with a regulatory subunit of the NC_{Ca-ATP} channel, particularly a SUR1, whereas in other embodiments the antagonist combines, binds and/or associates with a pore-forming unit of the channel, such as TRPM4, for example. Alternatively, the antagonist combines, binds, and/or associates with a pore-forming subunit of the NC_{Ca-ATP} channel, such that the NC_{Ca-ATP} channel is closed (deactivated). The terms antagonist or inhibitor can be used interchangeably. An antagonist of the NC_{Ca-ATP} channel is a compound *(e.g.,* a small molecule, protein, or nucleic acid) that reduces the activity of *(e.g.,* reduces current flow through) the NC_{Ca-ATP} channel. Examples of NC_{Ca-ATP} channel antagonists include SUR1 antagonists, TRPM4 antagonists, cation channel blockers, *etc.*

As used herein, the terms "brain abscess" or "cerebral abscess" refer to a circumscribed collection of purulent exudate that is typically associated with swelling.

As used herein, the terms "blood brain barrier" or "BBB" refer the barrier between brain blood vessels and brain tissues whose effect is to restrict what may pass from the blood into the brain.

As used herein, the term "cerebral ischemia" refers to a lack of adequate blood flow to an area, for example a lack of adequate blood flow to the brain or spinal cord, which may be the result of a blood clot, blood vessel constriction, a hemorrhage or tissue compression from an expanding mass.

As used herein, the term "a compound that inhibits the NC_{Ca-ATP} channel" refers to a compound, including without limitation small organic compounds, peptides, nucleic acids, or other compounds, that is effective inhibit the NC_{Ca-ATP} channel as defined herein, including a channel that includes SUR1 receptor and TRPM4.

As used herein, the term "depolarization" refers to a diminution in the membrane potential (becoming less negative); depolarization is caused by an increase in cation permeability (not merely sodium) with these channels

As used herein, the terms "effective amount" or "therapeutically effective amount" are interchangeable and refer to an amount that results in an improvement or remediation of at least one symptom of the disease or condition. Those of skill in the art understand that the effective amount may improve the patient's or subject's condition, but may not be a complete cure of the disease and/or condition.

As used herein, the term "endothelium" refers to a layer of cells that line the inside surfaces of body cavities, blood vessels, and lymph vessels or that form capillaries.

As used herein, the term "endothelial cell" refers to a cell of the endothelium or a cell that lines the surfaces of body cavities, for example, blood or lymph vessels or capillaries. In certain embodiments, the term endothelial cell refers to a neural endothelial cell or an endothelial cell that is part of the nervous system, for example the central nervous system or the brain or spinal cord.

As used herein, the term "gliotic capsule" refers to a physical barrier surrounding, in whole or in part, a foreign body, including a metastatic tumor, a cerebral abscess or other mass not normally found in brain except under pathological conditions. In certain embodiments, the gliotic capsule comprises an inner zone comprising neuronal cells, neuroglial cells (e.g., astrocytes) and/or endothelial cells expressing a NC_{Ca-ATP} channel.

As used herein, the terms "inhibit" and "antagonize" refer to the ability of the compound to block, partially block, interfere, decrease, reduce or deactivate an already existing channel such as the NC_{Ca-ATP} channel (including SUR1 receptor and TRPM4), to increase the closed time and/or closing rate of the NC_{Ca-ATP} channel, decrease the open time, and/or prevent or reduce expression or assembly of subunits of a channel such as the NC_{Ca-ATP} channel that would be expressed or assembled under a given circumstance were it not for the inhibitor. Thus, one of skill in the art understands that the term "inhibit" encompasses a complete and/or partial loss of activity of a channel, such as the NC_{Ca-ATP} channel. Channel activity may be inhibited by channel block (occlusion or closure of the pore region, preventing ionic current flow through the channel), by allosteric inhibition *via* interaction with a regulatory subunit, changing an opening rate or mean open time, or a closing rate or mean closed time, by interfering with expression of channel subunits, by interfering with assembly of channel subunits, by interfering with trafficking of channels or their subunits to the plasmalemmal membrane and formation of functional chanels, or by other means. For example, a complete and/or partial loss of activity of the NC_{Ca-ATP} channel as may be indicated by a reduction in cell depolarization, reduction in sodium ion influx or any other monovalent ion influx, reduction in an influx of water, reduction in extravasation of blood, reduction in cell death, as well as an improvement in cellular survival following an ischemic challenge.

As used herein, the term "inhibits the NC_{Ca-ATP} channel" refers to a reduction in, cessation of, or blocking of, the activity of the NC_{Ca-ATP} channel, including inhibition of current flow through the channel, inhibition of opening of the channel, increasing the closed time and/or closing rate, inhibition of activation of the channel, inhibition or reduction of the expression of the channel, including inhibition or reduction of genetic message encoding the channel and inhibition or reduction of the production channel proteins, inhibition or reduction of insertion of the channel into the plasma membrane of a cell, or other forms of reducing the physiologic activity of the NC_{Ca-ATP} channel.

As used herein, the term "ionic edema" in brain or nervous tissue refers to edema arising in tissue in which the blood-brain barrier remains substantially intact, and is associated with the movement of electrolytes (e.g. Na⁺, Cl⁻) plus water into brain parenchyma.

The term matrix metalloproteinase (MMP) as used herein refers to a zinc-dependent endopeptidase, and in specific aspects of the invention it refers to particular MMPs, including MMP-2 and/or MMP-9, for example. An antagonist of MMP is one or more molecules that inhibits the activity and/or expression of MMP.

The term "morbidity" as used herein is the state of being diseased. Yet further, morbidity can also refer to the disease rate or the ratio of sick subjects or cases of disease in to a given population.

The term "mortality" as used herein is the state of being mortal or causing death. Yet further, mortality can also refer to the death rate or the ratio of number of deaths to a given population.

As used herein, the term "neuron" refers to a nerve cell, also termed a neuronal cell.

As used herein, the term "neuronal cell" refers to a cell that is a morphologic and functional unit of the nervous system. The cell comprises a nerve cell body, the dendrites, and the axon. The terms neuron, nerve cell, neuronal, neurone, and neurocyte can be used interchangeably. Neuronal cell types can include, but are not limited to a typical nerve cell body showing internal structure, a horizontal cell (of Cajal) from cerebral cortex; Martinottic cell, biopolar cell, unipolar cell, Pukinje cell, and a pyramidal cell of motor area of cerebral cortex.

As used herein, the term "neural" refers to anything associated with the nervous system. As used herein, the term "neural cells" includes neurons and glia, including astrocytes. As used herein, the term "isolated neural cells" means neural cells isolated from brain or spinal cord.

As used herein, the terms "neuroglia" or "neuroglial cell" refers to a cell that is a non-neuronal cellular element of the nervous system. The terms neuroglia, neurogliacyte, and neuroglial cell can be used interchangeably. Neuroglial cells can include, but are not limited to ependymal cells, astrocytes, oligodendrocytes, or microglia.

The term "preventing" as used herein refers to minimizing, reducing or suppressing the risk of developing a disease state or parameters relating to the disease state or progression or other abnormal or deleterious conditions.

The term "reactive astrocytes" means astrocytes found in brain or spinal cord at the site of a lesion or ischemia. The term "native reactive astrocytes" or "NRAs" means reactive astrocytes that are freshly isolated from brain or spinal cord. The term "freshly isolated" as used herein refers to NRAs that have been purified from brain or spinal cord, particularly NRAs that were purified from about 0 to about 72 hours previously. When NRAs are referred to as being "purified from brain" the word "purified" means that the NRAs are isolated from other brain tissue and/or implanted gelatin or sponge and does not refer to a process that simply harvests a population of cells from brain without further isolation of the cells. As described herein, the NC_{Ca-ATP} channel found in reactive astrocytes is present only in freshly isolated cells; the NC_{Ca-ATP} channel is lost shortly after culturing the cells under typical normoxic (but not hypoxic) conditions. NRAs provide an *in vitro* model that is more similar to reactive astrocytes as they exist *in vivo* in the brain, than astrocytes grown in culture, unless special culture conditions are used. The terms "native" and "freshly isolated" are used synonymously.

As used herein, the term "reduces" refers to a decrease in cell death, inflammatory response, hemorrhagic conversion, extravasation of blood, *etc.* as compared to no treatment with the compound of the present invention. Thus, one of skill in the art is able to determine the scope of the reduction of any of the symptoms and/or conditions associated with a spinal cord injury in which the subject has received the treatment of the present invention compared to no treatment and/or what would otherwise have occurred without intervention.

As used herein, the term "stroke" refers to any acute, clinical event related to the impairment of cerebral circulation. The terms "acute cerebral ischemia" and "stroke" can be used interchangeably.

As used herein, the term "synergistically" refers to the combined actions of two or more agents, where the effects of two or more agents when acting in combination is greater than the effect of either agent when applied individually, or where the combined action of two or more agents has an additional effect or effects, in addition to those effects caused by the agents when applied individually.

The term "TRPM" as used herein refers to "transient receptor potential ion channels, melastatin" and, in particular concerns TRPM4. An antagonist of TRPM4 is one or more molecules that inhibit the activity and/or expression of TRPM4. In a specific embodiment, it is a component of the NC_{Ca-ATP} channel.

The terms "treating" and "treatment" as used herein refer to administering to a subject a therapeutically effective amount of a composition so that the subject has an improvement in the disease or condition. The improvement is any observable or measurable improvement. Thus, one of skill in the art realizes that a treatment may improve the patient's condition, but may not be a complete cure of the disease. Treating may also comprise treating subjects at risk of developing a disease and/or condition.

The term "tumor necrosis factor alpha (TNFα)" as used herein refers to a cytokine involved in a variety of cellular processes, including apoptotic cell death, cellular proliferation, differentiation, inflammation, tumorigenesis, and viral replication. An antagonist of TNFα is one or more molecules that inhibits the activity and/or expression of TNFα.

As used herein, the term "vasogenic edema" in brain or nervous tissue refers to edema arising in tissue in which the blood-brain barrier is not substantially intact, and in which macromolecules plus water enter into brain parenchyma in addition to any movement of electrolytes.

As used herein, the term "vascular endothelial growth factor (VEGF)" refers to a signaling protein, a mitogen, primarily for vascular endothelial cells. It is involved in both vasculogenesis and angiogenesis, yet it has effects on a number of cell types, including neural cells. An antagonist of VEGF is one or more molecules that inhibits the activity and/or expression of VEGF.

### II. General Embodiments of the Invention

The present invention relates to a novel ion channel whose function underlies at least the swelling of mammalian neural cells, for example, such as in response to ATP depletion. Treatment methods are provided that relate to diseases, trauma, and conditions that lead to the expression of such channels, including the use of inhibitors of the channel function to prevent this cell swelling response, which characterizes at least brain damage in cerebral ischemia and traumatic brain injury, for example.

The NC_{Ca-ATP} channel is distinguished by certain functional characteristics, the combination of which distinguishes it from known ion channels. The characteristics that distinguish the NC_{Ca-ATP} channel include, but are not necessarily limited to, the following: 1) it is a non-selective cation channel that readily allows passage of Na, K and other monovalent cations; 2) it is activated by an increase in intracellular calcium, and/or by a decrease in intracellular ATP; and 3) it is regulated by sulfonylurea receptor type 1 (SUR1), which heretofore had been considered to be associated exclusively with K_{ATP} channels such as those found in pancreatic β cells, for example. More specifically, the NC_{Ca-ATP} channel has a single-channel conductance to potassium ion (K⁺) between 20 and 50 pS at physiological K concentrations. The NC_{Ca-ATP} channel is also stimulated by Ca²⁺ on the cytoplasmic side of the cell membrane in a physiological concentration range, where said concentration range is from 10⁻⁸ to 10⁻⁵ M. The NC_{Ca-ATP} channel is also inhibited by cytoplasmic ATP in a physiological concentration range, where said concentration range is from about 10⁻¹ mM to about 5 mM. The NC_{Ca-ATP} channel is also permeable to the following cations; K⁺, Cs⁺, Li⁺, Na⁺; to the extent that the permeability ratio between any two of said cations is greater than 0.5 and less than 2.

In one embodiment, there is a mechanism that gives rise to PHN that involves expression and activation of NC_{Ca-ATP} channels (see Simard *et al.,* 2007). The data demonstrate that cells that express the NC_{Ca-ATP} channel following an ischemic or other injury-stimulus, later undergo oncotic (necrotic) cell death when ATP is depleted. This is shown explicitly for astrocytes (Simard *et al.,* 2006), and in specific embodiments it also occurs with capillary endothelial cells that express the channel. It follows that if capillary endothelial cells undergo this process leading to necrotic death, capillary integrity would be lost, leading to extravasation of blood and formation of petechial hemorrhages.

### III. NC_{Ca-ATP} Channel

A unique non-selective monovalent cationic ATP-sensitive channel (NC_{Ca-ATP} channel) was identified first in native reactive astrocytes (NRAs) and later in neurons and capillary endothelial cells after stroke or traumatic brain or spinal cord injury (see International application WO 03/079987 to Simard et al.*,* and Chen and Simard, 2001. As with the K_{ATP} channel in pancreatic β cells, the NC_{CaATP} channel is considered to be a heteromultimer structure comprised of sulfonylurea receptor type 1 (SUR1) regulatory subunits and pore-forming subunits (Chen *et al.,* 2003). The pore-forming subunits have been characterized biophysically and are molecularly indistinguishable from TRPM4.

The invention is based, in part, on the discovery of a specific channel, the NCca-ATP channel, defined as a channel on astrocytes in U.S. Application Publication No. 20030215889. More specifically, the present invention has further defined that this channel is not only expressed on astrocytes, it is expressed at least on neural cells, neuroglial cells, and/or neural endothelial cells after brain and spinal cord trauma, for example, an hypoxic event, an ischemic event, or other secondary neuronal injuries relating to these events.

The NC_{Ca-ATP} channel is activated by calcium ions (Ca²⁺) and is sensitive to ATP. Thus, this channel is a non-selective cation channel activated by intracellular Ca²⁺ and blocked by intracellular ATP. When opened by depletion of intracellular ATP, this channel is responsible for complete depolarization due to massive Na⁺ influx, which alters the membrane potential and creates an osmotic gradient, resulting in cytotoxic edema and cell death. When the channel is blocked or inhibited, massive Na⁺ does not occur, thereby preventing cytotoxic edema.

Certain functional characteristics distinguish the NC_{Ca-ATP} channel from other known ion channels. These characteristics can include, but are not limited to, at least some of the following: 1) it is a non-selective cation channel that readily allows passage of Na⁺, K⁺ and other monovalent cations; 2) it is activated by an increase in intracellular calcium, and/or by a decrease in intracellular ATP; 3) it is regulated by sulfonylurea receptor type 1 (SUR1), which heretofore had been considered to be associated exclusively with K_{ATP} channels such as those found in pancreatic β cells.

More specifically, the NC_{Ca-ATP} channel has a single-channel conductance to potassium ion (K⁺) between 20 and 50 pS at physiological K concentrations. The NC_{Ca-ATP} channel is also stimulated by Ca²⁺ on the cytoplasmic side of the cell membrane in a physiological concentration range, where concentration range is from about 10⁻⁸ to about 10⁻⁵ M. The NC_{Ca-ATP} channel is also inhibited by cytoplasmic ATP in a physiological concentration range, where the concentration range is from about 10⁻¹ mM to about 5 mM. The NC_{Ca-ATP} channel is also permeable to the following cations; K⁺, Cs⁺, Li⁺, Na⁺; to the extent that the permeability ratio between any two of the cations is greater than about 0.5 and less than about 2.

SUR imparts sensitivity to antidiabetic sulfonylureas such as glibenclamide and tolbutamide and is responsible for activation by a chemically diverse group of agents termed "K⁺ channel openers" such as diazoxide, pinacidil and cromakalin (Aguilar-Bryan *et al.,* 1995; Inagaki *et al.,* 1996; Isomoto *et al.,* 1996; Nichols *et al.,* 1996; Shyng *et al.,* 1997). In various tissues, molecularly distinct SURs are coupled to distinct pore-forming subunits to form different K_{ATP} channels with distinguishable physiological and pharmacological characteristics. The K_{ATP} channel in pancreatic β cells is formed from SUR1 linked with Kir6.2, whereas the cardiac and smooth muscle K_{ATP} channels are formed from SUR2A and SUR2B linked with Kir6.2 and Kir6.1, respectively (Fujita *et al.,* 2000). Despite being made up of distinctly different pore-forming subunits, the NC_{Ca-ATP} channel is also sensitive to sulfonylurea compounds and is believed to include a SUR1 receptor.

Also, unlike the K_{ATP} channel, the NC_{CA-ATP} channel conducts sodium ions, potassium ions, cesium ions and other monovalent cations with near equal facility (Chen and Simard, 2001) suggesting further that the characterization, and consequently the affinity to certain compounds, of the NC_{Ca-ATP} channel differs from the K_{ATP} channel.

Other nonselective cation channels that are activated by intracellular Ca²⁺ and inhibited by intracellular ATP have been identified by others but not in astrocytes or neurons as disclosed herein. Further, the NC_{Ca-ATP} channel expressed and found in neural cells differs physiologically from the other channels with respect to calcium sensitivity and adenine nucleotide sensitivity (Chen *et al*., 2001) and sensitivity to sulfonylureas (Chen *et al*., 2003).

### Summary of NC_{Ca-ATP} Channel Characteristics

At least some of the characteristics of cells expressing and composition comprising the NC_{Ca-ATP} channel are summarized in Table 1 (taken from experiments with freshly isolated native reactive astrocytes (NRA]).

**TABLE 1**

| | | |
|---|---|---|
| Properties of cells and membrane compositions containing the NC_{Ca-ATP} Channel of the Present Invention | | |
| | Reactive Astrocytes | Membrane Preparation derived from freshly isolated native reactive astrocytes |
| Monovalent cation permeable? | Yes: | Yes: |
| | Na⁺ | Na⁺ |
| | K⁺ | K⁺ |
| | Li⁺ | Li⁺ |
| | Rb⁺ | Rb⁺ |
| | Cs⁺ | Cs⁺ |
| | (Na⁺≈K⁺≈Li⁺≈Rb⁺) | (Na⁺≈K⁺≈Li⁺≈Rb⁺) |
| Anion permeable? | No | No |
| Divalent cation permeable? | No | No |
| Compounds blocking channel activity | SUR1 antagonists | SUR1 antagonists |
| Channel opening Requires: | - Intracell. ATP depletion - Intracell. Ca²⁺ | - Intracell ATP depletion - Intracell. Ca²⁺ |
| Single Channel Conductance | ∼25-35 pS | ∼25-35 PS |
| Activation [Ca²⁺] | <1.0 µM | <1.0 µM |
| [ATP]₁ EC₅₀ (um) | 0.79 µM | 0.79 µM |
| ADP AMP | No channel effect | No channel effect |
| Pore radius (nm) | 0.41 | 0.41 |

### IV. Exemplary Embodiments of The Present Invention

The present invention is directed to therapeutic methods involving TRPM4 antagonists as set out above, and as defined in the claims.

It is a further object of the present invention to provide a method of preventing and/or reducing neural cell swelling in the brain of a subject, said method comprising administering to the subject a formulation containing an effective amount of a singular or combinatorial therapeutic composition comprising a compound which is an antagonist of TRPM4, and a pharmaceutically acceptable carrier.

In certain aspects, a singular composition is provided to an individual in need thereof, whereas in other aspects, a combination composition is provided to an individual in need thereof. In particular cases, the singular composition comprises an antagonist of TRPM4, whereas in other cases the combination composition comprises an antagonist of TRPM4 with another composition, such as, for example, a compound selected from the group consisting of a) one or more cation channel blockers; and b) one or more of a compound selected from the group consisting of one or more antagonists of vascular endothelial growth factor (VEGF), one or more antagonists of matrix metalloprotease (MMP), one or more antagonists of nitric oxide synthase (NOS), one or more antagonists of thrombin, aquaporin, or a biologically active derivative thereof.

It is an object of the present invention to provide a method of alleviating the negative effects of traumatic brain injury or cerebral ischemia stemming from neural cell swelling in a subject trauma (e.g., traumatic brain or spinal cord injury (TBI or SCI), concussion) ischemic brain injury, hemorrhagic infarction, stroke, atrial fibrillations, clotting disorders, pulmonary emboli, arterio-venous malformations, mass-occupying lesions (e.g., hematomas), subjects undergoing treatments that increase the risk of stroke, for example, surgery (vascular or neurological), treatment of myocardial infarction with thrombolytics, cerebral/endovascular treatments, stent placements, angiography, *etc.,* comprising administering to the subject a formulation comprising an effective amount of a combinatorial therapeutic composition comprising a TRPM4 antagonist that at least in part blocks the NC_{Ca-ATP} channel, and a pharmaceutically acceptable carrier. Such administration may be delivery directly to the brain, intravenously, subcutaneously, intramuscularly, intracutaneously, intragastrically and orally. Examples of such compounds include an inhibitor of the channel, such as, for example, an antagonist of a type 1 sulfonylurea receptor, such as sulfonylureas like glibenclamide and tolbutamide, as well as other insulin secretagogues such as repaglinide, nateglinide, meglitinide, midaglizole, LY397364, LY389382, gliclazide, glimepiride, MgADP, and combinations thereof.

It is yet another object of the present invention to provide a formulation for preventing or inhibiting neural cell swelling in the brain of a subject, using a formulation that includes a singular or combinatorial therapeutic composition comprising a TRPM4 antagonist that at least in part inhibits the NC_{Ca-ATP} channel and a pharmaceutically acceptable carrier, wherein in certain embodiments the quantity of the compound is less than the quantity of the compound in formulations for treating diabetes, in certain cases.

In addition to the sulfonylurea receptor 1 (SUR1) being expressed in R1 astrocytes as part of the NC_{Ca-ATP} channel, the present invention further describes that the SUR1 regulatory subunit of this channel is up-regulated in at least neurons, neural cells and capillary endothelial cells following ischemia, and inhibiting this receptor reduces stroke size, cerebral edema and mortality. Thus, antagonists of the NC_{Ca-ATP} channel have an important role in preventing, alleviating, inhibiting and/or abrogating the formation of cytotoxic edema, ionic edema, vasogenic edema, and hemorrhagic conversion.

Antagonists are contemplated for use in treating adverse conditions associated with hypoxia and/or ischemia that result in increased intracranial pressure and/or cytotoxic edema of the central nervous system. Such conditions include trauma, ischemic brain injury, namely secondary neuronal injury, and hemorrhagic infarction, for example. Antagonists protect the cells expressing the NC_{Ca-ATP} channel, which is desirable for clinical treatment in which gliotic capsule integrity is relevant and must be maintained to prevent the spread of infection, such as with a brain abscess. The protection *via* inhibition of the NC_{Ca-ATP} channel is associated with at least a reduction in cerebral edema.

In one aspect, the NC_{Ca-ATP} channel is blocked, inhibited, or otherwise is decreased in activity. In such examples, an antagonist of the NC_{Ca-ATP} channel is administered and/or applied. The antagonist modulates the NC_{Ca-ATP} channel such that flux through the channel is reduced, ceased, decreased and/or stopped. The antagonist may have a reversible or an irreversible activity with respect to the activity of the NC_{Ca-ATP} channel of the neuronal cell, neuroglial cell, endothelial cell or a combination thereof. The antagonist may prevent or lessen the depolarization of the cells, thereby lessening cell swelling due to osmotic changes that can result from depolarization of the cells. Thus, inhibition of the NC_{Ca-ATP} channel can reduce cytotoxic edema and death of endothelial cells.

Subjects that can be treated with the singular or combinatorial therapeutic composition of the present invention include, but are not limited to, subjects suffering from or at risk of developing conditions associated with hypoxia and/or ischemia that result in increased intracranial pressure and/or with cytotoxic edema of the central nervous system (CNS). Such conditions include, but are not limited to, trauma *(e.g.,* traumatic brain or spinal cord injury (TBI or SCI), concussion) ischemic brain injury, hemorrhagic infarction, germinal matrix hemorrhage, stroke, atrial fibrillations, clotting disorders, pulmonary emboli, arterio-venous malformations, mass-occupying lesions *(e.g.,* hematomas), *etc.* Still further, subjects at risk of developing such conditions can include subjects undergoing treatments that increase the risk of stroke, for example, surgery (vascular or neurological), treatment of myocardial infarction with thrombolytics, cerebral/endovascular treatments, stent placements, angiography, or individuals without a medical condition who engage in sport activities that put them at risk for brain and spinal cord injury *etc.*

In other embodiments, antagonists are contemplated for use in treating adverse conditions associated with intracranial pressure and/or ionic or cytotoxic edema of the central nervous system, in specific embodiments. Such conditions include trauma *(e.g.,* traumatic brain or spinal cord injury (TBI or SCI, respectively)), ischemic brain or spinal cord injury, primary and secondary neuronal injury, stroke, arteriovenous malformations (AVM), mass-occupying lesion *(e.g.,* hematoma), and hemorrhagic infarction. Antagonists protect the cells expressing the NC_{CA-ATP} channel, which is desirable for clinical treatment in which ionic or cytotoxic edema is formed, in which capillary integrity is lost following ischemia, and in which gliotic capsule integrity is important and must be maintained to prevent the spread of infection, such as with a brain abscess. Those of skill in the art realize that a brain abscess is a completely enclosed and results in cerebral swelling. The protection *via* inhibition of the NC_{Ca-ATP} channel is associated with a reduction in cerebral ionic and cytotoxic edema. Thus, the compound that inhibits the NC_{Ca-ATP} channel is neuroprotective, in certain aspects.

In one aspect, the NC_{Ca-ATP} channel is blocked, inhibited, or otherwise is decreased in activity. In such examples, an antagonist of the NC_{Ca-ATP} channel is administered and/or applied. The antagonist modulates the NC_{Ca-ATP} channel such that flux (ion and/or water) through the channel is reduced, ceased, decreased and/or stopped. The antagonist may have a reversible or an irreversible activity with respect to the activity of the NC_{Ca-ATP} channel of the neuronal cell, neuroglial cell, a neural endothelial cell or a combination thereof. Thus, inhibition of the NC_{Ca-ATP} channel can reduce cytotoxic edema and death of endothelial cells which are associated with formation of ionic edema and with hemorrhagic conversion.

Accordingly, the present invention is useful in the treatment or alleviation of acute cerebral ischemia. According to a specific embodiment of the present invention the administration of effective amounts of the active compound can block the channel, which if remained open leads to neuronal cell swelling and cell death. A variety of antagonists to SUR1 are suitable for blocking the channel. Examples of suitable SUR1 antagonists include, but are not limited to mitiglinide, iptakalim, endosulfines, glibenclamide, tolbutamide, repaglinide, nateglinide, meglitinide, midaglizole, LY397364, LY389382, glyclazide, glimepiride, estrogen, estrogen related-compounds and combinations thereof. In a preferred embodiment of the invention the SUR1 antagonists is selected from the group consisting of glibenclamide and tolbutamide. Another antagonist that can be used is MgADP. Still other therapeutic "strategies" for preventing neural cell swelling and cell death can be adopted including, but not limited to methods that maintain the neural cell in a polarized state and methods that prevent strong depolarization.

In further embodiments, inhibitors or antagonists of the NC_{Ca-ATP} channel can be used to reduce or alleviate or abrogate hemorrhagic conversion. The pathological sequence that takes place in capillaries after ischemia can be divided into 3 stages, based on the principal constituents that move from the intravascular compartment into brain parenchyma (Ayata 2002; Betz, 1996; Betz 1989). The first stage is characterized by formation of "ionic" edema, during which the BBB remains intact, with movement of electrolytes (Na⁺, Cl⁻) plus water into brain parenchyma. The second stage is characterized by formation of "vasogenic" edema, due to breakdown of the BBB, during which macromolecules plus water enter into brain parenchyma. The third stage is characterized by hemorrhagic conversion, due to catastrophic failure of capillaries, during which all constituents of blood extravasate into brain parenchyma. In accordance with Starling's law, understanding these phases requires that 2 things be identified:
(i) the driving force that "pushes" things into parenchyma; and (ii) the permeability pore that allows passage of these things into parenchyma.

In specific aspects, the use of the antagonist or related-compounds thereof can reduce the risk of mortality of a subject suffering from a stroke and/or rescue the penumbra area or prevent damage in the penumbra area which comprises areas of tissue that are at risk of becoming irreversibly damaged.

With the administration of an antagonist of the NC_{Ca-ATP} channel, endothelial cell depolarization is abrogated, slowed, reduced or inhibited due to the opening of the NC_{Ca-ATP} channel. Thus, abrogation of cell depolarization results in abrogation or inhibition of Na⁺ influx, which prevents a change in osmotic gradient, thereby preventing an influx of water into the endothelial cell and stopping cell swelling, blebbing and cytotoxic edema. Thus, preventing or inhibiting or attenuating endothelial cell depolarization can prevent or reduce hemorrhagic conversion.

Subjects that may be treated with the antagonist or related-compound thereof include those that are suffering from or at risk of developing trauma (e.g., traumatic brain or spinal cord injury (TBI or SCI)), ischemic brain or spinal cord injury, primary and secondary neuronal injury, stroke, arteriovenous malformations (AVM), brain abscess, mass-occupying lesion, hemorrhagic infarction, or any other condition associated with cerebral hypoxia or cerebral ischemia resulting in cerebral edema and/or increased intracranial pressure, for example, but not limited to brain mass, brain edema, hematoma, end stage cerebral edema, encephalopathies, *etc.* Thus, the antagonist can be a therapeutic treatment in which the therapeutic treatment includes prophylaxis or a prophylactic treatment. The antagonist or related-compounds thereof are neuroprotective.

Some subjects that may be treated with the antagonist of the present invention include those subjects that are at risk or predisposed to developing a stroke. Such subjects can include, but are not limited to subjects that suffer from atrial fibrillations, clotting disorders, and/or risk of pulmonary emboli. In certain embodiments, a subject at risk for developing a stroke may include subjects undergoing treatments, for example, but not limited to cerebral/endovascular treatments, surgery (e.g., craniotomy, cranial surgery, removal of brain tumors (e.g., hematoma), coronary artery bypass grafting (CABG), angiography, stent replacement, other vascular surgeries, and/or other CNS or neurological surgeries), and treatment of myocardial infarction (MI) with thrombolytics, as well as surgeries on aortic abdominal aneurysms and major vessels that provide blood supply to the spinal cord. In such cases, the subject may be treated with the antagonist or related-compound of the present invention prior to the actual treatment. Pretreatment can include administration of the antagonist and/or related-compound months (1, 2, 3, *etc.),* weeks (1, 2, 3, *etc.),* days (1, 2, 3, *etc*.), hours (1, 2, 3, 4, 5, 6, 7, 8, 9, 10, 11, 12), or minutes (15, 30, 60, 90, *etc.)* prior to the actual treatment or surgery. Treatment of the antagonist and/or related-compound can continue during the treatment and/or surgery and after the treatment and/or surgery until the risk of developing a stroke in the subject is decreased, lessened or alleviated. In further embodiments, the antagonist of the present invention can be given to a subject at risk of developing head/neck trauma, such as a subject involved in sports or other activities that have an increased risk of head/neck trauma.

An effective amount of an antagonist that may be administered to a cell includes a dose of about 0.0001 nM to about 2000µM. More specifically, doses of an agonist to be administered are from about 0.01 nM to about 2000µM; about 0.01 µM to about 0.05 µM; about 0.05 µM to about 1.0 µM; about 1.0 µM to about 1.5 µM; about 1.5 µM to about 2.0 µM; about 2.0 µM to about 3.0 µM; about 3.0 µM to about 4.0 µM; about 4.0 µM to about 5.0 µM; about 5.0 µM to about 10 µM; about 10 µM to about 50 µM; about 50 µM to about 100 µM; about 100 µM to about 200 µM; about 200 µM to about 300 µM; about 300 µM to about 500 µM; about 500 µM to about 1000 µM; about 1000 µM to about 1500 µM and about 1500 µM to about 2000 µM. Of course, all of these amounts are exemplary, and any amount in-between these dosages is also expected to be of use in the invention.

The antagonist or related-compound thereof can be administered parenterally or alimentarily. Parenteral administrations include, but are not limited to, intravenously, intradermally, intramuscularly, intraarterially, intrathecally, subcutaneous, or intraperitoneally U.S. Pat. Nos. 6,613,308, 5,466,468, 5,543,158; 5,641,515; and 5,399,363. Alimentary administrations include, but are not limited to, orally, buccally, rectally, or sublingually. The administration of the therapeutic compounds and/or the therapies of the present invention may include systemic, local and/or regional administrations, for example, topically (dermally, transdermally), *via* catheters, implantable pumps, *etc.* Alternatively, other routes of administration are also contemplated such as, for example, arterial perfusion, intracavitary, intraperitoneal, intrapleural, intraventricular and/or intrathecal. The skilled artisan is aware of determining the appropriate administration route using standard methods and procedures. Other routes of administration are discussed elsewhere in the specification.

Treatment methods will involve treating an individual with an effective amount of a composition comprising an antagonist of NC_{Ca-ATP} channel which is an antagonist of TRPM4. An effective amount is described, generally, as that amount sufficient to detectably and repeatedly ameliorate, reduce, minimize or limit the extent of a disease or its symptoms. More specifically, it is envisioned that the treatment with an antagonist of NC_{Ca-ATP} channel which is an antagonist of TRPM4 will inhibit cell depolarization, inhibit Na⁺ influx, inhibit an osmotic gradient change, inhibit water influx into the cell, inhibit cytotoxic cell edema, decrease stroke size, inhibit hemorrhagic conversion, and/or decrease mortality of the subject, in specific embodiments.

The effective amount of an antagonist of NC_{Ca-ATP} channel which is an antagonist of TRPM4 to be used are those amounts effective to produce beneficial results, for example, with respect to stroke treatment, in the recipient animal or patient. Such amounts may be initially determined by reviewing the published literature, by *conducting in vitro* tests and/or by conducting metabolic studies in healthy experimental animals. Before use in a clinical setting, it may be beneficial to conduct confirmatory studies in an animal model, preferably a widely accepted animal model of the particular disease to be treated. Preferred animal models for use in certain embodiments are rodent models, which are preferred because they are economical to use and, particularly, because the results gained are widely accepted as predictive of clinical value.

As is well known in the art, a specific dose level of active compounds such as an antagonist of the NC_{Ca-ATP} channel which is an antagonist of TRPM4 for any particular patient depends upon a variety of factors including the activity of the specific compound employed, the age, body weight, general health, sex, diet, time of administration, route of administration, rate of excretion, drug combination, and the severity of the particular disease undergoing therapy. The person responsible for administration will determine the appropriate dose for the individual subject. Moreover, for human administration, preparations should meet sterility, pyrogenicity, general safety and purity standards as required by FDA Office of Biologics standards.

One of skill in the art realizes that the effective amount of the antagonist or related-compound thereof can be the amount that is required to achieve the desired result: reduction in the risk of stroke, reduction in the amount of damage following stroke, reduction in intracranial pressure, reduction in cell death, reduction in stroke size, and/or reduction in spinal cord injury, *etc.* This amount also is an amount that maintains a reasonable level of blood glucose in the patient, for example, the amount of the antagonist maintains a blood glucose level of at least 60 mmol/l, more preferably, effective to maintain blood glucose levels within an acceptable range, such as, for example, between about 60 mmol.l and about 150 mmol/l. Thus, the amount prevents the subject from becoming hypoglycemic. If glucose levels are not normal, then one of skill in the art would administer either insulin or glucose or glucagon, depending upon if the patient is hypoglycemic or hyperglycemic.

Administration of the therapeutic antagonist of TRPM4 of the present invention to a patient or subject will follow general protocols for the administration of therapies used in stroke treatment, such as thrombolytics, taking into account the toxicity, if any, of the antagonist of TRPM4. It is expected that the treatment cycles would be repeated as necessary. It also is contemplated that various standard therapies, as well as surgical intervention, may be applied in combination with the described therapy.

Another aspect for the treatment of ischemia, brain trauma, or other brain injury comprises administration of an effective amount of a SUR1 antagonist and administration of glucose or glucagon. Glucose or glucagon administration may precede the time of treatment with an antagonist of the NC_{Ca-ATP} channel, may be at the time of treatment with an antagonist of the NC_{Ca-ATP} channel, such as a SUR1 and/or TRPM4 antagonist, or may follow treatment with an antagonist of the NC_{Ca-ATP} channel *(e.g.,* at 15 minutes after treatment with an antagonist of the NC_{Ca-ATP} channel, or at one half hour after treatment with an antagonist of the NC_{Ca-ATP} channel, or at one hour after treatment with an antagonist of the NC_{Ca-ATP} channel, or at two hours after treatment with an antagonist of the NC_{Ca-ATP} channel, or at three hours after treatment with an antagonist of the NC_{Ca-ATP} channel). Glucose or glucagon administration may be by intravenous, or intraperitoneal, or other suitable route and means of delivery. Additional glucose or glucagon allows administration of higher doses of an antagonist of the NC_{Ca-ATP} channel than might otherwise be possible, so that combined glucose or glucagon with an antagonist of the NC_{Ca-ATP} channel provides greater protection, and may allow treatment at later times, than with an antagonist of the NC_{Ca-ATP} channel alone. Greater amounts of glucose are administered where larger doses of an antagonist of the NC_{Ca-ATP} channel are administered.

Another aspect of the present invention comprises co-administration of an antagonist of the NC_{Ca-ATP} channel which is an antagonist of TRPM4 with a thrombolytic agent. Co-administration of these two compounds increases the therapeutic window of the thrombolytic agent by reducing hemorrhagic conversion. The therapeutic window for thrombolytic agents may be increased by several (4-8, for example) hours by co-administering an antagonist of the NC_{Ca-ATP} channel which is an antagonist of TRPM4. In addition to a thrombolytic agent, other agents can be used in combination with the antagonist of the present invention, for example, but not limited to antiplatelets, anticoagulants, vasodilators, statins, diuretics, *etc.*

Yet further, the compositions of the present invention can be used to produce neuroprotective kits that are used to treat subjects at risk or suffering from conditions that are associated with cytotoxic cerebral edema, brain injury or spinal cord injury.

### V. Non-selective cation channels, transient receptor potential channels, and ischemic stroke

A number of different mechanisms have been implicated in cell death in CNS ischemia and stroke, including excitotoxicity, oxidative stress, apoptosis, and oncotic (necrotic) cell death. Each of these mechanisms is thought to propagate through largely distinct, mutually exclusive signal transduction pathways (Won *et al.,* 2002). However, in some measure, each of these mechanisms requires cation influx into neural cells. Unchecked influx of Na⁺ gives rise to oncotic cell swelling (cytotoxic edema), which predisposes to oncotic (necrotic) cell death. Unchecked influx of Ca²⁺ can trigger apoptotic as well as necrotic death. Because cation channels are responsible for most cation influx, it is evident that cation channels are key to life-death processes in neural cells during ischemic stroke.

A variety of cation channels have been implicated in neural cell death induced by ischemia/hypoxia. Among them are channels that are highly selective for permeant cations, such as voltage-dependent Na⁺ and Ca²⁺ channels, as well as channels that are not selective for any given cation - non-selective cation (NC) channels. In ischemic stroke, much attention has been directed to dihydropyridine-sensitive L-type voltage-dependent Ca²⁺ channels (CaV1.2), but block of this channel in patients with acute ischemic stroke has shown little benefit (Horn and Limburg, 2000). Arguably, the best studied channels in ischemic stroke belong to the group of receptor operated cation channels opened by glutamate, including N-methyl-D-aspartate (NMDA) and α-amino-3-hydroxy-5-methyl-4-isoxazolepropionate (AMPA) receptor channels, which are involved in excitotoxic cell death (Choi, 1988; Planells-Cases *et al.,* 2006).

Apart from neural cell death, other critically important pathophysiological processes that contribute to adverse outcome in ischemic stroke include formation of ionic edema, vasogenic edema and hemorrhagic conversion - all processes involving capillary endothelial cells (Simard *et al.,* 2007). In the case of ionic edema formation, transcapillary flux of Na⁺ constitutes the seminal process that drives inflow of H₂O into brain parenchyma, resulting in edema and swelling. In specific embodiments, NC channels play a role in this process. Thus, NC channels are implicated not only in primary neural cell death but in secondary neural cell death caused by endothelial dysfunction.

In recent years, study of ischemia/hypoxia-induced cell death has been dominated by discussion of apoptosis, a form of "delayed" programmed cell death that involves transcriptional up-regulation of death-related gene products, such as caspases. However, in stroke, only a fraction of cells undergo apoptotic death, with the majority of cells dying by oncotic/necrotic death (Lipton, 1999). The lesson from studies on apoptosis is that death, like so many other cellular events, is driven by gene expression and synthesis of new gene products, a concept that has not been fully embraced in studies on oncotic/necrotic death. Comprehensive understanding of the pathophysiology of ischemic stroke requires a focus not only on constitutively expressed NC channels in neurons, astrocytes and endothelial cells, but perhaps more importantly, on newly expressed NC channels whose transcription is driven by mechanisms involved in ischemic stroke, namely, hypoxia and oxidative stress.

### A. Non-specific NC channel blockers in ischemic stroke

A number of studies have shown that pharmacological inhibition of NC channels reduces focal ischemic injury in rodent models of ischemic stroke. Although none of these pharmacological agents is uniquely specific for any single molecular entity, some have been shown to block transient receptor potential (TRP) channels.

### 1. The NC channel blocker, pinokalant

The isoquinoline derivative pinokalant (LOE 908 MS, (R,S)-(3,4-dihydro-6,7-dimethoxy-isoquinoline-1-yl)-2-phenyl-N,N-di[2-(2,3,4-trimethoxyphenyl)ethyl]-acetamide) blocks a variety of NC channels, including both receptor- and store-operated NC channels that mediate Ca²⁺-entry, including:
(i) norepinephrine-activated Ca²⁺-entry channels in adrenergic receptor-expressing Chinese hamster ovary cells (Kawanabe *et al*., 2001);
(ii) endothelin-1 (ET-1)-activated Ca²⁺-entry channels in rat aorta myocytes (Zhang *et al*., 1999), A7r5 cells (Iwamuro *et al*., 1999; Miwa *et al*., 2000), rabbit internal carotid artery myocytes (Kawanabe *et al*., 2003), in C6 glioma cells (Kawanabe *et al.,* 2001), in ET-1-expressing CHO cells (Kawanabe *et al*., 2002; Kawanabe *et al*., 2003) and in bovine adrenal chromaffin cells (Lee *et al*., 1999);
(iii) ATP- and N-formyl-L-methionyl-L-leucyl-L-phenylalanine (fMLP)-stimulated cation currents in HL-60 cells (Krautwurst et a., 1993);
(iv) vasopressin-induced cation current in A7r5 cells (Krautwurst *et al*., 1994);
(v) store-operated NC channels in human endothelial cells (Encabo *et al*., 1996); (however, in some cells, store-operated NC channels are resistant to pinokalant, reflecting a significant diversity of molecular constituents of these channels (Miwa *et al*., 1999; Flemming *et al*., 2003).

The primary candidate subunits of mammalian receptor- and store-operated NC channels are TRP proteins. Some of the above receptor- and store-operated NC channels that are blocked by pinokalant have been shown to be mediated by members of the TRP family, indicating that pinokalant, at least in part, is targeting some TRP channels. Thus, TRPC6 is a component of the norepinephrine-activated channel in rabbit portal vein, and it is believed that TRP6 plays an important role in mediating Ca²⁺ influx in vascular smooth muscle (Large, 2002). TRPC1 has been implicated in ET-1-evoked arterial contraction (Beech, 2005). TRPC are thought to function as Ca²⁺ entry channels operated by store-depletion as well as receptor-activated channels in a variety of cell types, including endothelial cells (Ahmmed and Malik, 2005). In the cockroach, Periplaneta Americana, the TRPγ (pTRPy) channel is blocked by pinokalant (Wicher *et al*., 2006). However, block by pinokalant cannot be taken as evidence in and of itself that a TRP channel is involved in any given cationic current. Voltage-activated delayed rectifier K⁺ channels in PC12 cells and cortical neurons (Krause *et al*., 1998) and in HL-60 cells (Krautwurst *et al*., 1993) are also blocked by pinokalant.

Given its pharmacological profile as an inhibitor of NC channels, pinokalant has been evaluated as a potential neuroprotectant in rodent models of stroke (Christensen *et al*., 2005; Hoehn-Berlage *et al*., 1997; Li *et al*., 1999; Tatlisumak *et al*., 2000; Tatlisumak *et al*., 2000). Magnetic resonance imaging (MRI) was used to study the effect of pinokalant in a permanent (suture occlusion) middle cerebral artery occlusion (MCAO) model (Hoehn-Berlage *et al*., 1997). In untreated animals, the ischemic lesion volume [defined as the region in which the apparent diffusion coefficient (ADC) of water decreased to below 80% of control] steadily increased by approximately 50% during the initial 6 h of vascular occlusion. In treated animals, the ADC lesion volume decreased by approximately 20% during the same interval. After 6 h of vascular occlusion, blood flow was significantly higher in treated animals, and the volume of ATP-depleted and morphologically injured tissue representing the infarct core was 60-70% smaller. The volume of severely acidic tissue did not differ, indicating that pinokalant does not reduce the size of ischemic penumbra. These findings were interpreted as demonstrating that post-occlusion treatment delays the expansion of the infarct core into the penumbra for a duration of at least 6 h.

MRI was also used to study the effect of pinokalant in a temporary (90-min suture occlusion) MCAO model (Li *et al*., 1999; Tatlisumak *et al*., 2000; Tatlisumak *et al*., 2000). Before treatment, the DWI-derived infarct volume did not differ between the groups, whereas at 4 h after MCAO, it was significantly smaller in the treated group. A significant difference in ischemic lesion size was detected beginning 1.5 h after treatment. The size of the ischemic core was significantly smaller in the treatment group, while the size of the ischemic penumbra was similar in the two groups at 85 min after arterial occlusion. Postmortem, 2,3,5-tuphenyltetrazolium chloride (TTC)-derived infarct volume was significantly attenuated in the pinokalant group and the neurological scores at 24 h were significantly better among the treated rats.

### 2. The NC channel blockers, the fenamates

The fenamates, flufenamic acid, mefenamic acid, meclofenamic acid, and niflumic acid, for example, block Ca²⁺-activated non-selective cation channels in a variety of cells (Gogelein *et al*., 1990; cho *et al*., 2003; Koivisto *et al*., 1998). Recently, it was shown in Chinese hamster ovary cells that flufenamic acid inhibits TRPM2 activated by extracellular H₂O₂ (Naziroglu *et al*., 2006), although other channels are also blocked by these compounds.

Three fenamates (flufenamic acid, meclofenamic acid and mefenamic acid) were examined for their protective effect on neurons under ischemic (glucose/oxygen deprivation) or excitotoxic conditions, using the isolated retina of chick embryo as a model (Chen *et al*., 1998). The fenamates protected the retina against the ischemic or excitotoxic insult, with only part of the neuroprotection attributed to inhibition of NMDA receptor-mediated currents, implicating non-NMDA NC channels in the response.

The effect of pre-treatment or post-treatment with mefenamate was evaluated in a rodent model of transient focal ischemia (Kelly and Auer, 2003). Neither pre- nor post-ischemic administration of a dose previously shown effective in preventing epileptic neuronal necrosis was found to reduce necrosis in cortex, nor in any subcortical structures, which forced the authors to conclude that NC channel blockade with mefenamate affords no neuroprotection in this model.

### 3. The NC channel blocker, SKF 96365

SKF 96365 (SK&F 96365) (1-(beta-[3-(4-methoxy-phenyl)propoxy]-4-methoxyphenethyl)-1H- imidazole hydrochloride) is structurally distinct from the known Ca²⁺ antagonists and shows selectivity in blocking receptor-mediated Ca²⁺ entry, compared with receptor-mediated internal Ca²⁺ release (Merritt *et al*., 1990. However, SKF 96365 is not as potent (IC₅₀ ∼10 µM) or selective (also inhibits voltage-gated Ca²⁺ entry) as would be desirable, so caution has been advised when using this compound (Merritt *et al*., 1990).

Measurements of intracellular Ca²⁺ in human embryonic kidney (HEK)293 cells that stably expressed human TRP3 were used to show that SKF 96365 blocks TRP channels (Zhu *et al*., 1998). Expression of TRP3 in these cells forms a non-selective cation channel that opens after the activation of phospholipase C, but not after store depletion. Increased Ca²⁺ entry in TRP3-expressing cells is blocked by high concentrations of SKF 96365 (Zhu *et al.,* 1998).

The blood-brain barrier (BBB) serves as a critical organ in the maintenance of CNS homeostasis and is disrupted in a number of neurological disorders, including ischemic stroke. SKF 96365 was used to determine if Ca²⁺ flux was important in mediating hypoxic/aglycemic effects on endothelial cells of the BBB (Brown and Davis, 2005; Brown *et al*., 2004; Abbruscato and Davis, 1999), which do not express voltage-dependent Ca²⁺ channels. Expression of the tight junction protein occludin increased after hypoxic/aglycemic stress when cells were exposed to SKF 96365, which correlated with inhibition of the hypoxia-induced increase in permeability. Treatment with SKF 96365 increased intracellular Ca²⁺ under normoglycemic conditions, and was protective against hypoxia-induced BBB disruption under normoglycemia.

### B. The cannabinoid 1 receptor blocker, rimonabant and the vanilloid agonist, capsaicin

Rimonabant (SR141716A) is a compound that interacts with the G-protein coupled cannabinoid 1 (CB1) receptor (Henness *et al*., 2006). Rimonabant has also been suggested to block TRP channel vanilloid subfamily member 1 (TRPV1) (Pegorini *et al*., 2006). The link between CB1 and TRPV1 is reinforced by evidence that anandamide, an endogenous CB1 ligand, also activates TRPV1 (Pertwee, 2005). Capsaicin as well as H⁺ (pH ≤ 5.9) are agonists known to activate TRPV1 (Gunthorpe *et al*., 2002; Van Der and Di, 2004).

In a rat model of ischemic stroke, rimonabant, given 30 min after initiation of permanent MCAO, reduced infarct volume by ∼40% (Berger *et al*., 2004). The effects of rimonabant and capsaicin were investigated, with the aim of assessing the potential role of TRPV1 in a model of global cerebral ischemia in gerbils (Pegorini *et al*., 2006; Pegorini *et al*., 2005). Both compounds were found to antagonize the electroencephalographic changes, hyperlocomotion and memory impairment induced by global ischemia, and both were associated with a progressive survival of pyramidal cells in the CA1 subfield of the hippocampus. Notably, capsazepine, a selective TRPV1 antagonist, reversed both rimonabant-induced and capsaicin-induced neuroprotective effects. The authors interpreted their findings as suggesting that neuroprotection associated with capsaicin might be attributable, at least in part, to TRPV1 desensitization.

### C. SUR1-regulated NC_{Ca-ATP} channel

The NC_{Ca-ATP} channel is a 35 pS cation channel that conducts all inorganic monovalent cations (Na⁺, K⁺, Cs⁺, Li⁺, Rb⁺), but is impermeable to Ca²⁺ and Mg²⁺ (Chen and Simard, 2001). The fact that it conducts Cs⁺ makes it easy to distinguish from K_{ATP} channels with which it shares several properties (see below). Channel opening requires nanomolar concentrations of Ca²⁺ on the cytoplasmic side. Channel opening is blocked by ATP (EC₅₀, 0.79 µM), but is unaffected by ADP or AMP. Studies using a variety of organic monovalent cations indicate that the channel has an equivalent pore radius of 0.41 nm.

The NC_{Ca-ATP} channel is composed of pore-forming and regulatory subunits. The regulatory subunit is sulfonylurea receptor 1 (SUR1), the same as that for K_{ATP} channels in pancreatic β cells (Chen *et al*., 2003), and so NC_{Ca-ATP} and pancreatic K_{ATP} channels have pharmacological profiles that resemble each other closely. NC_{Ca-ATP} channel opening is blocked by tolbutamide (EC₅₀, 16.1 µM at pH 7.4) and glibenclamide (EC₅₀, 48 nM at pH 7.4). Block by sulfonylurea is due to prolongation of and an increase in the probability of long closed states, with no effect on open channel dwell times or channel conductance. The potency of block by glibenclamide is increased ∼8-fold at pH 6.8 (EC₅₀, 6 nM), consistent with the weak acid needing to enter the lipid phase of the membrane to cause block (Simard *et al*., 2006). In the presence of ATP, channel opening is increased by diazoxide, but not pinacidil or cromakalin, as expected for SUR1 but not SUR2. The inhibitory effect of glibenclamide on opening of the NC_{Ca-ATP} channel is prevented by antibody directed against one of the cytoplasmic loops of SUR1. Knockdown of SUR1 using antisense-oligodeoxynucleotide reduces SUR1 expression (Simard *et al*., 2006) and prevents expression of functional NC_{Ca-ATP} channels.

The NC_{Ca-ATP} channel is not constitutively expressed, but is expressed in the CNS (brain and spinal cord) under conditions of hypoxia or injury and in certain aspects is expressed in other tissues and cells in response to other injuries or medical conditions (such as cardiac tissues or cells or blood vessels, for example). The channel was first discovered in freshly isolated reactive astrocytes obtained from the hypoxic inner zone of the gliotic capsule (Chen and Simard, 2001; Chen *et al*., 2003). Since then, it has also been identified in neurons from the core of an ischemic stroke (Simard *et al*., 2006) and in cultured brain endothelial cells (bEnd.3 cells) subjected to hypoxia. In rodent models of ischemic stroke and of spinal cord injury, the SUR1 regulatory subunit is transcriptionally up-regulated in neurons, astrocytes and capillary endothelial cells.

The consequence of channel opening has been studied in isolated cells that express the channel, by depleting ATP using Na azide or Na cyanide plus 2-deoxyglucose, or by using diazoxide. These treatments induce a strong inward current that depolarizes the cell completely to 0 mV. Morphological studies demonstrate that cells subsequently undergo changes consistent with cytotoxic edema (oncotic cell swelling), with formation of membrane blebs. Bleb formation is reproduced without ATP depletion by diazoxide (Chen and Simard, 2001). Cells later die predominantly by non-apoptotic, propidium iodide-positive necrotic death (Simard *et al*., 2006).

The effect of channel block by glibenclamide has been studied *in vitro* in reactive astrocytes that express the channel (Chen *et al*., 2003; Simard *et al*., 2006). In cells exposed to Na azide to deplete ATP, glibenclamide blocks membrane depolarization, significantly reduces blebbing associated with cytotoxic edema, and significantly reduces necrotic cell death.

The effect of channel block by glibenclamide has also been studied in 2 rodent models of ischemic stroke (Simard *et al*., 2006). Specificity of the drug for the target was based on administering a low dose by constant infusion (75-200 ng/h), which was predicted to yield serum concentrations of ∼1-3 ng/ml (2-6 nM), coupled with the low pH of the ischemic tissues, to take advantage of the fact that glibenclamide is a weak acid that would preferentially target acidic tissues. In a rodent model of massive ischemic stroke with malignant cerebral edema associated with high mortality (68%), glibenclamide reduced mortality and cerebral edema (excess water) by half. In a rodent model of stroke induced by thromboemboli with delayed spontaneous reperfusion, glibenclamide reduced lesion volume by half, and its use was associated with cortical sparing attributed to improved leptomeningeal collateral blood flow due to reduced mass effect from edema.

In summary, the salient features of the NC_{Ca-ATP} channel are that: (i) it is not constitutively expressed, but is transcriptionally up-regulated in association with an hypoxic injurious insult; (ii) when expressed, it is not active but becomes activated when intracellular ATP is depleted, leading to cell depolarization, cytotoxic edema and necrotic cell death; (iii) block of the channel *in vitro* results in block of depolarization, cytotoxic edema and necrotic cell death induced by ATP depletion; (iv) block of the channel *in vivo* results in significant improvement in rodent models of ischemic stroke and spinal cord injury.

### VI. TRP channels in ischemic stroke

There is a dearth of studies addressing the potential role of TRP channels in ischemic stroke *in vivo,* which is attributable, in part, to a paucity of appropriate tools. Although *in vitro* studies (reviewed below) suggest a possible role, demonstrating a role *in vivo* is considerably more difficult, as it requires demonstrating the presence of one or more of the following: (i) salutary effect of pharmacological block; (ii) salutary effect of gene silencing; (iii) transcriptional up-regulation under conditions of hypoxia/oxidative stress *in vitro*; (iv) transcriptional up-regulation under conditions of ischemic stroke *in vivo.*

### A. In vitro studies - TRPM2/TRPM7

Restoring extracellular Ca²⁺ after a period of low Ca²⁺ concentrations has long been known to cause a paradoxical increase in intracellular Ca²⁺ levels that can lead to cell death ('Ca²⁺ paradox'). Until recently, entry of Ca²⁺ through NMDA receptor channels was considered to be the major pathway leading to the excitotoxic, delayed cell death associated with ischemia. There is now evidence, however, that TRP channels, specifically TRPM2 and TRPM7, may be important contributors to both the Ca²⁺ paradox and the delayed death of neurons following ischemia (Nicotera and Bano, 2003; Aarts *et al*., 2005; Aarts and Tymianski, 2005; McNulty and Fonfria, 2005; MacDonald *et al*., 2006). Aarts and coworkers studied the mechanism of death in cultures of mixed cortical neurons subjected to 1.5 h of oxygen/glucose deprivation, followed by return to normoxic conditions and treatment with an anti-excitotoxic cocktail (MK-801, CNQX, and nimodipine) (2003). Treatment unmasked a Ca²⁺-mediated death mechanism associated with a Ca²⁺-permeable NC conductance that was shown to be carried by TRPM7, based on siRNA-inhibition of TRPM7 expression. Suppressing TRPM7 expression blocked TRPM7 currents, anoxic ⁴⁵Ca²⁺ uptake, production of reactive oxygen species (ROS), and anoxic death. In addition, TRPM7 suppression eliminated the need for the anti-excitotoxic cocktail to rescue anoxic neurons and permitted the survival of neurons previously destined to die from prolonged anoxia. Notably, TRPM7 current is potentiated by acidosis (Jiang and Yue, 2005), a condition that is present with cerebral ischemia and that makes this mechanism all the more relevant in ischemic stroke.

### B. In vivo studies - TRPC4

Involvement of TRPC4 was studied in a rodent model of MCAO (Gao *et al*., 2004). The authors used a commercial antibody directed against TRPC4 (from Alamone Labs) for Western immunoblots and immunohistochemistry. Some have cautioned that unequivocal detection of endogenous TRPC4 proteins may be difficult (Flockerzi *et al*., 2005). In any case, TRPC4 protein was found to be significantly elevated in striatum and hippocampus 12 h - 3 d after MCAO, with TRPC4 immunoreactivity being present in neuronal membranes.

### C. TRPM4 - an NC_{Ca-ATP} channel

Applicants disclose herein that TRPM4 is linked to ischemic stroke. Many of its biophysical properties are similar to those of the SUR1-regulated NC_{Ca-ATP} channel (see Table 2), which has also been implicated in ischemic stroke. TRPM4, together with TRPM5, are believed to be members of the class of non-selective, Ca²⁺-impermeable cation channels that are activated by intracellular Ca²⁺ and blocked by intracellular ATP, *i*.*e*., NC_{Ca-ATP} channels.

Both the SUR1-regulated NC_{Ca-ATP} channel and TRPM4 are highly selective for monovalent cations, have no significant permeation of Ca²⁺, are activated by internal Ca²⁺ and blocked by internal ATP. The two channels have several features in common (Table 2).

Applicants herein disclose that SUR1 and TRPM4 may combine into a heteromeric assembly of SUR1 and TRPM4; Applicants note that SUR1 is known to be a promiscuous regulatory subunit. SUR1 is known to play a role in forming K_{ATP} channels by assembling with Kir6.1 or Kir6.2 pore-forming subunits, and for forming heterologous constructs of SUR1 with another inwardly rectifying K⁺ channel, Kir1.1a (Ammala *et al*., 1996).

### D. Drivers of TRP expression - hypoxia and oxidative stress

### 1. Hypoxia

Stroke is a pathological condition marked by ischemia-induced hypoxia. Thus, we consider TRP channel expression that is induced by hypoxia.

In pulmonary arterial smooth muscle cells, hypoxia induces a 2-3-fold increase in TRPC1 and TRPC6 mRNA and protein levels, but has no effect on TRPC3/TRPC4 expression (Wang *et al*., 2006; Lin *et al*., 2004). Notably, hypoxia-induced up-regulation of TRPC1 and TRPC6 is correlated with and is dependent on the transcription factor, hypoxia inducible factor 1 (HIF-1) (Wang *et al.,* 2006).

### 2. Redox state

Apart from ischemia/hypoxia, stroke is also a condition characterized by reperfusion, which is associated with oxidant stress. Overproduction of ROS is one of the major causes of cell death in ischemic-reperfusion injury. ROS as well as reactive nitrogen species (RNS) play a pivotal role in CNS pathophysiology, especially in the context of ischemia/reperfusion.

TRPM2, which is abundantly expressed in the brain, is a Ca²⁺-permeant, non-selective cation channel that senses and responds to oxidative stress levels in the cell. Comprehensive reviews of the involvement of TRPM2 (a.k.a. TRPC7 or LTRPC2) in oxidative stress-induced cell death have been published (Perraud *et al*., 2003; Miller, 2004; Kuhn *et al*., 2005; Miller, 2006; Miller, 2006). TRPM2 functions as a cell death-mediating Ca²⁺-permeable cation channel that possesses both ion channel and ADP-ribose hydrolase functions. Oxidative and nitrosative stress lead to the accumulation of cytosolic ADP-ribose released from mitochondria, and accumulation of ADP-ribose is required for oxidative- and nitrosative-stress-induced gating of TRPM2 cation channels (Perraud *et al*., 2005). Inhibition of TRPM2 function by poly(ADP-ribose)polymerase-1 (PARP-1) inhibitors protects cells from oxidative stress-induced death (Miller, 2004). In heterologous cells, TRPM2 expression enhances and TRPM2 suppression reduces vulnerability to H₂O₂ toxicity (Hara *et al*., 2002). Although specific involvement of TRPM2 in ischemic reperfusion injury in CNS has not been shown, its interdependent expression with TRPM7, coupled with the demonstrated role of TRPM7 in neuronal death (Aarts *et al*., 2003), make it likely that TRPM2 indeed plays a role.

### 3. Analysis of promoter regions

Applicants have considered that additional insights might be gained by examining transcriptional mechanisms shown to be active in ischemic stroke, and performed studies and obtained data supporting a link between these mechanisms and TRP protein expression. Cerebral ischemia is associated with hypoxia and oxidant stress, which activate a transcriptional program in brain that may include the transcription factors, AP-1 (Yoneda *et al*., 1997; Yoneda *et al*., 1998; Domanska-Janik *et al*., 1999; Cho *et al*., 2001), Sp-1 (Simard *et al*., 2006), HIF-1 (Marti *et al*., 2000; Prass *et al*., 2003), NF-κB (Koong *et al*., 1994; Mattson, 1997), PPAR α&γ (Deplanque *et al*., 2003; Shimazu *et al*., 2005; Sundararajan *et al*., 2006), egr-1 (Honkaniemi and Sharp, 1996; Honkaniemi *et al*., 1997), c-Myc (Huang *et al*., 2001; Lubec *et al*., 2002).

The promoter regions of members of the TRPC and TRPM subfamilies were analyzed, searching for consensus binding site sequences for the transcription factors listed above. Surprisingly, the promoter regions of all TRP proteins examined, TRPC1-7 and TRPM1-8, were found to possess multiple consensus sites for one or more of the transcription factors linked to ischemic stroke (Table 2). Although this analysis cannot be taken as evidence for involvement of any of the factors in transcriptional regulation of the TRP proteins, it indicates that none can be excluded, suggesting that further work on the role of TRP proteins in ischemic stroke is warranted.

### VII. TRPM4 Antagonists

In the present invention, antagonists of TRPM4 are employed in methods and/or compositions. Antagonists may be of any kind, but in particular embodiments the antagonists are proteins, nucleic acids, small molecules, and so forth. In specific cases, the TRPM4 nucleic acid antagonists comprise flufenamic acid and/or RNAi, such as siRNA or antisense oligodeoxynucleotides (AS-ODN). A pair of AS-ODNs found to be highly effective in reducing TRPM4 expression and in improving outcome from spinal cord injury when used together, have the following sequences: (TRPM4-AS1: 5'-GTGTGCATCGCTGTCCCACA-3' (SEQ ID NO:1); and TRPM4-AS2: 5'-CTGCGATAGCACTCGCCAAA-3' (SEQ ID NO:2); sense (SE) or antisense (AS) oligodeoxynucleotides (ODN) were administered that were phosphorothioated to protect from endogenous nucleases.

### VIII. Molecular pathophysiology of brain edema in focal ischemia

Dysfunction of cerebral capillaries due to ischemia and post-ischemic reperfusion results in a progressive alteration in permeability of the blood brain barrier (BBB), leading to formation of ionic edema, vasogenic edema and hemorrhagic conversion. When capillaries that form the BBB can no longer retain intravascular constituents such as Na⁺, H₂O, serum proteins and blood, these substances enter into the extracellular space of the brain and cause swelling. It is common to divide edema into different subtypes (Joo and Klatzo, 1989; Betz *et al*., 1989; Ayata and Ropper, 2002) but it is not typical to include hemorrhagic conversion in the same discussion. Yet, it now appears that ionic edema, vasogenic edema and hemorrhagic conversion share important molecular antecedents, both transcriptional and pre-transcriptional, suggesting that hemorrhagic conversion may represent an end-stage in a process that manifests initially as edema.

Brain edema and hemorrhagic conversion are topics of great importance to neurologists and neurosurgeons who cope daily with their damaging consequences. Excellent reviews on these subjects have appeared (Ayata and Ropper, 2002; Young *et al*., 1994; Betz, 1996; Rosenberg, 1999). The present disclosure concerns embodiments related to edema formation and hemorrhagic conversion.

### A. Edema versus swelling

Edema is detrimental because it causes swelling (FIG. 1). Swelling means that the volume occupied by a given mass of tissue is increased, due to tumor, edema, blood, etcetera. Swelling is harmful because of its effects on adjacent tissues, with these effects magnified by the fixed volume of the skull. Swollen tissues exert mechanical force on a surrounding shell of tissue, displacing it and increasing tissue pressure within it. When tissue pressure exceeds capillary pressure, capillary inflow is compromised, leading to ischemia, formation of edema and swelling of the shell (Hossmann and Schuier, 1980). Edema and swelling are both indicators and causes of injury.

### B. Swelling requires active blood flow

Swelling implies that a new constituent is added to the extracellular space of the brain. Excluding tumor, the new constituent can only come from the vascular space. The absolute requirement for active blood flow is easily appreciated with a simple thought-experiment. Excision of a piece of tissue from a live brain, whether in the operating room or laboratory, will cause the cells within the tissue to die, exhibiting shifts in ionic and water content between extracellular and intracellular spaces that are characteristic of cytotoxic edema. However, such tissues will not swell, will not become heavier, and will show no ionic edema, vasogenic edema, or hemorrhagic conversion, simply because there is no source of new water, ions and blood. This thought-experiment reinforces the distinction between cytotoxic edema and the three pathophysiological processes (ionic edema, vasogenic edema and hemorrhagic conversion), with the latter three requiring blood flow to cause swelling.

With post-ischemic reperfusion, the requirement for active blood flow is fulfilled. In the case of unperfused tissue, there is a spatial gradient of ischemia/hypoxia, ranging from profound hypoxia in the core, to near-critical hypoxia in the penumbra, to normoxia further away. These zones are associated with different molecular and physiological responses (Hossmann, 1994). Ionic edema forms in the zone of perfused but severely ischemic tissue. In a rodent model of malignant cerebral edema studied 8 hours after permanent middle cerebral artery occlusion (FIG. 1B), the excess water of edema is localized overwhelmingly in perfused TTC(+) regions adjacent to the core, with minimal excess water in the poorly-perfused TTC(-) core (Simard *et al*., 2006). Magnetic resonance imaging confirms that edema is found first in peri-infarct regions that are perfused (Quast *et al*., 1993).

Edema fluid moves by bulk flow (convection) into the unperfused tissue. The driving force for this movement is the concentration gradient for the constituents that are moving, including Na⁺ and Cl⁻, and H₂O. Before equilibration, areas within the core will contain little or no excess electrolytes, whereas penumbral areas adjacent to infarct will contain an excess of electrolytes and water. The rate of accumulation of excess Na⁺ in the core may be used to estimate the age of the infarct (Wang *et al*., 2000).

### C. Starling's principle

Over a century ago, Starling established the basic principles involved in formation of edema (Starling, 1896). According to Starling, understanding edema formation requires that two things be identified: (i) the driving force that "pushes" substances into the brain; and (ii) the permeability pore that allows transcapillary passage of these substances from the intravascular to the extracellular space.

The driving force is determined by the sum of hydrostatic and osmotic pressure gradients (FIG. 2). Hydrostatic pressure is determined by the difference between pre-capillary arteriolar and post-capillary venular pressures, which are influenced by blood pressure and tissue pressure. Osmotic pressure is determined by the concentrations of osmotically active particles in blood versus extracellular tissues. In the normal brain capillary, osmotic pressure plays a much more important role than hydrostatic pressure, due to the existence of tight junctions between endothelial cells that minimize this mechanism of fluid transfer across the capillary. Under pathological conditions, both osmotic and hydrostatic pressure gradients play critical roles in fluid transfer.

The second factor, the permeability pore, is determined by "passages" through and between the capillary endothelial cells that form the BBB (Hawkins and Davis, 2005). Passages through endothelial cells can be formed by ion channels, if those channels are expressed on both luminal and abluminal sides of endothelial cells. Also, reverse pinocytosis has been put forth as a mechanism by which substances can undergo transcapillary movement. Formation of passages between capillary endothelial cells implies either that cells contract, partially "retracting" cell borders, that cells loose tight junctions between themselves, or that the cells are totally lost, *e*.*g*., by necrotic death.

### D. Cytotoxic edema

Cytotoxic edema is a premorbid process that involves oncotic swelling of cells due to movement of osmotically active molecules (principally Na⁺, Cl⁻ and H₂O) from the extracellular to the intracellular space (Klatzo, 1987; Kimelberg, 1995; Go, 1997; Kempski, 2001). The terms "cytotoxic edema", "cellular edema", "oncosis" and "necrotic volume increase" are synonymous and refer to pathophysiological processes at the cellular level. With cytotoxic edema, no new constituent from the intravascular space is added and tissue swelling does not occur. However, cytotoxic edema creates the "driving force" for transcapillary formation of ionic and vasogenic edema, which do cause swelling.

An older definition of cytotoxic edema encompassed not only the definition as given here involving a strictly cellular disturbance, but also the concept of transcapillary water and electrolyte transport into brain parenchyma, *i*.*e*., ionic edema. Because distinct physiological processes are involved, however, we regard it as important to maintain independent definitions.

Movements of osmotically active molecules into the cell can occur either by primary active transport or secondary active transport. Primary active transport (ATP-dependent, Na⁺/K⁺ ATPase, etcetera) requires continuous expenditure of energy, which is not readily available under conditions of ischemia (Sweeney *et al*., 1995; White *et al*., 2000). Secondary active transport uses energy stored in a pre-existing ionic gradients across the cell membrane (ion channels, Na⁺/K⁺/Cl⁻ cotransporter, etcetera.) Because of the dysfunctional energy state that exists with ischemia, we focus on mechanisms that are largely independent of continuous expenditure of energy.

Two types of substances are involved in cytotoxic edema - primary drivers and secondary participants. Primary drivers are molecules that are more concentrated outside compared to inside the cell and that are normally extruded from the cell by primary active transport. Secondary participants are molecules for which no pre-existing electrochemical gradient normally exists, but for which a gradient is created by the primary drivers. If Na⁺ is the primary driver, Cl⁻ and H₂O would be the secondary participants that move in order to maintain electrical and osmotic neutrality. Many types of Cl⁻ channels normally exist in all cells of the CNS. Aquaporin channels that may aid bulk flow of H₂O are up-regulated, at least in astrocytes, in CNS ischemia (Badaut *et al*., 2002; Amiry-Moghaddam and ottersen, 2003).

Different molecular mechanisms may be utilized for secondary active transport. For Na⁺, conventional thinking asserts that in neurons and astrocytes, constitutively expressed Na⁺ influx pathways, including tetrodotoxin-sensitive Na⁺ channels, Na⁺/K⁺/Cl⁻ cotransporter, N-methyl-D-aspartate receptor channels etcetera, admit Na⁺ during the course of normal activity or during "pathological depolarization" (Banasiak *et al*., 2004; Breder *et al*., 2000; Beck *et al*., 2003) and that, because of ischemia, newly admitted Na⁺ cannot be extruded due to failure of Na⁺/K⁺ ATPase and other ATP-dependent transporters (yang *et al*., 1992).

Apart from constitutively expressed pathways, non-selective cation channels up-regulated by ischemia or oxidative stress may provide new pathways for Na⁺ influx. Transient receptor potential channels (Aarts and Tymianski, 2005) and the sulfonylurea receptor 1 (SUR1)-regulated NC_{Ca-ATP} channel (Simard *et al*., 2006; Chen and Simard, 2001; Chen *et al*., 2003) can act in this manner. The NC_{Ca-ATP} channel is transcriptionally up-regulated within 2-3 hr of ischemia (FIG. 3). Opening of this channel, which is triggered by ATP depletion, causes cell depolarization, cell blebbing (FIG. 4), cytotoxic edema and oncotic cell death (FIG. 5), all of which are prevented by blocking the channel.

Opening non-selective cation channels allows egress of K⁺ from the cell, but movements of Na⁺ and K⁺ do not simply neutralize one another, because the cell is full of negatively charged proteins and other macromolecules that act to bind K⁺, (Young and Constantini, 1994) resulting in a significantly greater inflow of Na⁺ than outflow of K⁺. The net inflow of Na⁺ generates an osmotic force that drives influx of H₂O typical of cytotoxic edema.

Cytotoxic edema is tied to cell death. With the inflow of Na⁺ down its concentration gradient, and the resultant inflow of Cl⁻ and H₂O, the cell depolarizes, blebs or outpouchings form in the cell membrane, and eventually the membrane ruptures as the cell undergoes lysis -necrotic cell death (FIG. 5) (Barros *et al*., 2001; Barros *et al*., 2002).

Cytotoxic edema (oncotic volume increase) may be contrasted with "apoptotic volume decrease" (Okada and Maeno, 2001). The former involves influx of Na⁺, Cl⁻ and H₂O, whereas the latter involves opening of K⁺ selective channels resulting in K⁺ efflux, which is accompanied by Cl⁻ efflux and by loss of H₂O from the cell. Apoptotic volume decrease results in cell shrinkage, which presages apoptotic cell death.

### E. Driving force for edema formation

The extracellular space of the brain is small compared to the intracellular space, constituting only 12-19% of brain volume (Go, 1997). Thus, movements of ions and water into cells during formation of cytotoxic edema results in depletion of these constituents from the extracellular space (Stiefel and Marmarou, 2002; Mori *et al*., 2002). Cytotoxic edema sets up a new gradient for Na⁺, now across the BBB, between the intravascular space and the extracellular space, which acts as a driving force for transcapillary movement of edema fluid. If neurons and astrocytes undergo necrotic death, joining their intracellular contents to that of the extracellular space, a concentration gradient for Na⁺ is still set up across the BBB, again because the extracellular space of the brain is small compared to the intracellular space, as reflected by the high K⁺ concentration and low Na⁺ concentration of normal homogenized brain tissue (Young and Constantini, 1994), coupled with the fact that K⁺ ions remain largely bound to negatively charged intracellular proteins and other macromolecules (Young and Constantini, 1994). Thus, whether or not cells are intact, cytotoxic edema and cell death create a transcapillary gradient that acts to drive subsequent movement of edema fluid.

### F. Permeability pores

In accordance with Starling's principle, the driving force across the BBB that is newly created by cytotoxic edema represents a form of potential energy that will not be expended unless the permeability properties of the BBB are changed. In the following sections, the permeability pore(s) are considered that permit fluxes to occur down concentration gradients across the capillary wall. The ischemia-induced changes in capillary permeability may be organized into three distinct phases (ionic edema, vasogenic edema and hemorrhagic conversion), based on the principal constituents that undergo transcapillary movement (FIGS. 2 and 5). The 3 phases are considered to occur sequentially, but the likelihood and rapidity of transition from one phase to another probably depend on such factors as duration and depth of hypoxia during perfusion or prior to reperfusion. Thus, the reperfused capillary in the core that was completely ischemic is more likely to go on to the third phase than the hypoxic capillary at the edge of the penumbra.

### 1. First phase - formation of ionic edema

The earliest phase of endothelial dysfunction in ischemia is characterized by formation of ionic edema (FIGS. 2 and 5) (Betz *et al*., 1989; Young and Constantini, 1994; Gotoh *et al*., 1985; Young *et al*., 1987; Betz *et al*., 1990). Formation of ionic edema involves transport of Na⁺ across the BBB, which generates an electrical gradient for Cl⁻ and an osmotic gradient for H₂O, thus replenishing Na⁺, Cl⁻ and water in the extracellular space that was depleted by formation of cytotoxic edema. As with cytotoxic edema, in ionic edema, the amount of Na⁺ accumulation exceeds the amount of K⁺ lost, giving a net inflow of Na⁺ into edematous brain (Young and Constantini, 1994; Young *et al*., 19987).

Formation of ionic edema is clearly distinct from formation of vasogenic edema, as it involves abnormal Na⁺ transport in the face of normal exclusion of protein by the BBB (Schuier and Hossmann, 1980; Todd *et al*., 1986; Goto *et al*., 1985; Todd *et al*., 1986). Early water influx (stage of ionic edema) is well correlated with Na⁺ accumulation and precedes albumin influx (stage of vasogemic edema) by 6 hours or more. In this phase of ionic edema, the BBB remains "intact", *i*.*e*., macromolecules do not permeate it. Thus, influx of Na⁺ cannot be accounted for by leakiness of the BBB, reverse pinocytosis, loss of tight junctions or other physical processes that would also allow transport of serum macromolecules along with Na⁺.

As with cytotoxic edema, two mechanisms can account for selective flux of Na⁺ across the BBB, primary active transport and secondary active transport, but again, we focus only on secondary active transport mechanisms that depend on preexisting electrochemical gradients. Unlike neurons and astrocytes, endothelial cells do not express voltage-dependent channels that conduct Na⁺ (Nilius and Droogmans, 2001). They express ligand-gated channels that could act in this manner (Nilius and Droogmans, 2001), but no evidence exists to show their involvement.

The secondary active Na⁺/K⁺/Cl⁻ cotransporter (Russell, 2000), located mostly on the luminal side of endothelium, has been postulated to be involved in formation of ionic edema, based on salutary effects of pre-ischemic administration of the cotransporter inhibitor, bumetanide (O'Donnell *et al*., 2004). However, this mechanism is said to require the participation of abluminal Na⁺/K⁺ ATPase to complete transcapillary flux of Na⁺ (O'Donnell *et al*., 2004). Thus, invoking this mechanism in the context of ischemia is problematic, although it may be relevant should energy restoration occur with timely reperfusion.

Data from the inventor's laboratory implicate SUR1-regulated NC_{Ca-ATP} channels in formation of ionic edema (FIG. 3). Post-ischemic block of the channel by low-dose glibenclamide reduces edema by half (Simard *et al*., 2006). Involvement of NC_{Ca-ATP} channels would imply that formation of ionic edema does not proceed by co-opting existing membrane proteins, but requires instead the expression of new protein by endothelial cells of ischemic but perfused capillaries.

A mechanism involving Na⁺-conducting channels in transcapillary flux of Na⁺ represents a description of cytotoxic edema of endothelial cells. Channels on the luminal side contribute to cytotoxic edema of endothelial cells, providing an influx pathway for Na⁺, whereas channels on the abluminal side act to relieve this cytotoxic edema by providing an efflux pathway for Na⁺ down its concentration gradient from the cell into the extracellular space. Obviously, this relief mechanism completes the pathway for transcapillary flux of Na⁺. As noted previously, Cl⁻ and H₂O follow *via* their own respective channels, completing the process of formation of ionic edema. Although Cl⁻ channels are present (Nilius and Droogmans, 2001), expression of aquaporin channels by endothelium *in situ* remains to be clarified, with aquaporin-1 but not aquaporin-4 possibly playing a role in ischemia (Dolman *et al*., 2005).

In this stage of ionic edema, BBB integrity is maintained, capillary tight junctions are preserved, and macromolecules are excluded from brain parenchyma. Thus, the driving force for formation of edema is determined only by osmotic pressure gradients, with hydrostatic pressure gradients being essentially irrelevant (FIG. 2).

### 2. Second phase - formation of vasogenic edema

The second phase of endothelial dysfunction is characterized by "breakdown" of the BBB, with leakage of plasma proteins into extracellular space (FIGS. 2 and 5). Macromolecules such as albumin, IgG and dextran, to which the BBB is normally impermeable, now pass readily across the endothelial barrier.

Vasogenic edema may be considered an ultrafiltrate of blood (Vorbrodt *et al.,* 1985), suggesting that the permeability pore is now quite large. The permeability pore that allows passage of larger molecules across the BBB has not been uniquely identified, and may have contributions from more than one mechanism. Any physical disruption of the capillary must be relatively limited, however, to account for egress of a proteinacious ultratrafiltrate without passage of erythrocytes.

Several mechanisms have been proposed to account for changes in permeability that gives rise to vasogenic edema, including reverse pinocytosis (Castejon *et al*., 1984), disruption of Ca²⁺ signaling (Brown and Davis, 2002), actin polymerization-dependent endothelial cell rounding or retraction with formation of inter-endothelial gaps, uncoupling of tight junctions, and enzymatic degradation of basement membrane. Formation of inter-endothelial gaps is observed with many inflammatory mediators (Ahmmed and Malik, 2005), including mediators up-regulated in cerebral ischemia such as thrombin (Satpathy *et al*., 2004). Thrombin-induced endothelial cell retraction may account for vasogenic edema associated not only with focal ischemia but also with intracerebral hematoma (Lee *et al*., 1996; Hua *et al*., 2003). Uncoupling of endothelial tight junctions is observed following up-regulation of vascular endothelial growth factor (VEGF), which increases hydraulic conductivity in isolated perfused microvessels, increases vascular permeability and promotes formation of edema (Weis and Cheresh, 2005). Antagonism of VEGF reduces edema associated with post-ischemia reperfusion (Van *et al*., 1999). Degradation of basement membrane required for structural integrity of capillaries is observed with enzymes that are up-regulated in cerebral ischemia, especially the matrix metalloproteinases (MMP), MMP-9 (gelatinase B) and MMP-2 (gelatinase A) (FIG. 2) (Asahi *et al*., 2001; Asahi *et al*., 2000; Mun-Bryce and rosenberg, 1998; Fukuda *et al*., 2004). Ischemia activates latent MMPs and causes *de novo* synthesis and release of MMPs (Asahi *et al*., 2001; Romanic *et al*., 1998; Kolev *et al*., 2003). MMP inhibitors reduce ischemia/reperfusion-related brain edema(Lapchak *et al*., 2000; Pfefferkorn and Rosenberg, 2003). Other proteins that are up-regulated and whose function results in degradation of the BBB include nitric oxide synthase (NOS), either iNOS (Iadecola *et al*., 1996) or nNOS (Sharma *et al*., 2000). Notably, these various molecular mechanisms establish the specific embodiment that constitutively expressed participants play only a limited role, and up-regulation of a family of proteins that alter BBB permeability is the norm.

Once BBB integrity is lost, capillaries behave like "fenestrated" capillaries, and both the hydrostatic and osmotic pressure gradients must be considered to understand edema formation (FIG. 2). Determinants of hydrostatic pressure, including systemic blood pressure and intracranial pressure, now assume an important role. Determinants of osmotic pressure now consist of all osmotically active molecules, including Na⁺ and macromolecules. There are implications regarding clinical management: (i) systemic blood pressure must be sufficient to perfuse the brain, but excess pressure will promote edema formation (Kogure *et al*., 1981); (ii) intracranial pressure, which determines tissue pressure, must be lowered to appropriate levels, but lowering it too much will promote edema formation. Optimization of parameters to achieve these conflicting goals is difficult. Treatments generally include use of osmotically active agents such as mannitol, but their effects may only be temporizing.

These concepts shed light on reasons for mixed outcomes following decompressive craniectomy (Kilincer *et al*., 2005; Mori *et al*., 2004), a procedure that abruptly lowers tissue pressure. In contrast to the stage of ionic edema, when hydrostatic pressure and therefore tissue pressure are unimportant for edema formation, in the stage of vasogenic edema, tissue pressure is a critical determinant of edema formation. Decompressive craniectomy may be safe if performed early, during the stage of ionic edema when the BBB is intact, as it may aid in restoring reperfusion by reducing intracranial pressure. By contrast, decompressive craniectomy performed later, during the stage of vasogenic edema, will decrease tissue pressure, drive formation of vasogenic edema, and thus may have an unintended deleterious effect. Brain imaging may guide the timing of treatment based on detection of these stages. Diffusion restriction on MRI correlates with the cytotoxic stage, while early hypodensity prior to mass effect on CT scan may be useful to assess ionic versus vasogenic edema prior to decompressive craniectomy (Knight *et al*., 1998; Latour *et al*., 2004).

### 3. Third phase - hemorrhagic conversion

The third phase of endothelial dysfunction is marked by catastrophic failure of capillary integrity, during which all constituents of blood, including erythrocytes, extravasate into brain parenchyma (FIGS. 5 and 6). Up to 30-40% of ischemic strokes undergo spontaneous hemorrhagic conversion, a complication that is more prevalent and more severe with use of thrombolytic stroke therapy (Asahi *et al*., 2000; Jaillard *et al*., 1999; Larrue *et al*., 1997). Hemorrhagic conversion, the transformation of a bland infarct into a hemorrhagic infarct after restoration of circulation, accounts for a major cause of early mortality in acute-stroke patients, ranging from 26-154 extra deaths per 1000 patients (Hacke *et al*., 1995; Hacke *et al*., 1998; Multicentre Acute Stroke Trial, 1995; National Institute of Neurological Disorders and Stroke rt-PA Stroke Study Group, 1995; Donnan *et al*., 1996.

Prolonged ischemia, aggravated by reperfusion, causes initial dysfunction and later death of capillary endothelial cells (del Zoppo *et al*., 1998; Hamann*et al*., 1999; Lee and Lo, 2004). As this process evolves, the BBB is increasingly compromised, capillaries become leaky, and eventually they lose their physical integrity. In the end, capillaries can no longer contain circulating blood, resulting in formation of petechial hemorrhages - hemorrhagic conversion. The close connection between BBB compromise and hemorrhagic conversion is supported by both animal (Knight *et al*., 1998) and human studies (Latour *et al*., 2004; Warach and Latour, 2004; NINDS t-PA Stroke Study Group, 1997) that predict hemorrhagic conversion following thrombolytic therapy based on pre-existing BBB dysfunction manifested either as gadolinium enhancement or hypodensity on computed tomographic imaging.

Hemorrhagic conversion is probably a multifactorial phenomenon due to reperfusion injury and oxidative stress. Mechanisms may include plasmin-generated laminin degradation, endothelial cell activation, transmigration of leukocytes through the vessel wall and other processes (Hamann *et al*., 1999; Wang and Lo, 2003). Factors important during the phase of vasogenic edema also participate. Exogenous VEGF administered intravascularly early following reperfusion aggravates hemorrhagic transformation (Abumiya *et al*., 2005). Dysregulation of extracellular proteolysis plays a key role in hemorrhagic transformation, with MMPs being critical participants (Fukuda *et al*., 2004; Wang and Lo, 2003; Heo *et al.,* 1999; Sumii and Lo, 2002). As with vasogenic edema, inhibition of BBB proteolysis reduces hemorrhagic conversion with reperfusion (Lapchak *et al*., 2000; Pfefferkorn and Rosenberg, 2003). Finally, oncotic death of endothelial cells, mediated by SUR1-regulated NC_{Ca-ATP} channels, would also be expected to give rise to hemorrhagic conversion (FIGS. 5 and 6). Additional research will be required to determine the relative contribution of these various mechanisms, and to uncover new ones likely involved.

As regards driving force, everything noted above for the "fenestrated capillary" associated with vasogenic edema holds in this phase as well. Theoretically, adding blood into the parenchyma and thereby increasing tissue pressure may reduce the hydrostatic driving force, but it does so at an untenable cost to the organ, adding mass that contributes to increased intracranial pressure, adding the exquisitely toxic oxidant, hemoglobin, and inciting a robust inflammatory response, all of which contribute adversely to outcome (Rosenberg, 2002; Zheng and Yenari, 2004; Price *et al.,* 2003). Implications for clinical management are similar to those for the previous stage, but optimization of parameters to achieve the conflicting goals is now appreciably more difficult.

### G. Energy considerations

The conceptualization of edema formation depicted here is grounded on physiological principals originally enunciated over a century ago. The power of this conceptualization lies in its ability to explain massive fluxes of ions and water into brain parenchyma despite the severe energy constraints typically encountered with ischemia. During formation of ionic edema, movements of ions and water occur by secondary active transport mechanisms, powered by concentration gradients originally formed by exclusion of Na⁺ from neurons and astrocytes. During formation of vasogenic edema as well as during hemorrhagic conversion, movements of plasma and blood into parenchyma are driven by hydrostatic pressure generated by the heart. Thus, vast quantities of ions, macromolecules, water and blood can move into the parenchyma with no new energy expenditure by the brain.

On the other hand, this conceptualization requires new protein synthesis induced by ischemia in order to alter permeability of the BBB. One important example is aquaporin 4 (AQP4), now strongly implicated in ischemia-induced edema (Badaut *et al*., 2002; Taniguchi *et al*., 2000). As for the SUR1-regulated NC_{Ca-ATP} channel, which is believed to be integral to formation of ionic edema, the need for protein synthesis has been shown at least for the SUR1 regulatory subunit of this channel, which is transcriptionally up-regulated in ischemia (Simard *et al*., 2006). In addition, the need for protein synthesis is true for prothrombin (Riek-Burchardt *et al*., 2002; Striggow *et al*., 2001), MMP-9 (Asahi *et al*., 2001; Asahi *et al*., 2000; Planas *et al*., 2000). VEGF (Croll and Wiegand, 2001) and iNOS, which play important roles in vasogenic edema and hemorrhagic conversion. New protein synthesis requires what is presumably a limited, perhaps "one-time" energy expenditure - what may ultimately be the last such expenditure on the way to self destruction of capillaries. Notably, the burden for new protein synthesis is left largely, though not exclusively, to endothelial cells in capillaries that are still perfused, and thus most likely to maintain a positive energy balance the longest in the face an ischemic insult.

### H. Transcriptional program

What links the various proteins, newly synthesized by ischemic endothelium, that are tied to progressive capillary dysfunction? Because the 3 phases of capillary dysfunction arise from a severe hypoxic insult, with or without free radicals generated upon reperfusion, synthesis of these proteins must be regulated by a transcriptional program involving hypoxia- or redox-sensitive transcription factors such as activator protein-1 (AP-1) (dimers of Fos, Jun and related oncoproteins that activate immediate early genes (IEGs) (Sng *et al*., 2004)), hypoxia inducible factor-1 (HIF-1), Sp-1 and nuclear factor-κB (NF-κB). Each of these factors is activated by focal cerebral ischemia (Simard *et al.,* 2006; Kogure and Kato, 1993; Salminene *et al.,* 1995; Han *et al*., 2003; Matrone *et al*., 2004; Schneider *et al.,* 1999; Hermann*et al*., 2005). HIF is activated when oxygen tension falls below 5% (40 mmHg), and is progressively activated with a decrease in oxygen tension down to 0.2-0.1% (1.6-0.8 mmHg), close to anoxia (Pouyssegur *et al*., 2006). Analysis of the promoter regions of the various proteins reveals the presence of one or more putative binding sites for each of these transcription factors (FIG. 7). Definitive evidence for involvement of all 4 factors in transcriptional regulation of proteins involved in cerebral edema remains to be obtained, but some pieces of the matrix have been filled in, including for AQP4 (AP-1, Sp-1) (Umenishi and Verkman, 1998), SUR1 (Sp-1) (Simard *et al.,* 2006; Ashfield and Ashcroft, 1998; Hernandez-Sanchez *et al.,* 1999), prothrombin (Sp-1) (Ceelie *et al.,* 2003), VEGF (Sp-1, HIF-1, AP-1) (Hasegawa *et al.,* 2006; Pore *et al.,* 2006; Nordal *et al.,* 2004; Sainikow *et al.,* 2002) and MMP-9 (NF-κB) (Kolev *et al.,* 2003; Bond *et al.,* 2001).

Other hypoxia- or redox-activated transcription factors that are involved may be determined by standard methods in the art.. Nevertheless, the functional grouping of these 4 factors affirms the concept of a transcriptional program which, when unleashed, initiates a sequential dynamic alteration in BBB characteristics that can lead to demise of the organ and ultimately, demise of the organism.

### IX. Combinatorial Therapeutic Compositions

The present invention includes a combinatorial therapeutic composition comprising an antagonist of the NC_{Ca-ATP} channel which is an antagonist of TRPM4 and another therapeutic compound, such as a cation channel blocker and/or an antagonist of a specific molecule, such as VEGF, MMP, NOS, thrombin, and so forth.

### A. Inhibitors of NC_{Ca-ATP} Channel

According to a specific embodiment of the present invention, the administration of effective amounts of the active compound can block the channel, which if it remained open would lead to neural cell swelling and cell death. The inhibitors of the channel inhibit TRPM4. Examples of suitable SUR1 antagonists include, but are not limited to glibenclamide, tolbutamide, repaglinide, nateglinide, meglitinide, midaglizole, LY397364, LY3 89382, gliclazide, glimepiride, MgADP, and combinations thereof. In a preferred embodiment the SUR1 antagonists is selected from the group consisting of glibenclamide and tolbutamide. Still other therapeutic "strategies" for preventing neural cell swelling and cell death can be adopted including, but not limited to methods that maintain the neural cell in a polarized state and methods that prevent strong depolarization.

Examples of antagonists of the present invention may encompass respective antagonists identified in US Application Publication No. 20030215889. One of skill in the art is aware that the NC_{Ca-ATP} channel is comprised of at least two subunits: the regulatory subunit, SUR1, and the pore-forming subunit, TRPM4.

### 1. Exemplary SUR1 Inhibitors

In certain embodiments, antagonists to sulfonylurea receptor-1 (SUR1) are suitable for blocking the channel. Examples of suitable SUR1 antagonists include, but are not limited to mitiglinide, iptakalim, endosulfines (alpha- and/or beta-endosulfine, for example; Heron *et al*., 1998), glibenclamide, tolbutamide, repaglinide, nateglinide, meglitinide, midaglizole, LY397364, LY389382, glyclazide, glimepiride, estrogen, estrogen related-compounds estrogen related-compounds (estradiol, estrone, estriol, genistein, non-steroidal estrogen (*e*.*g*., diethystilbestrol), phytoestrogen (*e*.*g*., coumestrol), zearalenone, *etc*.) and combinations thereof. In a preferred embodiment the SUR1 antagonist is selected from the group consisting of glibenclamide and tolbutamide. Yet further, another antagonist can be MgADP. Other antagonist include blockers of K_{ATP} channels, for example, but not limited to tolbutamide, glyburide (1[p-2[5-chloro-O-anisamido)ethyl] phenyl] sulfonyl] -3-cyclohexyl-3-urea); chlopropamide (1-[[(p-chlorophenyl)sulfonyl] -3-propylurea; glipizide (1-cyclohexyl-3[[p-[2(5-methylpyrazine carboxamido) ethyl] phenyl] sulfonyl] urea); or tolazamide(benzenesulfonamide-N-[[(hexahydro-1H-azepin-1yl)amino] carbonyl] -4-methyl). Exemplary blockers include pinokalant (LOE 908 MS); rimonabant (SR141716A); fenamates (flufenamic acid, mefenamic acid, meclofenamic acid, and niflumic acid, for example); SKF 96365 (1-(beta-[3-(4-methoxy-phenyl)propoxy]-4-methoxyphenethyl)-1H- imidazole hydrochloride); meclofenamic acid; and/or a combination or mixture thereof.

### 2. Modulators of SUR1 Transcription and/or Translation

In certain embodiments, the modulator can comprise a compound (protein, nucleic acid, siRNA, *etc*.) that modulates transcription and/or translation of SUR1 (regulatory subunit) and/or the molecular entities that comprise the pore-forming subunit.

### 3. Transcription Factors

Transcription factors are regulatory proteins that binds to a specific DNA sequence (e.g., promoters and enhancers) and regulate transcription of an encoding DNA region. Thus, transcription factors can be used to modulate the expression of SUR1. Typically, a transcription factor comprises a binding domain that binds to DNA (a DNA-binding domain) and a regulatory domain that controls transcription. Where a regulatory domain activates transcription, that regulatory domain is designated an activation domain. Where that regulatory domain inhibits transcription, that regulatory domain is designated a repression domain. More specifically, transcription factors such as Sp1, HIF1α, and NFκB can be used to modulate expression of SUR1.

In particular embodiments of the invention, a transcription factor may be targeted by a composition of the invention.
The transcription factor may be targeted with an antagonist of the invention, including siRNA to downregulate the transcription factor. Such antagonists can be identified by standard methods in the art, and in particular embodiments the antagonist is employed for treatment and or prevention of an individual in need thereof. In an additional embodiment, the antagonist is employed in conjunction with an additional compound, such as a composition that modulates the NC_{Ca-ATP} channel of the invention. For example, the antagonist may be used in combination with an inhibitor of the channel of the invention.

### 4. Antisense and Ribozymes

An antisense molecule that binds to a translational or transcriptional start site, or splice junctions, are ideal inhibitors. Antisense, ribozyme, and double-stranded RNA molecules target a particular sequence to achieve a reduction or elimination of a particular polypeptide, such as TRPM4. Thus, it is contemplated that antisense, ribozyme, and double-stranded RNA, and RNA interference molecules are constructed and used to modulate TRPM4 expression.

### 5. Antisense Molecules

Antisense methodology takes advantage of the fact that nucleic acids tend to pair with complementary sequences. By complementary, it is meant that polynucleotides are those which are capable of base-pairing according to the standard Watson-Crick complementarity rules. That is, the larger purines will base pair with the smaller pyrimidines to form combinations of guanine paired with cytosine (G:C) and adenine paired with either thymine (A:T) in the case of DNA, or adenine paired with uracil (A:U) in the case of RNA. Inclusion of less common bases such as inosine, 5-methylcytosine, 6-methyladenine, hypoxanthine and others in hybridizing sequences does not interfere with pairing.

Targeting double-stranded (ds) DNA with polynucleotides leads to triple-helix formation; targeting RNA will lead to double-helix formation. Antisense polynucleotides, when introduced into a target cell, specifically bind to their target polynucleotide and interfere with transcription, RNA processing, transport, translation and/or stability. Antisense RNA constructs, or DNA encoding such antisense RNAs, are employed to inhibit gene transcription or translation or both within a host cell, either *in vitro* or *in vivo*, such as within a host animal, including a human subject.

Antisense constructs are designed to bind to the promoter and other control regions, exons, introns or even exon-intron boundaries of a gene. It is contemplated that the most effective antisense constructs may include regions complementary to intron/exon splice junctions. Thus, antisense constructs with complementarity to regions within 50-200 bases of an intron-exon splice junction are used. It has been observed that some exon sequences can be included in the construct without seriously affecting the target selectivity thereof. The amount of exonic material included will vary depending on the particular exon and intron sequences used. One can readily test whether too much exon DNA is included simply by testing the constructs *in vitro* to determine whether normal cellular function is affected or whether the expression of related genes having complementary sequences is affected.

It is advantageous to combine portions of genomic DNA with cDNA or synthetic sequences to generate specific constructs. For example, where an intron is desired in the ultimate construct, a genomic clone will need to be used. The cDNA or a synthesized polynucleotide may provide more convenient restriction sites for the remaining portion of the construct and, therefore, would be used for the rest of the sequence.

### 6. RNA Interference

It is also contemplated in the present invention that double-stranded RNA is used as an interference molecule, e.g., RNA interference (RNAi). RNA interference is used to "knock down" or inhibit a particular gene of interest by simply injecting, bathing or feeding to the organism of interest the double-stranded RNA molecule. This technique selectively "knock down" gene function without requiring transfection or recombinant techniques (Giet, 2001; Hammond, 2001; Stein P, *et al*., 2002; Svoboda P*, et al*., 2001; Svoboda P, *et al*., 2000).

Another type of RNAi is often referred to as small interfering RNA (siRNA), which may also be utilized to inhibit TRPM4. A siRNA may comprise a double stranded structure or a single stranded structure, the sequence of which is "substantially identical" to at least a portion of the target gene (See WO 04/046320). "Identity," as known in the art, is the relationship between two or more polynucleotide (or polypeptide) sequences, as determined by comparing the sequences. In the art, identity also means the degree of sequence relatedness between polynucleotide sequences, as determined by the match of the order of nucleotides between such sequences. Identity can be readily calculated. See, for example: Computational Molecular Biology, Lesk, A.M., ed. Oxford University Press, New York, 1988; Biocomputing: Informatics and Genome Projects, Smith, D.W., ea., Academic Press, New York, 1993, and the methods disclosed in WO 99/32619, WO 01/68836, WO 00/44914, and WO 01/36646. While a number of methods exist for measuring identity between two nucleotide sequences, the term is well known in the art. Methods for determining identity are typically designed to produce the greatest degree of matching of nucleotide sequence and are also typically embodied in computer programs. Such programs are readily available to those in the relevant art. For example, the GCG program package (Devereux *et al*.), BLASTP, BLASTN, and FASTA (Atschul *et al*.,) and CLUSTAL (Higgins *et al*., 1992; Thompson, *et al*., 1994).

Thus, siRNA contains a nucleotide sequence that is essentially identical to at least a portion of the target gene. One of skill in the art is aware that the nucleic acid sequences for SUR1 are readily available in GenBank®, for example, GenBank® accession L40624 (rat) or AF087138 (human) (SEQ ID NO:5). Preferably, the siRNA contains a nucleotide sequence that is completely identical to at least a portion of the target gene. Of course, when comparing an RNA sequence to a DNA sequence, an "identical" RNA sequence will contain ribonucleotides where the DNA sequence contains deoxyribonucleotides, and further that the RNA sequence will typically contain a uracil at positions where the DNA sequence contains thymidine.

One of skill in the art will appreciate that two polynucleotides of different lengths may be compared over the entire length of the longer fragment. Alternatively, small regions may be compared. Normally sequences of the same length are compared for a final estimation of their utility in the practice of the present invention. It is preferred that there be 100% sequence identity between the dsRNA for use as siRNA and at least 15 contiguous nucleotides of the target gene (e.g., SUR1), although a dsRNA having 70%, 75%, 80%, 85%, 90%, or 95% or greater may also be used in the present invention. A siRNA that is essentially identical to a least a portion of the target gene may also be a dsRNA wherein one of the two complementary strands (or, in the case of a self-complementary RNA, one of the two self-complementary portions) is either identical to the sequence of that portion or the target gene or contains one or more insertions, deletions or single point mutations relative to the nucleotide sequence of that portion of the target gene. siRNA technology thus has the property of being able to tolerate sequence variations that might be expected to result from genetic mutation, strain polymorphism, or evolutionary divergence.

There are several methods for preparing siRNA, such as chemical synthesis, *in vitro* transcription, siRNA expression vectors, and PCR expression cassettes. Irrespective of which method one uses, the first step in designing an siRNA molecule is to choose the siRNA target site, which can be any site in the target gene. In certain embodiments, one of skill in the art may manually select the target selecting region of the gene, which may be an ORF (open reading frame) as the target selecting region and may preferably be 50-100 nucleotides downstream of the "ATG" start codon. However, there are several readily available programs available to assist with the design of siRNA molecules, for example siRNA Target Designer by Promega, siRNA Target Finder by GenScript Corp., siRNA Retriever Program by Imgenex Corp., EMBOSS siRNA algorithm, siRNA program by Qiagen, Ambion siRNA predictor, Ambion siRNA predictor, Whitehead siRNA prediction, and Sfold. Thus, it is envisioned that any of the above programs may be utilized to produce siRNA molecules that can be used in the present invention.

### 7. Ribozymes

Ribozymes are RNA-protein complexes that cleave nucleic acids in a site-specific fashion. Ribozymes have specific catalytic domains that possess endonuclease activity (Kim and Cech, 1987; Forster and Symons, 1987). For example, a large number of ribozymes accelerate phosphoester transfer reactions with a high degree of specificity, often cleaving only one of several phosphoesters in an oligonucleotide substrate (Cech *et al*., 1981; Reinhold-Hurek and Shub, 1992). This specificity has been attributed to the requirement that the substrate bind *via* specific base-pairing interactions to the internal guide sequence ("IGS") of the ribozyme, prior to chemical reaction.

Ribozyme catalysis has primarily been observed as part of sequence specific cleavage/ligation reactions involving nucleic acids (Joyce, 1989; Cech *et al*., 1981). For example, U.S. Patent 5,354,855 reports that certain ribozymes can act as endonucleases with a sequence specificity greater than that of known ribonucleases and approaching that of the DNA restriction enzymes. Thus, sequence-specific ribozyme-mediated inhibition of gene expression is particularly suited to therapeutic applications (Scanlon *et al*., 1991; Sarver *et al*., 1990; Sioud *et al*., 1992). Most of this work involved the modification of a target mRNA, based on a specific mutant codon that is cleaved by a specific ribozyme. In light of the information included herein and the knowledge of one of ordinary skill in the art, the preparation and use of additional ribozymes that are specifically targeted to a given gene will now be straightforward.

Other suitable ribozymes include sequences from RNase P with RNA cleavage activity (Yuan *et al*., 1992; Yuan and Altman, 1994), hairpin ribozyme structures (Berzal-Herranz *et al*., 1992; Chowrira *et al*., 1993) and hepatitis δ virus based ribozymes (Perrotta and Been, 1992). The general design and optimization of ribozyme directed RNA cleavage activity has been discussed in detail (Haseloff and Gerlach, 1988; Symons, 1992; Chowrira, *et al*., 1994; and Thompson, *et al.,* 1995).

The other variable on ribozyme design is the selection of a cleavage site on a given target RNA. Ribozymes are targeted to a given sequence by virtue of annealing to a site by complimentary base pair interactions. Two stretches of homology are required for this targeting. These stretches of homologous sequences flank the catalytic ribozyme structure defined above. Each stretch of homologous sequence can vary in length from 7 to 15 nucleotides. The only requirement for defining the homologous sequences is that, on the target RNA, they are separated by a specific sequence which is the cleavage site. For hammerhead ribozymes, the cleavage site is a dinucleotide sequence on the target RNA, uracil (U) followed by either an adenine, cytosine or uracil (A,C or U; Perriman, *et al.,* 1992; Thompson, *et al.,* 1995). The frequency of this dinucleotide occurring in any given RNA is statistically 3 out of 16.

Designing and testing ribozymes for efficient cleavage of a target RNA is a process well known to those skilled in the art. Examples of scientific methods for designing and testing ribozymes are described by Chowrira *et al*. (1994) and Lieber and Strauss (1995). The identification of operative and preferred sequences for use in SUR1 targeted ribozymes is simply a matter of preparing and testing a given sequence, and is a routinely practiced screening method known to those of skill in the art.

### 8. Inhibition of post-translational assembly and trafficking

Following expression of individual regulatory and pore-forming subunit proteins of the channel, and in particular aspects of the invention, these proteins are modified by glycosylation in the Golgi apparatus of the cell, assembled into functional heteromultimers that comprise the channel, and then transported to the plasmalemmal membrane where they are inserted to form functional channels. The last of these processes is referred to as "trafficking".

In specific embodiments of the invention, molecules that bind to any of the constituent proteins interfere with post-translational assembly and trafficking, and thereby interfere with expression of functional channels. One such example is with glibenclamide binding to SUR1 subunits. In additional embodiments, glibenclamide, which binds with subnanomolar affinity to SUR1, interferes with post-translational assembly and trafficking required for functional channel expresson.

### B. Cation Channel Blockers

In some embodiments of the present invention, the combinatorial therapeutic composition comprises one or more cation channel blockers. Exemplary blockers include pinokalant (LOE 908 MS); rimonabant (SR141716A); fenamates (flufenamic acid, mefenamic acid, meclofenamic acid, niflumic acid, for example); SKF 96365 (1-(beta-[3-(4-methoxy-phenyl)propoxy]-4-methoxyphenethyl)-1H- imidazole hydrochloride); and/or a combination or mixture thereof.

In specific embodiments, Ca²⁺ channel blockers are employed, such as, for example, Amlodipine besylate, (R)-(+)-Bay K, Cilnidipine, ω-Conotoxin GVIA, ω-Conotoxin MVIIC, Diltiazem hydrochloride, Gabapentin, Isradipine, Loperamide hydrochloride, Mibefradil dihydrochloride, Nifedipine, (R)-(-)-Niguldipine hydrochloride, (S)-(+)-Niguldipine hydrochloride, Nimodipine, Nitrendipine, NNC 55-0396 dihydrochloride, Ruthenium Red, SKF 96365 hydrochloride, SR 33805 oxalate, and/or Verapamil hydrochloride. The Ca²⁺ channel blockers may be L-type Ca²⁺ channel blockers, for example. In specific embodiments, the Ca channel blockers could be N-type calcium channel blockers (GVIA, MVIIA) and/or may be P-type calcium channel blockers or P/Q-type Ca channel blockers.

In specific embodiments, K+ channel blockers are employed, including, for example, Apamin, Charybdotoxin, Dequalinium dichloride, Iberiotoxin, Paxilline, UCL 1684, Tertiapin-Q, AM 92016 hydrochloride, Chromanol 293B, (-)-[3R,4S]-Chromanol 293B, CP 339818 hydrochloride, DPO-1, E-4031 dihydrochloride, KN-93, Linopirdine dihydrochloride, XE 991 dihydrochloride, 4-Aminopyridine, DMP 543, and/or YS-035 hydrochloride.

In other specific embodiments, Na⁺ channel blockers are employed, including, for example, Ambroxol hydrochloride, Amiloride hydrochloride, Flecainide acetate, Flunarizine dihydrochloride, Mexiletine hydrochloride, QX 222, QX 314 bromide, QX 314 chloride, Riluzole hydrochloride, Tetrodotoxin, and/or Vinpocetine.

Non-specific cation channel blockers may be utilized, such as Lamotrigine or Zonisamide, for example.

In additional embodiments, glutamate receptor blockers are employed, such as D-AP5, DL-AP5, L-AP5, D-AP7, DL-AP7, (R)-4-Carboxyphenylglycine, CGP 37849, CGP 39551, CGS 19755, (2R,3S)-Chlorpheg, Co 101244 hydrochloride, (R)-CPP, (RS)-CPP, D-CPP-ene, LY 235959, PMPA, PPDA, PPPA, Ro 04-5595 hydrochloride, Ro 25-6981 maleate, SDZ 220-040, SDZ 220-581, (±)-1-(1,2-Diphenylethyl)pipeidine maleate, IEM 1460, Loperamide hydrochloride, Memantine hydrochloride, (-)-MK 801 maleate, (+)-MK 801 maleate, N20C hydrochloride, Norketamine hydrochloride, Remacemide hydrochloride, ACBC, CGP 78608 hydrochloride, 7-Chlorokynurenic acid, CNQX, 5,7-Dichlorokynurenic acid, Felbamate, Gavestinel, (S)-(-)-HA-966, L-689,560, L-701,252, L-701,324, Arcaine sulfate, Eliprodil, N-(4-Hydroxyphenylacetyl)spermine, N-(4-Hydroxyphenylpropanoyl) spermine trihydrochloride, Ifenprodil hemitartrate, Synthalin sulfate, CFM-2, GYKI 52466 hydrochloride, IEM 1460, ZK 200775, NS 3763, UBP 296, UBP 301, UBP 302, CNQX, DNQX, Evans Blue tetrasodium salt, NBQX, SYM 2206, UBP 282, and/or ZK 200775, for example.

### C. Antagonists of Specific Molecules

Antagonists of specific molecules may be employed, for example, those related to endothelial dysfunction.

### 1. Antagonists of VEGF

Antagonists of VEGF may be employed. The antagonists may be synthetic or natural, and they may antagonize directly or indirectly. VEGF Trap_{R1R2} (Regeneron Pharmaceuticals, Inc.); Undersulfated, low-molecular-weight glycol-split heparin (Pisano *et al*., 2005); soluble NRP-1 (sNRP-1); Avastin (Bevacizumab); HuMV833; s-Flt-1, s-Flk-1; s-Flt-1/Flk-1; NM-3; and/or GFB 116.

### 2. Antagonists of MMP

Antagonists of any MMP may be employed. The antagonists may be synthetic or natural, and they may antagonize directly or indirectly. Exemplary antagonists of MMPs include at least (2R)-2-[(4-biphenylsulfonyl)amino]-3-phenylproprionic acid (compound 5a), an organic inhibitor of MMP-2/MMP-9 (Nyormoi *et al.,* 2003); broad-spectrum MMP antagonist GM-6001 (Galardy *et al.,* 1994;Graesser *et al.,* 1998); TIMP-1 and/or TIMP-2 (Rolli *et al.,* 2003); hydroxamate-based matrix metalloproteinase inhibitor (RS 132908) (Moore *et al.,* 1999); batimastat (Corbel *et al.,* 2001); those identified in United States Application 20060177448; and/or marimastat (Millar *et al*., 1998); peptide inhibitors that comprise HWGF (including CTTHWGFTLC; SEQ ID NO:6) (Koivunen *et al.,* 1999); and combinations thereof.

### 3. Antagonists of NOS

Antagonists of NOS may be employed. The antagonists may be synthetic or natural, and they may antagonize directly or indirectly. The antagonists may be antagonists of NOS I, NOS II, NOS III,or may be nonselective NOS antagonists. Exemplary antagonists include at least the following: aminoguanidine (AG); 2-amino-5,6-dihydro-6-methyl-4H-1,3 thiazine (AMT); S-ethylisothiourea (EIT) (Rairigh *et al*., 1998); asymmetric dimethylarginine (ADMA) (Vallance *et al*., 1992); N-nitro-L-arginine methylester (L-NAME) (Papapetropoulos *et al.,* 1997; Babaei *et al.,* 1998); nitro-L-arginine (L-NA) (Abman *et al,* 1990; Abman *et al*., 1991; Cornfield *et al.,* 1992; Fineman *et al*., 1994; McQueston *et al.,* 1993; Storme *et al*., 1999); the exemplary selective NOS II antagonists, aminoguanidine (AG) and N-(3-aminomethyl) benzylacetamidine dihydrochloride (1400W); N^{G}-monomethyl-L-arginine (L-NMMA); the exemplary selective NOS I antagonist, 7-nitroindazole (7-NINA), and a nonselective NOS antagonist, N-nitro-L-arginine (L-NNA), or a mixture or combination thereof.

### 4. Antagonists of Thrombin

Antagonists of thrombin may be employed. The antagonists may be synthetic or natural, and they may antagonize directly or indirectly. Exemplary thrombin antagonists include at least the following: ivalirudin (Kleiman *et al.,* 2002); hirudin (Hoffman *et al.,* 2000); SSR182289 (Duplantier *et al*., 2004); antithrombin III; thrombomodulin; Lepirudin (Refludan, a recombinant therapeutic hirudin); P-PACK II (d-Phenylalanyl-L-Phenylalanylarginine- chloromethyl ketone 2 HCl); Thromstop® (BNas-Gly-(pAM)Phe-Pip); Argatroban (Carr *et al*., 2003); and mixtures or combinations thereof.

### D. 'Others

Non-limiting examples of an additional pharmacological therapeutic agent that may be used in the present invention include an antacid, an antihyperlipoproteinemic agent, an antiarteriosclerotic agent, an anticholesterol agent, an antiinflammatory agent, an antithrombotic/fibrinolytic agent, anticoagulant, antiplatelet, vasodilator, and/or diuretics. Thromoblytics that are used can include, but are not limited to prourokinase, streptokinase, and tissue plasminogen activator (tPA) Anticholesterol agents include but are not limited to HMG-CoA Reductase inhibitors, cholesterol absorption inhibitors, bile acid sequestrants, nicotinic acid and derivatives thereof, fibric acid and derivatives thereof. HMG-CoA Reductase inhibitors include statins, for example, but not limited to atorvastatin calcium (Lipitor®), cerivastatin sodium (Baycol®), fluvastatin sodium (Lescol®), lovastatin (Advicor®), pravastatin sodium (Pravachol®), and simvastatin (Zocor®). Agents known to reduce the absorption of ingested cholesterol include, for example, Zetia®. Bile acid sequestrants include, but are not limited to cholestryramine, cholestipol and colesevalam. Other anticholesterol agents include fibric acids and derivatives thereof (e.g., gemfibrozil, fenofibrate and clofibrate); nicotinic acids and derivatives thereof (e.g., nician, lovastatin) and agents that extend the release of nicotinic acid, for example niaspan. Antiinflammatory agents include, but are not limited to non-sterodial anti-inflammatory agents (e.g., naproxen, ibuprofen, celeoxib) and sterodial anti-inflammatory agents (e.g., glucocorticoids). Anticoagulants include, but are not limited to heparin, warfarin, and coumadin. Antiplatelets include, but are not limited to aspirin, and aspirin related-compounds, for example acetaminophen. Diuretics include, but are not limited to such as furosemide (Lasix®), bumetanide (Bumex®), torsemide (Demadex®), thiazide & thiazide-like diuretics (e.g., chlorothiazide (Diuril®) and hydrochlorothiazide (Esidrix®), benzthiazide, cyclothiazide, indapamide, chlorthalidone, bendroflumethizide, metolazone), amiloride, triamterene, and spironolacton. Vasodilators include, but are not limited to nitroglycerin.

Thus, in certain embodiments, the present invention comprises co-administration of an antagonist of the NC_{Ca-ATP} channel which is an antagonist of TRPM4 with a thrombolytic agent. Co-administration of these two compounds will increase the therapeutic window of the thrombolytic agent. Examples of suitable thrombolytic agents that can be employed in the methods and pharmaceutical compositions of this invention are prourokinase, streptokinase, and tissue plasminogen activator (tPA).

In certain embodiments, the present invention comprises co-administration of an antagonist of the NC_{Ca-ATP} channel which is an antagonist of TRPM4 with glucose or related carbohydrate or glucagon to maintain appropriate levels of serum glucose. Appropriate levels of blood glucose are within the range of about 60 mmol/l to about 150 mmol/liter. Thus, glucose or glucagon or a related carbohydrate or glucagon is administered in combination to maintain the serum glucose within this range.

To inhibit hemorrhagic conversion, reduce cell swelling, *etc*., using the methods and compositions of the present invention, one would generally contact a cell with antagonist of NC_{Ca-ATP} channel which is an antagonist of TRPM4 in combination with an additional therapeutic agent, such as tPA, aspirin, statins, diuretics, warfarin, coumadin, mannitol, *etc*. These compositions would be provided in a combined amount effective to inhibit hemorrhagic conversion, cell swelling and cerebral edema. This process may involve contacting the cells with agonist of NC_{CA-ATP} channel or related-compounds thereof in combination with an additional therapeutic agent or factor(s) at the same time. This may be achieved by contacting the cell with a single composition or pharmacological formulation that includes both agents, or by contacting the cell with two distinct compositions or formulations, at the same time, wherein one composition includes an antagonist of the NC_{Ca-ATP} channel which is an antagonist of TRPM4 or derivatives thereof and the other includes the additional agent.

Further embodiments include treatment with SUR1 antagonist, thrombolytic agent, and glucose. Glucose or glucagon administration may be at the time of treatment with SUR1 antagonist, or may follow treatment with SUR1 antagonist (e.g., at 15 minutes after treatment with SUR1 antagonist, or at one half hour after treatment with SUR1 antagonist, or at one hour after treatment with SUR1 antagonist, or at two hours after treatment with SUR1 antagonist, or at three hours after treatment with SUR1 antagonist). Glucose or glucagon administration may be by intravenous, or intraperitoneal, or other suitable route and means of delivery. Additional glucose or glucagon allows administration of higher doses of SUR1 antagonist than might otherwise be possible. Treatment with glucose or glucagon in conjunction with treatment with SUR1 antagonist (at the same time as treatment with SUR1 antagonist, or at a later time after treatment with SUR1 antagonist) may further enlarge the time window after stroke, trauma, or other brain injury when thrombolytic treatment may be initiated.

Yet further, the combination of the antagonist and tPA results in a decrease or prevention of hemorrhagic conversion following reperfusion. Hemorrhagic conversion is the transformation of a bland infarct into a hemorrhagic infarct after restoration of circulation. It is generally accepted that these complications of stroke and of reperfusion are attributable to capillary endothelial cell dysfunction that worsens as ischemia progresses. Thus, the present invention is protective of the endothelial cell dysfunction that occurs as a result of an ischemic event.

### X. Exemplary Pharmaceutical Formulations and Methods of Use

In particular embodiments, the invention employs pharmaceutical formulations comprising a singular or combinatorial composition that inhibits a NC_{Ca-ATP} channel which is an antagonist of TRPM4.

### A. Exemplary Compositions of the Present Invention

The present invention also contemplates therapeutic methods employing compositions comprising the active substances disclosed herein. Preferably, these compositions include pharmaceutical compositions comprising a therapeutically effective amount of one or more of the active compounds or substances along with a pharmaceutically acceptable carrier.

As used herein, the term "pharmaceutically acceptable" carrier means a non-toxic, inert solid, semi-solid liquid filler, diluent, encapsulating material, formulation auxiliary of any type, or simply a sterile aqueous medium, such as saline. Some examples of the materials that can serve as pharmaceutically acceptable carriers are sugars, such as lactose, glucose and sucrose, starches such as corn starch and potato starch, cellulose and its derivatives such as sodium carboxymethyl cellulose, ethyl cellulose and cellulose acetate; powdered tragacanth; malt, gelatin, talc; excipients such as cocoa butter and suppository waxes; oils such as peanut oil, cottonseed oil, safflower oil, sesame oil, olive oil, corn oil and soybean oil; glycols, such as propylene glycol, polyols such as glycerin, sorbitol, mannitol and polyethylene glycol; esters such as ethyl oleate and ethyl laurate, agar; buffering agents such as magnesium hydroxide and aluminum hydroxide; alginic acid; pyrogen-free water; isotonic saline, Ringer's solution; ethyl alcohol and phosphate buffer solutions, as well as other non-toxic compatible substances used in pharmaceutical formulations.

Wetting agents, emulsifiers and lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. Examples of pharmaceutically acceptable antioxidants include, but are not limited to, water soluble antioxidants such as ascorbic acid, cysteine hydrochloride, sodium bisulfite, sodium metabisulfite, sodium sulfite, and the like; oil soluble antioxidants, such as ascorbyl palmitate, butylated hydroxyanisole (BHA), butylated hydroxytoluene (BHT), lecithin, propyl gallate, aloha-tocopherol and the like; and the metal chelating agents such as citric acid, ethylenediamine tetraacetic acid (EDTA), sorbitol, tartaric acid, phosphoric acid and the like.

In certain aspects of the invention, there is co-administration with one or more antacids, for example enteric coatings, time-release compositions, *etc*., that are effective to avoid stomach acid.

### B. Dose Determinations

By a "therapeutically effective amount" or simply "effective amount" of an active compound, such as glibenclamide or tolbutamide, is meant a sufficient amount of the compound to treat or alleviate the brain swelling at a reasonable benefit/risk ratio applicable to any medical treatment. It will be understood, however, that the total daily usage of the active compounds and compositions of the present invention will be decided by the attending physician within the scope of sound medical judgment. The specific therapeutically effective dose level for any particular patient will depend upon a variety of factors including the disorder being treated and the severity of the brain injury or ischemia; activity of the specific compound employed; the specific composition employed; the age, body weight, general health, sex and diet of the patient; the time of administration, route of administration, and rate of excretion of the specific compound employed; the duration of the treatment; drugs used in combination or coinciding with the specific compound employed; and like factors well known in the medical arts.

Toxicity and therapeutic efficacy of such compounds can be determined by standard pharmaceutical procedures in cell assays or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell based assays. A dose may be formulated in animal models to achieve a circulating plasma concentration range that includes the IC50 (*i*.*e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

The total daily dose of the active compounds of the present invention administered to a subject in single or in divided doses can be in amounts, for example, from 0.01 to 25 mg/kg body weight or more usually from 0.1 to 15 mg/kg body weight. Single dose compositions may contain such amounts or submultiples thereof to make up the daily dose. In general, treatment regimens according to the present invention comprise administration to a human or other mammal in need of such treatment from about 1 mg to about 1000 mg of the active substance(s) of this invention per day in multiple doses or in a single dose of from 1 mg, 5 mg, 10 mg, 100 mg, 500 mg or 1000 mg.

In certain situations, it may be important to maintain a fairly high concentration of the active agent in the blood stream of the patient relative to those in the animal models. A dose leading to such fairly high concentrations may include a dose that is similar or even several times greater than its use in other indications. For example, the typical anti-diabetic dose of oral or intravenous (IV) glibenclamide is about 1.25mg to about 20 mg per day; the typical anti-diabetic dose of oral or IV tolbutamide is about to 0.5 gm/day to about 3.0 gm/day; the typical anti-diabetic dose for oral gliclazide is about 30 mg/day to about 120 mg/day. Doses in these ranges or even larger doses may be required to block neural cell swelling and brain swelling e.g., about 0.5 mg/day to about 10 mg/day IV glibenclamide. Initial doses may be higher than later-administered doses, for example, in order to quickly obtain effective doses for rapid therapeutic effect, while lower, later doses may be at levels sufficient to maintain effective concentrations of the drug in body fluids and tissues. Intravenous doses (and any other administration route) designed to match the exposure, mean levels, peak levels, or any other feature or derivation of oral dosing, need to be adjusted with respect to the oral dose to adjust for the bioavailability of the oral dosage forms that can range from about 85% to about 100%.

For example, in one embodiment of the present invention directed to a method of preventing neuronal cell swelling in the brain of a subject by administering to the subject a formulation containing an effective amount of a compound that blocks the NC_{Ca-ATP} channel which is an antagonist of TRPM4 and a pharmaceutically acceptable carrier; such formulations may contain from about 0.1 to about 100 grams of tolbutamide or from about 0.5 to about 150 milligrams of glibenclamide. In another embodiment of the present invention directed to a method of alleviating the negative effects of traumatic brain injury or cerebral ischemia stemming from neural cell swelling in a subject by administering to the subject a formulation containing an effective amount of a compound that blocks the NC_{Ca-ATP} channel and a pharmaceutically acceptable carrier.

In situations of traumatic brain injury or cerebral ischemia (such as stroke), or cerebral hypoxia, it may be important to maintain a fairly high dose of the active agent to ensure delivery to the brain of the patient, particularly early in the treatment. Hence, at least initially, it may be important to keep the dose relatively high and/or at a substantially constant level for a given period of time, preferably, at least about six or more hours, more preferably, at least about twelve or more hours and, most preferably, at least about twenty-four or more hours. In situations of traumatic brain injury or cerebral ischemia (such as stroke), it may be important to maintain a fairly high dose of the active agent to ensure delivery to the brain of the patient, particularly early in the treatment.

When the method of the present invention is employed to treat conditions involving bleeding in the brain, such as traumatic brain injury or cerebral ischemia (such as stroke), delivery *via* the vascular system is available and the compound is not necessarily required to readily cross the blood-brain barrier.

### C. Formulations and Administration

The compounds of the present invention may be administered alone or in combination or in concurrent therapy with other agents which affect the central or peripheral nervous system, particularly selected areas of the brain.

Liquid dosage forms for oral administration may include pharmaceutically acceptable emulsions, microemulsions, solutions, suspensions, syrups and elixirs containing inert diluents commonly used in the art, such as water, isotonic solutions, or saline. Such compositions may also comprise adjuvants, such as wetting agents; emulsifying and suspending agents; sweetening, flavoring and perfuming agents.

In specific embodiments, including for oral administration, for example, there may be co-administration with antacids, H2 blockers, proton blockers and related compounds that neutralize or affect stomach pH, in order to enhance absorption of sulfonylureas.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulation can be sterilized, for example, by filtration through a bacteria-retaining filter, or by incorporating sterilizing agents in the form of sterile solid compositions, which can be dissolved or dispersed in sterile water or other sterile injectable medium just prior to use.

In order to prolong the effect of a drug, it is often desirable to slow the absorption of a drug from subcutaneous or intramuscular injection. The most common way to accomplish this is to inject a suspension of crystalline or amorphous material with poor water solubility. The rate of absorption of the drug becomes dependent on the rate of dissolution of the drug, which is, in turn, dependent on the physical state of the drug, for example, the crystal size and the crystalline form. Another approach to delaying absorption of a drug is to administer the drug as a solution or suspension in oil. Injectable depot forms can also be made by forming microcapsule matrices of drugs and biodegradable polymers, such as polylactide-polyglycoside. Depending on the ratio of drug to polymer and the composition of the polymer, the rate of drug release can be controlled. Examples of other biodegradable polymers include polyorthoesters and polyanhydrides. The depot injectables can also be made by entrapping the drug in liposomes or microemulsions, which are compatible with body tissues.

Suppositories for rectal administration of the drug can be prepared by mixing the drug with a suitable non-irritating excipient, such as cocoa butter and polyethylene glycol which are solid at ordinary temperature but liquid at the rectal temperature and will, therefore, melt in the rectum and release the drug.

Solid dosage forms for oral administration may include capsules, tablets, pills, powders, gelcaps and granules. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such as magnesium stearate and microcrystalline cellulose. In the case of capsules, tablets and pills, the dosage forms may also comprise buffering agents. Tablets and pills can additionally be prepared with enteric coatings and other release-controlling coatings.

Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferably, in a certain part of the intestinal tract, optionally in a delayed manner. Examples of embedding compositions which can be used include polymeric substances and waxes.

Dosage forms for topical or transdermal administration of a compound of this invention further include ointments, pastes, creams, lotions, gels, powders, solutions, sprays, inhalants or patches. Transdermal patches have the added advantage of providing controlled delivery of active compound to the body. Such dosage forms can be made by dissolving or dispersing the compound in the proper medium. Absorption enhancers can also be used to increase the flux of the compound across the skin. The rate can be controlled by either providing a rate controlling membrane or by dispersing the compound in a polymer matrix or gel. The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

The method of the present invention employs the compounds identified herein for both *in vitro* and *in vivo* applications. For *in vivo* applications, the invention compounds can be incorporated into a pharmaceutically acceptable formulation for administration. Those of skill in the art can readily determine suitable dosage levels when the invention compounds are so used.

As employed herein, the phrase "suitable dosage levels" refers to levels of compound sufficient to provide circulating concentrations high enough to effectively block the NC_{Ca-ATP} channel by antagonist of TRPM4 and prevent or reduce neural cell swelling *in vivo.*

In accordance with a particular embodiment, compositions comprising at least one SUR1 antagonist compound (as described above), and a pharmaceutically acceptable carrier are contemplated.

Exemplary pharmaceutically acceptable carriers include carriers suitable for oral, intravenous, subcutaneous, intramuscular, intracutaneous, and the like administration. Administration in the form of creams, lotions, tablets, dispersible powders, granules, syrups, elixirs, sterile aqueous or non-aqueous solutions, suspensions or emulsions, and the like, is contemplated.

For the preparation of oral liquids, suitable carriers include emulsions, solutions, suspensions, syrups, and the like, optionally containing additives such as wetting agents, emulsifying and suspending agents, sweetening, flavoring and perfuming agents, and the like.

For the preparation of fluids for parenteral administration, suitable carriers include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized, for example, by filtration through a bacteria-retaining filter, by incorporating sterilizing agents into the compositions, by irradiating the compositions, or by heating the compositions. They can also be manufactured in the form of sterile water, or some other sterile injectable medium immediately before use. The active compound is admixed under sterile conditions with a pharmaceutically acceptable carrier and any needed preservatives or buffers as may be required.

The treatments may include various "unit doses." Unit dose is defined as containing a predetermined quantity of the therapeutic composition (an antagonist of the NC_{Ca-ATP} channel or its related-compounds thereof) calculated to produce the desired responses in association with its administration, *e.g*., the appropriate route and treatment regimen. The quantity to be administered, and the particular route and formulation, are within the skill of those in the clinical arts. Also of import is the subject to be treated, in particular, the state of the subject and the protection desired. A unit dose need not be administered as a single injection but may comprise continuous infusion over a set period of time.

### D. Formulations and Routes for Administration of Compounds

Pharmaceutical compositions of the present invention comprise an effective amount of one or more modulators of NC_{Ca-ATP} channel which are antagonists of TRPM4 or related-compounds or additional agent dissolved or dispersed in a pharmaceutically acceptable carrier. The phrases "pharmaceutical or pharmacologically acceptable" refers to molecular entities and compositions that do not produce an adverse, allergic or other untoward reaction when administered to an animal, such as, for example, a human, as appropriate. The preparation of a pharmaceutical composition that contains at least one modulator of NC_{Ca-ATP} channel (antagonist and/or agonist) or related-compounds or additional active ingredient will be known to those of skill in the art in light of the present disclosure, as exemplified by Remington's Pharmaceutical Sciences, 18th Ed. Mack Printing Company, 1990. Moreover, for animal (*e.g.,* human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (*e.g.,* antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329. Except insofar as any conventional carrier is incompatible with the active ingredient, its use in the pharmaceutical compositions is contemplated.

The modulators of NC_{Ca-ATP} channel which are antagonists of TRPM4 may comprise different types of carriers depending on whether it is to be administered in solid, liquid or aerosol form, and whether it need to be sterile for such routes of administration as injection. The present invention can be administered intravenously, intradermally, transdermally, intrathecally, intraventricularly, intraarterially, intraperitoneally, intranasally, intravaginally, intrarectally, topically, intramuscularly, subcutaneously, mucosally, orally, topically, locally, inhalation (*e.g.,* aerosol inhalation), injection, infusion, continuous infusion, localized perfusion bathing target cells directly, *via* a catheter, *via* a lavage, in cremes, in lipid compositions (*e.g.,* liposomes), or by other method or any combination of the forgoing as would be known to one of ordinary skill in the art (see, for example, Remington's Pharmaceutical Sciences, 18th Ed. Mack Printing Company, 1990).

The modulators of NC_{Ca-ATP} channel which are antagonists of TRPM4 may be formulated into a composition in a free base, neutral or salt form. Pharmaceutically acceptable salts, include the acid addition salts, *e.g.,* those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Upon formulation, solutions will be administered in a manner compatible with the dosage formulation and in such amount as is therapeutically effective. The formulations are easily administered in a variety of dosage forms such as formulated for parenteral administrations such as injectable solutions, or aerosols for delivery to the lungs, or formulated for alimentary administrations such as drug release capsules and the like.

Further in accordance with the present invention, the composition of the present invention suitable for administration is provided in a pharmaceutically acceptable carrier with or without an inert diluent. The carrier should be assimilable and includes liquid, semi-solid, *i.e.,* pastes, or solid carriers. Except insofar as any conventional media, agent, diluent or carrier is detrimental to the recipient or to the therapeutic effectiveness of the composition contained therein, its use in administrable composition for use in practicing the methods of the present invention is appropriate. Examples of carriers or diluents include fats, oils, water, saline solutions, lipids, liposomes, resins, binders, fillers and the like, or combinations thereof. The composition may also comprise various antioxidants to retard oxidation of one or more component. Additionally, the prevention of the action of microorganisms can be brought about by preservatives such as various antibacterial and antifungal agents, including but not limited to parabens (*e.g.,* methylparabens, propylparabens), chlorobutanol, phenol, sorbic acid, thimerosal or combinations thereof.

In accordance with the present invention, the composition is combined with the carrier in any convenient and practical manner, *i.e.,* by solution, suspension, emulsification, admixture, encapsulation, absorption and the like. Such procedures are routine for those skilled in the art.

In a specific embodiment of the present invention, the composition is combined or mixed thoroughly with a semi-solid or solid carrier. The mixing can be carried out in any convenient manner such as grinding. Stabilizing agents can be also added in the mixing process in order to protect the composition from loss of therapeutic activity, *i.e.,* denaturation in the stomach. Examples of stabilizers for use in an the composition include buffers, amino acids such as glycine and lysine, carbohydrates such as dextrose, mannose, galactose, fructose, lactose, sucrose, maltose, sorbitol, mannitol, *etc.*

In further embodiments, the present invention may concern the use of a pharmaceutical lipid vehicle compositions that include modulators of NC_{Ca-ATP} channel which are antagonists of TRPM4, one or more lipids, and an aqueous solvent. As used herein, the term "lipid" will be defined to include any of a broad range of substances that is characteristically insoluble in water and extractable with an organic solvent. This broad class of compounds is well known to those of skill in the art, and as the term "lipid" is used herein, it is not limited to any particular structure. Examples include compounds which contain long-chain aliphatic hydrocarbons and their derivatives. A lipid may be naturally occurring or synthetic (*i.e.,* designed or produced by man). However, a lipid is usually a biological substance. Biological lipids are well known in the art, and include for example, neutral fats, phospholipids, phosphoglycerides, steroids, terpenes, lysolipids, glycosphingolipids, glycolipids, sulphatides, lipids with ether and ester-linked fatty acids and polymerizable lipids, and combinations thereof. Of course, compounds other than those specifically described herein that are understood by one of skill in the art as lipids are also encompassed by the compositions and methods of the present invention.

One of ordinary skill in the art would be familiar with the range of techniques that can be employed for dispersing a composition in a lipid vehicle. For example, the modulators of NC_{Ca-ATP} channel which are antagonists of TRPM4 may be dispersed in a solution containing a lipid, dissolved with a lipid, emulsified with a lipid, mixed with a lipid, combined with a lipid, covalently bonded to a lipid, contained as a suspension in a lipid, contained or complexed with a micelle or liposome, or otherwise associated with a lipid or lipid structure by any means known to those of ordinary skill in the art. The dispersion may or may not result in the formation of liposomes.

The actual dosage amount of a composition of the present invention administered to an animal or patient can be determined by physical and physiological factors such as body weight, severity of condition, the type of disease being treated, previous or concurrent therapeutic and/or prophylatic interventions, idiopathy of the patient and on the route of administration. Depending upon the dosage and the route of administration, the number of administrations of a preferred dosage and/or an effective amount may vary according to the response of the subject. The practitioner responsible for administration will, in any event, determine the concentration of active ingredient(s) in a composition and appropriate dose(s) for the individual subject.

In certain embodiments, pharmaceutical compositions may comprise, for example, at least about 0.1% of an active compound. In other embodiments, the an active compound may comprise between about 2% to about 75% of the weight of the unit, or between about 25% to about 60%, for example, and any range derivable therein. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

Pharmaceutical formulations may be administered by any suitable route or means, including alimentary, parenteral, topical, mucosal or other route or means of administration. Alimentary routes of administration include administration oral, buccal, rectal and sublingual routes. Parenteral routes of administration include administration include injection into the brain parenchyma, and intravenous, intradermal, intramuscular, intraarterial, intrathecal, subcutaneous, intraperitoneal, and intraventricular routes of administration. Topical routes of administration include transdermal administration.

### E. Alimentary Compositions and Formulations

In preferred embodiments of the present invention, the modulators of NC_{Ca-ATP} channel which are antagonists of TRPM4 are formulated to be administered *via* an alimentary route. Alimentary routes include all possible routes of administration in which the composition is in direct contact with the alimentary tract. Specifically, the pharmaceutical compositions disclosed herein may be administered orally, buccally, rectally, or sublingually. As such, these compositions may be formulated with an inert diluent or with an assimilable edible carrier, or they may be enclosed in hard- or soft- shell gelatin capsule, or they may be compressed into tablets, or they may be incorporated directly with the food of the diet.

In certain embodiments, the active compounds may be incorporated with excipients and used in the form of ingestible tablets, buccal tables, troches, capsules, elixirs, suspensions, syrups, wafers, and the like (Mathiowitz *et al.,* 1997; Hwang *et al.,* 1998; U.S. Pat. Nos. 5,641,515; 5,580,579 and 5,792, 451). The tablets, troches, pills, capsules and the like may also contain the following: a binder, such as, for example, gum tragacanth, acacia, cornstarch, gelatin or combinations thereof; an excipient, such as, for example, dicalcium phosphate, mannitol, lactose, starch, magnesium stearate, sodium saccharine, cellulose, magnesium stearate, sodium saccharine, cellulose, magnesium carbonate or combinations thereof; a disintegrating agent, such as, for example, corn starch, potato starch, alginic acid or combinations thereof; a lubricant, such as, for example, magnesium stearate; a sweetening agent, such as, for example, sucrose, lactose, saccharin or combinations thereof; a flavoring agent, such as, for example peppermint, oil of wintergreen, cherry flavoring, orange flavoring, *etc.* When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. When the dosage form is a capsule, it may contain, in addition to materials of the above type, carriers such as a liquid carrier. Gelatin capsules, tablets, or pills may be enterically coated. Enteric coatings prevent denaturation of the composition in the stomach or upper bowel where the pH is acidic. See, *e.g.,* U.S. Pat. No. 5,629,001. Upon reaching the small intestines, the basic pH therein dissolves the coating and permits the composition to be released and absorbed by specialized cells, *e.g.,* epithelial enterocytes and Peyer's patch M cells. A syrup of elixir may contain the active compound sucrose as a sweetening agent methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations.

For oral administration the compositions of the present invention may alternatively be incorporated with one or more excipients in the form of a mouthwash, dentifrice, buccal tablet, oral spray, or sublingual orally- administered formulation. For example, a mouthwash may be prepared incorporating the active ingredient in the required amount in an appropriate solvent, such as a sodium borate solution (Dobell's Solution). Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth.

Additional formulations which are suitable for other modes of alimentary administration include suppositories. Suppositories are solid dosage forms of various weights and shapes, usually medicated, for insertion into the rectum. After insertion, suppositories soften, melt or dissolve in the cavity fluids. In general, for suppositories, traditional carriers may include, for example, polyalkylene glycols, triglycerides or combinations thereof. In certain embodiments, suppositories may be formed from mixtures containing, for example, the active ingredient in the range of about 0.5% to about 10%, and preferably about 1% to about 2%.

In specific embodiments, there may be co-administration with antacids, H2 blockers, proton blockers and related compounds that neutralize or reduce stomach pH, in order to enhance absorption of sulfonylureas.

### F. Parenteral Compositions and Formulations

In further embodiments, modulators of NC_{Ca-ATP} channel which are antagonists of TRPM4 may be administered *via* a parenteral route. As used herein, the term "parenteral" includes routes that bypass the alimentary tract. Specifically, the pharmaceutical compositions disclosed herein may be administered for example, but not limited to intravenously, intradermally, intramuscularly, intraarterially, intraventricularly, intrathecally, subcutaneously, or intraperitoneally U.S. Pat. Nos. 6,613,308, 5,466,468, 5,543,158; 5,641,515; and 5,399,363).

Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable use include sterile aqueous solutions or dispersions and sterile powders for the extemporaneous preparation of sterile injectable solutions or dispersions (U.S. Patent 5,466,468). In all cases the form must be sterile and must be fluid to the extent that easy injectability exists. It must be stable under the conditions of manufacture and storage and must be preserved against the contaminating action of microorganisms, such as bacteria and fungi. The carrier can be a solvent or dispersion medium containing, for example, water, ethanol, DMSO, polyol (*i.e.,* glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous, and intraperitoneal administration. In this connection, sterile aqueous media that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. A powdered composition is combined with a liquid carrier such as, *e.g.,* water or a saline solution, with or without a stabilizing agent.

### G. Miscellaneous Pharmaceutical Compositions and Formulations

In other preferred embodiments of the invention, the active compound modulators of NC_{CaATP} channel which are antagonists of TRPM4 may be formulated for administration *via* various miscellaneous routes, for example, topical (*i.e.,* transdermal) administration, mucosal administration (intranasal, vaginal, *etc.*) and/or inhalation.

Pharmaceutical compositions for topical administration may include the active compound formulated for a medicated application such as an ointment, paste, cream or powder. Ointments include all oleaginous, adsorption, emulsion and water-solubly based compositions for topical application, while creams and lotions are those compositions that include an emulsion base only. Topically administered medications may contain a penetration enhancer to facilitate adsorption of the active ingredients through the skin. Suitable penetration enhancers include glycerin, alcohols, alkyl methyl sulfoxides, pyrrolidones and luarocapram. Possible bases for compositions for topical application include polyethylene glycol, lanolin, cold cream and petrolatum as well as any other suitable absorption, emulsion or water-soluble ointment base. Topical preparations may also include emulsifiers, gelling agents, and antimicrobial preservatives as necessary to preserve the active ingredient and provide for a homogenous mixture. Transdermal administration of the present invention may also comprise the use of a "patch". For example, the patch may supply one or more active substances at a predetermined rate and in a continuous manner over a fixed period of time.

In certain embodiments, the pharmaceutical compositions may be delivered by eye drops, intranasal sprays, inhalation, and/or other aerosol delivery vehicles. Methods for delivering compositions directly to the lungs *via* nasal aerosol sprays has been described *e.g.,* in U.S. Pat. Nos. 5,756,353 and 5,804,212. Likewise, the delivery of drugs using intranasal microparticle resins (Takenaga *et al.,* 1998) and lysophosphatidyl-glycerol compounds (U.S. Pat. No. 5,725, 871) are also well-known in the pharmaceutical arts. Likewise, transmucosal drug delivery in the form of a polytetrafluoroetheylene support matrix is described in U.S. Pat. No. 5,780,045.

The term aerosol refers to a colloidal system of finely divided solid of liquid particles dispersed in a liquefied or pressurized gas propellant. The typical aerosol of the present invention for inhalation will consist of a suspension of active ingredients in liquid propellant or a mixture of liquid propellant and a suitable solvent. Suitable propellants include hydrocarbons and hydrocarbon ethers. Suitable containers will vary according to the pressure requirements of the propellant. Administration of the aerosol will vary according to subject's age, weight and the severity and response of the symptoms.

### XI. Combination Treatments

In the context of the present invention, it is contemplated that an antagonist of the NC_{Ca-ATP} channel which is an antagonist of TRPM4 is used in combination with an additional therapeutic agent to more effectively treat any disease or medical condition in an individual in need thereof, such as a cerebral ischemic event, and/or decrease intracranial pressure, for example. In some embodiments, it is contemplated that a conventional therapy or agent, including but not limited to, a pharmacological therapeutic agent may be combined with the antagonist or related-compound of the present invention. The combined therapeutic agents may work synergistically, although in alternative embodiments they work additively.

Pharmacological therapeutic agents and methods of administration, dosages, *etc.* are well known to those of skill in the art (see for example, the "Physicians Desk Reference", Goodman & Gilman's "The Pharmacological Basis of Therapeutics", "Remington's Pharmaceutical Sciences", and "The Merck Index, Eleventh Edition"), and may be combined with the invention in light of the disclosures herein. Some variation in dosage will necessarily occur depending on the condition of the subject being treated. The person responsible for administration will, in any event, determine the appropriate dose for the individual subject, and such individual determinations are within the skill of those of ordinary skill in the art.

When an additional therapeutic agent is employed, as long as the dose of the additional therapeutic agent does not exceed previously quoted toxicity levels, the effective amounts of the additional therapeutic agent may simply be defined as that amount effective to improve at least one symptom in an animal when administered to an animal in combination with an antagonist of NC_{Ca-ATP} channel which is an antagonist of TRPM4. This may be easily determined by monitoring the animal or patient and measuring those physical and biochemical parameters of health and disease that are indicative of the success of a given treatment. Such methods are routine in animal testing and clinical practice.

Treatment with an antagonist of NC_{Ca-ATP} channel which is an antagonist of TRPM4 may precede or follow the additional agent treatment by intervals ranging from minutes to hours to weeks to months. In some embodiments, the antagonist of the NC_{Ca-ATP} channel which is an antagonist of TRPM4 is administered prior to the additional therapeutic compound, and in other embodiments, the antagonist of the NC_{Ca-ATP} channel which is an antagonist of TRPM4 is administered subsequent to the additional therapeutic compound. The difference in time between onset of administration of either part of the combinatorial composition may be within seconds, such as about 60 or less, within minutes, such as about 60 or less, within hours, such as about 24 or less, within days, such as about 7 or less, or within weeks of each other.

In embodiments where the additional agent is applied separately to the cell, one would generally ensure that a significant period of time did not expire between the time of each delivery, such that the agent would still be able to exert an advantageously combined effect on the cell. In such instances, it is contemplated that one would contact the cell with both modalities within about 1-24 hr of each other and, more preferably, within about 6-12 hr of each other.

Typically, for maximum benefit of the additional agent, the therapy must be started within three hours of the onset of stroke symptoms, making rapid diagnosis and differentiation of stroke and stroke type critical. However, in the present invention, administration of the NC_{Ca-ATP} channel which is an antagonist of TRPM4 with an additional agent increases this therapeutic window. The therapeutic window for thrombolytic agents, for example, may be increased by several (4-8) hours by co-administering an antagonist of the NC_{Ca-ATP} channel which is an antagonist of TRPM4.

### XII. Kits of the Invention

Any of the compositions described herein may be comprised in a kit. The kit may be a therapeutic kit and/or a preventative kit. In a specific embodiment, a combinatorial therapeutic composition is provided in a kit, and in some embodiments the two or more compounds that make up the composition are housed separately or as a mixture. Antagonists of the channel that may be provided include but are not limited to antibodies (monoclonal or polyclonal, for example to TRPM4), TRPM4 oligonucleotides, TRPM4 polypeptides, small molecules or combinations thereof, antagonist, agonist, *etc.*

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there is more than one components in the kit, the kit also may generally contain a second, third or other additional container into which additional components may be separately placed. However, various combinations of components may be comprised in a vial. The kits of the present invention also will typically include a means for containing the TRPM4 inhibitor, lipid, additional agent, and any other reagent containers in close confinement for commercial sale. Such containers may include injection or blow molded plastic containers into which the desired vials are retained.

Therapeutic kits of the present invention are kits comprising an antagonist, agonist or a related-compound thereof. Depending upon the condition and/or disease that is being treated, the kit may comprise a TRPM4 antagonist or related-compound thereof to block and/or inhibit the NC_{Ca-ATP} channel which is an antagonist of TRPM4. Such kits will generally contain, in suitable container means, a pharmaceutically acceptable formulation of SUR1 or TRPM4 antagonist, agonist or related-compound thereof. The kit may have a single container means, and/or it may have distinct container means for each compound. For example, the therapeutic compound and solution may be contained within the same container; alternatively, the therapeutic compound and solution may each be contained within different containers. A kit may include a container with the therapeutic compound that is contained within a container of solution.

When the components of the kit are provided in one and/or more liquid solutions, the liquid solution is an aqueous solution, with a sterile aqueous solution being particularly preferred. The TRPM4 antagonist may also be formulated into a syringeable composition. In which case, the container means may itself be a syringe, pipette, and/or other such like apparatus, from which the formulation may be applied to an infected area of the body, injected into an animal, and/or even applied to and/or mixed with the other components of the kit.

Examples of aqueous solutions include, but are not limited to aqueous solutions including ethanol, DMSO and/or Ringer's solution. In certain embodiments, the concentration of DMSO or ethanol that is used is no greater than 0.1% or (1 ml/1000 L).

However, the components of the kit may be provided as dried powder(s). When reagents and/or components are provided as a dry powder, the powder can be reconstituted by the addition of a suitable solvent. It is envisioned that the solvent may also be provided in another container means.

The container means will generally include at least one vial, test tube, flask, bottle, syringe and/or other container means, into which the TRPM4 antagonist, agonist or related-compounds thereof is suitably allocated. The kits may also comprise a second container means for containing a sterile, pharmaceutically acceptable buffer and/or other diluent.

The kits of the present invention will also typically include a means for containing the vials in close confinement for commercial sale, such as, *e.g.*, injection and/or blow-molded plastic containers into which the desired vials are retained.

Irrespective of the number and/or type of containers, the kits of the invention may also comprise, and/or be packaged with, an instrument for assisting with the injection/administration and/or placement of the TRPM4 antagonist within the body of an animal. Such an instrument may be a syringe, pipette, forceps, and/or any such medically approved delivery vehicle.

In addition to the TRPM4 antagonist, agonist or related-compounds thereof, the kits may also include a second active ingredient. Examples of the second active ingredient include substances to prevent hypoglycemia (*e.g.,* glucose, 5% of dextrose in water (D5W), glucagon, *etc.*)*,* thrombolytic agents, anticoagulants, antiplatelets, statins, diuretics, vasodilators, *etc.* These second active ingredients may be combined in the same vial as the SUR1 antagonist, agonist or related-compounds thereof or they may be contained in a separate vial.

Still further, the kits of the present invention can also include glucose testing kits. Thus, the blood glucose of the patient is measured using the glucose testing kit, then the TRPM4 antagonist, agonist or related-compounds thereof can be administered to the subject followed by measuring the blood glucose of the patient.

In addition to the above kits, the therapeutic kits of the present invention can be assembled such that an IV bag comprises a septum or chamber which can be opened or broken to release the compound into the IV bag. Another type of kit may include a bolus kit in which the bolus kit comprises a pre-loaded syringe or similar easy to use, rapidly administrable device. An infusion kit may comprise the vials or ampoules and an IV solution (*e.g.,* Ringer's solution) for the vials or ampoules to be added prior to infusion. The infusion kit may also comprise a bolus kit for a bolus/loading dose to be administered to the subject prior, during or after the infusion.

### EXAMPLES

### EXAMPLE 1

### TRPM4 - THE PORE-FORMING SUBUNIT OF THE NC_{CA-ATP} CHANNEL

The SUR1-regulated NC_{Ca-ATP} channel is a novel cation channel. Its importance lies in the fact that it is critically involved in cell death in CNS tissues, including brain and spinal cord. The SUR1-regulated NC_{Ca-ATP} channel is not normally expressed in the CNS, but is expressed only following hypoxia, injury or inflammation.

The channel has been extensively studied in rodent models of disease. It was first discovered in reactive astrocytes obtained from the gliotic capsule surrounding a foreign body implanted into the rat brain (Chen *et al.,* 2001; Chen *et al.,* 2003). Since then, it has also been identified in neurons from the core of an ischemic stroke (Simard *et al.,* 2006) and in spinal cord neurons and capillaries following traumatic injury (Simard *et al.,* 2007) and in cultures of murine CNS capillary endothelial (bEnd.3) cells subjected to hypoxia. Evidence of the channel, as indicated by SUR1 expression, is also found in astrocytes in a rodent model of brain abscess, and in neurons, capillaries and venules in a rodent model of subarachnoid hemorrhage.

The channel has also been studied in human disease. Evidence of the channel, as indicated by SUR1 expression, has been uncovered in human brain tissues from patients with a variety of hypoxia-related conditions, including preterm infants with germinal matrix hemorrhage, gliotic capsule from metastatic tumor, and brain tissue following gun shot wound to the head. It has also been identified in a human glioblastoma cell line (ATCC, U-118 MG), where it may be constitutively expressed, as well as in human brain microvascular endothelial cells and human aortic endothelial cells (both from ScienCell Research Laboratories) when these cells are exposed to hypoxia or tumor necrosis factor alpha (TNFα).

When expressed, the NC_{Ca-ATP} channel is not active but it becomes activated when intracellular ATP is depleted, leading to cell depolarization, cytotoxic edema and oncotic (necrotic) cell death.

The NC_{Ca-ATP} channel is composed of pore-forming plus regulatory subunits. The regulatory subunit is sulfonylurea receptor 1 (SUR1), the same as that for K_{ATP} channels in pancreatic β cells (Chen *et al.,* 2003). Thus, pharmacological agents used as oral antihyperglycemics to block pancreatic K_{ATP} channels, such as glibenclamide (also known as glyburide), also block the NC_{Ca-ATP} channel. The pore-forming subunit may be TRPM4, for example

Block of the NC_{Ca-ATP} channel by glibenclamide has been shown to be an important therapy for stroke (Simard *et al.,* 2007). In rodent models of ischemic stroke, glibenclamide reduces mortality, cerebral edema and lesion volume by half (Simard *et al.,* 2006). In humans with diabetes mellitus, use of sulfonylureas before and during hospitalization for ischemic stroke is associated with markedly better stroke outcomes (Kunte *et al.,* 2007).

Block of the NC_{Ca-ATP} channel by glibenclamide has also been shown to be an important therapy for spinal cord injury. In a rodent model of cervical spinal cord injury, glibenclamide significantly reduces progressive hemorrhagic necrosis and tissue loss, and significantly improves neurological outcome (Simard *et al.,* 2007).

### EXAMPLE 2

### TRPM4 AND THE SUR1-REGULATED NC_{CA-ATP} CHANNEL

The SUR1-regulated NC_{Ca-ATP} channel is composed of pore-forming plus regulatory subunits. The pore-forming subunits were not previously identified at the molecular level, but it was noted that many of the biophysical properties of the SUR1-regulated NC_{Ca-ATP} channel are similar to those of TRPM4 (see Table 2). TRPM4, together with TRPM5, are the only molecular candidates presently known for the class of non-selective, Ca²⁺-impermeable cation channels that are activated by intracellular Ca²⁺ and blocked by intracellular ATP, *i.e.,* NC_{Ca-ATP} channels.

Both the SUR1-regulated NC_{Ca-ATP} channel and TRPM4 are highly selective for monovalent cations, have no significant permeation of Ca²⁺, are activated by internal Ca²⁺ and blocked by internal ATP. The two channels have several features in common (Table 2).

The high sensitivity to glibenclamide exhibited by the SUR1-regulated NC_{Ca-ATP} channel requires expression of SUR1. In certain aspects, SUR1 forms complexes with TRPM4, resulting in an increase in sensitivity to sulfonylurea. Upon embodiments wherein such heteromeric assembly occurs, in certain aspects heteromers of SUR1 and TRPM4 may exhibit somewhat different properties than homomeric TRPM4.

Applicants disclose herein the heteromeric assembly of SUR1 and TRPM4. SUR1 is known to be a promiscuous regulatory subunit. SUR1 is best known for its role in forming K_{ATP} channels by assembling with Kir6.1 or Kir6.2 pore-forming subunits; in addition, heterologous constructs of SUR1 with another inwardly rectifying K⁺ channel, Kir1.1a, have also been described (Ammala *et al.,* 1996).

**Table 2. Properties of the SUR1-regulated NC_{Ca-ATP} channel and of the TRPM4 channel**

| | SUR1-regulated NC_{Ca-ATP} | TRPM4 |
|---|---|---|
| channel conductance | 35 pS | 25 pS |
| divalent cation conductivity | no | no |
| pore radius | 0.41 nm | |
| Ca²⁺ activation (EC₅₀) | 0.12-1.5 µM | 1.3 µM |
| ATP block (EC₅₀) | 0.8 µM | 0.13-1.7 µM |
| ADP, AMP block | no | yes |
| voltage dependent | no | yes |
| PIP₂ activation | yes^{*} | yes |
| PKC activation | no | yes |
| glibenclamide block (EC₅₀) | 48 nM | 10-100 µM |

Table 2. Data for SUR1-regulated NC_{Ca-ATP} channel are from published Chen *et al.,* 2001; 2003 and unpublished observations (*Chen and Simard, unpublished). Data for TRPM4 channel (Nilius *et al.,* 2006; Nilius *et al.,* 2003; Nilius *et al.,* 2004a; Nilius *et al.,* 2004b; Nilius *et al.,* 2005a; Nilius *et al.,* 2005b; Ullrich *et al.,* 2005; Guinamard *et al.,* 2004; Guinamard *et al.,* 2006; Demion *et al.,* 2007) were selected to most closely approximate those of the SUR1-regulated NC_{Ca-ATP} channel.

A pore-forming subunit of the NC_{Ca-ATP} channel was sought to be identified as TRPM4. Overall, several aspects indicate TRPM4 is the pore-forming subunit of the SUR1-regulated NC_{Ca-ATP} channel, including at least one of the following:
(i) SUR1 and TRPM4 should be newly co-expressed and co-localized;
(ii) immuno-isolation of one should "pull-down" the other, to show physical interaction between SUR1 and TRPM4;
(iii) pharmacological block of SUR1 and of TRPM4 should have similar effects inhibiting NC_{Ca-ATP} channels in patch clamp experiments;
(iv) preventing expression of either one, using antisense or a similar knock-down strategy, should prevent expression functional NC_{Ca-ATP} channels in patch clamp experiments;
(v) pharmacological block of SUR1 and of TRPM4 should have similar effects in injury models where the channel is up-regulated; and
(vi) preventing expression of either one, using antisense or a similar knock-down strategy, should have similar effects in injury models where the channel is up-regulated;

The studies described below were performed utilizing commercially available antibodies for SUR1 and TRPM4 (both from Santa Cruz Biotechnology), as an example.
(i) Co-expression of SUR1 and TRPM4. SUR1 and TRPM4 are both up-regulated under the same conditions and are co-expressed by the same cells.
   In the gliotic capsule surrounding a gelatin sponge implant, immunolabeling shows the same temporal and spatial pattern of expression of SUR1 and TRPM4. Both are up-regulated and co-expressed in reactive astrocytes of the hypoxic inner zone, as was shown previously for SUR1 (Chen *et al.,* 2003) and as shown here for TRPM4 (FIG. 8).
   In SCI, immunolabeling shows the same pattern of expression for SUR1 and TRPM4, with both up-regulated and co-expressed in capillaries and other cells in the core of the impact site (FIG. 9).
   In bEnd.3 cells exposed to TNFα, but not bEnd.3 cells under control conditions, immunolabeling and Western blots show up-regulation of expression for SUR1 and TRPM4 (FIG. 10).
   In addition, the only other molecular candidate, TRPM5, is not expressed under these conditions, in either bEnd.3 cells without or with exposure to TNFα or spinal cord pre- or post-injury (not shown).
(ii) Co-immunoprecipitation studies may be performed. One uses suitable antibodies for SUR1 and TRPM4, which may be obtained commercially or otherwise.
(iii) Pharmacological block of SUR1 and TRPM4 / patch clamp. In bEnd.3 cells, exposure to TNFα causes expression of a new channel that is not present before exposure to TNFα, and whose biophysical properties are consistent with the NC_{Ca-ATP} channel, including activation by ATP depletion following exposure to Na azide plus 2-deoxyglucose and a reversal potential near 0 mV (FIG. 11). This channel is blocked by glibenclamide (FIG. 4), as expected for SUR1, and by flufenamic acid (FIG. 11), as expected for TRPM4.
(iv) Antisense for SUR1 and TRPM4 / patch clamp. These studies are performed for SUR1 and TRPM4. For these experiments, antisense oligodeoxynucleotide (for example, SEQ ID NO:1 or SEQ ID NO:2) is infused into the injury site where a gliotic capsule forms around an implanted gelatin sponge.
   Antisense knock-down of SUR1 reduces SUR1 protein expression and prevents expression of functional NC_{Ca-ATP} channels in freshly isolated astrocytes, as reported (Simard *et al.,* 2007). The same study occurs for TRPM4, in specific embodiments.
(v) Pharmacological block of SUR1 and TRPM4 / injury model.
   In SCI, treatment with the SUR1 blockers, glibenclamide or repaglinide, reduces progressive hemorrhagic necrosis and improves neurological function, as reported (Simard *et al.,* 2007).
   In SCI, treatment with the TRPM4 blocker, flufenamic acid, reduces progressive hemorrhagic necrosis (FIG. 12) and improves neurological function (FIG. 13), although the neurobehavioral effect is not as pronounced as with the SUR1 blockers, in specific cases because flufenamic acid is not as specific for its target.
(vi) Antisense for SUR1 and TRPM4 / injury model.
   In SCI, treatment with antisense directed against SUR1 reduces expression of SUR1, reduces progressive hemorrhagic necrosis and improves neurological function, as reported (Simard *et al.,* 2007).
   In SCI, treatment with antisense directed against TRPM4 eliminates expression of TRPM4 in capillaries (FIG. 9) and it significantly reduces progressive hemorrhagic necrosis (not shown) and improves neurological function (FIG. 13). In these studies, residual TRPM4 expression is still noted in astrocytes in the core, but no labeling is present in the penumbra (FIG. 9).
(vii) Glibenclamide block of SUR1 reduces expression of TRPM4 in SCI.

In SCI, treatment with the SUR1 blockers, glibenclamide or repaglinide, reduces progressive hemorrhagic necrosis and improves neurological function, as reported (Simard *et al.,* 2007). Immunolabeling spinal cord sections of animals treated with glibenclamide demonstrates that TRPM4 expression is significantly reduced, consistent with a necessary co-association between SUR1 and TRPM4, as expected if SUR1 is required for proper trafficking of TRPM4 to the membrane.

Several lines of evidence, as reviewed above, show that TRPM4 constitutes the pore-forming subunit of the SUR1-regulated NC_{Ca-ATP} channel. Given the channel's important role in CNS disease, it is clear that targeting TRPM4 is a useful alternative or complementary strategy to targeting SUR1 in these diseases. The data provided herein clearly show that antisense oligodeoxynucleotide directed against SUR1 or TRPM4 have similar, strongly positive, beneficial effects in SCI.

### EXAMPLE 3

### TRPM4 CHANNEL IN SPINAL CORD INJURY

### Spinal cord injury - the clinical problem

Acute spinal cord injury (SCI) results in physical disruption of spinal cord neurons and axons leading to deficits in motor, sensory, and autonomic function. The concept of secondary injury in SCI arises from the observation that the volume of injured tissue increases with time after injury, i.e., the lesion itself expands and evolves over time (Tator and Fehlings, 1991; Kwon *et al.,* 2004). Whereas primary injured tissues are irrevocably damaged from the very beginning, right after impact, tissues that are destined to become "secondarily" injured are considered to be potentially salvageable. Older observations based on histological studies that gave rise to the concept of lesion-evolution have been confirmed with non-invasive MRI (Bilgen *et al.,* 2000; Weirich *et al.,* 1990; Ohta *et al.,* 1999; Sasaki *et al.,* 1978).

Numerous mechanisms of secondary injury are recognized, including edema, ischemia, oxidative stress and inflammation. In SCI, however, one pathological entity in particular is recognized that is relatively unique to the spinal cord and that has especially devastating consequences - progressive hemorrhagic necrosis (PHN) (Tator and Fehlings, 1991; Nelson *et al.,* 1977; Tator , 1991; Fitch *et al.,* 1999; Tator, 1991; Kraus, 1996).

PHN is a rather mysterious condition, first recognized over 3 decades ago, that had previously eluded understanding and treatment (Simard *et al.,* 2007). Following impact, petechial hemorrhages form in surrounding tissues and later emerge in more distant tissues, eventually coalescing into the characteristic lesion of hemorrhagic necrosis. The cord exhibits a progressive increase in hemorrhage (Khan, 1985; Kawata *et al.,* 1993). After injury, a small hemorrhagic lesion involving primarily the capillary-rich central gray matter is observed at 15 min, but hemorrhage, necrosis and edema in the central gray matter enlarge progressively over a period of 3-24 h (Kawata *et al.,* 1993; Balentine, 1978; Iizuka *et al.,* 1987). The white matter surrounding the hemorrhagic gray matter shows a variety of abnormalities, including decreased H&E staining, disrupted myelin, and axonal and periaxonal swelling. White matter lesions extend far from the injury site, especially in the posterior columns (Tator and Koyanagi, 1997). The evolution of hemorrhage and necrosis has been referred to as "autodestruction". PHN eventually causes loss of vital spinal cord tissue and, in some species including humans, leads to post-traumatic cystic cavitation surrounded by glial scar tissue.

### TRPM4 in SCI

Transient receptor potential channels are increasingly recognized as playing important roles in disease. (Nilius, 2007; Nilius *et al.,* 2007). In a recent series of invited reviews on the role of TRP channels in disease (Simard *et al.,* 2007), it is discussed that the biophysical properties of the NC_{Ca-ATP} channel resemble closely the biophysical properties of TRPM4, especially as regards: (i) non-selective monovalent conductivity; (ii) single channel conductance; (iii) absence of divalent cation conductivity; (iv) regulation by intracellular Ca²⁺; and (v) regulation by intracellular ATP. In certain aspects, NC_{Ca-ATP} channels are heteromultimers of SUR1 and TRPM4.

As described below, the invention regards at least the following aspects: (i) TRPM4 (but not TRPM5) is up-regulated in parallel with SUR1 in 2 different models, the gliotic capsule model first used for discovery of the NC_{Ca-ATP} channel, and in the SCI model first used to show the channel's role in PHN; (ii) exposure of brain microvascular endothelial cells (bEnd.3) to TNFα up-regulates both SUR1 and TRPM4 mRNA and protein, and causes expression of SUR1-regulated NC_{Ca-ATP} channels that are blocked by the TRP4 blocker, flufenamic acid; (iii) post-SCI PHN is blocked by post-injury administration of flufenamic acid and anti-TRPM4 AS-ODN just as effectively as was reported with glibenclamide, repaglinide and anti-SUR1-AS-ODN.

### Overview

Spinal cord injury (SCI) results in "progressive hemorrhagic necrosis" (PHN), a poorly understood pathological entity described over 30 years ago that leads to devastating loss of spinal cord tissue and debilitating neurological dysfunction. In embodiments of the invention, the regulatory subunit of the non-selective cation channel, the NC_{Ca-ATP} channel, is critically involved in PHN, but the pore-forming subunit of the channel was not molecularly identified. In further embodiments of the invention, TRPM4 is the pore-forming subunit of the channel. The invention expands upon these embodiments by further characterizing the role of TRPM4 in post-SCI PHN. For example, in a rat model of contusion SCI it was demonstrated that hemorrhage and progressive lesion expansion were dramatically reduced by pharmacological block and gene suppression of TRPM4, and these effects were associated with a dramatic improvement in neurobehavioral functional outcome. In particular aspects of the invention, the cells most critically involved in PHN are capillary and post-capillary venular endothelial cells.

In certain aspects, patch clamp recordings of freshly isolated spinal cord capillaries post-SCI and cultured CNS microvascular endothelial cells exposed to TNFα are utilized, and the physiological regulation and the functional role of TRPM4 channels in endothelial cells is determined. The inventors also demonstrate that NFκB, which is the downstream effector of TNFα and which is known to be prominently involved in SCI, acts as an important transcriptional regulator of TRPM4 channels, in particular aspects of the invention.

In other embodiments using tissues from a rat SCI model and cultures of CNS microvascular endothelial cells, the role of the transcription factor, NFκB, is determined in expression of TRPM4 channels, and the effect of NFκB suppression is examined on outcome in SCI vis-à-vis TRPM4 expression. Overall, an understanding of the role of TRPM4 channels in SCI leads to novel molecular insights and novel treatments for this devastating human condition.

Thus, using a rodent model of spinal cord injury, the inventors discovered that pharmacological and antisense inhibition of TRPM4 channels cause a striking reduction in hemorrhagic necrosis and a dramatic improvement of neurological function.

In specific embodiments of the invention, *de novo* expression of TRPM4 channels in endothelial cells is required for PHN. In particular aspects, establishing the role of TRPM4 in PHN; determining the regulation and the functional role of TRPM4 channels in freshly isolated spinal cord capillaries and cultured microvascular endothelial cells; and elucidating transcriptional regulation of the channel leads to novel treatments for this devastating human condition.

### EXAMPLE 4

### INVOLVEMENT OF TRPM4 CHANNELS IN POST-SCI PHN

**Up-regulation of TRPM4 in SCI.** The model of unilateral cervical SCI that is employed involves a "severe" injury (10-gm weight dropped 25 mm; NYU impactor) (Soblosky *et al.,* 2001) that results in development of a progressively expansive necrotic lesion characteristic of "progressive hemorrhagic necrosis" (PHN) (Tator *et al.,* 1991; Nelson *et al.,* 1977; Tator, 1991; Fitch *et al.,* 1999). TRPM4 expression was studied in uninjured controls and in rats post-SCI (3 female Long Evans rats/group) using commercially available antibodies. Low levels of TRPM4 expression were found in uninjured controls (FIG. 14A,C), but 24 h post-SCI, TRPM4 was heavily up-regulated in tissues surrounding the injury (FIG. 14B,D). TRPM4 up-regulation extended to distant tissues, including into the contralateral hemi-cord. TRPM4 up-regulation was apparent in various cell types, but was especially prominent in elongated structures that co-labeled with vimentin, consistent with "activated" capillaries (FIG. 14E,F).

*In situ* hybridization confirmed expression of TRPM4 after injury, especially in microvessels in the penumbra, whereas controls, including uninjured cords and injured cords labeled with "sense" probes, showed no comparable labeling (FIG. 14G,H). These findings with TRPM4 parallel precisely recent observations of an increase in SUR1 post-SCI, providing evidence of a link between the 2 subunits that are believed to form the NC_{Ca-ATP} channel (Simard *et al.,* 2007a; Simard *et al.,* 2007b).

In other studies, exemplary sense (SE) or antisense (AS) oligodeoxynucleotides (ODN) were administered that were phosphorothioated to protect from endogenous nucleases. (TRPM4-AS1: 5'-GTGTGCATCGCTGTCCCACA-3' (SEQ ID NO:1); and TRPM4-AS2: 5'-CTGCGATAGCACTCGCCAAA-3' (SEQ ID NO:2); complementary sequences were used as sense ODNs.) ODN was administered i.v. *via* mini-osmotic pumps, with infusion beginning 2 days prior to SCI and continuing until the animal was sacrificed. When cords were examined 24 h post-SCI, rats treated with SE-ODN exhibited widespread up-regulation of TRPM4 (FIG. 15A), similar to untreated rats (FIG. 14B). However, rats treated with AS-ODN exhibit virtually no TRPM4 (FIG. 15B), demonstrating highly effective gene suppression. These studies gave further validation that TRPM4 was in fact up-regulated post-SCI, and also confirmed that the commercially available antibody employed for immunolabeling was appropriately specific.

**Pharmacological and anti-sense block of TRPM4 ameliorates PHN post-**SCI. To assess the role of TRPM4 in SCI, the effect of flufenamic acid (FFA) was studied, a blocker of TRPM4, as well as of AS-ODN directed against TRPM4, which was highly effective in down-regulating TRPM4 expression (see above). Immediately post-SCI, animals were given either vehicle or FFA (35 mg/kg i.p., every 6 hours). For the ODN experiments rats were either pre-treated for 2 days (as above) or were implanted with mini-osmotic pumps for i.v. delivery beginning immediately post-SCI.

The hallmark of PHN is progressive extravasation of blood. Cords of vehicle-treated animals examined 24 h post-SCI showed prominent bleeding at the surface as well as internally, with internal bleeding consisting of a central region of hemorrhage plus numerous petechial hemorrhages remote from the impact (FIG. 16A, CTR, arrows). By contrast, cords of FFA-treated animals showed less hemorrhage both at the surface and internally, with internal bleeding consisting of a central region of hemorrhage but with few or no petechial hemorrhages (FIG. 16A, FFA). Cords of animals pre-treated with SE-ODN showed a prominent contusion with numerous petechial hemorrhages (FIG. 16A, SE), whereas cords from rats pre-treated with AS-ODN showed very little hemorrhage (FIG. 16A, AS).

For the 4 groups of rats, the amount of blood in cord homogenates was quantified 24-hr post-SCI, after first perfusing animals to remove intravascular blood (FIG. 16B,C). Values were compared to the inventor's published findings in untreated rats showing a progressive increase over the first 12 h post SCI (FIG. 16C filled circles and curved line). Post-treatment with SE-ODN resulted in an amount of hemorrhage comparable to untreated controls. By contrast, post-treatment with either FFA or AS-ODN resulted in a significant decrease in blood to levels comparable to those found right after impact (FIG. 16C). These findings with TRPM4 inhibition mirror exactly findings recently reported with glibenclamide (Simard *et al.,* 2007), again providing evidence of a link between the 2 subunits that are believed to form the NC_{Ca-ATP} channel.

Formation of petechial hemorrhages can be equated to catastrophic failure of capillary integrity. Capillaries were examined in the region of injury by immunolabeling with vimentin, which is up-regulated in endothelium following injury (Haseloff *et al.,* 2006). In controls post-SCI, vimentin(+) capillaries appeared foreshortened or fragmented, whereas in rats treated with FFA or pre-treated with AS-ODN, the capillaries were elongated and more intact (FIG. 17). The improved capillary integrity with inhibition of TRPM4 correlates with the decrease in hemorrhage, indicating an important role of TRPM4 in PHN.

Treatments that maintained capillary integrity and reduced hemorrhage were also associated with improved neurobehavioral outcomes 24 h post-SCI. Animals used to assess hemorrhage on an inclined plane were tested with a standard test that requires more-and-more dexterous function of the limbs and paws as the angle of the plane is increased (Rivlin and Tator, 1977). Vertical exploratory behavior ("rearing") was also quantified, which is a complex exercise that requires balance, truncal stability, bilateral hindlimb dexterity and strength, and at least unilateral forelimb dexterity and strength that, in combination, are excellent markers of cervical spinal cord function. Rats treated with FFA or pre- and post-treated with AS-ODN showed significantly better performance than controls when tested 24 h after SCI (FIG. 18).

In one embodiment, the reduction, but not elimination, of hemorrhage and progressive lesion expansion that was observed by the inventors with flufenamic acid and anti-TRPM4 AS-ODN was due to incomplete block of TRPM4; in a specific embodiment, complete block of TRPM4 channel function by knock-out maximally reduces PHN and other forms of secondary injury.

The justification for these studies rests on several observations. First, the data already in hand show a very strong beneficial effect of pharmacological block of TRPM4 using the flufenamic acid, and of gene suppression using anti-TRPM4 AS-ODN. The similar outcomes obtained with agents that act *via* distinct molecular mechanisms underscores the important role of TRPM4. Nevertheless, these post-injury treatments did not result in complete elimination of PHN. On the one hand, flufenamic acid has low selectivity for TRPM4 molecule, and so it is not assured that the beneficial effect of this compound was due strictly to inhibition of TRPM4 or whether some other potential molecular target(s) might also have been involved. By contrast, anti-TRPM4 AS-ODN, which by its very nature is highly specific for its target, was highly effective in reducing post-SCI PHN. However, as is generally observed with AS-ODN, it did not completely suppress expression of the targeted gene.

In certain embodiments of the invention, the pore-forming subunit of the channel is a better therapeutic target in SCI than the regulatory subunit, since targeting the regulatory subunit (SUR1) alone may be associated with undesirable side effects, including hypoglycemia and hypertension, and a may yield a submaximal effect, since TRPM4 can form functional channels by itself without SUR1(Vennekens and Nilius, 2007; Nilius *et al.,* 2006, Nilius *et al.,* 2005). In other aspects, it is useful to determine the role that TRPM4 plays in recovery post-SCI.

*The extent to which TRPM4 channels are involved in PHN and other forms of secondary injury in SCI is determined.*

Certain studies are employed that determine whether TRPM4 plays a critical role in PHN (and in post-SCI recovery). Multiple groups of mice are studied, untreated and flufenamic acid treated wild-type (129/SvJ) and other TRPM4 mice models, for example other knockdown models using antisense oligonucleotides other than SEQ ID NO:1: Group 1: wild-type, untreated; Group2: wild-type, treated with flufenamic acid beginning right after SCI; Group3: TRPM4 Antisense (AS), untreated; and Group4: TRPM4 AS, treated with flufenamic acid beginning right after SCI.

The group with flufenamic acid provides a link to the work in rats already in hand, and allows comparison of what is the best available pharmacological blocker of TRPM4, with the idealized situation of gene knockdown. The group with TRPM4 AS plus flufenamic acid helps to ascertain whether drug has a beneficial (or deleterious) effect independent of TRPM4.

In the following 4 series of studies, all animals undergo SCI at T9, as previously described in C57B1/6 mice61-63 or 129/SvJ mice. Adult female mice (3-4 mo old, 20-24 g in weight) are anesthetized with 0.5 ml/20 gm Avertin i.p., and undergo laminectomy at T9, with the dura left intact. SCI contusions are induced using a force-driven impactor (Infinite Horizon) that creates graded contusion injuries by incrementing the force applied to the surface of the cord, with a force sensor to detect the actual force during impact (Scheff *et al.,* 2003). Animals not receiving an adequate impact, as judged by the sensor recording, are eliminated from the study. After surgery, animals are given Ringer's solution s.q. to prevent dehydration, are treated with topical antibiotic at the incision and prophylactic antibiotic (gentocin, 50 mg i.p.) to prevent urinary tract infections. Manual bladder expression is performed twice daily. Blood glucose levels are checked using a droplet of blood from tail pricks and a standard glucometer.

For the two untreated groups, groups 1 and 3, no intervention is implemented after SCI. For the other two, groups 2 and 4, immediately after injury, within 2-3 min of SCI, animals receive flufenamic acid (35 mg/kg i.p., q 6 h). This dose is based on experience with flufenamic acid treatment of rats with SCI.

IN SERIES 1, the "dose-response" relationship is assessed between magnitude of the injury force *vs*. neurobehavioral outcome at 7 d in the 4 groups of mice. At 7 d, BMS scores reach ∼half of the maximal values that will be achieved, with maximal values reached at 14 d. Scores at 7 d discriminate well between groups (Basso *et al.,* 2006). Injuries with the Infinite Horizon impactor are performed at force settings of 20, 40, 60, 80 kdyn which, based on published data in C57B1/6 mice (Basso *et al.,* 2006; Cummings *et al.,* 2006) is expected to result in mild to severe injuries (30, 50 and 60 kdyn impact forces yield 70%, 50% and 35% tissue sparing at the epicenter, respectively) (Cummings *et al.,* 2006). Neurobehavioral functional outcome is measured as described below using the open field BMS scale and the ladder beam test.

An exemplary purpose with these studies is to determine what level of impact force should be used in subsequent experiments that will best separate outcomes in the 4 groups of mice.

The exemplary studies of Series 1 may require 30 mice per group (7-8 mice/dose x 4 doses) or 120 mice total, for example. In 3 subsequent series of experiments (Series 2-4), one can use a single impact force, to be determined above, to perform experiments to compare edema, hemorrhage, expansion of the necrotic lesion, inflammation and neural function in the 4 groups, at various times after SCI.

IN SERIES 2, measures of capillary endothelial dysfunction are assessed, *i.e.*, edema formation, "blood brain barrier" (BBB) leakiness and hemorrhage at 1½, 3, 6, 12 h in the 3 groups of mice. Previous studies in rat indicate that maximum hemorrhage is observed at 12 h.

Using separate groups of animals for each measurement, the following are measured: (i) excess Na⁺; (ii) excess H₂O; (iii) BBB leakiness; and (iv) hemorrhage in each of the 4 groups of animals at 4 time points after injury. Excess Na⁺ and H₂O are measured using flame photometry and the wet weight/dry weight method, respectively. BBB leakiness are measured spectrophotometrically using Evans blue. Hemorrhage is measured spectrophotometrically after conversion of hemoglobin to cyanomethemoglobin using Drabkin's reagent. Details of each method are given below. Separate animals are needed for each measurement because no two measurements can be performed on tissues from a single animal.

A purpose of these studies is to determine the time course of the change in capillary leakiness and failure of capillary integrity following SCI, and the effect of TRPM4 inhibition on these parameters. For each of the 4 individual measurements, the studies of Series 2 are expected to require 40 mice per group (10 mice/time point x 4 times) or 160 mice per measurement. With 4 measurements in all, 640 mice are used for these experiments.

IN SERIES 3, one can assess the inflammatory response by immunohistochemistry and myeloperoxidase activity at 1, 2, 4, 7 d in the 4 groups of mice. In SCI, maximum neutrophil activity occurs 24 h after injury (Carlson *et al.,* 1998), both microglia and macrophages are noted by 12 h post-injury (Popovich *et al.,* 1997) and peak microglial activation is observed 3-7 d post-injury (Popovich *et al.,* 1997).

To assess the degree of inflammatory cell infiltration, 10-µm sections of spinal cord from the lesion epicenter and rostral and caudal penumbra are immunostained with OX42 for resident and activated microglia, OX-6 for activated microglia, GFAP for activated astrocytes, F4/80 for macrophages, and ab2557 for neutrophils, as described below. Selected sections are co-immunolabeled for TRPM4, NeuN and von Willebrand factor for cellular identification. In addition, cord segments are evaluated for myeloperoxidase activity as a measure of neutrophil activation/infiltration (La Rosa *et al.,* 2004). A purpose of these studies is to determine the time course of inflammation and the effect of TRPM4 suppression on the inflammatory response.

In Series 4, serial neurological outcome is assessed at 1, 3, 7, 14 d, and lesion volume at 14 d in the 4 groups of mice. At 7 days, BMS scores are ∼half maximal and at 14 days, they reach maximal values (Basso *et al.,* 2006).

To assess neurological outcome, the open field BMS scale and the ladder beam test are used. To assess lesion volume, 10-µm serial paraffin sections of spinal cord are used at 100 µm intervals through the entire lesion. Lesion volumes are calculated based on measurements of residual tissue. A purpose with these studies is to determine the time course of neurobehavioral recovery after injury, and the effect of TRPM4 suppression on neurobehavioral functional recovery and on lesion size. The studies of Series 4 are expected to require 40 mice per group (10 mice/time point x 4 times) or 160 mice total.

### Specific Methods:

NEUROBEHAVIORAL FUNCTION IN MICE - OPEN FIELD TEST AND LADDER BEAM TEST. Several instruments are available to assess locomotor recovery following SCI in mice. Due to the range of possible outcomes following SCI, from complete hindlimb paralysis to normal locomotion with slightly impaired trunk stability or paw position, a single instrument cannot differentiate with equal sensitivity across the entire broad spectrum of recovery. Arguably, the best broad-range instrument for assessing locomotor recovery is the open-field locomotor rating scale specialized for mice, the Basso mouse scale (BMS) (Basso *et al.,* 2006), which was adapted from the better known BBB developed for rats.

Open field testing and scoring will be done using the BMS for locomotion, as described (Basso et al, 2006). The BMS is a sensitive, valid and reliable locomotor measure in SCI mice The BMS detects significant differences in locomotor outcomes between severe contusion and transection, and between SCI severity gradations. Also, the BMS demonstrates significant predictive and concurrent validity, with novice BMS raters with training scoring within 0.5 points of experts, and it demonstrates high reliability (0.92-0.99) (Basso *et al.,* 2006).

Mice are tested in an open field (metal watering tank, 115-cm diameter, 30-cm side wall height; Behlen Manufacturing, Columbus NE). The BMS is performed by 2 raters located on opposite sides of the open field. Each test lasts 4 min. For accurate evaluation, mice are first acclimated to the open field over multiple sessions prior to testing. Scoring is based on 7 locomotor categories for early (ankle movement), intermediate (plantar placement, stepping) and late (coordination paw position, trunk instability tail) phases of recovery. The BMS score and subscore are calculated from data entered on a standardized score sheet.

Neurobehavioral function is evaluated using a ladder beam test and score (LBS) as described (Cummings *et al.,* 2006). The LBS is most advantageous for separating animals within the 5-7 range on the BMS or the 9-13 point range on the "mouse BBB". This is a range where animals with large differences in functional recovery can often receive similar or identical open-field scores.

The apparatus consists of a metal horizontal ladder beam (74 rungs of 4 mm diameter, spaced 12 mm apart) suspended 18 in. above the ground with a hollow black escape box at one end (Columbus Instruments, OH). A digital video camcorder is used to film the trials. Animals are pre-handled for 1 week before their first videotaping and trained on the ladder beam apparatus 3 days prior to assessment. Scoring is performed as described (Cummings *et al.,* 2006).

TISSUE WATER AND SODIUM CONTENT. Tissue water is quantified by the wet/dry weight method as described (Hua *et al.,* 2003; Sribnick *et al.,* 2005). The excised cord is carefully blotted to remove droplets of fluid and is carefully weighed on a precision scale to obtain the wet weight (WW). The tissues are then dried to constant weight at 80° C and reweighed to obtain the dry weight (WD). Tissue water, expressed as percent of WW, is computed as (WW-WD)/WW x100.

Dehydrated cord samples are digested in 1 ml of 1 N nitric acid for 1 week. Sodium content is measured by flame photometry (Instrumentation Laboratory, Inc), as described (Xi *et al.,* 2001). Ion contents are expressed in microequivalents per gram of dehydrated brain tissue (µEq/g DW).

BBB LEAKINESS. BBB leakiness is quantified using the Evans blue technique as described (Kaptanoglu *et al.,* 2004; Warnick *et al.,* 1995; Kakinuma *et al.,* 1998). Evans blue (EB) is dissolved in saline (2 g/100 ml). The tail vein is cannulated to administer 50 mg/kg of EB. The EB is allowed to circulate for 30 min, and then is washed out using saline cardiac perfusion. Cord samples are taken and frozen at -20 °C until spectrophotometric evaluation. The tissues are weighed and the EB dye is extracted in formamide at room temperature for 18 h. The absorbance of extracted dye is measured at 620 nm using a plate reader. Parallel measurements at 740 nm are used to correct for turbidity.

HEMOGLOBIN MEASUREMENTS. Hemoglobin (Hgb) in cord tissue is quantified spectrophotometrically after conversion to cyanomethemoglobin using Drabkin's reagent. This method allows determination of hemoglobin concentrations below 0.1 mg/dL (Choudhri *et al.,* 1997; Pfefferkorn and Rosenberg, 2003), has been validated for CNS tissue for use in assessing hemorrhagic conversion in stroke (Pfefferkorn and Rosenberg, 2003) and has been used by us for quantifying hemorrhagic necrosis following SCI in rats (Simard *et al.,* 2007). A 5-mm segment of cord tissue encompassing the injury is placed in a volume of water (molecular grade) that is 3x its weight, followed by homogenization for 30 sec, sonication on ice with a pulse ultrasonicator for 1 min, and centrifugation at 13,000 rpm for 45 min. After the Hgb-containing supernatant is collected, 80 µL of Drabkin's reagent (Sigma; K3Fe(CN)₆ 200 mg/L, KCN 50 mg/L, NaHCO₃ 1 g/L, pH 8.6) is added to a 20-µL aliquot and allowed to stand for 15 min. This reaction converts hemoglobin to cyanomethemoglobin, which has an absorbance peak at 540 nm, and whose concentration can then be assessed by the OD of the solution at 540 nm using a microplate reader. Values of Hgb are converted into equivalent microliters of blood using a standardized curve made from measurements on normal perfused spinal cords "doped" with known volumes of blood.

IMMUNOHISTOCHEMISTRY. Cryosections are immunolabeled using standard techniques used in the inventors' lab (Chen *et al.,* 2003; Simard *et al.,* 2006). After permeabilizing (0.3% Triton X-100 for 10 min), sections are blocked (2% donkey serum for 1 hr; Sigma D-9663), then incubated with primary antibody directed against: TRPM4 (1:200; Santa Cruz Biotechnology); for neuron, NeuN (1:100; MAB377; Chemicon, CA); for astrocyte, anti-mouse monoclonal GFAP (1:2500, Sigma); for endothelium, von Willebrand factor (1:200; F3520, Sigma); for reactive astrocyte and capillary, vimentin (1:200; CY3 conjugated; C-9060, Sigma); for microglia, anti-rat OX42 (1:500; Harlan Sera Labs); for microglia, anti-mouse monoclonal OX-6 (1:1000, BD Transduction); for tissue macrophages, anti-mouse macrophages F4/80 (clone BM8, Cell Sciences); for neutrophil, anti-mouse neutrophil antibody (ab2557, Abcam). After washing, sections are incubated with species-appropriate fluorescent secondary antibody. Fluorescent signals are visualized using epifluorescence microscopy (Nikon Eclipse E1000). Images are captured using a Sensys digital camera.

LESION VOLUME. Animals undergo intracardiac perfusion with 0.1 M PBS and 4% formaldehyde in PBS. The region of cord containing the injury is embedded in paraffin and transverse sectioned (10 µm), with sections every 100 µm stained with H&E to evaluate lesion size. Myelin integrity is examined using Luxol fast blue and eriochrome cyanine staining. The section with the largest extent of lesion and the least amount of white matter is designated the epicenter. The area of white matter sparing and the total cross sectional area of the lesion are measured using image analysis software (Scion Image, Scion Corporation).

MYELOPEROXIDASE ACTIVITY. (Jimenez-Garza *et al.,* 2005) Cord segments are sonicated in 500 µl of 50 mM phosphate buffer, pH 6.0, containing 0.5% hexadecyltrimethylammonium bromide to extract the MPO from the neutrophil granules. The homogenates are frozen and thawed 3x, with brief sonication each time. After extraction, the samples are centrifuged at 40,000xg for 15 min at 4°C, and the supernatant saved to a new tube. The supernatant (50-µl aliquot) is assayed for MPO using o-dianisidine (0.167 mg/ml) and H2O2 (0.0005%). Absorbance at 460 nm is recorded with a spectrophotometer at 30-s intervals for 3 min. The change in absorbance per minute is calculated, and the units of MPO activity per 1-mm spinal cord segment determined according to the formula for peroxidase activity. One unit of MPO activity is defined as that which degrades one micromole of peroxide per minute.

DATA ANALYSIS. Standard statistical methods are used, including ANOVA and t-test, as appropriate for individual experiments. As usual, *p*<0.05 is taken as the measure of significance.

In specific embodiments, these studies further characterize the role of TRPM4 in post-SCI PHN. The strategy that is employed in the study design is intended to allow direct comparison of outcome in flufenamic acid treated wild-type mice *vs.* TRPM4 AS mice. These studies are carefully designed to determine if effects of flufenamic acid are attributable principally or exclusively to TRPM4. If this were true and if the dose of flufenamic acid were optimal in terms of producing a maximal effect, then one would expect similar outcomes for animals in groups 2-4, i.e., wild-type treated with flufenamic acid should be indistinguishable from untreated TRPM4 AS, and treatment of TRPM4 AS with flufenamic acid should have no added effect. In other embodiments, drug and AS are not equivalent. If TRPM4AS is better than flufenamic acid, this would indicate a suboptimal drug effect, for example, dose too low or treatment too late or untoward side-effect of drug. If flufenamic acid is better than TRPM4 AS, this would indicate an effect of drug unrelated to TRPM4, a finding that could potentially be picked up also in the group of TRPM4 AS plus drug.

The wild-type (129/SvJ) mice suitable for use here have previously been used in studies of SCI (Ma *et al.,* 2004). Interestingly, this strain exhibits an attenuated inflammatory response compared to other mouse strains (C57B1/6). Their early response to injury is quite similar to other strains, but the reduced inflammatory response yields less cavitation at the site of injury (Ma *et al.,* 2004), which may facilitate axonal regrowth past the site of injury. Its attenuated inflammatory response may make it particularly suitable for studies related to NFκB, whose role is frequently viewed exclusively within an inflammatory context, but which is interesting for characterizing its role in transcriptional regulation of TRPM4.

### EXAMPLE 5

### CHARACTERIZATION OF THE PHYSIOLOGICAL REGULATION AND FUNCTIONAL ROLE OF TRPM4 CHANNELS

Using freshly isolated spinal cord capillaries post-SCI, it is confirmed that the channel up-regulated by injury is TRPM4, and using capillary endothelial cell cultures exposed to TNFα, the physiological regulation and functional role of TRPM4 channels is characterized.

In one certain embodiment, the channel up-regulated in spinal cord capillaries by SCI is TRPM4. In another embodiment, the channel up-regulated in capillary endothelial cell cultures by TNFα is TRPM4. In an additional embodiment, the TRPM4 channel in endothelial cells is inhibited by PIP2 depletion induced by estrogen-mediated phospholipase C activation, which may account in part for salutary effects of estrogen reported in SCI. In another embodiment, expression and activation of TRPM4 channels in endothelial cells leads to cytotoxic edema and cell death.

Studies are undertaken with freshly isolated spinal cord capillaries and cultured microvascular endothelial cells derived from murine CNS to determine expression, functional regulation, and the functional role of TRPM4 channels. In Series 1, the biophysical properties of the channels are characterized in the various preparations of freshly isolated spinal cord capillaries and cultured endothelial cells. In Series 2, the channel in endothelial cell cultures that is up-regulated by TNFα is characterized, and its inhibition by estrogen and PIP2 depletion is studied. In Series 3, the role of the channel in death of endothelial cells is assessed.

**TNFα causes up-regulation NC_{Ca}-_{ATP} (putative TRPM4) channel in bEnd.3.** In certain aspects of the invention, injury leads to NFκB activation, such as SCI, and can result in up-regulation of functional TRPM4 channels. Murine CNS microvascular endothelial cells (bEnd.3, ATTC, Rockville, MD) were studied under control conditions and after 6-h exposure to the pro-inflammatory cytokine, TNFα (20 ng/ml), which activates NFκB. Cultures were immunolabeled for TRPM4 and TRPM5, the only 2 molecularly identified non-selective cation channels with exclusive monovalent conductivity. Only very faint labeling for TRPM4 was observed in controls (FIG. 19A), but cultures exposed to TNFα showed very prominent labeling for TRPM4 (FIG. 19B). Immunolabeling for TRPM5 was negative. Western blots confirmed robust up-regulation of TRPM4 (3-fold increase, FIG. 19C,D).

A characteristic feature of TRPM4 channels in expression systems is that they are blocked by intracellular ATP in the low micromolar range (Nilius *et al.,* 2006; Nilius *et al.,* 2005; Nilius *et al.,* 2003; Nilius *et al.,* 2005; Nilius and Vennekens, 2006) and so are activated by ATP depletion. To detect this feature in TNFα-treated bEnd.3 cells, the inventors depleted ATP combination of Na azide, a mitochondrial uncoupler (Chen and Simard, 2001) and 2-deoxyglucose (2-DG) to inhibit glycolysis. Patch clamp was performed using a nystatin-perforated patch technique to maintain the metabolic integrity of the cells. Control bEnd.3 cells showed no effect of ATP depletion on membrane currents (not shown), but TNFα-treated cells responded with activation of a robust inward current at the holding potential (-50 mV), which was ohmic and had a reversal potential ∼0 mV (FIG. 20), consistent with TRPM4 channels. This current was blocked by 100 µM flufenamic acid (FIG. 20), the best available blocker of TRPM4 channels (Nilius *et al.,* 2006; Nilius *et al.,* 2005; Nilius *et al.,* 2003; Nilius *et al.,* 2005; Nilius and Vennekens, 2006).

Single channel recordings were performed using inside-out patches, with Cs⁺ as the only permeant cation. These experiments confirmed the presence of a 31-pS non-selective cation channel that was reversibly blocked by ATP on the cytoplasmic side (FIG. 21). In addition, these experiments showed that the channel required physiological concentrations of Ca²⁺ on the intracellular side, and that it was not permeable to Ca²⁺ (FIG. 21C). This channel was blocked by FFA (FIG. 21D), as expected for TRPM4.

The observations on the channel up-regulated by TNFα in bEnd.3 cells, including: (i) activation by depletion of cellular ATP; (ii) a reversal potential ∼0 mV; (iii) conductance of Cs⁺; (iv) single channel conductance of ∼30 pS; (v) impermeability to Ca²⁺; (vi) block by flufenamic acid; and (vii) correlation of channel activity with expression of TRPM4 (but not TRPM5) protein, in combination, strongly support the hypothesis that TNFα causes up-regulation of TRPM4 and that it forms the pore-forming subunit of the NC_{Ca-ATP} channel. Knock-down experiments using siRNA may be performed to confirm this, but the biophysical properties observed best fit TRPM4 or TRPM5 channels, and TRPM5 was not expressed.

**Isolation of spinal cord capillaries and patch clamp.** Microvascular complexes were isolated from normal (uninjured) rat spinal cord using a method based on perfusion with magnetic particles (details of method given below). Magnetic separation yielded microvascular complexes that typically included a precapillary arteriole plus attached capillaries (FIG. 22A, arrows).

Capillary endothelial cells were patch clamped still attached to intact microvascular complexes using a conventional whole cell method. Cells were studied with standard physiological solutions in the bath and in the pipette, with no ATP in the pipette solution (FIG. 22B,C). Under these conditions, currents turned on instantaneously (FIG. 22B), the current-voltage relationship was linear and it reversed near -20 mV (FIG. 22C). These recordings demonstrate the feasibility of patch clamping freshly isolated capillary endothelial cells that are still attached to intact microvascular complexes from spinal cord.

**Primary cultures of spinal cord capillary endothelial cells and patch clamp.** Studies on primary cultures of murine spinal cord microvascular endothelial (scEnd) cells are undertaken, which are phenotypically closer to native spinal cord capillary endothelial cells than bEnd.3 cells. Murine spinal cord microvessels were isolated and cultured as described (Ge and Pachter, 2006; Wu *et al.,* 2003). Labeling with CD31(+) beads as well as for von Willebrand factor confirmed their endothelial identity (FIG. 23A,B). As with bEnd.3 cells, patch clamp of scEnd cells cultured under normal conditions showed that no membrane current is activated by ATP depletion. However, in scEnd cells exposed to 20 ng/ml TNFα for 6 h, ATP depletion activated an inward current that reverses at 0 mV, consistent with the NC_{Ca-ATP} channel (FIG. 23C-E).

**Estrogen inhibits the NC_{Ca-ATP} channel.** One of the characteristic features of TRPM4 channels in expression systems is that the channel is activated by PIP₂ and is inhibited by depletion of PIP₂ (Nilius *et al.,* 2006; Nilius *et al.,* 2007). In certain aspects of the invention, TRPM4 channels in a native system would respond to PIP₂ in the same manner. For these experiments, freshly isolated reactive astrocytes were isolated from hypoxic gliotic capsule (Chen and Simard, 2001; Chen *et al.,* 2003) and TNFα-treated bEnd.3 cells, which in both cases express SUR1-regulated NC_{Ca-ATP} channels whose pore-forming subunit is TRPM4, in certain embodiments of the invention.

Membrane patches studied in the presence of high concentration of ATP on the cytoplasmic side showed little channel activity, as expected, but addition of PIP₂ resulted in robust channel activation, despite the high level of ATP (FIG. 24a). This finding is consistent with PIP₂ causing an apparent decrease in affinity of the channel for ATP, as reported for TMPM4 (Nilius *et al.,* 2006; Nilius *et al.,* 2007.

PIP₂ is the principal substrate for phospholipase C (PLC). Thus, PLC activation depletes levels of PIP₂, whereas PLC inhibition augments levels of PIP₂. Studies for a receptor mechanism that would activate PLC and thereby deplete PIP₂ to reduce channel opening are employed. Estrogen is studied, which is known to activate PLC in neurons and other cells from both males and females (Qiu *et al.,* 2003; Beyer *et al.,* 2002; Le Mellay *et al.,* 1999). When NC_{Ca-ATP} channel activity was prominent due to a low concentration of ATP, addition of estrogen (E2; 10 nM) caused a rapid reduction in channel activity (FIG. 24b). In a specific embodiment, estrogen depletes PIP₂ *via* activation of PLC, resulting in an apparent increase in affinity of the channel for ATP.

Apart from direct application of PIP2 (FIG. 24a), PIP2 levels can be augmented by inhibiting spontaneously active PLC. In control bEnd.3 cells, which do not express NC_{Ca-ATP} channels, addition of the PLC inhibitor, U73122, was without effect (FIG. 24c). However, in TNFα-stimulated bEnd.3 cells that do express the channel (see above), addition of U73122 activated an ohmic current that reversed at 0 mV, consistent with previous observations on TRPM4.

*Using freshly isolated spinal cord capillaries post-SCI, it is conformed that the channel up-regulated by injury is TRPM4, and using capillary endothelial cell cultures exposed to TNFα, the physiological regulation and functional role of TRPM4 channels are characterized.*

In one embodiment of the invention, the channel up-regulated in spinal cord capillaries by SCI is TRPM4; the channel up-regulated in capillary endothelial cell cultures by TNFα is TRPM4; the TRPM4 channel in endothelial cells is inhibited by PIP2 depletion induced by estrogen-mediated phospholipase C activation, which may account in part for salutary effects of estrogen reported in SCI; and/or expression and activation of TRPM4 channels in endothelial cells leads to cytotoxic edema and cell death.

The biophysical properties of the SUR1-regulated NC_{Ca-ATP} channel that was documented in astrocytes (Chen and Simard, 2001; Chen *et al.,* 2003), neurons (Simard *et al.,* 2006), and cultured CNS capillary endothelial cells (Simard *et al.,* 2007) closely resemble those of TRPM4. These similarities were carefully laid out in a recent review (Simard *et al.,* 2007). However, in the case of spinal cord capillaries post-SCI, the NC_{Ca-ATP} channel itself has not yet been recorded, and for all cases listed, definitive evidence that the pore-forming subunit is TRPM4 is obtained. To address this definitively for capillaries post-SCI, the following are isolated and recorded from spinal cord capillary endothelial cells from 3 situations: (i) normal rats; (ii) rats post-SCI; (iii) rats post-SCI treated with anti-TRPM4 AS-ODN. The data show that microvascular complexes and endothelial cells were isolated that are part of capillaries still attached to microvascular complexes (FIG. 22). The data also show that post-SCI, capillaries express abundant TRPM4, and that post-SCI, treatment with anti-TRPM4 AS-ODN significantly attenuates TRPM4 expression (FIG. 14 and 15). Patch clamp study of capillary endothelial cells from the 3 preparations shows that a new channel is up-regulated post-SCI, that its biophysical properties are characteristic of TRPM4, and when TRPM4 expression is suppressed, a channel with those biophysical properties is absent.

A similar set of studies are performed to assess for up-regulation of TRPM4 using 2 preparations of cultured endothelial cells: (i) primary cultures of murine spinal cord capillary endothelial cells; (ii) immortalized murine brain endothelial cells (bEnd.3 cells). Exposure to TNFα is used to induce channel expression, and cells silenced for TRPM4 expression will be used for molecular confirmation of TRPM4 involvement. The advantage of the primary cultures is their "phenotypic proximity" to native cells; their disadvantage is that preparation is time-consuming and difficult, and the yield is small. The advantage of the bEnd.3 cells is the ease of working with them; their disadvantage is their "phenotypic distance" from native cells, and the uncertainty whether regulatory machinery (e.g., G-protein coupled membrane estrogen receptors; phospholipase) is maintained in these immortalized cells. The data show that there has been successfully established primary cultures of murine spinal cord capillary endothelial cells, which have been patch clamped (FIG. 23) and that the inventors have successfully patch clamped bEnd.3 cells exposed to TNFα that express TRPM4 mRNA and protein, as well as functional NCCa-ATP /putative TRPM4 channels (FIGS. 20, 21).

Using cultured endothelial cells, modulation of channel opening by estrogen is also studied. Estrogen has emerged as a potentially important treatment for SCI. The severity of the initial injury as well as the ultimate recovery of motor function after SCI are significantly influenced by gender, being remarkably better in females (Farooque *et al.,* 2006). Administration of 17β-estradiol to ovariectomized rats improves recovery of hind-limb locomotion, increases white matter sparing, and decreases apoptosis in both post- and pre-menopausal rats (Chaovipoch *et al.,* 2006; Yune *et al.,* 2004). When compared to sham, vehicle-treated animals show increased tissue edema, increased infiltration of inflammatory cells and increased myelin loss, whereas estrogen-treated rats have reduced edema, decreased inflammation and decreased myelin loss (Sribnick *et al.,* 2005). Estrogen-mediated neuroprotection may be due to one of several characteristics of this steroid hormone, including non-specific mechanisms involving lipid membrane fluidity, and receptor-dependent mechanisms involving immediate signaling cascades as well as genomic effects (Sribnick *et al.,* 2005; Sribnick *et al.,* 2003; Roog and Hall, 2000). Estrogen is a potent anti-oxidant (Moosmann and Behl, 1999) and anti-inflammatory agent (Dimayuga *et al.,* 2005). Estrogen treatment may attenuate ischemia after injury (Roog and Hall, 2000) and *in vitro* studies indicate that estrogen prevents Ca²⁺ influx (Nilsen *et al.,* 2002), calpain activation (Sribnick *et al.,* 2004), calpain activity (Sur *et al.,* 2003), and apoptosis (Linford and Dorsa, 2002). Notably, no unique mechanism has yet been identified to fully account for the beneficial effect of estrogen in SCI.

However, TRPM4 is known to be modulated by PIP2 (Nilius *et al.,* 2006), and the data indicate that estrogen may reduce channel opening due to phospholipase C-dependent, PIP2 depletion (FIG. 24). These findings indicate that, in specific embodiments of the invention, TRPM4 is an important target of estrogen in SCI. Further characterization of this mechanism coupling estrogen receptors on endothelium (Kim and Bender, 2005) to TRPM4 expressed by endothelium in injured tissues yields important mechanistic insights and has important therapeutic implications, including providing therapy for SCI and related conditions.

Finally, the relationship between TRPM4 expression and death of endothelial cells is studied. Astrocytes that express the NC_{Ca-ATP} channel undergo oncotic (necrotic) death due to opening of the channel when ATP is depleted (Simard *et al.,* 2006). Induction of endothelial cell death *in vivo* by channel opening leads to hemorrhage and PHN, in specific embodiments of the invention. The studies in this series are designed to determine if channel opening in endothelial cells results in their dysfunction and death.

Studies: Studies with freshly isolated rat spinal cord capillary endothelial cells as well as cultured endothelial cells derived from murine brain and spinal cord microvessels are useful.

IN SERIES 1, one can characterize the biophysical properties of the channels in the various preparations:
1. freshly isolated spinal cord capillaries from (i) uninjured controls; (ii) post-SCI; (iii) post-SCI plus treatment with AS-ODN (sense-ODN as control) to suppress expression of TRPM4. Capillaries are isolated. Patch clamp study utilizes nystatin whole-cell recording as well as cell-attached and inside-out patches made from endothelial cells still attached to intact microvascular complexes for unambiguous identification of the cell under study.
2. primary cultures of murine spinal cord capillary endothelial cells under (i) control conditions; (ii) post exposure to TNFα; (iii) post exposure to TNFα plus treatment with siRNA to suppress expression of TRPM4.
3. bEnd.3 cells under (i) control conditions; (ii) post exposure to TNFα; (iii) post exposure to TNFα plus treatment with siRNA to suppress expression of TRPM4.

Biophysical properties attributable to TRPM4 may be demonstrated by the studies disclosed herein. The essential properties that characterize TRPM4 include single channel conductance of 25-35 pS, monovalent but not divalent cation conductance, sensitivity to intracellular Ca²⁺ and ATP. In combination, the specific values of these properties allow exclusion of all other TRP channels (Simard *et al.,* 2007). Measurement of these characteristics, in combination with the knock-down experiments, provides unambiguous channel identification. Thus, the following studies are undertaken, in specific embodiments:
SERIES 1A. With the 3 preparations under 3 conditions each, initial screening experiments are performed to demonstrate a current activated by depletion of cellular ATP. One can perform whole-cell and cell-attached patch recordings during application of Na azide plus 2-DG to deplete intracellular ATP. Absence of current activatable by ATP-depletion signifies that TRPM4 is not expressed. Presence of a current activatable by ATP-depletion with Cs⁺ as the charge carrier strongly indicates the presence of TRPM4, which is further characterized in subsequent studies (SERIES 1B-E).
A general approach to channel activation by ATP-depletion is similar to previous studies with Na azide in astrocytes (Simard *et al.,* 2006), but because of the possibility that endothelial cells are more resistant to mitochondrial uncoupling than astrocytes, both mitochondrial function as well as glycolysis are inhibited. In many systems, tolerance to stress (e.g., hypoxia) is conferred by up-regulation of glycolytic pathways, with a key player in this response being the GLUT-1 glucose transporter, which is abundantly expressed by CNS endothelium (Abbott, 2002). Moreover, in some systems, glycolysis alone without mitochondrial function is sufficient to maintain high level cell function (Beckner *et al.,* 1990). It is useful to determine whether endothelial cells are more tolerant than astrocytes to mitochondrial poisoning, and therefore less likely to open ATP-sensitive channels unless glycolysis is suppressed.
   These screening studies on all preparations under all conditions (9 total) determines whether a particular preparation/condition is associated with an ATP-sensitive current and thus is worthy of further study. If so, then other properties of this conductance are characterized as follows:
SERIES 1B. The single channel slope conductance is obtained by measuring single channel currents at various membrane potentials. The slope conductance of NC_{Ca-ATP} and TRPM4 in different preparations is 25-35 pS. The slope conductance is measured using Na⁺, K⁺ and Cs⁺ as the charge carrier, at different pH's including pH 7.9, 7.4, 6.9 and 6.4. The slope conductance with Na⁺ is relevant to normal physiological function with normal ionic gradients found *in vivo.* The slope conductance with Cs⁺ assures that a K⁺ channel is not involved. Study of conductance at different values of pH is useful for determining channel properties in CNS injury, which is associated with acidic pH.
SERIES 1C. The probability of channel opening (nPₒ) is measured at different concentrations of intracellular calcium ([Ca²⁺]ᵢ), at different pH's including pH 7.9, 7.4, 6.9 and 6.4. Both the NC_{Ca-ATP} and TRPM4 channels are regulated by [Ca²⁺]ᵢ. However, in many systems, Ca²⁺ binding is opposed by H⁺, and thus an important aspect is whether channel opening is impeded at low pH due to interference with Ca²⁺ activation of the channel.
SERIES 1D. The concentration-response relationship for channel inhibition by ATP is measured at pH 7.9, 7.4, 6.9 and 6.4. Both the NC_{Ca-ATP} and TRPM4 channels are inhibited by ATP (Simard *et al.,* 2007). There is a potentially important interaction between hydrogen ion and nucleotide binding that may also be very important in the context of CNS injury, and thus these measurements are performed at various values of pH.
SERIES 1E. The divalent cation permeability is determined by measuring whole cell currents at various membrane potentials before and after replacing extracellular monovalent cations with 75 mM Ca²⁺ or Mg²⁺. This study demonstrates only outward current, no inward current, consistent with: (i) monovalent outward current; (ii) absent inward current; and (iii) no anion current (Chen and simard, 2001; Chen *et al.,* 2003; Simard *et al.,* 2006).
   IN SERIES 2, TRPM4 channel inhibition is studied by estrogen and PIP2 depletion, using the 2 culture systems detailed above.
SERIES 2A. Concentration-response data is obtained for estrogen (increasing concentrations from 10⁻¹ to 10⁺⁵ pM; 5 cells per concentration) using cell-attached patches (Cs⁺ as the charge carrier) of cells in which the NC_{Ca-ATP} channel is activated by ATP depletion induced by 1 mM Na azide plus 5 mM 2-deoxyglucose (2-DG). For these studies, single exposure to 17β-estradiol is used, with no cumulative dosing, to prevent desensitization of the response. From these data, measurements of the fractional reduction in the probability of channel opening are obtained. These values are normalized to compute the dose-response relationship.
SERIES 2B. Estrogen is known to activate PLC, resulting in generation of IP3 and DAG (Le Mellay *et al.,* 1999). Blockers of PLC are tested to ascertain whether they will inhibit the effect of estrogen on the NC_{Ca-ATP} channel. Effects of two-isoform specific inhibitors of PLC are tested, D609 (200 µM) and U73122 (100 µM), which selectively inhibit phosphatidylcholine-specific PLC (PC-PLC) and phosphatidylinositide-specific PLC (PI-PLC), respectively (Halstead *et al.,* 1995; Kucich *et al.,* 2000). In specific embodiments, PLC is crucial to the estrogen effect, since in some embodiments of the invention the PLC substrate, PIP₂, becomes depleted following estrogen-receptor occupation.
SERIES 2C. PKCδ, a downstream product of PLC activation, is translocated from cytosol to membrane fractions upon activation of the PLC-PKC pathway in R1 astrocytes (Perillan *et al.,* 2002). First, translocation of PKCδ in endothelial cells is confirmed following exposure to estrogen. For this, confocal microscopy and Western blots are utilized, as done previously (Perillan *et al.,* 2002). Next, it is assessed whether the effect of estrogen can be mimicked using the PKC activator, PMA (500 nM), and whether the effect of estrogen can be blocked with the pan-specific PKC inhibitor, calphostin C (100 nM). In one embodiment, PLC but not PKC is required for the estrogen effect on the channel, and so one can expect that, though PKC may be translocated, that inhibition of PKC will have no effect. Nevertheless, these are useful control experiments concerning PIP₂ depletion.
SERIES 2D. To verify that PIP₂ must be consumed for channel inhibition, one can mimic the effect of estrogen-mediated PLC activation by introducing exogenous PLC into the cell. These studies are carried out like the studies of SERIES 2A with estrogen, except that, instead of estrogen, cells are exposed to exogenous PLC (Ignotz and Honeyman, 2000) The purpose of these studies is to ascertain whether (presumed) PIP₂ depletion accompanying estrogen-receptor occupancy inhibits channel activation. Unfortunately, direct measurement of PIP₂ levels is not feasible, and so the approach taken here mimics that used by others (Xie *et al.,* 1999). Use of exogenous PLC helps establish the central role of PLC activity, devoid of other potentially confounding effects of estrogen-receptor occupancy.
SERIES 2E. The previous studies show that depletion of PIP₂ by endogenous or exogenous PLC activity decreases channel opening. In these studies, the opposite is shown, that adding PIP₂ increases channel activity. The concentration-response relationship is determined for ATP-inhibition of single channel NC_{Ca ATP} currents in inside-out patches before and after addition of PIP₂ (10 and 50 µM) to the bath. Incorporation of PIP₂ into the membrane is time-dependent (Xie *et al.,* 1999; Baukrowitz *et al.,* 1998), so one can wait 5 min after addition of PIP₂ to record data. The purpose of these studies is to establish that increased levels of PIP₂ facilitate activation of the channel, causing an apparent decrease in affinity for ATP. Single-channel measurements are performed of data from individual patches in which the probability of opening is similar with two different chemical conditions: (i) no added PIP₂ and low [ATP]; (ii) added PIP₂ and high [ATP]. This analysis allows further functional comparison between the pore-forming subunit of the NC_{Ca-ATP} channel and Kir6.x, both of which appear to possess an integral binding site for ATP (Proks *et al.,* 1999) distinct from SUR1, and that is not sensitive to trypsin (see Fig. 10 of Chen *et al.,* 2003).
IN SERIES 3, the role of the TRPM4 channel in death of endothelial cells is assessed using the 2 culture systems detailed above. For the cell death experiments, a general approach is similar to previous studies with Na azide in astrocytes (Simard *et al.,* 2006), but as detailed above, one can induce ATP depletion by exposure to 1 mM Na azide plus 5 mM 2-deoxyglucose. In parallel studies, one can confirm ATP depletion using a standard chemoluminescent technique (Sigma).

ONCOTIC BLEBBING AND CELL DEATH WITH ATP DEPLETION. It was previously reported that Na azide-induced ATP depletion caused cell blebbing and swelling and eventually cell death due to activation of NC_{Ca-ATP} channels (Chen and Simard, 2001). Here, one examines the effect of ATP depletion, obtained as described above, on morphology and death of endothelial cells. Phase contrast, DIC and scanning are used as well as transmission electron microscopy to assess morphological changes associated with ATP depletion. In cells expressing the channel, one can expect to see progressive bleb formation, cytoplasmic clearing and eventually membrane rupture signifying oncotic cell death, with changes consistent with necrotic rather than apoptotic death. Studies using the TRPM4 blocker, flufenamic acid, are performed to examine the effect of pharmacological block. However, flufenamic acid is not a specific inhibitor.

Definitive evidence of TRPM4 involvement in cell death can come from parallel studies using cells in which TRPM4 expression is suppressed by siRNA. As with native cells treated with flufenamic acid, one can expect that gene suppression will yield complete protection from blebbing and oncotic death expected with ATP depletion.

These morphological observations are supplemented with measurements relevant to cell death, including labeling with propidium iodide, and labeling for annexin V and activated caspase-3, as well as measuring LDH release in the medium, and measuring caspase activity.

### Specific methods:

ISOLATION OF SPINAL CORD MICROVESSELS WITH ATTACHED CAPILLARIES. The method used is adapted from others (Harder *et al.,* 1994), with modifications (Seidel *et al.,* 1991). Briefly, a rat undergoes transcardiac perfusion of 50 ml of heparinized PBS containing a 1% suspension of iron oxide particles (10 µm; Aldrich Chemical Co.). The contused spinal cord is removed, the pia and pial vessels are stripped away, the cord is split longitudinally and white matter bundles are stripped away to leave mostly gray matter tissue, which is minced into pieces 1-2 mm³. Tissue pieces are incubated with dispase II (2.4 U/ml; Roche) for 30 min with agitation in the incubator and are triturated with a fire-polished Pasteur pipette. Microvessels are adhered to the sides of 1.5 ml Eppendorf tubes by rocking 20 min adjacent to a magnet (Dynal MPC-S magnetic particle concentrator; Dynal Biotech, Oslo, Norway). Isolated microvessels are washed in PBS x2 to remove cellular debris and are stored at 4° C in physiological solution (Seidel *et al.,* 1991). Capillary endothelial cells near the end of the visualized microvascular tree are targeted for patch clamping.

PRIMARY CULTURES OF SPINAL CORD CAPILLARY ENDOTHELIAL CELLS. The method has been described in detail (Ge and Pachter, 2006; Wu *et al.,* 2003) 10-w old C57BL/6 mice are used. Cords from 5-6 mice are collected and rinsed aseptically. Tissues are homogenized using a Dounce tissue grinder with 3 strokes, in complete ECM 1001 solution. The homogenate is resuspended in 15% Dextran, centrifuge at 10,000xg for 15 min at 4°C. The resuspended pellet is placed in 0.1% collagenase/dispase and incubate at 37°C 3 hr. The digestate is centrifuge at 1,000xg for 3 min, the pellet is resuspended in 45% Percoll and centrifuge at 20000xg for 10 min at 4°C. The top layer is transferred to a new 50 ml conical tube and washed once with PBS, once with HBSS and centrifuge at 1000xg for 3 min. The pellet is resuspended with ECM 1001 and plated on collagen-coated surfaces. Cultures are incubated in 5% CO₂. When confluent, cells are detached with dissociation buffer. Cells are incubated with rat anti-mouse CD31 for 20 min and washed with HBSS twice. Cells are then incubated with Dynabeads M-450 sheep anti-rat IgG for 20 min. Cell-bound beads are collect with a magnet and are plated on collagen-coated dishes.

ENDOTHELIAL CELL CULTURE. bEnd.3 cells (ATCC) are cultured under normoxic conditions (5% CO₂/95% room air) as recommended by the supplier.

PATCH CLAMP. Details of the protocols that is used for inside-out patches may be found in Chen and Simard (2001); Chen *et al.* (2003); Perillan *et al.* (2002); and Perillan *et al.* (2000). In general, recordings are carried out using a Cs⁺ rich solution, to preclude recording activity from any K⁺ channel including K_{ATP} channel.

RNAi. Expression of TRPM4 will be directly inhibited by transfecting siRNA of TRPM4 in bEnd.3 cells, using methods as previously reported with similar endothelial cells. siRNA transfection are carried out using Hiperfect transfection reagent (Qiagen) according to the manufacturer's protocol. Targeting RNA duplex are purchased (Dharmacon).

WESTERN BLOTS are performed as described (Simard *et al.,* 2006; Perillan *et al.,* 2002; Gerzanich *et al.,* 2003; Gerzanich *et al.,* 2003).

CONFOCAL MICROSCOPY. Cells are plated on chamber slides (LAB-TEK, Naperville, IL) for 24-48 h as previously described (Perillan *et al.,* 2002). Cultures are exposed to estrogen (10 nM) for 5 min, rinsed and then fixed. For confocal imaging, the samples are examined using a Zeiss LSM510 confocal microscope. Details of the protocol for confocal microscopy for assessment of PKC isoform translocation may be found in Perilan *et al.* (2002).

SCANNING ELECTRON MICROSCOPY (SEM) One can study cell blebbing and swelling as previously described (Chen and Simard, 2001). Cell cultures are exposed at room temperature to Na azide plus 2-DG then, after various time intervals, cells are fixed using iced 4% formaldehyde + 1% glutaraldehyde for 24 h then dehydrated using serial concentrations (Simard *et al.,* 2007; Beyer *et al.,* 2002; Sribnick *et al.,* 2005; Beckner *et al.,* 1990), 100%) of ethanol (Jewell *et al.,* 1982). Specimens are critical point dried (Tousimis), gold coated (Technics), and viewed using an AMR 1000 scanning electron microscope.

LACTATE DEHYDROGENASE (LDH) RELEASE ASSAY. LDH release is measured using a commercially available assay (Cytotoxicity Detection Kit; Roche Molecular Biochemicals) (Wang *et al.,* 2006).

CASPASE ACTIVITY ASSAY. Caspase activity is measured as described. (Wang *et al.,* 2006). Caspase-3 fluorogenic substrate, Ac-DEVD-AFC and Caspase-8 fluorogenic substrate, Ac-IETD-AFC were from BD Biosciences (Franklin Lakes, NJ). Caspase activity in cell lysates is determined according to the manufacturer's instructions, using a plate reader and expressed as arbitrary fluorescence units.

CELL DEATH ASSAY. Methods to be used are as previously described (Simard *et al.,* 2006). Endothelial cell cultures are plated on 4-well chamber slides (Lab-Tek, Nalge Nunc International) in physiological solution (10⁴ cells/100µl/well) supplemented with either: vehicle; Na azide plus 2-DG; or 100 µM flufenamic acid followed 5 min later with Na azide plus 2-DG. Ten min after adding Na azide plus 2-DG, plates are assayed using propidium iodide (PI) and annexin V (Vybrant Apoptosis Assay Kit 2, Molecular Probes).

DATA ANALYSIS. Standard statistical methods are used, including ANOVA and t-test, as appropriate for individual experiments. As usual, p<0.05 is taken as the measure of significance.

### EXAMPLE 6

### ROLE OF THE TRANSCRIPTION FACTOR, NUCLEAR FACTOR-κB (NFκB), IN EXPRESSION OF TRPM4

Using various cultured cell lines and tissues from a rat SCI model, the role of the transcription factor, nuclear factor-κB (NFκB), is determined in expression of TRPM4.

NFκB plays a role in transcriptional regulation of TRPM4, in particular embodiments of the invention. Studies using reporter gene analysis, electrophoretic mobility shift assay (EMSA), chromatin immunoprecipitation (ChIP) and gene suppression *in vitro* are employed to establish the role of specific NFκB subunits in expression of TRPM4 protein and functional TRPM4 channels, and experiments with EMSA, ChIP and gene suppression are employed *in vivo* to corroborate the *in vitro* experiments and to establish the role of NFκB vis-à-vis TRPM4 expression in PHN following SCI.

**Nuclear translocation of NFκB to endothelium *in vivo* in SCI.** NFκB is well known to undergo nuclear translocation in SCI, where it contributes to transcriptional up-regulation of inflammatory cytokines (Bethea *et al.,* 1998; Brambilla *et al.,* 2002). Evidence has been presented that intrinsic NFκB originates mostly from astrocytes (Brambilla *et al.,* 2005). However, astrocyte-related activity would not easily account in any direct manner for PHN, which by necessity involves capillaries and post-capillary venules.

Cords were immunolabeled for NFκB at different times post-SCI. Nuclear localization of NFκB was found as early as 45 min, including in endothelial cells identified by vimentin co-labeling (FIG. 25). Nuclear Westerns corroborated nuclear translocation of the p65 subunit of NFκB at 45 min (FIG. 25).

**NFκB binds to rat TRPM4 promoter.** Sequence analysis of the rat TRPM4 promoter region within ∼2 kb from the transcription start site in 5' flanking region, revealed 1 consensus NFκB binding site (GGGRNNYYCC (SEQ ID NO:3); R=purine, Y=pyrimidine, and N=any nucleotide) at position -73. EMSA was used to assess the potential interaction between the NFκB consensus site on the TRPM4 promoter and the NFκB subunit, p65. EMSA showed a specific binding of NFκB to the consensus sequence of the TRPM4 promoter (FIG. 26).

NFκB inhibitor reduces TRPM4 expression post-SCI. Blockade of NFκB activity either indirectly by methylprednisone (Xu *et al.,* 1998) or by pyrrolidine dithiocarbamate (PDTC), a specific NFκB inhibitor shown to decrease NFκB activity (Jimenez-Garza *et al.,* 2005; La Rosa *et al.,* 2004), is beneficial in SCI, increasing the amount of spared tissue. Rats were treated post-SCI with PDTC (100 mg/kg, ip) (Jimenez-Garza *et al.,* 2005). The animals had improved functional outcome (not shown) as reported (Jimenez-Garza *et al.,* 2005). Of note, immunolabeling cord sections showed that TRPM4 was significantly decreased (FIG. 27), which indicates NFκB is an important regulator of TRPM4 transcription.

*Using various cultured cell lines and tissues from a rat SCI model, the role of the transcription factor, nuclear factor-κB (NFκB), in expression of TRPM4 and of functional TRPM4 channels is determined.*

In a specific embodiment, NFκB is employed in transcriptional regulation of TRPM4. In another specific embodiment, there is identification of transcription factors involved in expression of NC_{Ca-ATP} channel subunits, including TRPM4, because transcriptional mechanisms are useful therapeutic targets (Simard *et al.,* 2007).

An analysis of the promoter regions of TRP proteins of the TRPM and TRPC families was recently published (Simard *et al.,* 2007). Analysis of the 5' flanking region of the mouse TRPM4 promoter showed the presence of a consensus NFκB binding site (GGGRNNYYCC (SEQ ID NO:3) at position -73 relative to the start site). NFκB has been shown to play a central role in the induction of genes mediating the inflammatory response, development, cellular growth, and apoptosis.

In SCI, NFκB is activated in neurons, microglia, and endothelial cells, with protein seen in the nucleus as early as 30-45 min (Bethea *et al.,* 1998). Blockade of NFκB activity indirectly by either methylprednisone (Xu *et al.,* 1998) or PDTC (Jimenez-Garza *et al.,* 2005; L Rosa *et al.,* 2004) is beneficial in SCI. Genetic block of NFκB activity through transgenic expression of a constitutively active IκB in astrocytes leads to reduced glial scar formation, preserved white matter, and improved functional outcome (Brambilla *et al.,* 2005). The increased expression of NFκB in SCI is due in part to the release of pro-inflammatory cytokines by all classes of neural cells (Pineau and Lacroix, 2007). Among other things, activation of NFκB in the endothelium causes expression of cell adhesion molecules resulting in the recruitment of immune cells that then continue cytokine synthesis for days.

In a specific embodiment, NFκB is critical for transcription of TRPM4 and for secondary injury mediated by TRPM4 channel in SCI *in vivo.*

Studies: studies using reporter gene analysis, electrophoretic mobility shift assay (EMSA), chromatin immunoprecipitation (ChIP), and gene suppression *in vitro* are performed to establish the role of NFκB in expression of TRPM4 channels, and studies with EMSA, ChIP and gene suppression *in vivo* to corroborate the *in vitro* experiments and to establish the role of NFκB in PHN following SCI.

SCI MODEL. For some of the studies below, one can use cord tissue obtained following SCI. For these studies, adult female Long Evans rats are subjected to cervical SCI under anesthesia using the NYU impactor (10 gm x 25 mm).

IN SERIES 1, one can use reporter gene analysis of *cis*-acting TRPM4 promoter elements to show that activating NFκB drives TRPM4 expression. Point mutations of putative NFκB binding sites in the promoter region are used to confirm involvement of putative sites. Appropriate site-directed mutagenesis of putative NFκB binding sites in the TRPM4 promoter are performed, as directed by these studies, and transfections repeated to test the necessity of these *cis*-acting sequences. Functionally important sites are further analyzed by gel-mobility shift assay using nuclear extracts from the cell lines and the proteins in the bound complexes identified by antibody supershift analysis. Control mobility shift reactions include TNFα stimulated HeLa cell extracts (positive) and no extract (negative).

The mouse TRPM4 promoter is cloned, and sequential deletions of putative binding sites for NFκB are made using appropriate PCR primers to generate a set of nested promoter constructs driving firefly luciferase reporter gene expression. As proof of principle, lipid-mediated transient transfection of these constructs along with a CMV-Renilla luciferase plasmid are performed on HeLa cells (an immortalized human cervical epithelioid carcinoma cell line), because these cells have been shown to respond to NFκB activators and are well characterized. Mouse brain endothelial cells (bEnd.3; ATCC) are transfected as well, to more closely approximate *in vivo* endothelial cell activities. (Transfection methods in bEnd.3 cells, including for p50 and p65 NFκB subunits, have been described (Xiao *et al.,* 2005; Zhu *et al.,* 2003)) These studies confirm mouse TRPM4 promoter activity as well as assess important *cis-*acting transcription control elements.

Cells transfected with TRPM4 promoter reporter constructs are exposed to TNFα to determine the role of the putative NFκB binding sites 5' of the transcriptional start sites. p4xNF-κB Luc serves as a positive control.

To test the sufficiency of NFκB to activate the TRPM4 promoter, TRPM4 reporter plasmids are co-transfected with plasmids expressing the appropriate NFκB subunit(s) as determined in Series 3 experiments, described below. The necessity for NFκB is tested by RNAi co-transfection of cells using duplex RNA targeted to respective NFκB subunits (to reduce NFκB protein expression), along with reporter plasmids (for additional detail, see Series 4, below). These studies determine the importance of NFκB to TRPM4 promoter activation *in vitro.*

IN SERIES 2, nuclear Westerns and double labeling immunohistochemistry of SCI tissues are used to show temporal and spatial correlation between nuclear localization of NFκB and TRPM4 expression, with special emphasis on endothelial cells in cord post-SCI.

These studies are based on data that verify published findings that NFκB undergoes early nuclear translocation following SCI (Bethea *et al.,* 1998), although specific involvement of endothelium can be determined. Cords of rats subjected to contusion SCI are analyzed at ½, 1, 3, 6 and 24 h post-SCI by immunohistochemical analysis of frozen and paraffin-embedded sections and nuclear Western blots for NFκB subunits. Double labeling using antibodies directed against TRPM4, von Willebrand factor and vimentin are performed to co-localize NFκB subunits and TRPM4 in endothelial and other cells.

IN SERIES 3, electrophoretic mobility shift assay (EMSA) and chromatin immunoprecipitation (ChIP) are used to identify which NFκB subunits undergo a specific protein-DNA interaction with the consensus NFκB binding site identified in the TRPM4 promoter.

For these studies, EMSA and ChIP are carried out using both bEnd.3 cells and tissues from SCI. Nuclear extracts from bEnd.3 cells exposed to TNFα and from rat tissue post-SCI are analyzed. Cords are obtained at ½, 1, 3, 6 and 24 h post-SCI, with controls including sham operated and naive tissues. Studies with bEnd.3 cells establish the validity of the concept in endothelium, whereas experiments with the cord tissues establish its validity specifically *in vivo* in SCI.

Biotinylated ∼22 bp-long single-stranded oligonucleotides containing the respective NFκB binding site of the rat TRPM4 promoter are synthesized, annealed with each complementary oligonucleotide, and used for EMSA. EMSA initially determines which NFκB binding sites can be involved, and supershift assay of EMSA and ChIP analysis further identifies subunits of NFκB bound to the sites.

The binding specificity of NFκB subunits is verified by supershift assay using antibodies directed against the 5 known subunits of NFκB (all 5 are available from Santa Cruz Biotechnology and have been shown to work with EMSA (Hoffmann *et al.,* 2003)), with studies carried out as described (Hoffman *et al.,* 2003) Knowledge of involvement of specific NFκB subunits is useful for subsequent gene suppression experiments described below.

IN SERIES 4, gene suppression with siRNA is used to show that knocking down specific NFκB subunits *in vitro* results in concurrent decreases in TRPM4 mRNA and protein, and in functional TRPM4 channels.

For these studies, one can study bEnd.3 cells, which the inventors show up-regulate TRPM4 protein and functional channels under appropriate conditions, including exposure to TNFα. Gene silencing using siRNA has previously been reported with similar endothelial cells 10⁶. Here, expression of individual NFκB subunit(s) identified as involved in Series 3, are silenced, following which the cells are exposed to TNFα (and/or hypoxia). A mixture of siRNA duplex targeting 4 different regions of a mouse NFκB subunit mRNA are purchased and transfected into bEnd.3 cells to inhibit activity of NFκB (ON-TARGET plus siRNA from Dharmacon). Renilla luciferase-targeted duplex RNAi is used as a negative control (RL duplex from Dharmacon). It is also used to determine transfection efficiency by measuring Renilla luciferase activity after co-transfection with a CMV-Renilla luciferase plasmid.

To determine the efficiency of siRNA in blocking NFκB activity, immunoblots of appropriate NFκB subunits are performed.

After verifying the siRNA effect, mRNA and protein abundance of TRPM4 are determined by quantitative RT-PCR and immunoblot, respectively. These cells are also studied electrophysiologically to determine presence of functional channels.

IN SERIES 5, one can use a pharmacological agent (pyrrolidine dithiocarbamate) and gene suppression (AS-ODN), to show that inhibiting NFκB or selectively knocking down NFκB subunits *in vivo* results in a decrease in TRPM4, improvement in the various manifestations of PHN and a concomitant improvement in neurobehavioral function.

For pharmacological inhibition, pyrrolidine dithiocarbamate (PDTC, 100 mg/kg, ip), a specific NFκB inhibitor, is used to decrease NFκB activity (Jimenez-Garza *et al.,* 2005)

For *in vivo* gene suppression, AS-ODN is used because its utility has already been demonstrated in SCI, and because other strategies such as RNAi are significantly more costly to implement in the rat. AS-ODN is directed against the appropriate NFκB subunits (as determined from supershift assay of EMSA and ChIP in Series 3). Scrambled ODN (Scr-ODN) serves as control. ODN is delivered i.v. (intra jugular) by mini-osmotic pump, as previously (see data with AS-ODN, FIG. 18).

Groups of rats subjected to SCI are treated to inhibit NFκB, as described. Cords are obtained at ½, 1, 3, 6 and 24 h post-SCI. To determine efficacy of the different treatment, cords are assessed for nuclear NFκB using nuclear Western blots. Treatment endpoints to be evaluated include: (i) TRPM4 protein and mRNA expression; (ii) inflammatory response, to be assessed using immunohistochemistry and measurement of myeloperoxidase enzymatic activity (MPO) or ED-1 as markers of PMN and macrophages/microglia, respectively (Carlson *et al.,* 1998); (iii) manifestations of PHN, including edema, hemorrhage, lesion size, and neurobehavioral function.

### Specific methods:

LUCIFERASE REPORTER ASSAY is carried out as previously described (Woo *et al.,* 2002) A ∼2 kb-long portion of the promoter region of the mouse TRPM4 gene (-1905 to +66 and/or -1544 to +66 relative to transcription start site) is cloned using polymerase chain reaction (PCR), and inserted into a reporter plasmid to drive expression of the luciferase gene. The NFκB luciferase reporter system (p4xNF-κB Luc; Clontech Laboratories) serves as a positive control for NFκB activity. Co-transfection of pRL-CMV, Renilla luciferase expression plasmid serves as a control for transfection efficiency. Twenty-four hours following transfection, cells are treated with and without 20 ng/mL TNFα for 12-24 hr. Luciferase activity of cell extracts is determined using the Dual Luciferase system from Promega. Cell viability is determined using the standard Trypan blue dye exclusion method.

SITE-DIRECTED MUTAGENESIS is carried out as previously described (Woo *et al.,* 2002). The NFκB binding sites of the TRPM4 promoter are inactivated by site-directed mutagenesis using the Quick Change II XL site-directed mutagenesis kit (Stratagene): GGGRNNYYCC (SEQ ID NO:3) to cccRNNYYCC (SEQ ID NO:4) (mutated nucleotides are indicated by lowercase letters). All the mutations are verified by sequencing, and the mutated TRPM4 promoter is moved into the luciferase reporter plasmid, pGL3-basic. HeLa cells are transfected with the wild-type and mutant versions of the reporter plasmids, and luciferase activity is compared.

WESTERN BLOTS. Nuclear or cytoplasmic proteins isolated using the CelLytic Nu-CLEAR (Sigma) are analyzed by quantitative Western blot, as described (Simard *et al.,* 2006).

QUANTITATIVE RT-PCR for TRPM4 is performed using probes as previously described (Simard *et al.,* 2006).

ELECTROPHORETIC MOBILITY SHIFT ASSAYS (EMSA) / SUPERSHIFT ASSAY. Nuclear extracts are prepared from confluent cultured cells exposed to 20 ng/ml of TNFα or from post-SCI cords, as previously described (Simard *et al.,* 2006). To prepare probes for EMSA, 22 bp-long single-stranded oligonucleotides containing the NFκB binding site of the rat TRPM4 promoter region are synthesized and purified. To obtain a double-stranded probe, 200 pmol of each complementary oligonucleotide is annealed in 100 µl containing 150 mM NaCl, 10 mM MgCl₂, and 50 mM Tris-Cl, pH 7.9. Then, 10 µg of protein of nuclear extract is incubated for 10 min at 25°C in 20 µl containing 20 mM HEPES, pH 7.9, 50 mM KCl, 1 mM EDTA, 5% glycerol, 1 mM dithiothreitol, 5 mM MgCl₂, and 1.5 µg of poly(dA-dT). After that, 10 fmol of biotinylated probe is added and the mixture is incubated for an additional 20 min. The reaction mixtures are electrophoresed on a 6% polyacrylamide gel in a buffer containing 45 mM Tris, 45 mM borate, and 1 mM EDTA, transferred to nylon membrane, cross-linked, and the biotinylated DNA detected *via* Western blot using strepavidin-horseradish peroxidase as per manufacturer's instructions. Supershift assay is performed using EMSA-validated (Hoffman *et al.,* 2003) antibodies for all 5 known subunits of NFκB, which are available from Santa Cruz Biotechnology. Specific binding to the target sequence is verified by competition assay with excess amount of unlabeled probe.

CHROMATIN IMMUNOPRECIPITATION is performed as previously described (Simard *et al.,* 2006) using them same antibodies for the 5 known subunits of NFκB, available from Santa Cruz Biotechnology.

RNAi. Expression of NFκB is directly inhibited by transfecting siRNA of NFκB subunits in bEnd.3 cells, using methods as previously reported with similar endothelial cells (Culmsee *et al.,* 2006), siRNA transfection is carried out using Hiperfect transfection reagent (Qiagen) according to the manufacturer's protocol. Targeting RNA duplexes are purchased (Dharmacon) or if necessary are custom designed with the BLOCK-iT™ RNAi Express search engine (Invitrogen).

While these studies are targeted to identify the role of NFκB in TRPM4 regulation, in certain aspects NFκB does not directly affect TRPM4 transcription *in vivo* (or *in vitro*)*.* This is why studies can include reporter constructs with gross deletions to the 5' flanking region to identify potential regulatory sites by function and not merely by sequence.

### EXAMPLE 7

### TRPM4 AS THE PORE-FORMING SUBUNIT OF THE NC_{Ca}-_{ATP} CHANNEL

Immunolabeling for TRPM4 in rat models of intracerebral hemorrhage, stroke, blast head injury and spinal cord injury demonstrate expression of TRPM4 therein. FIG. 28 shows that TRPM4 is upregulated in cells and capillaries of penumbral tissues in rat models of hemorrhagic stroke (upper panels) and in ischemic stroke (lower panels). FIGS. 29A-29F demonstrate that TRPM4 is upregulated in cortex and thalamus in a rat model of traumatic brain injury induced by gunshot blast. FIGS. 31A-31H show that TRPM4 is up-regulated in capillaries in SCI. 31A,31B: Immunohistochemical localization of TRPM4 in control and 24 h post-SCI, with montages constructed from multiple individual images, and positive labeling shown in black pseudocolor; arrow points to impact site; red asterisks show sampling areas for panels 31C-31F. 31C-31E: Magnified views of TRPM4 immunolabeled sections taken from control (C) and from the "penumbra" (31D,31E). 31F: Immunolabeling of capillaries with vonWillebrand factor; same field as E. 31G: In situ hybridization for TRPM4 in the penumbra 24 h post-SCI. 31H: PCR of spinal cord tissue from control (CTR) and post-SCI from 3 regions, the impact site (SCI) and rostral (R), caudal (C). Images of immunohistochemistry and in situ hybridization are representative of findings in 3 rats/group.

In addition, capillary fragmentation and bleeding are prevented by exemplary TRPM4 antisense molecules. FIGS. 30A-30B show that TRPM4 up-regulation post-SCI is prevented by gene suppression using AS-ODN. FIGS. 30A,30B: Montages showing immunohistochemical localization of TRPM4 24 h post-SCI in a rat treated with sense (SE) ODN (30A) or with antisense (AS) ODN (30B); i.v. infusions of ODN were started 48 h before SCI; arrows point to impact sites. Furthermore, FIGS. 32A-32D demonstrate that capillary fragmentation is prevented by TRPM4 blockers. In FIGS. 32A-32D, the sections are immunolabeled for vimentin to show capillaries near the impact site in rats treated with vehicle (32A), flufenamic acid (FFA) (32B), sense (SE) ODN (32C) or antisense (AS) ODN (32D); there are fragmented capillaries in 32A and 32C *vs.* elongated capillaries in 32B and 32D. In addition, FIGS. 33A-33C illustrate that progressive hemorrhage is prevented by TRPM4 blockers. In FIGS. 33A and 33B, cord sections (33A) and cord homogenates (33B) from control rats (CTR or vehicle-treated), and rats treated with flufenamic acid (FFA), sense (SE) ODN, antisense (AS) ODN are provided (arrows point to distant petechial hemorrhages). In FIG. 33C, there is quantification of extravasated blood in cord homogenates in controls (●), in rats treated post-SCI with FFA (n=3), or post-SCI with SE-ODN (n=4) or AS-ODN (n=5).

TNFα causes upregulation of TRPM4 mRNA and protein and functional TRPM4 channels in bEnd3 cells. For example, FIGS 34A-34D show that the biophysical properties of the NC_{Ca-ATP} channel in bEnd.3 cells after exposure to TNFα are identical to TRPM4. In FIG. 34A, there is recording of inside-out patch showing 31 pS channel studied with Cs⁺ as the only permeant cation; the channel was reversibly blocked by ATP. In FIG. 34B, there is plot of single channel conductance. In FIG. 34C, outward cationic single channel currents at the membrane potential of +60 mV, in an inside-out patch with multiple channels, recorded in the presence on the cytoplasmic side of 0 CaCl₂/140 mM CsCl, 1 µM CaCl₂/140 mM CsCl and 75 mM CaCl₂/0 CsCl as indicated are provided, showing that: (i) Cs⁺ is permeable; (ii) physiological levels of Ca²⁺ are required for channel activity; (iii) Ca²⁺ is not permeable; (iv) Cl-is not permeable. In FIG. 34D, single channel activity recorded in the absence of ATP, blocked by the TRPM4 blocker, flufenamic acid (FFA) is demonstrated. Also, FIGS. 38A-38D demonstrate that TNFα causes up-regulation of TRPM4 mRNA and protein in bEnd.3 cells. 38A-38D: PCR (38A), immunolabeling (38B), and Western blots (38C,38D) for TRPM4 in bEnd.3 cells under control conditions and after 6-h exposure to 20 ng/mL TNFα.

Also, cells that express TRPM4 are susceptible to ATP-depletion induced death. In FIG. 35, for example, TRPM4 expression and opening predisposes to oncotic/necrotic cell death. Plasmids of mTRPM4-GFP and enhanced green fluorescence protein (EGFP; Clontech) were transfected into COS7 cells. ATP was depleted using Na azide plus 2-DG, then cells were assayed with propidium iodide as a marker of oncotic/necrotic death. ATP depletion resulted in oncotic/necrotic death of 80% of cells transfected with TRPM4 *vs.* 2% of cells transfected with control EGFP.

In SCI, behavior is improved and lesion size is decreased by TRPM4 antisense. For example, FIG. 39 illustrates that exemplary TRPM4-AS reduces lesion volume in rats post-SCI. In addition, FIGS. 36A-36D provide that flufenamic acid (FFA) and TRPM4 AS-ODN improve neurobehavioral function post SCI. In FIG. 36A, performance on inclined plane 24 h post-SCI in rats treated after SCI with TRPM4 SE-ODN, AS-ODN and FFA. 36B-36D is provided. Rearing behavior 24 h post-SCI in rats either pre-treated for 48 h (36C) or treated post-SCI (36D) with TRPM4 SE-ODN versus AS-ODN is also shown. Finally, FIG. 37 demonstrates that an exemplary TRPM4-AS improves neuro-behavioral performance in rats post-SCI. Performance on up-angled and down-angled plane at various times post-SCI in rats administered TRPM4 antisense (AS) or TRPM4 sense (SE) is shown.

### REFERENCES

Full citations for the references cited herein are provided in the following list.

### PATENTS AND PATENT APPLICATIONS

U.S. Patent No. 5,399,363
U.S. Patent No. 5,354,855
U.S. Patent No. 5,466,468
U.S. Patent No. 5,543,158
U.S. Patent No, 5,580,579
U.S. Patent No. 5,629,001
U.S. Patent No. 5,637,085
U.S. Patent No. 5,641,515
U.S. Patent No. 5,725,871
U.S. Patent No. 5,756,353
U.S. Patent No. 5,780,045
U.S. Patent No. 5,792, 451
U.S. Patent No. 5,804,212
U.S. Patent No. 6,391,911
U.S. Patent No. 6,613,308
WO 99/32619
WO 00/44914
WO 01/68836
WO 01/36646
WO 03/079987
WO 04/046320

### PUBLICATIONS

Aarts MM, Tymianski M. TRPMs and neuronal cell death. Pflugers Arch 2005; 451:243-9.
Aarts, M. M., M. Tymianski, TRPM7 and ischemic CNS injury, Neuroscientist. 11 (2005) 116-123.
Aarts, M., K. Iihara, W. L. Wei, Z. G. Xiong, M. Arundine, W. Cerwinski, J. F. MacDonald, M. Tymianski, A key role for TRPM7 channels in anoxic neuronal death, Cell 115 (2003) 863-877.
Abbott NJ. Astrocyte-endothelial interactions and blood-brain barrier permeability. J Anat 2002;200:629-38.
Abbruscato, T. J., T. P. Davis, Combination of hypoxia/aglycemia compromises in vitro blood-brain barrier integrity, J. Pharmacol. Exp. Ther. 289 (1999) 668-675.
Abman SH, Chatfield BA, Hall SL, McMurtry IF. Role of endothelium-derived relaxing factor during transition of pulmonary circulation at birth. Am J Physiol. 1990;259:H1921-H1927.
Abman SH, Chatfield BA, Rodman DM, Hall SL, McMurtry IF. Maturational changes in endothelium-derived relaxing factor activity of ovine pulmonary arteries in vitro. Am J Physiol. 1991;260:L280-L285.
Abumiya T, Yokota C, Kuge Y, Minematsu K. Aggravation of hemorrhagic transformation by early intraarterial infusion of low-dose vascular endothelial growth factor after transient focal cerebral ischemia in rats. Brain Res 2005; 1049:95-103.
Ahmmed GU, Malik AB. Functional role of TRPC channels in the regulation of endothelial permeability. Pflugers Arch 2005; 451:131-42.
Allen, N. J., D. Attwell, Modulation of ASIC channels in rat cerebellar Purkinje neurons by ischaemia-related signals, J. Physiol 543 (2002) 521-529.
Amiry-Moghaddam M, Ottersen OP. The molecular basis of water transport in the brain. Nat Rev Neurosci 2003; 4:991-1001.
Ammala C, Moorhouse A, Gribble F, Ashfield R, Proks P, Smith PA, Sakura H, Coles B, Ashcroft SJ, Ashcroft FM. Promiscuous coupling between the sulphonylurea receptor and inwardly rectifying potassium channels. Nature. 1996;379:545-548.
Anderson KD, Abdul M, Steward O. Quantitative assessment of deficits and recovery of forelimb motor function after cervical spinal cord injury in mice. Exp Neurol 2004;190:184-91.
Asahi M, Asahi K, Jung JC, del Zoppo GJ, Fini ME, Lo EH. Role for matrix metalloproteinase 9 after focal cerebral ischemia: effects of gene knockout and enzyme inhibition with BB-94. J Cereb Blood Flow Metab 2000; 20:1681-9.
Asahi M, Asahi K, Wang X, Lo EH. Reduction of tissue plasminogen activator-induced hemorrhage and brain injury by free radical spin trapping after embolic focal cerebral ischemia in rats. J Cereb Blood Flow Metab 2000; 20:452-7.
Asahi M, Wang X, Mori T, Sumii T, Jung JC, Moskowitz MA et al. Effects of matrix metalloproteinase-9 gene knock-out on the proteolysis of blood-brain barrier and white matter components after cerebral ischemia. J Neurosci 2001; 21:7724-32.
Ashfield R, Ashcroft SJ. Cloning of the promoters for the beta-cell ATP-sensitive K-channel subunits Kir6.2 and SUR1. Diabetes 1998; 47:1274-80.
Askwith, C. C., J. A. Wemmie, M. P. Price, T. Rokhlina, M. J. Welsh, Acid-sensing ion channel 2 (ASIC2) modulates ASIC1 H+-activated currents in hippocampal neurons, J. Biol. Chem. 279 (2004) 18296-18305.
Ayata C, Ropper AH. Ischaemic brain oedema. J Clin Neurosci 2002; 9:113-24.
Babaei S, Teichert-Kuliszewska K, Monge JC, et al. Role of nitric oxide in the angiogenic response in vitro to basic fibroblast growth factor. Circ Res. 1998;82:1007-1015
Badaut J, Lasbennes F, Magistretti PJ, Regli L. Aquaporins in brain: distribution, physiology, and pathophysiology. J Cereb Blood Flow Metab 2002; 22:367-78.
Balentine JD. Pathology of experimental spinal cord trauma. I. The necrotic lesion as a function of vascular injury. Lab Invest 1978;39:236-53.
Balzer, M., B. Lintschinger, K. Groschner, Evidence for a role of Trp proteins in the oxidative stress-induced membrane conductances of porcine aortic endothelial cells, Cardiovasc. Res. 42 (1999) 543-549.
Banasiak KJ, Burenkova O, Haddad GG. Activation of voltage-sensitive sodium channels during oxygen deprivation leads to apoptotic neuronal death. Neuroscience 2004; 126:31-44.
Barros LF, Castro J, Bittner CX. Ion movements in cell death: from protection to execution. Biol Res 2002; 35:209-14.
Barros LF, Hermosilla T, Castro J. Necrotic volume increase and the early physiology of necrosis. Comp Biochem Physiol A Mol Integr Physiol 2001; 130:401-9.
Bassler, E. L., T. J. Ngo-Anh, H. S. Geisler, J. P. Ruppersberg, S. Grunder, Molecular and functional characterization of acid-sensing ion channel (ASIC) 1b, J. Biol. Chem. 276 (2001) 33782-33787.
Basso DM, Fisher LC, Anderson AJ, Jakeman LB, McTigue DM, Popovich PG. Basso Mouse Scale for locomotion detects differences in recovery after spinal cord injury in five common mouse strains. J Neurotrauma 2006;23:635-59.
Baukrowitz T, Schulte U, Oliver D, Herlitze S, Krauter T, Tucker SJ, Ruppersberg JP, Fakler B. PIP2 and PIP as determinants for ATP inhibition of KATP channels. Science 1998;282:1141-4.
Beck J, Lenart B, Kintner DB, Sun D. Na-K-Cl cotransporter contributes to glutamate-mediated excitotoxicity. J Neurosci 2003; 23:5061-8.
Beckner ME, Stracke ML, Liotta LA, Schiffmann E. Glycolysis as primary energy source in tumor cell chemotaxis. J Natl Cancer Inst 1990;82:1836-40.
Beech, D. J., TRPC1: store-operated channel and more, Pflugers Arch. 451 (2005) 53-60.
Benveniste, M., R. Dingledine, Limiting stroke-induced damage by targeting an acid channel, N. Engl. J. Med. 352 (2005) 85-86.
Berger, C., P. C. Schmid, W. R. Schabitz, M. Wolf, S. Schwab, H. H. Schmid, Massive accumulation of N-acylethanolamines after stroke. Cell signalling in acute cerebral ischemia?, J. Neurochem. 88 (2004) 1159-1167.
Bethea JR, Castro M, Keane RW, Lee TT, Dietrich WD, Yezierski RP. Traumatic spinal cord injury induces nuclear factor-kappaB activation. J Neurosci 1998;18:3251-60.
Betz AL, Ennis SR, Schielke GP, Hoff JT. Blood-to-brain sodium transport in ischemic brain edema. Adv Neurol 1990; 52:73-80.
Betz AL, Iannotti F, Hoff JT. Brain edema: a classification based on blood-brain barrier integrity. Cerebrovasc Brain Metab Rev 1989; 1:133-54.
Betz AL. Alterations in cerebral endothelial cell function in ischemia. Adv Neurol 1996; 71:301-11.
Bevan, S., J. Yeats, Protons activate a cation conductance in a sub-population of rat dorsal root ganglion neurones, J. Physiol 433 (1991) 145-161.
Beyer C, Ivanova T, Karolczak M, Kuppers E. Cell type-specificity of nonclassical estrogen signaling in the developing midbrain. J Steroid Biochem Mol Biol 2002;81:319-25.
Bilgen M, Abbe R, Liu SJ, Narayana PA. Spatial and temporal evolution of hemorrhage in the hyperacute phase of experimental spinal cord injury: in vivo magnetic resonance imaging. Magn Reson Med 2000;43:594-600.
Blanco JE, Anderson KD, Steward O. Recovery of forepaw gripping ability and reorganization of cortical motor control following cervical spinal cord injuries in mice. Exp Neurol 2006.
Bond M, Chase AJ, Baker AH, Newby AC. Inhibition of transcription factor NF-kappaB reduces matrix metalloproteinase-1, -3 and -9 production by vascular smooth muscle cells. Cardiovasc Res 2001; 50:556-65.
Bracken MB, Shepard MJ, Holford TR, Leo-Summers L, Aldrich EF, Fazl M, Fehlings MG, Herr DL, Hitchon PW, Marshall LF, Nockels RP, Pascale V, Perot PL, Jr., Piepmeier J, Sonntag VK, Wagner F, Wilberger JE, Winn HR, Young W. Methylprednisolone or tirilazad mesylate administration after acute spinal cord injury: 1-year follow up. Results of the third National Acute Spinal Cord Injury randomized controlled trial. J Neurosurg 1998;89:699-706.
Bracken MB, Shepard MJ, Holford TR, Leo-Summers L, Aldrich EF, Fazl M, Fehlings M, Herr DL, Hitchon PW, Marshall LF, Nockels RP, Pascale V, Perot PL, Jr., Piepmeier J, Sonntag VK, Wagner F, Wilberger JE, Winn HR, Young W. Administration of methylprednisolone for 24 or 48 hours or tirilazad mesylate for 48 hours in the treatment of acute spinal cord injury. Results of the Third National Acute Spinal Cord Injury Randomized Controlled Trial. National Acute Spinal Cord Injury Study. JAMA 1997;277:1597-604.
Bracken MB. Steroids for acute spinal cord injury. Cochrane Database Syst Rev 2002;CD001046.
Brambilla R, Bracchi-Ricard V, Hu WH, Frydel B, Bramwell A, Karmally S, Green EJ, Bethea JR. Inhibition of astroglial nuclear factor kappaB reduces inflammation and improves functional recovery after spinal cord injury. J Exp Med 2005;202:145-56.
Breder J, Sabelhaus CF, Opitz T, Reymann KG, Schroder UH. Inhibition of different pathways influencing Na(+) homeostasis protects organotypic hippocampal slice cultures from hypoxic/hypoglycemic injury. Neuropharmacology 2000; 39:1779-87.
Brown RC, Davis TP. Calcium modulation of adherens and tight junction function: a potential mechanism for blood-brain barrier disruption after stroke. Stroke 2002; 33:1706-11.
Brown, R. C., K. S. Mark, R. D. Egleton, T. P. Davis, Protection against hypoxia-induced blood-brain barrier disruption: changes in intracellular calcium, Am. J. Physiol Cell Physiol 286 (2004) C1045-C1052.
Brown, R. C., T. P. Davis, Hypoxia/aglycemia alters expression of occludin and actin in brain endothelial cells, Biochem. Biophys. Res. Commun. 327 (2005) 1114-1123.
Carlson SL, Parrish ME, Springer JE, Doty K, Dossett L. Acute inflammatory response in spinal cord following impact injury. Exp Neurol 1998;151:77-88.
Castejon OJ. Formation of transendothelial channels in traumatic human brain edema. Pathol Res Pract 1984; 179:7-12.
Ceelie H, Spaargaren-Van Riel CC, De JM, Bertina RM, Vos HL. Functional characterization of transcription factor binding sites for HNF1-alpha, HNF3-beta (FOXA2), HNF4-alpha, Sp1 and Sp3 in the human prothrombin gene enhancer. J Thromb Haemost 2003; 1:1688-98.
Chaovipoch P, Jelks KA, Gerhold LM, West EJ, Chongthammakun S, Floyd CL. 17beta-estradiol is protective in spinal cord injury in post- and pre-menopausal rats. J Neurotrauma 2006;23:830-52.
Chen AA, Derfus AM, Khetani SR, Bhatia SN. Quantum dots to monitor RNAi delivery and improve gene silencing. Nucleic Acids Res 2005;33:e190.
Chen M, Dong Y, Simard JM. Functional coupling between sulfonylurea receptor type 1 and a nonselective cation channel in reactive astrocytes from adult rat brain. J Neurosci 2003;23:8568-77.
Chen M, Simard JM. Cell swelling and a nonselective cation channel regulated by internal Ca2+ and ATP in native reactive astrocytes from adult rat brain. J Neurosci 2001; 21:6512-21.
Chen, Q., J. W. Olney, P. D. Lukasiewicz, T. Almli, C. Romano, Fenamates protect neurons against ischemic and excitotoxic injury in chick embryo retina, Neurosci. Lett. 242 (1998) 163-166.
Cheng H, Beck A, Launay P, Gross SA, Stokes AJ, Kinet JP, Fleig A, Penner R. TRPM4 controls insulin secretion in pancreatic beta-cells. Cell Calcium 2007;41:51-61.
Cho, H., M. S. Kim, W. S. Shim, Y. D. Yang, J. Koo, U. Oh, Calcium-activated cationic channel in rat sensory neurons, Eur. J. Neurosci. 17 (2003) 2630-2638.
Cho, S., E. M. Park, Y. Kim, N. Liu, J. Gal, B. T. Volpe, T. H. Joh, Early c-Fos induction after cerebral ischemia: a possible neuroprotective role, J. Cereb. Blood Flow Metab 21 (2001) 550-556.
Choi, D. W., Glutamate neurotoxicity and diseases of the nervous system, Neuron 1 (1988) 623-634.
Choudhri TF, Hoh BL, Solomon RA, Connolly ES, Jr., Pinsky DJ. Use of a spectrophotometric hemoglobin assay to objectively quantify intracerebral hemorrhage in mice. Stroke 1997;28:2296-302.
Christensen, T., M. Wienrich, H. A. Ensinger, N. H. Diemer, The broad-spectrum cation channel blocker pinokalant (LOE 908 MS) reduces brain infarct volume in rats: a temperature-controlled histological study, Basic Clin. Pharmacol. Toxicol. 96 (2005) 316-324.
Chu, X. P., N. Close, J. A. Saugstad, Z. G. Xiong, ASIC1a-specific modulation of acid-sensing ion channels in mouse cortical neurons by redox reagents, J. Neurosci. 26 (2006) 5329-5339.
Colak A, Soy O, Uzun H, Aslan O, Barut S, Belce A, Akyildiz A, Tasyurekli M. Neuroprotective effects of GYKI 52466 on experimental spinal cord injury in rats. J Neurosurg 2003;98:275-81.
Cooper PR, Hagler H, Clark WK, Barnett P. Enhancement of experimental cerebral edema after decompressive craniectomy: implications for the management of severe head injuries. Neurosurgery 1979; 4:296-300.
Corbel M, Caulet-Maugendre S, Germain N, Molet S, Lagente V, Boichot E. Inhibition of bleomycin-induced pulmonary fibrosis in mice by the matrix metalloproteinase inhibitor batimastat. J Pathol. 2001 Apr;193(4):538-45.
Cornfield DN, Chatfield BA, McQueston JA, McMurtry IF, Abman SH. Effects of birth-related stimuli on L-arginine-dependent pulmonary vasodilation in the ovine fetus. Am J Physiol. 1992;262:H1363-H1368.
Coumans JV, Lin TT, Dai HN, MacArthur L, McAtee M, Nash C, Bregman BS. Axonal regeneration and functional recovery after complete spinal cord transection in rats by delayed treatment with transplants and neurotrophins. J Neurosci 2001;21:9334-44.
Croll SD, Wiegand SJ. Vascular growth factors in cerebral ischemia. Mol Neurobiol 2001; 23:121-35.
Csanady, L., V. Adam-Vizi, Ca(2+)- and voltage-dependent gating of Ca(2+)- and ATP-sensitive cationic channels in brain capillary endothelium, Biophys. J. 85 (2003) 313-327.
Culmsee C, Gasser E, Hansen S, Tonn JC, Wagner E, Goldbrunner R. Effects of Raf-1 siRNA on human cerebral microvascular endothelial cells: a potential therapeutic strategy for inhibition of tumor angiogenesis. Brain Res 2006;1125:147-54.
Cummings BJ, Engesser-Cesar C, Cadena G, Anderson AJ. Adaptation of a ladder beam walking task to assess locomotor recovery in mice following spinal cord injury. Behav Brain Res 2006.
Davis, S. M., G. W. Albers, H. C. Diener, K. R. Lees, J. Norris, Termination of Acute Stroke Studies Involving Selfotel Treatment. ASSIST Steering Committed, Lancet 349 (1997)32.
Davis, S. M., K. R. Lees, G. W. Albers, H. C. Diener, S. Markabi, G. Karlsson, J. Norris, Selfotel in acute ischemic stroke : possible neurotoxic effects of an NMDA antagonist, Stroke 31 (2000) 347-354.
del Zoppo GJ, von Kummer R, Hamann GF. Ischaemic damage of brain microvessels: inherent risks for thrombolytic treatment in stroke. J Neurol Neurosurg Psychiatry 1998; 65:1-9.
Demion M, Bois P, Launay P, Guinamard R. TRPM4, a Ca2+-activated nonselective cation channel in mouse sino-atrial node cells. Cardiovasc Res. 2007;73:531-538.
Deplanque, D., P. Gele, O. Petrault, I. Six, C. Furman, M. Bouly, S. Nion, B. Dupuis, D. Leys, J. C. Fruchart, R. Cecchelli, B. Staels, P. Duriez, R. Bordet, Peroxisome proliferator-activated receptor-alpha activation as a mechanism of preventive neuroprotection induced by chronic fenofibrate treatment, J. Neurosci. 23 (2003) 6264-6271.
Dimayuga FO, Reed JL, Carnero GA, Wang C, Dimayuga ER, Dimayuga VM, Perger A, Wilson ME, Keller JN, Bruce-Keller AJ. Estrogen and brain inflammation: effects on microglial expression of MHC, costimulatory molecules and cytokines. J Neuroimmunol 2005;161:123-36.
Dolman D, Dmdarski S, Abbott NJ, Rattray M. Induction of aquaporin 1 but not aquaporin 4 messenger RNA in rat primary brain microvessel endothelial cells in culture. J Neurochem 2005; 93:825-33.
Domanska-Janik, K., P. Bong, A. Bronisz-Kowalczyk, H. Zajac, B. Zablocka, AP1 transcriptional factor activation and its relation to apoptosis of hippocampal CA1 pyramidal neurons after transient ischemia in gerbils, J. Neurosci. Res. 57 (1999) 840-846.
Donnan GA, Davis SM, Chambers BR, Gates PC, Hankey GJ, McNeil JJ et al. Streptokinase for acute ischemic stroke with relationship to time of administration: Australian Streptokinase (ASK) Trial Study Group. JAMA 1996; 276:961-6.
Encabo, A., C. Romanin, F. W. Birke, W. R. Kukovetz, K. Groschner, Inhibition of a store-operated Ca2+ entry pathway in human endothelial cells by the isoquinoline derivative LOE 908, Br. J. Pharmacol. 119 (1996) 702-706.
Faden AI, Lemke M, Simon RP, Noble LJ. N-methyl-D-aspartate antagonist MK801 improves outcome following traumatic spinal cord injury in rats: behavioral, anatomic, and neurochemical studies. J Neurotrauma 1988;5:33-45.
Farooque M, Suo Z, Arnold PM, Wulser MJ, Chou CT, Vancura RW, Fowler S, Festoff BW. Gender-related differences in recovery of locomotor function after spinal cord injury in mice. Spinal Cord 2006;44:182-7.
Fehlings MG, Baptiste DC. Current status of clinical trials for acute spinal cord injury. Injury 2005;36 Suppl 2:B113-B122.
Fineman JR, Wong J, Morin FC III, Wild LM, Soifer SJ. Chronic nitric oxide inhibition in utero produces persistent pulmonary hypertension in newborn lambs. J Clin Invest. 1994;93:2675-2683.
Fitch MT, Doller C, Combs CK, Landreth GE, Silver J. Cellular and molecular mechanisms of glial scarring and progressive cavitation: in vivo and in vitro analysis of inflammation-induced secondary injury after CNS trauma. J Neurosci 1999;19:8182-98.
Flemming, R., S. Z. Xu, D. J. Beech, Pharmacological profile of store-operated channels in cerebral arteriolar smooth muscle cells, Br. J. Pharmacol. 139 (2003) 955-965.
Flockerzi, V., C. Jung, T. Aberle, M. Meissner, M. Freichel, S. E. Philipp, W. Nastainczyk, P. Maurer, R. Zimmermann, Specific detection and semi-quantitative analysis of TRPC4 protein expression by antibodies, Pflugers Arch. 451 (2005) 81-86.
Freichel, M., S. Philipp, A. Cavalie, V. Flockerzi, TRPC4 and TRPC4-deficient mice, Novartis. Found. Symp. 258 (2004) 189-199.
Fukuda S, Fini CA, Mabuchi T, Koziol JA, Eggleston LL, Jr., del Zoppo GJ. Focal cerebral ischemia induces active proteases that degrade microvascular matrix. Stroke 2004; 35:998-1004.
Galardy RE, et al. Low molecular weight inhibitors in corneal ulceration. Ann NY Acad Sci. 1994;732:315-323.
Gao, Y. Q., H. Gao, Z. Y. Zhou, S. D. Lu, F. Y. Sun, [Expression of transient receptor potential channel 4 in striatum and hippocampus of rats is increased after focal cerebral ischemia], Sheng Li Xue. Bao. 56 (2004) 153-157.
Ge S, Pachter JS. Isolation and culture of microvascular endothelial cells from murine spinal cord. J Neuroimmunol 2006;177:209-14.
Gensel JC, Tovar CA, Hamers FP, Deibert RJ, Beattie MS, Bresnahan JC. Behavioral and histological characterization of unilateral cervical spinal cord contusion injury in rats. J Neurotrauma 2006;23:36-54.
Gerzanich V, Ivanova S, van der Heijden MS, Zhou H, Simard JM. Trans-cellular proliferating cell nuclear antigen gene activation in cerebral vascular smooth muscle by endothelial oxidative injury in vivo. Arterioscler Thromb Vasc Biol 2003;23:2048-54.
Gerzanich V, Ivanova S, Zhou H, Simard JM. Mislocalization of eNOS and upregulation of cerebral vascular Ca2+ channel activity in angiotensin-hypertension. Hypertension 2003;41:1124-30.
Gillibert, J. Duplantier, L Dubuisson, N Senant, G Freyburger, I Laurendeau, J-M Herbert, A Desmoulière and J Rosenbaum A role for thrombin in liver fibrosis, Gut 2004;53:1682-1687.
Go KG. The normal and pathological physiology of brain water. Adv Tech Stand Neurosurg 1997; 23:47-142.
Gogelein, H., D. Dahlem, H. C. Englert, H. J. Lang, Flufenamic acid, mefenamic acid and niflumic acid inhibit single nonselective cation channels in the rat exocrine pancreas, FEBS Lett. 268 (1990) 79-82.
Gotoh O, Asano T, Koide T, Takakura K. Ischemic brain edema following occlusion of the middle cerebral artery in the rat. I: The time courses of the brain water, sodium and potassium contents and blood-brain barrier permeability to 125I-albumin. Stroke 1985; 16:101-9.
Gotoh O, Asano T, Koide T, Takakura K. Ischemic brain edema following occlusion of the middle cerebral artery in the rat. I: The time courses of the brain water, sodium and potassium contents and blood-brain barrier permeability to 125I-albumin. Stroke 1985; 16:101-9.
Graesser D, et al. The interrelationship of alpha4 integrin and matrix metalloproteinase-2 in the pathogenesis of experimental autoimmune encephalomyelitis. Lab Invest. 1998;78:1445-1458.
Greenberg DA. Angiogenesis and stroke. Drug News Perspect 1998; 11:265-70.
Groschner, K., C. Rosker, M. Lukas, Role of TRP channels in oxidative stress, Novartis. Found. Symp. 258 (2004) 222-230.
Guinamard R, Chatelier A, Demion M, Potreau D, Patri S, Rahmati M, Bois P. Functional characterization of a Ca(2+)-activated non-selective cation channel in human atrial cardiomyocytes. J Physiol. 2004;558:75-83.
Guinamard R, Demion M, Chatelier A, Bois P. Calcium-activated nonselective cation channels in mammalian cardiomyocytes. Trends Cardiovasc Med. 2006;16:245-250.
Gunthorpe, M. J., C. D. Benham, A. Randall, J. B. Davis, The diversity in the vanilloid (TRPV) receptor family of ion channels, Trends Pharmacol. Sci. 23 (2002) 183-191.
Hacke W, Kaste M, Fieschi C, Toni D, Lesaffre E, von Kummer R et al. Intravenous thrombolysis with recombinant tissue plasminogen activator for acute hemispheric stroke. The European Cooperative Acute Stroke Study (ECASS). JAMA 1995; 274:1017-25.
Hacke W, Kaste M, Fieschi C, von Kummer R, Davalos A, Meier D et al. Randomised double-blind placebo-controlled trial of thrombolytic therapy with intravenous alteplase in acute ischaemic stroke (ECASS II). Second European-Australasian Acute Stroke Study Investigators. Lancet 1998; 352:1245-51.
Halstead J, Kemp K, Ignotz RA. Evidence for involvement of phosphatidylcholine-phospholipase C and protein kinase C in transforming growth factor-b signaling. J Biol Chem 1995;270:13600-3.
Hamann GF, del Zoppo GJ, von Kummer R. Hemorrhagic transformation of cerebral infarction--possible mechanisms. Thromb Haemost 1999; 82 Suppl 1:92-4.
Han HS, Karabiyikoglu M, Kelly S, Sobel RA, Yenari MA. Mild hypothermia inhibits nuclear factor-kappaB translocation in experimental stroke. J Cereb Blood Flow Metab 2003; 23:589-98.
Hara, Y., M. Wakamori, M. Ishii, E. Maeno, M. Nishida, T. Yoshida, H. Yamada, S. Shimizu, E. Mori, J. Kudoh, N. Shimizu, H. Kurose, Y. Okada, K. Imoto, Y. Mori, LTRPC2 Ca2+-permeable channel activated by changes in redox status confers susceptibility to cell death, Mol. Cell 9 (2002) 163-173.
Harder DR, Gebremedhin D, Narayanan J, Jefcoat C, Falck JR, Campbell WB, Roman R. Formation and action of a P-450 4A metabolite of arachidonic acid in cat cerebral microvessels. Am J Physiol 1994;266:H2098-H2107.
Harteneck, C., Function and pharmacology of TRPM cation channels, Naunyn Schmiedebergs Arch. Pharmacol. 371 (2005) 307-314.
Hasegawa K, Wakino S, Tanaka T, Kimoto M, Tatematsu S, Kanda T et al. Dimethylarginine Dimethylaminohydrolase 2 Increases Vascular Endothelial Growth Factor Expression Through Sp1 Transcription Factor in Endothelial Cells. Arterioscler Thromb Vasc Biol 2006.
Haseloff RF, Krause E, Bigl M, Mikoteit K, Stanimirovic D, Blasig IE. Differential protein expression in brain capillary endothelial cells induced by hypoxia and posthypoxic reoxygenation. Proteomics 2006;6:1803-9.
Hawkins BT, Davis TP. The blood-brain barrier/neurovascular unit in health and disease. Pharmacol Rev 2005; 57:173-85.
Hayes KC, Kakulas BA. Neuropathology of human spinal cord injury sustained in sports-related activities. J Neurotrauma 1997;14:235-48.
Henness, S., D. M. Robinson, K. A. Lyseng-Williamson, Rimonabant, Drugs 66 (2006) 2109-2119.
Heo JH, Lucero J, Abumiya T, Koziol JA, Copeland BR, del Zoppo GJ. Matrix metalloproteinases increase very early during experimental focal cerebral ischemia. J Cereb Blood Flow Metab 1999; 19:624-33.
Hernandez-Sanchez C, Ito Y, Ferrer J, Reitman M, LeRoith D. Characterization of the mouse sulfonylurea receptor 1 promoter and its regulation. J Biol Chem 1999; 274:18261-70.
Heron L, Virsolvy, A, Peyrollier, K, Gribble, FM, Cam, AL, Ashcroft, FM, and Bataille D. Human -endosulfine, a possible regulator of sulfonylurea-sensitive KATP channel: Molecular cloning, expression and biological properties. Proc. Natl. Acad Sci Vol. 95, Issue 14, 8387-8391, July 7, 1998.
Herrmann O, Baumann B, de LR, Muhammad S, Zhang W, Kleesiek J et al. IKK mediates ischemia-induced neuronal death. Nat Med 2005; 11:1322-9.
Hess DC, Howard E, Cheng C, Carroll J, Hill WD, Hsu CY. Hypertonic mannitol loading of NF-kappaB transcription factor decoys in human brain microvascular endothelial cells blocks upregulation of ICAM-1. Stroke 2000;31:1179-86.
Hoehn-Berlage, M., K. A. Hossmann, E. Busch, M. Eis, B. Schmitz, M. L. Gyngell, Inhibition of nonselective cation channels reduces focal ischemic injury of rat brain, J. Cereb. Blood Flow Metab 17 (1997) 534-542.
Hoffman, Johannes N.; Vollmar, Brigitte ; Inthorn, Dietrich; Schildberg, Friedrich W.; Menger, Michael D. The thrombin antagonist hirudin fails to inhibit endotoxin-induced leukocyte/endothelial cell interaction and microvascular perfusion failure. Shock 2000, vol. 14, no5, pp. 528-534.
Hoffmann A, Leung TH, Baltimore D. Genetic analysis of NF-kappaB/Rel transcription factors defines functional specificities. EMBO J 2003;22:5530-9.
Honkaniemi, J., B. A. States, P. R. Weinstein, J. Espinoza, F. R. Sharp, Expression of zinc finger immediate early genes in rat brain after permanent middle cerebral artery occlusion, J. Cereb. Blood Flow Metab 17 (1997) 636-646.
Honkaniemi, J., F. R. Sharp, Global ischemia induces immediate-early genes encoding zinc finger transcription factors, J. Cereb. Blood Flow Metab 16 (1996) 557-565.
Horn, J., M. Limburg, Calcium antagonists for acute ischemic stroke, Cochrane. Database. Syst. Rev. (2000) CD001928.Hossmann KA, Schuier FJ. Experimental brain infarcts in cats. I. Pathophysiological observations. Stroke 1980; 11:583-92.
Hossmann KA. Viability thresholds and the penumbra of focal ischemia. Ann Neurol 1994; 36:557-65.
Hua Y, Keep RF, Hoff JT, Xi G. Thrombin preconditioning attenuates brain edema induced by erythrocytes and iron. J Cereb Blood Flow Metab 2003;23:1448-54.
Hua Y, Wu J, Keep RF, Hoff JT, Xi G. Thrombin exacerbates brain edema in focal cerebral ischemia. Acta Neurochir Suppl 2003; 86:163-6.
Huang, C. Y., M. Fujimura, N. Noshita, Y. Y. Chang, P. H. Chan, SOD1 down-regulates NF-kappaB and c-Myc expression in mice after transient focal cerebral ischemia, J. Cereb. Blood Flow Metab 21 (2001) 163-173.
Huang, Y., J. O. McNamara, Ischemic stroke: "acidotoxicity" is a perpetrator, Cell 118 (2004) 665-666.
Iadecola C, Zhang F, Casey R, Clark HB, Ross ME. Inducible nitric oxide synthase gene expression in vascular cells after transient focal cerebral ischemia. Stroke 1996; 27:1373-80.
Ignotz RA, Honeyman T. TGF-b signaling in A549 lung carcinoma cells: lipid second messengers. J Cell Biochem 2000;78:588-94.
Iizuka H, Yamamoto H, Iwasaki Y, Yamamoto T, Konno H. Evolution of tissue damage in compressive spinal cord injury in rats. J Neurosurg 1987;66:595-603.
Immke, D. C., E. W. McCleskey, Lactate enhances the acid-sensing Na+ channel on ischemia-sensing neurons, Nat. Neurosci. 4 (2001) 869-870.
Immke, D. C., E. W. McCleskey, Protons open acid-sensing ion channels by catalyzing relief of Ca2+ blockade, Neuron 37 (2003) 75-84.
Intracerebral hemorrhage after intravenous t-PA therapy for ischemic stroke. The NINDS t-PA Stroke Study Group. Stroke 1997; 28:2109-18.
Iwamuro, Y., S. Miwa, X. F. Zhang, T. Minowa, T. Enoki, Y. Okamoto, H. Hasegawa, H. Furutani, M. Okazawa, M. Ishikawa, N. Hashimoto, T. Masaki, Activation of three types of voltage-independent Ca2+ channel in A7r5 cells by endothelin-1 as revealed by a novel Ca2+ channel blocker LOE 908, Br. J. Pharmacol. 126 (1999) 1107-1114.
Jaillard A, Cornu C, Durieux A, Moulin T, Boutitie F, Lees KR et al. Hemorrhagic transformation in acute ischemic stroke. The MAST-E study. MAST-E Group. Stroke 1999; 30:1326-32.
Jewell SA, Bellomo G, Thor H, Orrenius S, Smith M. Bleb formation in hepatocytes during drug metabolism is caused by disturbances in thiol and calcium ion homeostasis. Science 1982;217:1257-9.
Jiang, J., M. Li, L. Yue, Potentiation of TRPM7 inward currents by protons, J. Gen. Physiol 126 (2005) 137-150.
Jimenez-Garza O, Camacho J, Ibarra A, Martinez A, Guizar-Sahagun G. Early effects of modulating nuclear factor-kappaB activation on traumatic spinal cord injury in rats. Ann N Y Acad Sci 2005;1053:148-50.
Johnson, M. B., K. Jin, M. Minami, D. Chen, R. P. Simon, Global ischemia induces expression of acid-sensing ion channel 2a in rat brain, J. Cereb. Blood Flow Metab 21 (2001) 734-740.
Jones, N. G., R. Slater, H. Cadiou, P. McNaughton, S. B. McMahon, Acid-induced pain and its modulation in humans, J. Neurosci. 24 (2004) 10974-10979.
Joo F, Klatzo I. Role of cerebral endothelium in brain oedema. Neurol Res 1989; 11:67-75.
Kakinuma Y, Hama H, Sugiyama F, Yagami K, Goto K, Murakami K, Fukamizu A. Impaired blood-brain barrier function in angiotensinogen-deficient mice. Nat Med 1998;4:1078-80.
Kaptanoglu E, Okutan O, Akbiyik F, Solaroglu I, Kilinc A, Beskonakli E. Correlation of injury severity and tissue Evans blue content, lipid peroxidation and clinical evaluation in acute spinal cord injury in rats. J Clin Neurosci 2004;11:879-85.
Kawanabe, Y., K. Nozaki, N. Hashimoto, T. Masaki, Characterization of Ca2+ channels and G proteins involved in arachidonic acid release by endothelin-1/endothelinA receptor, Mol. Pharmacol. 64 (2003) 689-695.
Kawanabe, Y., N. Hashimoto, T. Masaki, Ca(2+) influx through nonselective cation channels plays an essential role in endothelin-1-induced mitogenesis in C6 glioma cells, Neuropharmacology 41 (2001) 331-340.
Kawanabe, Y., N. Hashimoto, T. Masaki, Involvements of voltage-independent Ca2+ channels and phosphoinositide 3-kinase in endothelin-1-induced PYK2 tyrosine phosphorylation, Mol. Pharmacol. 63 (2003) 808-813.
Kawanabe, Y., N. Hashimoto, T. Masaki, S. Miwa, Ca(2+) influx through nonselective cation channels plays an essential role in noradrenaline-induced arachidonic acid release in Chinese hamster ovary cells expressing alpha(1A)-, alpha(1B)-, or alpha(1D)-adrenergic receptors, J. Pharmacol. Exp. Ther. 299 (2001) 901-907.
Kawanabe, Y., Y. Okamoto, S. Miwa, N. Hashimoto, T. Masaki, Molecular mechanisms for the activation of voltage-independent Ca2+ channels by endothelin-1 in chinese hamster ovary cells stably expressing human endothelin(A) receptors, Mol. Pharmacol. 62 (2002) 75-80.
Kawata K, Morimoto T, Ohashi T, Tsujimoto S, Hoshida T, Tsunoda S, Sakaki T. [Experimental study of acute spinal cord injury: a histopathological study]. No Shinkei Geka 1993;21:45-51.
Kelly, J. J., R. N. Auer, Mefenamate, an agent that fails to attenuate experimental cerebral infarction, Can. J. Neurol. Sci. 30 (2003) 259-262.
Kempski O. Cerebral edema. Semin Nephrol 2001; 21:303-7.
Khan M GRRBaPM. Hemorrhagic changes in experimental spinal cord injury models. Canadian Journal of Neuroscience 1985;12:259-62.
Kilincer C, Asil T, Utku U, Hamamcioglu MK, Turgut N, Hicdonmez T et al. Factors affecting the outcome of decompressive craniectomy for large hemispheric infarctions: a prospective cohort study. Acta Neurochir (Wien) 2005; 147:587-94.
Kim KH, Bender JR. Rapid, estrogen receptor-mediated signaling: why is the endothelium so special? Sci STKE 2005;2005:e28.
Kimelberg HK. Current concepts of brain edema. Review of laboratory investigations. J Neurosurg 1995; 83:1051-9.
Klatzo I. Blood-brain barrier and ischaemic brain oedema. Z Kardiol 1987; 76 Suppl 4:67-9.
Klatzo I. Pathophysiological aspects of brain edema. Acta Neuropathol (Berl) 1987; 72:236-9.
Kleiman Neal S.; Klem, Jeffrey; Fernandes, Laura S. ; Rubin, Howard ; Challa, Sarma ; Solomon, Stuart ; Maresh, Kelly; Arora, Umesh ; Klem, Elizabeth; Buergler, John; Mathew, Shiba; Browning, Adrianne; Delao, Tim; Pharmacodynamic profile of the direct thrombin antagonist bivalirudin given in combination with the glycoprotein IIb/IIIa antagonist eptifibatide The American Heart Journal; 2002, vol. 143, no4, pp. 585-593
Knight RA, Barker PB, Fagan SC, Li Y, Jacobs MA, Welch KM. Prediction of impending hemorrhagic transformation in ischemic stroke using magnetic resonance imaging in rats. Stroke 1998; 29:144-51.
Kogure K, Busto R, Scheinberg P. The role of hydrostatic pressure in ischemic brain edema. Ann Neurol 1981; 9:273-82.
Kogure K, Kato H. Altered gene expression in cerebral ischemia. Stroke 1993; 24:2121-7.
Koivisto, A., A. Klinge, J. Nedergaard, D. Siemen, Regulation of the activity of 27 pS nonselective cation channels in excised membrane patches from rat brown-fat cells, Cell Physiol Biochem. 8 (1998) 231-245.
Koivunen E, Arap W, Valtanen H, Rainisalo A, Medina OP, Heikkila P, Kantor C, Gahmberg CG, Salo T, Konttinen YT, Sorsa T, Ruoslahti E, Pasqualini R. Tumor targeting with a selective gelatinase inhibitor. Nat Biotechnol. 1999 Aug;17(8):768-74.
Kolev K, Skopal J, Simon L, Csonka E, Machovich R, Nagy Z. Matrix metalloproteinase-9 expression in post-hypoxic human brain capillary endothelial cells: H2O2 as a trigger and NF-kappaB as a signal transducer. Thromb Haemost 2003; 90:528-37.
Koong, A. C., E. Y. Chen, A. J. Giaccia, Hypoxia causes the activation of nuclear factor kappa B through the phosphorylation of I kappa B alpha on tyrosine residues, Cancer Res. 54 (1994) 1425-1430.
Kraus KH. The pathophysiology of spinal cord injury and its clinical implications. Semin Vet Med Surg (Small Anim) 1996;11:201-7.
Krause, E., F. Pfeiffer, A. Schmid, D. Arndts, I. Schulz, LOE 908 blocks delayed rectifier type potassium channels in PC 12 cells and cortical neurons in culture, Biochem. Biophys. Res. Commun. 244 (1998) 659-664.
Krautwurst, D., J. Hescheler, D. Arndts, W. Losel, R. Hammer, G. Schultz, Novel potent inhibitor of receptor-activated nonselective cation currents in HL-60 cells, Mol. Pharmacol. 43 (1993) 655-659.
Krautwurst, D., V. E. Degtiar, G. Schultz, J. Hescheler, The isoquinoline derivative LOE 908 selectively blocks vasopressin-activated nonselective cation currents in A7r5 aortic smooth muscle cells, Naunyn Schmiedebergs Arch. Pharmacol. 349 (1994) 301-307.
Krishtal, O. A., V. I. Pidoplichko, A "receptor" for protons in small neurons of trigeminal ganglia: possible role in nociception, Neurosci. Lett. 24 (1981) 243-246.
Krishtal, O. A., V. I. Pidoplichko, A receptor for protons in the nerve cell membrane, Neuroscience 5 (1980) 2325-2327.
Krishtal,, O. A., V. I. Pidoplichko, Receptor for protons in the membrane of sensory neurons, Brain Res. 214 (1981) 150-154.
Kucich U, Rosenbloom JC, Shen G, Abrams WR, Hamilton AD, Sebti SM, Rosenbloom J. TGF-b1 stimulation of fibronectin transcription in cultured human lung fibroblasts requires active geranylgeranyl transferase I, phosphatidylcholine-specific phospholipase C, protein kinase C-d, and p38, but not erk1/erk2. Arch Biochem Biophys 2000;374:313-24.
Kuhn, F. J., I. Heiner, A. Luckhoff, TRPM2: a calcium influx pathway regulated by oxidative stress and the novel second messenger ADP-ribose, Pflugers Arch. 451 (2005) 212-219.
Kunte H, Schmidt S, Eliasziw M, del Zoppo G, Simard JM, Masuhr M, Dirnagl U. Sulfonylureas Improve Outcome in Patients with Type 2 Diabetes and Acute Ischemic Stroke. Stroke. 2007; in press.
Kwon BK, Tetzlaff W, Grauer JN, Beiner J, Vaccaro AR. Pathophysiology and pharmacologic treatment of acute spinal cord injury. Spine J 2004;4:451-64.
La Rosa G, Cardali S, Genovese T, Conti A, Paola R, La Torre D, Cacciola F, Cuzzocrea S. Inhibition of the nuclear factor-kappaB activation with pyrrolidine dithiocarbamate attenuating inflammation and oxidative stress after experimental spinal cord trauma in rats. J Neurosurg Spine 2004;1:311-21.
Lapchak PA, Chapman DF, Zivin JA. Metalloproteinase inhibition reduces thrombolytic (tissue plasminogen activator)-induced hemorrhage after thromboembolic stroke. Stroke 2000; 31:3034-40.
Large, W. A., Receptor-operated Ca2(+)-permeable nonselective cation channels in vascular smooth muscle: a physiologic perspective, J. Cardiovasc. Electrophysiol. 13 (2002) 493-501.
Larrue V, von Kummer R, del Zoppo G, Bluhmki E. Hemorrhagic transformation in acute ischemic stroke. Potential contributing factors in the European Cooperative Acute Stroke Study. Stroke 1997; 28:957-60.
Latour LL, Kang DW, Ezzeddine MA, Chalela JA, Warach S. Early blood-brain barrier disruption in human focal brain ischemia. Ann Neurol 2004; 56:468-77.
Launay, P., A. Fleig, A. L. Perraud, A. M. Scharenberg, R. Penner, J. P. Kinet, TRPM4 is a Ca2+-activated nonselective cation channel mediating cell membrane depolarization, Cell 109 (2002) 397-407.
Le Mellay V, Lasmoles F, Lieberherr M. Gaq/11 and Gbg proteins and membrane signaling of calcitriol and estradiol. J Cell Biochem 1999;75:138-46.
Lee KR, Colon GP, Betz AL, Keep RF, Kim S, Hoff JT. Edema from intracerebral hemorrhage: the role of thrombin. J Neurosurg 1996; 84:91-6.
Lee SR, Kim HY, Rogowska J, Zhao BQ, Bhide P, Parent JM, Lo EH. Involvement of matrix metalloproteinase in neuroblast cell migration from the subventricular zone after stroke. J Neurosci 2006;26:3491-5.
Lee SR, Lo EH. Induction of caspase-mediated cell death by matrix metalloproteinases in cerebral endothelial cells after hypoxia-reoxygenation. J Cereb Blood Flow Metab 2004; 24:720-7.
Lee, K., H. Morita, Y. Iwamuro, X. F. Zhang, Y. Okamoto, T. Nakagawa, H. Hasegawa, H. Furutani, S. Miwa, T. Masaki, Pharmacological characterization of receptor-mediated Ca2+ entry in endothelin-1-induced catecholamine release from cultured bovine adrenal chromaffin cells, Naunyn Schmiedebergs Arch. Pharmacol. 360 (1999) 616-622.
Lees, K. R., K. Asplund, A. Carolei, S. M. Davis, H. C. Diener, M. Kaste, J. M. Orgogozo, J. Whitehead, Glycine antagonist (gavestinel) in neuroprotection (GAIN International) in patients with acute stroke: a randomised controlled trial. GAIN International Investigators, Lancet 355 (2000) 1949-1954.
Li, F., R. A. Carano, K. Irie, T. Tatlisumak, M. D. Silva, U. Pschorni, C. H. Sotak, M. Fisher, Neuroprotective effects of a novel broad-spectrum cation channel blocker, LOE 908 MS, on experimental focal ischemia: a multispectral study, J. Magn Reson. Imaging 10 (1999) 138-145.
Lin, M. J., G. P. Leung, W. M. Zhang, X. R. Yang, K. P. Yip, C. M. Tse, J. S. Sham, Chronic hypoxia-induced upregulation of store-operated and receptor-operated Ca2+ channels in pulmonary arterial smooth muscle cells: a novel mechanism of hypoxic pulmonary hypertension, Circ. Res. 95 (2004) 496-505.
Linford NJ, Dorsa DM. 17beta-Estradiol and the phytoestrogen genistein attenuate neuronal apoptosis induced by the endoplasmic reticulum calcium-ATPase inhibitor thapsigargin. Steroids 2002;67:1029-40.
Lipski, J., T. I. Park, D. Li, S. C. Lee, A. J. Trevarton, K. K. Chung, P. S. Freestone, J. Z. Bai, Involvement of TRP-like channels in the acute ischemic response of hippocampal CA1 neurons in brain slices, Brain Res. 1077 (2006) 187-199.
Lipton, P., Ischemic cell death in brain neurons, Physiol Rev. 79 (1999) 1431-1568.
Lubec, B., O. Labudova, H. Hoeger, L. Kirchner, G. Lubec, Expression of transcription factors in the brain of rats with perinatal asphyxia, Biol. Neonate 81 (2002) 266-278.
Ma M, Basso DM, Walters P, Stokes BT, Jakeman LB. Behavioral and histological outcomes following graded spinal cord contusion injury in the C57B1/6 mouse. Exp Neurol 2001;169:239-54.
Ma M, Wei P, Wei T, Ransohoff RM, Jakeman LB. Enhanced axonal growth into a spinal cord contusion injury site in a strain of mouse (129X1/SvJ) with a diminished inflammatory response. J Comp Neurol 2004;474:469-86.
MacDonald, J. F., Z. G. Xiong, M. F. Jackson, Paradox of Ca2+ signaling, cell death and stroke, Trends Neurosci. 29 (2006) 75-81.
Marcus E. Carr; Pantep Angchaisuksiri; Sheryl L. Carr; Erika J. Martin , Effect of Non-Heparin Thrombin Antagonists on Thrombin Generation, Platelet Function, and Clot Structure in Whole Blood, Cell Biochemistry and Biophysics, Volume 39, Number 2, October 2003, pp. 89-100(12)
Marti, H. J., M. Bernaudin, A. Bellail, H. Schoch, M. Euler, E. Petit, W. Risau, Hypoxia-induced vascular endothelial growth factor expression precedes neovascularization after cerebral ischemia, Am. J. Pathol. 156 (2000) 965-976.
Matrone C, Pignataro G, Molinaro P, Irace C, Scorziello A, Di Renzo GF et al. HIF-1alpha reveals a binding activity to the promoter of iNOS gene after permanent middle cerebral artery occlusion. J Neurochem 2004; 90:368-78.
Mattson, M. P., Neuroprotective signal transduction: relevance to stroke, Neurosci. Biobehav. Rev. 21 (1997) 193-206.
McNulty, S., E. Fonfria, The role of TRPM channels in cell death, Pflugers Arch. 451 (2005) 235-242.
McQueston JA, Cornfield DN, McMurtry IF, Abman SH. Effects of oxygen and exogenous L-arginine on EDRF activity in the fetal pulmonary circulation. Am J Physiol. 1993;264:H865-H871.
Merola A, O'Brien MF, Castro BA, Smith DA, Eule JM, Lowe TG, Dwyer AP, Haher TR, Espat NJ. Histologic characterization of acute spinal cord injury treated with intravenous methylprednisolone. J Orthop Trauma 2002;16:155-61.
Merritt, J. E., W. P. Armstrong, C. D. Benham, T. J. Hallam, R. Jacob, A. Jaxa-Chamiec, B. K. Leigh, S. A. McCarthy, K. E. Moores, T. J. Rink, SK&F 96365, a novel inhibitor of receptor-mediated calcium entry, Biochem. J. 271 (1990) 515-522.
Millar AW, Brown PD, Moore J, Galloway WA, Cornish AG, Lenehan TJ, Lynch KP. Results of single and repeat dose studies of the oral matrix metalloproteinase inhibitor marimastat in healthy male volunteers. Br J Clin Pharmacol. 1998 Jan;45(1):21-6.
Miller, B. A., Inhibition of TRPM2 function by PARP inhibitors protects cells from oxidative stress-induced death, Br. J. Pharmacol. 143 (2004) 515-516.
Miller, B. A., The role of TRP channels in oxidative stress-induced cell death, J. Membr. Biol. 209 (2006) 31-41.
Miwa, S., Y. Iwamuro, X. F. Zhang, T. Inoki, Y. Okamoto, M. Okazawa, T. Masaki, Ca2+ entry channels in rat thoracic aortic smooth muscle cells activated by endothelin-1, Jpn. J. Pharmacol. 80 (1999) 281-288.
Miwa, S., Y. Iwamuro, X. F. Zhang, Y. Okamoto, H. Furutani, T. Masaki, Pharmacological properties of calcium entry channels in A7r5 cells activated by endothelin-1, J. Cardiovasc. Pharmacol. 36 (2000) S107-S109.
Montell, C., Physiology, phylogeny, and functions of the TRP superfamily of cation channels, Sci. STKE. 2001 (2001) RE1.
Moore G, Liao S, Curci JA, Starcher BC, Martin RL, Hendricks RT, Chen JJ, Thompson RW. Suppression of experimental abdominal aortic aneurysms by systemic treatment with a hydroxamate-based matrix metalloproteinase inhibitor (RS 132908). J Vasc Surg. 1999 Mar;29(3):522-32.
Moosmann B, Behl C. The antioxidant neuroprotective effects of estrogens and phenolic compounds are independent from their estrogenic properties. Proc Natl Acad Sci USA 1999;96:8867-72.
Mori K, Miyazaki M, Iwase H, Maeda M. Temporal profile of changes in brain tissue extracellular space and extracellular ion (Na(+), K(+)) concentrations after cerebral ischemia and the effects of mild cerebral hypothermia. J Neurotrauma 2002; 19:1261-70.
Mori K, Nakao Y, Yamamoto T, Maeda M. Early external decompressive craniectomy with duroplasty improves functional recovery in patients with massive hemispheric embolic infarction: timing and indication of decompressive surgery for malignant cerebral infarction. Surg Neurol 2004; 62:420-9.
Mun-Bryce S, Rosenberg GA. Matrix metalloproteinases in cerebrovascular disease. J Cereb Blood Flow Metab 1998; 18:1163-72.
Na KY, Woo SK, Lee SD, Kwon HM. Silencing of TonEBP/NFAT5 transcriptional activator by RNA interference. J Am Soc Nephrol 2003;14:283-8.
Naves, L. A., E. W. McCleskey, An acid-sensing ion channel that detects ischemic pain, Braz. J. Med. Biol. Res. 38 (2005) 1561-1569.
Naziroglu, M., A. Luckhoff, E. Jungling, Antagonist effect of flufenamic acid on TRPM2 cation channels activated by hydrogen peroxide, Cell Biochem. Funct. (2006).
Nedergaard, M., R. P. Kraig, J. Tanabe, W. A. Pulsinelli, Dynamics of interstitial and intracellular pH in evolving brain infarct, Am. J. Physiol 260 (1991) R581-R588.
Nelson E, Gertz SD, Rennels ML, Ducker TB, Blaumanis OR. Spinal cord injury. The role of vascular damage in the pathogenesis of central hemorrhagic necrosis. Arch Neurol 1977;34:332-3.
Nicotera, P., D. Bano, The enemy at the gates. Ca2+ entry through TRPM7 channels and anoxic neuronal death, Cell 115 (2003) 768-770.
Nilius B, Droogmans G. Ion channels and their functional role in vascular endothelium. Physiol Rev 2001; 81:1415-59.
Nilius B, Mahieu F, Karashima Y, Voets T. Regulation of TRP channels: a voltage-lipid connection. Biochem Soc Trans 2007;35:105-8.
Nilius B, Mahieu F, Prenen J, Janssens A, Owsianik G, Vennekens R, Voets T. The Ca2+-activated cation channel TRPM4 is regulated by phosphatidylinositol 4,5-biphosphate. EMBO J 2006;25:467-78.
Nilius B, Owsianik G, Voets T, Peters JA. Transient receptor potential cation channels in disease. Physiol Rev 2007;87:165-217.
Nilius B, Prenen J, Droogmans G, Voets T, Vennekens R, Freichel M, Wissenbach U, Flockerzi V. Voltage dependence of the Ca2+-activated cation channel TRPM4. J Biol Chem. 2003;278:30813-30820.
Nilius B, Prenen J, Janssens A, Owsianik G, Wang C, Zhu MX, Voets T. The selectivity filter of the cation channel TRPM4. J Biol Chem. 2005;280:22899-22906.
Nilius B, Prenen J, Janssens A, Voets T, Droogmans G. Decavanadate modulates gating of TRPM4 cation channels. J Physiol. 2004;560:753-765.
Nilius B, Prenen J, Tang J, Wang C, Owsianik G, Janssens A, Voets T, Zhu MX. Regulation of the Ca2+ sensitivity of the nonselective cation channel TRPM4. J Biol Chem. 2005;280:6423-6433.
Nilius B, Prenen J, Voets T, Droogmans G. Intracellular nucleotides and polyamines inhibit the Ca2+-activated cation channel TRPM4b. Pflugers Arch. 2004;448:70-75.
Nilius B, Vennekens R. From cardiac cation channels to the molecular dissection of the transient receptor potential channel TRPM4. Pflugers Arch 2006;453:313-21.
Nilius B. TRP channels in disease. Biochim Biophys Acta 2007.
Nilsen J, Chen S, Brinton RD. Dual action of estrogen on glutamate-induced calcium signaling: mechanisms requiring interaction between estrogen receptors and src/mitogen activated protein kinase pathway. Brain Res 2002;930:216-34.
Nordal RA, Nagy A, Pintilie M, Wong CS. Hypoxia and hypoxia-inducible factor-1 target genes in central nervous system radiation injury: a role for vascular endothelial growth factor. Clin Cancer Res 2004; 10:3342-53.
O'Donnell ME, Tran L, Lam TI, Liu XB, Anderson SE. Bumetanide inhibition of the blood-brain barrier Na-K-Cl cotransporter reduces edema formation in the rat middle cerebral artery occlusion model of stroke. J Cereb Blood Flow Metab 2004; 24:1046-56.
Ohta K, Fujimura Y, Nakamura M, Watanabe M, Yato Y. Experimental study on MRI evaluation of the course of cervical spinal cord injury. Spinal Cord 1999;37:580-4.
Okada Y, Maeno E. Apoptosis, cell volume regulation and volume-regulatory chloride channels. Comp Biochem Physiol A Mol Integr Physiol 2001; 130:377-83.
Onifer SM, Zhang YP, Burke DA, Brooks DL, Decker JA, McClure NJ, Floyd AR, Hall J, Proffitt BL, Shields CB, Magnuson DS. Adult rat forelimb dysfunction after dorsal cervical spinal cord injury. Exp Neurol 2005;192:25-38.
Papapetropoulos A, Garcia-Cardena G, Madri JA, et al. Nitric oxide production contributes to the angiogenic properties of vascular endothelial growth factor in human endothelial cells. J Clin Invest. 1997;100:3131-3139.
Pearse DD, Lo TP, Jr., Cho KS, Lynch MP, Garg MS, Marcillo AE, Sanchez AR, Cruz Y, Dietrich WD. Histopathological and behavioral characterization of a novel cervical spinal cord displacement contusion injury in the rat. J Neurotrauma 2005;22:680-702.
Pegorini, S., A. Zani, D. Braida, C. Guerini-Rocco, M. Sala, Vanilloid VR1 receptor is involved in rimonabant-induced neuroprotection, Br. J. Pharmacol. 147 (2006) 552-559.
Pegorini, S., D. Braida, C. Verzoni, C. Guerini-Rocco, G. G. Consalez, L. Croci, M. Sala, Capsaicin exhibits neuroprotective effects in a model of transient global cerebral ischemia in Mongolian gerbils, Br. J. Pharmacol. 144 (2005) 727-735.
Perillan PR, Chen M, Potts EA, Simard JM. Transforming growth factor-beta 1 regulates Kir2.3 inward rectifier K+ channels via phospholipase C and protein kinase C-delta in reactive astrocytes from adult rat brain. J Biol Chem 2002;277:1974-80.
Perillan PR, Li X, Potts EA, Chen M, Bredt DS, Simard JM. Inward rectifier K+ channel Kir2.3 (IRK3) in reactive astrocytes from adult rat brain. Glia 2000;31:181-92.
Perraud, A. L. , C. Schmitz, A. M. Scharenberg, TRPM2 Ca2+ permeable cation channels: from gene to biological function, Cell Calcium 33 (2003) 519-531.
Perraud, A. L., C. L. Takanishi, B. Shen, S. Kang, M. K. Smith, C. Schmitz, H. M. Knowles, D. Ferraris, W. Li, J. Zhang, B. L. Stoddard, A. M. Scharenberg, Accumulation of free ADP-ribose from mitochondria mediates oxidative stress-induced gating of TRPM2 cation channels, J. Biol. Chem. 280 (2005) 6138-6148.
Pertwee, R. G., Pharmacological actions of cannabinoids, Handb. Exp. Pharmacol. (2005) 1-51.
Pfefferkorn T, Rosenberg GA. Closure of the blood-brain barrier by matrix metalloproteinase inhibition reduces rtPA-mediated mortality in cerebral ischemia with delayed reperfusion. Stroke 2003; 34:2025-30.
Pineau I, Lacroix S. Proinflammatory cytokine synthesis in the injured mouse spinal cord: multiphasic expression pattern and identification of the cell types involved. J Comp Neurol 2007;500:267-85.
Planas AM, Sole S, Justicia C, Farre ER. Estimation of gelatinase content in rat brain: effect of focal ischemia. Biochem Biophys Res Commun. 2000; 278:803-7.
Planells-Cases, R., J. Lerma, A. Ferrer-Montiel, Pharmacological intervention at ionotropic glutamate receptor complexes, Curr. Pharm. Des 12 (2006) 3583-3596.
Popovich PG, Wei P, Stokes BT. Cellular inflammatory response after spinal cord injury in Sprague-Dawley and Lewis rats. J Comp Neurol 1997;377:443-64.
Pore N, Jiang Z, Gupta A, Cerniglia G, Kao GD, Maity A. EGFR tyrosine kinase inhibitors decrease VEGF expression by both hypoxia-inducible factor (HIF)-1-independent and HIF-1-dependent mechanisms. Cancer Res 2006; 66:3197-204.
Poteser, M., A. Graziani, C. Rosker, P. Eder, I. Derler, H. Kahr, M. X. Zhu, C. Romanin, K. Groschner, TRPC3 and TRPC4 associate to form a redox-sensitive cation channel. Evidence for expression of native TRPC3-TRPC4 heteromeric channels in endothelial cells, J. Biol. Chem. 281 (2006) 13588-13595.
Pouyssegur J, Dayan F, Mazure NM. Hypoxia signalling in cancer and approaches to enforce tumour regression. Nature 2006; 441:437-43.
Prass, K., A. Scharff, K. Ruscher, D. Lowl, C. Muselmann, I. Victorov, K. Kapinya, U. Dirnagl, A. Meisel, Hypoxia-induced stroke tolerance in the mouse is mediated by erythropoietin, Stroke 34 (2003) 1981-1986.
Price CJ, Warburton EA, Menon DK. Human cellular inflammation in the pathology of acute cerebral ischaemia. J Neurol Neurosurg Psychiatry 2003; 74:1476-84.
Proks P, Gribble FM, Adhikari R, Tucker SJ, Ashcroft FM. Involvement of the N-terminus of Kir6.2 in the inhibition of the KATP channel by ATP. J Physiol 1999;514 (Pt 1):19-25.
Qiu J, Bosch MA, Tobias SC, Grandy DK, Scanlan TS, Ronnekleiv OK, Kelly MJ. Rapid signaling of estrogen in hypothalamic neurons involves a novel G-protein-coupled estrogen receptor that activates protein kinase C. J Neurosci 2003;23:9529-40.
Quast MJ, Huang NC, Hillman GR, Kent TA. The evolution of acute stroke recorded by multimodal magnetic resonance imaging. Magn Reson Imaging 1993; 11:465-71.
Randomised controlled trial of streptokinase, aspirin, and combination of both in treatment of acute ischaemic stroke. Multicentre Acute Stroke Trial--Italy (MAST-I) Group. Lancet 1995; 346:1509-14.
Rehncrona, S., Brain acidosis, Ann. Emerg. Med. 14 (1985) 770-776.
Riek-Burchardt M, Striggow F, Henrich-Noack P, Reiser G, Reymann KG. Increase of prothrombin-mRNA after global cerebral ischemia in rats, with constant expression of protease nexin-1 and protease-activated receptors. Neurosci Lett 2002; 329:181-4.
Rivlin AS, Tator CH. Objective clinical assessment of motor function after experimental spinal cord injury in the rat. J Neurosurg 1977;47:577-81.
Robyn L. Rairigh, Timothy D. Le Cras, D. Dunbar Ivy, John P. Kinsella, Gresham Richter, Marilee P. Horan, I-Da Fan, and Steven H. Abman, Role of Inducible Nitric Oxide Synthase in Regulation of Pulmonary Vascular Tone in the Late Gestation Ovine Fetus J. Clin. Invest. Volume 101, Number 1, January 1998, 15-21.
Rolli, Melanie, Emilia Fransvea, Jan Pilch, Alan Saven, and Brunhilde Felding-Habermann, Activated integrin αvβ3 cooperates with metalloproteinase. MMP-9 in regulating migration of metastatic breast cancer cells Proc Natl Acad Sci USA. 2003 August 5; 100(16): 9482-9487.
Romanic AM, White RF, Arleth AJ, Ohlstein EH, Barone FC. Matrix metalloproteinase expression increases after cerebral focal ischemia in rats: inhibition of matrix metalloproteinase-9 reduces infarct size. Stroke 1998; 29:1020-30.
Roof RL, Hall ED. Estrogen-related gender difference in survival rate and cortical blood flow after impact-acceleration head injury in rats. J Neurotrauma 2000;17:1155-69.
Roof RL, Hall ED. Gender differences in acute CNS trauma and stroke: neuroprotective effects of estrogen and progesterone. J Neurotrauma 2000;17:367-88.
Rosenberg GA. Ischemic brain edema. Prog Cardiovasc. Dis 1999; 42:209-16.
Rosenberg GA. Matrix metalloproteinases in neuroinflammation. Glia 2002; 39:279-91.
Russell JM. Sodium-potassium-chloride cotransport. Physiol Rev 2000; 80:211-76.
Salminen A, Liu PK, Hsu CY. Alteration of transcription factor binding activities in the ischemic rat brain. Biochem Biophys Res Commun 1995; 212:939-44.
Salnikow K, Kluz T, Costa M, Piquemal D, Demidenko ZN, Xie K et al. The regulation of hypoxic genes by calcium involves c-Jun/AP-1, which cooperates with hypoxia-inducible factor 1 in response to hypoxia. Mol Cell Biol 2002; 22:1734-41.
Sasaki S, Schneider H, Renz S. Microcirculatory disturbances during the early phase following experimental spinal cord trauma in the rat. Adv Neurol 1978;20:423-31.
Satpathy M, Gallagher P, Lizotte-Waniewski M, Srinivas SP. Thrombin-induced phosphorylation of the regulatory light chain of myosin II in cultured bovine corneal endothelial cells. Exp Eye Res 2004; 79:477-86.
Scheff SW, Rabchevsky AG, Fugaccia I, Main JA, Lumpp JE, Jr. Experimental modeling of spinal cord injury: characterization of a force-defined injury device. J Neurotrauma 2003;20:179-93.
Schneider A, Martin-Villalba A, Weih F, Vogel J, Wirth T, Schwaninger M. NF-kappaB is activated and promotes cell death in focal cerebral ischemia. Nat Med 1999; 5:554-9.
Schuier FJ, Hossmann KA. Experimental brain infarcts in cats. II. Ischemic brain edema. Stroke 1980; 11:593-601.
Schwartz G, Fehlings MG. Evaluation of the neuroprotective effects of sodium channel blockers after spinal cord injury: improved behavioral and neuroanatomical recovery with riluzole. J Neurosurg 2001;94:245-56.
Seidel MF, Simard JM, Hunter SF, Campbell GA. Isolation of arteriolar microvessels and culture of smooth muscle cells from cerebral cortex of guinea pig. Cell Tissue Res 1991;265:579-87.
Sharma HS, Drieu K, Alm P, Westman J. Role of nitric oxide in blood-brain barrier permeability, brain edema and cell damage following hyperthermic brain injury. An experimental study using EGB-761 and Gingkolide B pretreatment in the rat. Acta Neurochir Suppl 2000; 76:81-6.
Shimazu, T., I. Inoue, N. Araki, Y. Asano, M. Sawada, D. Furuya, H. Nagoya, J. H. Greenberg, A peroxisome proliferator-activated receptor-gamma agonist reduces infarct size in transient but not in permanent ischemia, Stroke 36 (2005) 353-359.
Simard JM, Chen M, Tarasov KV, Bhatta S, Ivanova S, Melnitchenko L et al. Newly expressed SUR1-regulated NC(Ca-ATP) channel mediates cerebral edema after ischemic stroke. Nat Med 2006; 12:433-40.
Simard JM, Kent TA, Chen M, Tarasov KV, Gerzanich V. Brain oedema in focal ischaemia: molecular pathophysiology and theoretical implications. Lancet Neurol 2007;6:258-68.
Simard JM, Tarasov KV, Gerzanich V. Non-selective cation channels, transient receptor potential channels and ischemic stroke. Biochim Biophys Acta 2007.
Simard JM, Tsymbalyuk O, Ivanov A, Ivanova S, Bhatta S, Geng Z, Woo SK, Gerzanich V. Endothelial sulfonylurea receptor 1-regulated NCCa-ATP channels mediate progressive hemorrhagic necrosis following spinal cord injury. J Clin Invest 2007; in press.
Simard, J. M., T. A. Kent, M. Chen, K. V. Tarasov, V. Gerzanich, Molecular pathophysiology of brain edema in focal ischemia - a focused review, Lancet Neurology (2007); 6:258-268.
Sng JC, Taniura H, Yoneda Y. A tale of early response genes. Biol Pharm Bull 2004; 27:606-12.
Soblosky JS, Song JH, Dinh DH. Graded unilateral cervical spinal cord injury in the rat: evaluation of forelimb recovery and histological effects. Behav Brain Res 2001;119:1-13.
Sribnick EA, Ray SK, Nowak MW, Li L, Banik NL. 17beta-estradiol attenuates glutamate-induced apoptosis and preserves electrophysiologic function in primary cortical neurons. J Neurosci Res 2004;76:688-96.
Sribnick EA, Wingrave JM, Matzelle DD, Ray SK, Banik NL. Estrogen as a neuroprotective agent in the treatment of spinal cord injury. Ann N Y Acad Sci 2003;993:125-33.
Sribnick EA, Wingrave JM, Matzelle DD, Wilford GG, Ray SK, Banik NL. Estrogen attenuated markers of inflammation and decreased lesion volume in acute spinal cord injury in rats. J Neurosci Res 2005;82:283-93.
Starling EH. On the absorption of fluids from connective tissue spaces. J Physiol 1896; 19:312-26.
Stiefel MF, Marmarou A. Cation dysfunction associated with cerebral ischemia followed by reperfusion: a comparison of microdialysis and ion-selective electrode methods. J Neurosurg 2002; 97:97-103.
Storme L, Rairigh RL, Parker TA, Kinsella JP, Abman SH. In vivo evidence for a myogenic response in the fetal pulmonary circulation. Pediatr Res. 1999;45:425-31.
Striggow F, Riek-Burchardt M, Kiesel A, Schmidt W, Henrich-Noack P, Breder J et al. Four different types of protease-activated receptors are widely expressed in the brain and are up-regulated in hippocampus by severe ischemia. Eur J Neurosci 2001; 14:595-608.
Sumii T, Lo EH. Involvement of matrix metalloproteinase in thrombolysis-associated hemorrhagic transformation after embolic focal ischemia in rats. Stroke 2002; 33:831-6.
Sundararajan, S., Q. Jiang, M. Heneka, G. Landreth, PPARgamma as a therapeutic target in central nervous system diseases, Neurochem. Int. 49 (2006) 136-144.
Sur P, Sribnick EA, Wingrave JM, Nowak MW, Ray SK, Banik NL. Estrogen attenuates oxidative stress-induced apoptosis in C6 glial cells. Brain Res 2003;971:178-88.
Sweeney MI, Yager JY, Walz W, Juurlink BH. Cellular mechanisms involved in brain ischemia. Can J Physiol Pharmacol 1995; 73:1525-35.
Taniguchi M, Yamashita T, Kumura E, Tamatani M, Kobayashi A, Yokawa T et al. Induction of aquaporin-4 water channel mRNA after focal cerebral ischemia in rat. Brain Res Mol Brain Res 2000; 78:131-7.
Tatlisumak, T., R. A. Carano, K. Takano, M. R. Meiler, F. Li, C. H. Sotak, U. Pschorn, M. Fisher, Broad-spectrum cation channel inhibition by LOE 908 MS reduces infarct volume in vivo and postmortem in focal cerebral ischemia in the rat, Acta Neurochir. Suppl 76 (2000) 329-330.
Tatlisumak, T., R. A. Carano, K. Takano, M. R. Meiler, F. Li, C. H. Sotak, D. Arndts, U. Pschorn, M. Fisher, Broad-spectrum cation channel inhibition by LOE 908 MS reduces infarct volume in vivo and postmortem in focal cerebral ischemia in the rat, J. Neurol. Sci. 178 (2000) 107-113.
Tator CH, Fehlings MG. Review of the secondary injury theory of acute spinal cord trauma with emphasis on vascular mechanisms. J Neurosurg 1991;75:15-26.
Tator CH, Koyanagi I. Vascular mechanisms in the pathophysiology of human spinal cord injury. J Neurosurg 1997;86:483-92.
Tator CH. Review of experimental spinal cord injury with emphasis on the local and systemic circulatory effects. Neurochirurgie 1991;37:291-302.
Teng YD, Choi H, Onario RC, Zhu S, Desilets FC, Lan S, Woodard EJ, Snyder EY, Eichler ME, Friedlander RM. Minocycline inhibits contusion-triggered mitochondrial cytochrome c release and mitigates functional deficits after spinal cord injury. Proc Natl Acad Sci U S A 2004;101:3071-6.
Teng YD, Wrathall JR. Local blockade of sodium channels by tetrodotoxin ameliorates tissue loss and long-term functional deficits resulting from experimental spinal cord injury. J Neurosci 1997;17:4359-66.
Tissue plasminogen activator for acute ischemic stroke. The National Institute of Neurological Disorders and Stroke rt-PA Stroke Study Group. N Engl J Med 1995; 333:1581-7.
Todd NV, Picozzi P, Crockard A, Russell RW. Duration of ischemia influences the development and resolution of ischemic brain edema. Stroke 1986; 17:466-71.
Todd NV, Picozzi P, Crockard HA, Russell RR. Reperfusion after cerebral ischemia: influence of duration of ischemia. Stroke 1986; 17:460-6.
Trescher K, Bernecker O, Fellner B, Gyongyosi M, Krieger S, Demartin R, Wolner E, Podesser BK. Adenovirus-mediated overexpression of inhibitor kappa B-alpha attenuates postinfarct remodeling in the rat heart. Eur J Cardiothorac Surg 2004;26:960-7.
Ugawa, S., T. Ueda, H. Yamamura, M. Nagao, S. Shimada, Coexpression of Vanilloid Receptor Subtype-1 and Acid-sensing Ion Channel Genes in the Human Trigeminal Ganglion Neurons, Chem. Senses 30 Suppl 1 (2005) i195.
Ugawa, S., T. Ueda, H. Yamamura, S. Shimada, In situ hybridization evidence for the coexistence of ASIC and TRPV1 within rat single sensory neurons, Brain Res. Mol. Brain Res. 136 (2005) 125-133.
Ugawa, S., T. Ueda, Y. Ishida, M. Nishigaki, Y. Shibata, S. Shimada, Amiloride-blockable acid-sensing ion channels are leading acid sensors expressed in human nociceptors, J. Clin. Invest 110 (2002) 1185-1190.
Ullrich, N. D., T. Voets, J. Prenen, R. Vennekens, K. Talavera, G. Droogmans, B. Nilius, Comparison of functional properties of the Ca2+-activated cation channels TRPM4 and TRPM5 from mice, Cell Calcium 37 (2005) 267-278.
Umenishi F, Verkman AS. Isolation and functional analysis of alternative promoters in the human aquaporin-4 water channel gene. Genomics 1998; 50:373-7.
Vallance P, Leone A, Calver A, et al. Endogenous dimethylarginine as an inhibitor of nitric oxide synthesis. J Cardiovasc Pharmacol. 1992;20(suppl 12):S60-S62.
van BN, Thibodeaux H, Palmer JT, Lee WP, Fu L, Cairns B et al. VEGF antagonism reduces edema formation and tissue damage after ischemia/reperfusion injury in the mouse brain. J Clin Invest 1999; 104:1613-20.
Van Der, S. M., M. Di, V, Endovanilloids. Putative endogenous ligands of transient receptor potential vanilloid 1 channels, Eur. J. Biochem. 271 (2004) 1827-1834.
Vennekens R, Nilius B. Insights into TRPM4 function, regulation and physiological role. Handb Exp Pharmacol 2007;269-85.
Vennekens R, Olausson J, Meissner M, Bloch W, Mathar I, Philipp SE, Schmitz F, Weissgerber P, Nilius B, Flockerzi V, Freichel M. Increased IgE-dependent mast cell activation and anaphylactic responses in mice lacking the calcium-activated nonselective cation channel TRPM4. Nat Immunol. 2007;8:312-320.
Vorbrodt AW, Lossinsky AS, Wisniewski HM, Suzuki R, Yamaguchi T, Masaoka H et al. Ultrastructural observations on the transvascular route of protein removal in vasogenic brain edema. Acta Neuropathol (Berl) 1985; 66:265-73.
Waldmann, R., G. Champigny, E. Lingueglia, W. De, Jr., C. Heurteaux, M. Lazdunski, H(+)-gated cation channels, Ann. N. Y. Acad. Sci. 868 (1999) 67-76.
Waldmann, R., M. Lazdunski, H(+)-gated cation channels: neuronal acid sensors in the NaC/DEG family of ion channels, Curr. Opin. Neurobiol. 8 (1998) 418-424.
Wang X, Lo EH. Triggers and mediators of hemorrhagic transformation in cerebral ischemia. Mol Nettrobiol 2003; 28:229-44.
Wang X, Wang Y, Kim HP, Nakahira K, Ryter SW, Choi AM. Carbon monoxide protects against hyperoxia-induced endothelial cell apoptosis by inhibiting reactive oxygen species formation. J Biol Chem 2006.
Wang Y, Hu W, Perez-Trepichio AD, Ng TC, Furlan AJ, Majors AW et al. Brain tissue sodium is a ticking clock telling time after arterial occlusion in rat focal cerebral ischemia. Stroke 2000; 31:1386-91.
Wang Y, Kilic E, Kilic U, Weber B, Bassetti CL, Marti HH et al. VEGF overexpression induces post-ischaemic neuroprotection, but facilitates haemodynamic steal phenomena. Brain 2005; 128:52-63.
Wang, J., L. Weigand, W. Lu, J. T. Sylvester, G. L. Semenza, L. A. Shimoda, Hypoxia inducible factor 1 mediates hypoxia-induced TRPC expression and elevated intracellular Ca2+ in pulmonary arterial smooth muscle cells, Circ. Res. 98 (2006) 1528-1537.
Warach S, Latour LL. Evidence of reperfusion injury, exacerbated by thrombolytic therapy, in human focal brain ischemia using a novel imaging marker of early blood-brain barrier disruption. Stroke 2004; 35:2659-61.
Warnick RE, Fike JR, Chan PH, Anderson DK, Ross GY, Gutin PH. Measurement of vascular permeability in spinal cord using Evans Blue spectrophotometry and correction for turbidity. J Neurosci Methods 1995;58:167-71.
Webb AA, Muir GD. Sensorimotor behaviour following incomplete cervical spinal cord injury in the rat. Behav Brain Res 2005;165:147-59.
Weirich SD, Cotler HB, Narayana PA, Hazle JD, Jackson EF, Coupe KJ, McDonald CL, Langford LA, Harris JH, Jr. Histopathologic correlation of magnetic resonance imaging signal patterns in a spinal cord injury model. Spine 1990;15:630-8.
Weis SM, Cheresh DA. Pathophysiological consequences of VEGF-induced vascular permeability. Nature 2005; 437:497-504.
Wemmie, J. A., C. C. Askwith, E. Lamani, M. D. Cassell, J. H. Freeman, Jr., M. J. Welsh, Acid-sensing ion channel 1 is localized in brain regions with high synaptic density and contributes to fear conditioning, J. Neurosci. 23 (2003) 5496-5502.
Wemmie, J. A., J. Chen, C. C. Askwith, A. M. Hruska-Hageman, M. P. Price, B. C. Nolan, P. G. Yoder, E. Lamani, T. Hoshi, J. H. Freeman, Jr., M. J. Welsh, The acid-activated ion channel ASIC contributes to synaptic plasticity, learning, and memory, Neuron 34 (2002) 463-477.
Weng, X. C., J. Q. Zheng, X. D. Gai, J. Li, W. B. Xiao, Two types of acid-sensing ion channel currents in rat hippocampal neurons, Neurosci. Res. 50 (2004) 493-499.
White BC, Sullivan JM, DeGracia DJ, O'Neil BJ, Neumar RW, Grossman LI et al. Brain ischemia and reperfusion: molecular mechanisms of neuronal injury. J Neurol Sci 2000; 179:1-33.
Wicher, D., H. J. Agricola, R. Schonherr, S. H. Heinemann, C. Derst, TRPgamma channels are inhibited by cAMP and contribute to pacemaking in neurosecretory insect neurons, J. Biol. Chem. 281 (2006) 3227-3236.
Won, S. J., D. Y. Kim, B. J. Gwag, Cellular and molecular pathways of ischemic neuronal death, J. Biochem. Mol. Biol. 35 (2002) 67-86.
Woo SK, Lee SD, Na KY, Park WK, Kwon HM. TonEBP/NFAT5 stimulates transcription of HSP70 in response to hypertonicity. Mol Cell Biol 2002;22:5753-60.
Wu Z, Hofman FM, Zlokovic BV. A simple method for isolation and characterization of mouse brain microvascular endothelial cells. J Neurosci Methods 2003;130:53-63.
Xi G, Hua Y, Bhasin RR, Ennis SR, Keep RF, Hoff JT. Mechanisms of edema formation after intracerebral hemorrhage: effects of extravasated red blood cells on blood flow and blood-brain barrier integrity. Stroke 2001;32:2932-8.
Xiao Q, Hsu CY, Chen H, Ma X, Xu J, Lee JM. Characterization of cis-regulatory elements of the vascular endothelial growth inhibitor gene promoter. Biochem J 2005;388:913-20.
Xie LH, Horie M, Takano M. Phospholipase C-linked receptors regulate the ATP-sensitive potassium channel by means of phosphatidylinositol 4,5-bisphosphate metabolism. Proc Natl Acad Sci U S A 1999;96:15292-7.
Xiong, Z. G., X. M. Zhu, X. P. Chu, M. Minami, J. Hey, W. L. Wei, J. F. MacDonald, J. A. Wemmie, M. P. Price, M. J. Welsh, R. P. Simon, Neuroprotection in ischemia: blocking calcium-permeable acid-sensing ion channels, Cell 118 (2004) 687-698.
Xiong, Z. G., X. P. Chu, R. P. Simon, Ca2+ -permeable acid-sensing ion channels and ischemic brain injury, J. Membr. Biol. 209 (2006) 59-68.
Xu J, Fan G, Chen S, Wu Y, Xu XM, Hsu CY. Methylprednisolone inhibition of TNF-alpha expression and NF-kB activation after spinal cord injury in rats. Brain Res Mol Brain Res 1998;59:135-42.
Yagi, J., H. N. Wenk, L. A. Naves, E. W. McCleskey, Sustained currents through ASIC3 ion channels at the modest pH changes that occur during myocardial ischemia, Circ. Res. 99 (2006) 501-509.
Yang GY, Chen SF, Kinouchi H, Chan PH, Weinstein PR. Edema, cation content, and ATPase activity after middle cerebral artery occlusion in rats. Stroke 1992; 23:1331-6.
Yao, X., C. J. Garland, Recent developments in vascular endothelial cell transient receptor potential channels, Circ. Res. 97 (2005) 853-863.
Yoneda, Y., N. Kuramoto, Y. Azuma, K. Inoue, K. Ogita, A. Mitani, H. Yanase, S. Masuda, L. Zhang, K. Kataoka, Prolongation by bifemelane of potentiation of AP1 DNA binding in hippocampal CA1 subfield of gerbils with transient forebrain ischemia, J. Neurosci. Res. 51 (1998) 574-582.
Yoneda, Y., Y. Azuma, K. Inoue, K. Ogita, A. Mitani, L. Zhang, S. Masuda, M. Higashihara, K. Kataoka, Positive correlation between prolonged potentiation of binding of double-stranded oligonucleotide probe for the transcription factor AP1 and resistance to transient forebrain ischemia in gerbil hippocampus, Neuroscience 79 (1997) 1023-1037.
Young W, Constantini S. Ionic and water shifts in injured central nervous tissues. In: Salzman SK, Faden AI, editors. The Neurobiology of Central Nervous System Trauma.New York: Oxford University Press; 1994. p. 123-30.
Young W, Rappaport ZH, Chalif DJ, Flamm ES. Regional brain sodium, potassium, and water changes in the rat middle cerebral artery occlusion model of ischemia. Stroke 1987; 18:751-9.
Yune TY, Kim SJ, Lee SM, Lee YK, Oh YJ, Kim YC, Markelonis GJ, Oh TH. Systemic administration of 17beta-estradiol reduces apoptotic cell death and improves functional recovery following traumatic spinal cord injury in rats. J Neurotrauma 2004;21:293-306.
Zhang, X. F., Y. Iwamuro, T. Enoki, M. Okazawa, K. Lee, T. Komuro, T. Minowa, Y. Okamoto, H. Hasegawa, H. Furutani, S. Miwa, T. Masaki, Pharmacological characterization of Ca2+ entry channels in endothelin-1-induced contraction of rat aorta using LOE 908 and SK&F 96365, Br. J. Pharmacol. 127 (1999) 1388-1398.
Zhao BQ, Wang S, Kim HY, Storrie H, Rosen BR, Mooney DJ et al. Role of matrix metalloproteinases in delayed cortical responses after stroke. Nat Med 2006; 12:441-5.
Zhao BQ, Wang S, Kim HY, Storrie H, Rosen BR, Mooney DJ, Wang X, Lo EH. Role of matrix metalloproteinases in delayed cortical responses after stroke. Nat Med 2006;12:441-5.
Zheng Z, Yenari MA. Post-ischemic inflammation: molecular mechanisms and therapeutic implications. Neurol Res 2004; 26:884-92.
Zhu Y, Sun Y, Xie L, Jin K, Sheibani N, Greenberg DA. Hypoxic induction of endoglin via mitogen-activated protein kinases in mouse brain microvascular endothelial cells. Stroke 2003;34:2483-8.
[**0947**] Zhu, X., M. Jiang, L. Birnbaumer, Receptor-activated Ca2+ influx via human Trp3 stably expressed in human embryonic kidney (HEK)293 cells. Evidence for a non-capacitative Ca2+ entry, J. Biol. Chem. 273 (1998) 133-142.

## Claims

1. An antagonist of TRPM4 for use in a method of (a) inhibiting neural cell swelling in an individual having traumatic brain injury, traumatic spinal cord injury, cerebral ischemia, spinal cord ischemia, central nervous system (CNS) damage, peripheral nervous system (PNS) damage, cerebral hypoxia, spinal cord hypoxia, or cerebral edema; or (b) treating or preventing spinal cord injury stemming from neural cell swelling.

2. An antagonist of TRPM4 for use according to claim 1, wherein the antagonist of TRPM4 is a nucleic acid, a protein, a small molecule, or a combination thereof.

3. An antagonist of TRPM4 for use according claim 2, wherein the nucleic acid is a TRPM4 siRNA.

4. An antagonist of TRPM4 for use according to claim 1, wherein the method further comprises delivering to the individual a therapeutically effective amount of an additional therapeutic compound selected from:
a) a SUR1 antagonist;
b) one or more cation channel blockers;
c) one or more of a compound selected from one or more antagonists of vascular endothelial growth factor (VEGF), one or more antagonists of matrix metalloprotease (MMP), one or more antagonists of nitric oxide synthase (NOS), one or more antagonists of thrombin, aquaporin, a biologically active derivative thereof, and a combination thereof; and
d) a combination thereof.

5. An antagonist of TRPM4 for use according to claim 4, wherein
- the TRPM4 antagonist and the additional therapeutic compound are delivered to the individual successively; or
- the TRPM4 antagonist is delivered to the individual prior to delivery of the additional therapeutic compound; or
- the TRPM4 antagonist is delivered to the individual subsequent to delivery of the additional therapeutic compound.

6. An antagonist of TRPM4 for use according to claim 4, wherein
- the TRPM4 antagonist and the additional therapeutic compound are delivered to the individual concomitantly; or
- the TRPM4 antagonist and the additional therapeutic compound being delivered as a mixture; or
- the TRPM4 antagonist and the additional therapeutic compound act synergistically in the individual.

## Patentansprüche

1. Antagonist von TRPM4 zur Verwendung in einem Verfahren zum (a) Hemmen eines Anschwellens von Nervenzellen in einem Individuum mit traumatischer Gehirnverletzung, traumatischer Rückenmarksverletzung, Rückenmarksischämie, Schaden am Zentralnervensystem (ZNS), Schaden am peripheren Nervensystem (PNS), zerebraler Hypoxie, Rückenmarkshypoxie oder zerebralem Ödem; oder (b) Behandeln oder Verhindern von Rückenmarksverletzung verursacht durch Anschwellen von Nervenzellen.

2. Antagonist von TRPM4 zur Verwendung nach Anspruch 1, wobei der Antagonist von TRPM4 eine Nukleinsäure, ein Protein, ein kleines Molekül oder eine Kombination davon ist.

3. Antagonist von TRPM4 zur Verwendung nach Anspruch 2, wobei die Nukleinsäure ein TRPM4 siRNA ist.

4. Antagonist von TRPM4 zur Verwendung nach Anspruch 1, wobei das Verfahren ferner eine Zuführung einer therapeutisch wirksamen Menge einer zusätzlichen therapeutischen Verbindung an das Individuum umfasst, ausgewählt von:
a) einem SUR1-Antagonisten;
b) einem oder mehreren Kationkanal-Blockern;
e) einer oder mehreren Verbindungen ausgewählt von einem oder mehreren Antagonisten von vaskulärem endothelialem Wachstumsfaktor (VEGF), einem oder mehreren Antagonisten von Matrix-Metalloprotease (MMP), einem oder mehreren Antagonisten von Stickstoffmonoxid-Synthase (NOS), einem oder mehreren Antagonisten von Thrombin, Aquaporin, einem biologisch aktiven Derivat davon und einer Kombination davon; und
d) einer Kombination davon.

5. Antagonist von TRPM4 zur Verwendung nach Anspruch 4, wobei
- der TRPM4-Antagonist und die zusätzliche therapeutische Verbindung dem Individuum nacheinander zugeführt werden; oder
- der TRPM4-Antagonist dem Individuum vor der Zuführung der zusätzlichen therapeutischen Verbindung zugeführt wird; oder
- der TRPM4-Antagonist dem Individuum nach der Zuführung der zusätzlichen therapeutischen Verbindung zugeführt wird.

6. Antagonist von TRPM4 zur Verwendung nach Anspruch 4, wobei
- der TRPM4-Antagonist und die zusätzliche therapeutische Verbindung dem Individuum gleichzeitig zugeführt werden; oder
- der TRPM4-Antagonist und die zusätzliche therapeutische Verbindung als ein Gemisch zugeführt werden; oder
- der TRPM4-Antagonist und die zusätzliche therapeutische Verbindung synergistisch im Individuum wirken.

## Revendications

1. Antagoniste de TRPM4 à utiliser dans un procédé permettant (a) d'inhiber le gonflement de cellules neuronales chez un individu présentant une lésion traumatique cérébrale, un lésion traumatique de la moelle épinière, une ischémie cérébrale, une ischémie de la moelle épinière, un endommagement du système nerveux central (SNC), un endommagement du système nerveux périphérique (SNP), une hypoxie cérébrale, une hypoxie de la moelle épinière ou un oedème cérébral ; ou (b) de traiter ou prévenir une lésion de la moelle épinière consécutive à un gonflement de cellules.

2. Antagoniste de TRPM4 à utiliser selon la revendication 1, l'antagoniste de TRPM4 étant un acide nucléique, une protéine, une petite molécule ou une combinaison de ceux-ci.

3. Antagoniste de TRPM4 à utiliser selon la revendication 2, dans lequel l'acide nucléique est un ARNsi de TRPM4.

4. Antagoniste de TRPM4 à utiliser selon la revendication 1, dans lequel le procédé comprend en outre l'administration à l'individu d'une quantité thérapeutiquement efficace d'un composé thérapeutique supplémentaire choisi parmi :
a) un antagoniste de SUR1 ;
b) au moins un inhibiteur de canaux cationiques ;
c) au moins un composé choisi parmi au moins un antagoniste du facteur de croissance endothéliale vasculaire (VEGF), au moins antagoniste de métalloprotéase matricielle (MMP), au moins un antagoniste de l'oxyde nitrique synthase (NOS), au moins antagoniste de la thrombine, l'aquaporine, un dérivé biologiquement actif de ceux-ci et une combinaison de ceux-ci ; et
d) une combinaison de ceux-ci.

5. Antagoniste de TRPM4 à utiliser selon la revendication 4,
- l'antagoniste de TRPM4 et le composé thérapeutique supplémentaire étant administrés à l'individu successivement ; ou
- l'antagoniste de TRPM4 étant administré à l'individu avant l'administration du composé thérapeutique supplémentaire ; ou
- l'antagoniste de TRPM4 étant administré à l'individu à la suite de l'administration du composé thérapeutique supplémentaire.

6. Antagoniste de TRPM4 à utiliser selon la revendication 4,
- l'antagoniste de TRPM4 et le composé thérapeutique supplémentaire étant administrés à l'individu de manière concomitante ; ou
- l'antagoniste de TRPM4 et le composé thérapeutique supplémentaire étant administrés sous forme de mélange ; ou
- l'antagoniste de TRPM4 et le composé thérapeutique supplémentaire agissant de manière synergique chez l'individu.
